# EUROPEAN PATENT SPECIFICATION

(11) **EP 1 891 064 B1**
(45) Date of publication and mention of the grant of the patent: **02.09.2009**
(21) Application number: 06753965.0
(22) Date of filing: 30.05.2006
(51) Int. Cl.: C07D 451/04, A61K 31/439, A61P 25/28

(54) **N-AMIDE DERIVATIVES OF 8-AZABICYCLO[3.2.1]OCT-3-YL AS CCR1 ANTAGONISTS**
N-AMIDDERIVATE VON 8-AZABICYCLO[3.2.1]OCT-3-YL ALS CCR1-ANTAGONISTEN
DERIVES AMIDE N DE 8-AZABICYCLO[3.2.1]OCT-3-YLE UTILISES COMME ANTAGONISTES CCR1

(30) Priority: 14.06.2005 ES 200501431
(43) Date of publication of application: 27.02.2008
(73) Proprietor: Almirall, S.A., 08022 Barcelona (ES)
(72) Inventor: TERRICABRAS BELART, Emma, 08190 Sant Cugat Del Valles Barcelona (ES); FERNANDEZ FORNER, Maria, Dolors, E-08025 Barcelona (ES); LOZOYA TORIBIO, Maria, Estrella, E-08100 Mollet del Valles Barcelona (ES)
(74) Representative: Winkler, Andreas Fritz Ernst
(86) International application number: PCT/EP2006/005129
(87) International publication number: WO 2006/133802

(56) References cited:
- WO-A-00/38680
- WO-A-03/080574
- US-A1- 2004 063 688

## Description

The present invention relates to new antagonists of the interaction between the CCR1 Chemokine receptor and its ligands, including MIP-1α (CCL3).

Chemokines are a large family of small proteins (8-10KDa) synthesized by leukocytes and resident tissue cells that control leukocyte trafficking. They are structurally divided into four classes: C, CC, CXC and CX3C, depending on the relative position of their amino terminal cysteine residues. In the last decade, it was clearly established that chemokines play a significant role in diverse processes such as embryonic development, host defence, immune surveillance, inflammation, angiogenesis, autoimmunity and cancer *(Kunkel SL, Godessart N. Chemokines in autoimmunity: from pathology to therapeutics. Autoimm Rev 2002; 1 (6): 313-320).

Chemokines exert their biological function by binding to receptors expressed on the cell membrane. Chemokine receptors are themselves grouped into CR, CCR, CXCR or CX3CR classes, according to the chemokines they bind. They belong to the G-protein-coupled receptor (GPCR) superfamily, that span the membrane 7 times and are coupled to heterotrimeric G proteins. Upon ligand-receptor interaction, the α and βγ subunits dissociate and activate different intracellular signalling pathways, leading to cell mobilization and activation.

The gene encoding human CCR1 has been mapped to chromosome 3p21, in a cluster with other chemokine receptor genes such as CCR2, CCR3, CCR4, CCR5, CCR8, CCR9, XCR1 and CX3CR1. The receptor is expressed in leukocytes: monocytes, neutrophils, eosinophils, basophils, platelets, T cells, NK cells and dendritic cells, although not in B cells, as well as in stromal cells (endothelial cells, chondrocytes and osteoclasts).

The expression of the receptor depends on the state of cell differentiation or activation. Thus, among T cells, CD45RO+ cells express a larger number of CCR1 than CD45RO-cells. The differentiation of monocytes into macrophages is accompanied by an upregulation of CCR1 and a downregulation of CCR2. IL-10 upregulates CCR1 on monocytes whereas TLR2 and TLR4 stimulation downregulates it. In dendritic cells, immature cells express high levels of CCR1. Following maturation, CCR1 is replaced by CCR7. IFNγ and GM-CSF upregulate CCR1 expression on neutrophils. IL-2, IL-4. IL-10 and IL-12 increases and TCR stimulation downregulates CCR1 expression on T cells.

multiple chemokines have been reported to bind CCR1 with high affinity: CCL3, CCL3L1, CCL5, CCL6, CCL7, CCL9, CCL10, CCL14, CCL16, CCL23. The chemokine CCL3/MIP-1α is by for the most well known ligand of CCR1 and probably the most relevant in vivo. Binding studies have determined that both the NH2-terminal extracellular domain and the third extracellular loop of the receptor contain binding sites for CCL3.

CCL3 has been reported to play a role in a number of diseases including rheumatoid arthritic, myositis; atopic dermatitis, cutaneous leishmaniasis, allergic asthma*, pulmonary fibrosis*, sarcoidosis, bronchitis, acute respiratory distress syndrome, systemic sclerosis, respiratory virus infection*, Helicobacter Pylori, ulcerative colitis, alcoholic hepatitis/cirrhosis, liver transplant rejection, glomerulonephritis*, renal transplant rejection* dialysis-associated amyloidosis, sepsis*, atherosderosis *, systemic lupus erythematosus (SLE)*, acute coronary syndrome, hypereosinophilia, HIV-infection, malaria, aplastic anemia, chronic myeloid leucemia, multiple myeloma, acute myeloid leucemia, myelodysplastic syndrome, multiple sclerosis*
Alzheimer's disease*, schizophrenia, maniac depressive illness, bacterial meningitis, Behcet's disease, heart transplant rejection*. Since CCL3 may bind both CCR1 and CCR5, those diseases on which a role for CCR1 has also been demonstrated are indicated with an asterisk. (See Menten P, Wuyts A, Van Damme J. Macrophage inflammatory protein-1. Cytokine Growth factor Reviews 2002; 13: 455-481)

The use of small molecule antagonists of chemokine receptors has demonstrated to be a valid approach for the treatment of inflammatory conditions in animal models.

US 2004/063688 A1 is directed to piperidine derivatives which are useful as potent and selective inhibitors of CCR3 binding to the CCR1 receptor.

WO 03/080574 A discloses 8-aza-bicyclo-[3.2.1]oct-3-yl derivatives for use as modulators of CCR, especially CCR5.

It has now been found that certain N-amide derivatives of 8-azabicyclo[3.2.1]oct-3-yl are novel potent CCR1 antagonists and can therefore be used In the treatment or prevention of diseases susceptible to amelioration by antagonism of the CCR1 receptor.

Further objectives of the present invention are to provide a method for preparing said compounds; pharmaceutical compositions comprising an effective amount of said compounds; the use of the compounds in the manufacture of a medicament for the treatment of pathological conditions or diseases susceptible of being improved by antagonism of an chemokine receptor, in particular by antagonism of the CCR1 receptor;

Thus, the present invention is directed to new N-amide derivatives of 8-azabicyclo[3.2.1]oct-3-yl of formula (I)

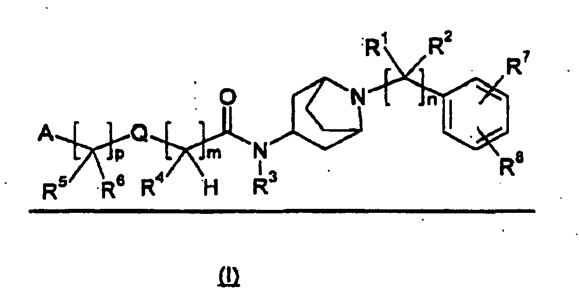

in the fom of the free base, a pharmaceutically acceptable salt, or a zwitterionic form thereof wherein
•n is an integer from 1 to 4
• m is an integer from 1 to 3
• p is an integer from 0 to 2
• R¹, R² and R³ each independently represent a hydrogen atom or a straight or branched C₁₋₄ alkyl group
• Q represents a direct bond or a group selected from -CONR⁹, -OCONR⁹-, - NR⁹CONR¹⁰-, -NR⁸-, -NR⁹COO-, -O-, -SO₂NR⁸- and -NR⁹CO
• R⁹ and R¹⁰ each independently represent a hydrogen atom or a straight or branched C₁₋₄ alkyl group
• R⁴ represents a hydrogen atom or a group selected from straight or branched C₁₋₄ alkyl which is unsubstitued or substituted with a group selected from -COOH, phenyl or -SCH₃ with the proviso that when m is 2 or 3, only one R⁴ is as defined above and the rest of R⁴ are hydrogen atoms
• R⁵ and R⁶ each independently represent a hydrogen atom, a straight or branched C₁₋₄ alkyl or a phenyl group, or
• R⁵ and R⁶ together with the carbon atom to which they are attached form a C₃₋₈ cycloalkyl group
• R⁷ and R⁸ each independently represent a hydrogen or halogen atom or a C₁₋₄ alkoxy group
• A represents a group of formula (i) or (ii) 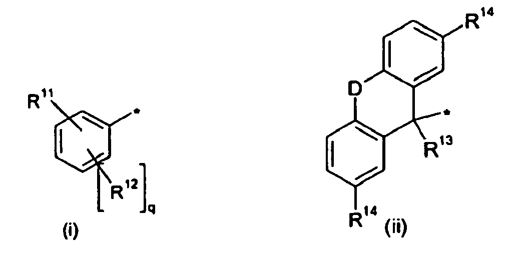
• D represents a direct bond or a group selected from **-**O**-** and -CH₂CH₂
• R¹¹ represents a hydrogen or halogen atom or a group selected from **(a)** straight or branched C₁₋₄ alkyl which is unsubstituted or substituted by a hydroxy, cyano, phenyl or a group -CONRaRb wherein Ra and Rb are independently selected from hydrogen atoms and C₁₋₄ alkyl groups, **(b)** hydroxy, phenoxy or straight or branched C₁₋₄ alkoxy, **(c)** nitro, **(d)** hydroxycarbonyl or straight or branched C₁₋₄ alkoxycarbonyl, **(e)** a group -NRcRd wherein Rc represents a hydrogen atom or a straight or branched C₁₋₄ alkyl and Rd represents represents a hydrogen atom, a straight or branched C₁₋₄ alkyl or a phenyl group which is optionally substituted by one or more groups selected from -CF₃, and halogen atoms, **(f)** a group - NHCORe wherein Re represents a group selected from -NH₂, straight or branched C₁₋₄ alkyl which is unsubstituted or substituted by one or more fluorine atoms or - NH₂, straight or branched C₂₋₄ alkenyl and 3-piperidinyl groups, **(g)** a group - CONRcRf wherein Rc is as hereinabove defined and Rf is a group selected from hydrogen atoms, straight or branched C₁₋₄ alkyl groups and 4-piperidinyl groups or Rc and Rf form together with the nitrogen atom to which they are attached a piperidinyl group which may be unsubstituted or substituted by a group selected from -NH₂, -CONH₂, -CH₂NH₂ and -CH₂NHCOCF₃, **(h)** a group -NHSO₂Rg
wherein Rg is selected from the group comprising a straight or branched C₁₋₄ alkyl and C₁₋₄ alkylphenyl groups and **(i)** a guanidino group;
- R¹² each independently represent a cyano, straight or branched C₁₋₄ alkyl, straight or branched C₁₋₄ alkoxy, a halogen atom or a group -CF₃
- or R¹¹ and one of R¹² form a methanediol group
- q is an integer from 0 to 2
- R¹³ represents a hydrogen atom or a straight or branched C₁₋₄ alkyl
- R¹⁴ each independently represent a hydrogen or halogen atom.

Other aspects of the present invention are: a) pharmaceutical compositions comprising an effective amount of said compounds, b) the use of said compounds in the manufacture of a medicament for the treatment of diseases susceptible of being improved by antagonism of a the CCR1 receptor.

As used herein the term C₁₋₄ alkyl embraces linear or branched radicals having 1 to 4 carbon atoms.

Examples include methyl, ethyl, n-propyl, i-propyl, n-butyl, see-butyl and tert-butyl radicals.

As used herein, the term C₃₋₈ cycloakyl group embraces alicyclic, monocyclic, saturated radicals having from 3 to 8 carbon atoms.

Examples indude cyclopropyl cyclobutyl, cyclopentyl, cyclohexyl, cycloheptyl and cyclooctyl radicals.

As used herein, the C₁₋₄ alkoxy embraces linear or brached oxy-containing radicals each having alkyl portions of 1 to 4 carbon atoms.

Preferred alkoxy radicals include methoxy, ethoxy, n-propoxy, i-propoxy, n-butoxy, sec-butoxy and t-butoxy.

As used herein, the term halogen atom embraces chlorine, fluorine, bromine or iodine atoms typically a fluorine, chlorine or bromine atom, most preferably chlorine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkylamines, arylalkyl amines and heterocyclic amines.

Other preferred salts according to the invention are quaternary ammonium compounds
wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

According to one embodiment of the present invention in the compounds of formula (I), Q represents a group selected from -CONR⁹-, -O- and -NR⁹CO-. The compounds wherein Q is selected from -CONR⁹- and -O- are particularly preferred.

According to a preferred embodiment of the present invention in the compounds of formula (I), n is 1.

According to another embodiment of the present invention in the compounds of formula (I), R³ is a hydrogen atom or a methyl group. The compounds wherein R³ is a hydrogen atom are particularly preferred.

According to another preferred embodiment of the present invention in the compounds of formula (I), R¹ each independently represent a hydrogen atom and R² is selected from hydrogen atoms and methyl groups. The compounds wherein both R¹ and R² are hydrogen atoms are particularly preferred.

According to still another embodiment of the present invention in the compounds of formula (I), R⁹ is a hydrogen atom.

According to still another preferred embodiment of the present invention in the compounds of formula (I), R⁴ each independently represent a hydrogen atom or a methyl group. The compounds wherein R⁴ is a hydrogen atom are particularly preferred.

According to still another preferred embodiment of the present invention in the compounds of formula (I), R⁵ and R⁶ each independently represent a hydrogen atom or a phenyl group.

According to another preferred embodiment of the present invention in the compounds of formula (I), A represents a group of formula **(i)** and R¹¹ represents a hydrogen or halogen atom or a group selected from **(a)** methyl which is substituted by a -CONRaRb group wherein Ra and Rb are independently selected from hydrogen atoms and C₁₋₄ alkyl groups, **(b)** hydroxy or phenoxy, **(c)** nitro, **(d)** methoxycarbonyl, **(e)** a group -NHRd
wherein Rd represents a hydrogen atom, a methyl group or a phenyl group which substituted by one or more groups selected from -CF₃, and halogen atoms, **(f)** a group - NHCORe wherein Re represents a group selected from -NH₂, -CF₃ and straight C₁₋₄ alkyl which is unsubstituted or substituted by a -NH₂ group, **(g)** a group -CONHRf wherein Rf is a group selected from hydrogen atoms, methyl and 4-piperidinyl groups or Rc and Rf form together with the nitrogen atom to which they are attached a piperidinyl group which may be unsubstituted or substituted by a group selected from -NH₂, -CONH₂ - and - CH₂NH₂, **(h)** a group -NHSO₂Rg wherein Rg is selected from the group comprising methyl and methylphenyl groups and **(i)** a guanidino group. It is particularly preferred that R¹¹ represents a hydrogen or halogen atom or a group selected from **(a)** methyl which is substituted by a -CONRaRb group wherein Ra and Rb are independently selected from hydrogen atoms and C₁₋₄ alkyl groups, **(c)** nitro, **(f)** a group -NHCORe wherein Re represents a group selected from -NH₂, -CF₃ and straight C₁₋₄ alkyl which is unsubstituted or substituted by a -NH₂ group, **(g)** a group -CONHRf wherein Rf is a group selected from hydrogen atoms, methyl and 4-piperidinyl groups or Rc and Rf form together with the nitrogen atom to which they are attached a piperidinyl group which may be unsubstituted or substituted by a group selected from -NH₂, -CONH₂ - and -CH₂NH₂, **(h)** a group - NHSO₂Rg wherein Rg is selected from the group comprising methyl and methylphenyl groups and **(i)** a guanidino group.

According to another preferred embodiment of the present invention in the compounds of formula (I), Q represents an oxygen atom, A represents a group of formula (i), q is 1 or 2 and R¹¹ represents a group selected from **(a)** methyl which is substituted by a -CONRaRb group wherein Ra and Rb are independently selected from hydrogen atoms and C₁₋₄ alkyl groups, **(b)** a group -NRcRd wherein Rc represents a hydrogen atom or a straight or branched C₁₋₄ alkyl and Rd represents represents a hydrogen atom, a straight or branched C₁₋₄ alkyl or a phenyl group which is optionally substituted by one or more groups selected from -CF₃, and halogen atoms, **(c)** a group -NHCORe wherein Re represents a group selected from -NH₂, straight or branched C₁₋₄ alkyl which is unsubstituted or substituted by one or more fluorine atoms or -NH₂, straight or branched C₁₋₄ alkenyl and 3-piperidinyl groups, **(d)** a group-CONRcRf wherein Rc is as hereinabove defined and Rf is a group selected from hydrogen atoms, straight or branched C₁₋₄ alkyl groups and 4-piperidinyl groups or Rc and Rf form together with the nitrogen atom to which they are attached a piperidinyl group which may be unsubstituted or substituted by a group selected from -NH₂, -CONH₂, -CH₂NH₂ and -CH₂NHCOCF₃, **(e)** a group -NHSO₂Rg wherein Rg is selected from the group comprising C₁₋₄ alkyl and C₁₋₄ alkylphenyl groups and **(f)** a guanidino group

According to still another preferred embodiment of the present invention in the compounds of formula (I), A represents a group of formula (i) and R¹² each independently represent a halogen atom or a group -CF₃.

According to still another preferred embodiment of the present invention in the compounds of formula (I), A represents a group of formula (i) and q is an integer from 0 to 1.

According to still another preferred embodiment of the present invention in the compounds of formula (I), A represents a group of formula (ii) and R¹³ represents a hydrogen atom or a methyl group.

According to still another preferred embodiment of the present invention in the compounds of formula (I), A represents a group of formula (ii) and R¹⁴ represents a hydrogen atom or a chlorine atom.

According to still another preferred embodiment of the present invention in the compounds of formula (I), the group 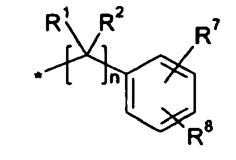 represents a group 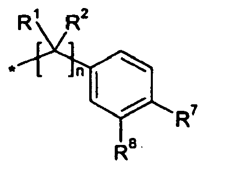 wherein R⁷ represents a fluorine or chlorine atom and R⁸ represents a hydrogen or chlorine atom. Particularly preferred are the compounds wherein R⁷ represents a chlorine atom and R⁸ represents a hydrogen atom.

Particular individual compounds of the invention include:
5-chloro-2-(3-((3-endo)-8-(4-chlorobenzyl)-8-azabicyclo[3.2.1]octan-3-ylamino)-3-oxopropoxy)-N-methylbenzamide
3-(4-chloro-2-ureidophenoxy)-N-((3-endo)-8-(4-chlorobenzyl)-8-azabicyclo[3.2.1]octan-3-yl)propanamide
5-chloro-2-(4-((3-endo)-8-(4-chlorobenzyl)-8-azabicyclo[3.2.1]octan-3-ylamino)-4-oxobutoxy)-N-methylbenzamide
N'-((3-endo)-8-(4-chlorobenzyl)-8-azabicyclo[3.2.1]octan-3-yl)-N3-(3-chlorophenyl)malonamide
2-(4-chloro-2-ureidophenoxy)-N-((3-endo)-8-(4-fluorobenzyl)-8-azabicyclo[3.2.1]octan-3-yl)acetamide
5-chloro-2-(2-((3-endo)-8-(4-chlorobenzyl)-8-azabicyclo[3.2.1]octan-3-ylamino)-2-oxoethoxy)-N-methylbenzamide
2-(4-bromo-2-ureidophenoxy)-N-((3-endo)-8-(4-chlorobenzyl)-8-azabicyclo[3.2.1]octan-3-yl)acetamide
2-(5-chloro-2-(2-((3-endo)-8-(4-chlorobenzyl)-8-azabicyclo[3.2.1]octan-3-ylamino)-2-oxoethoxy)phenyl)-N-methylacetamide
2-(2-(2-amino-2-oxoethyl)-4-chlorophenoxy)-N-((3-endo)-8-(4-chlorobenzyl)-8-azabicyclo[3.2.1]octan-3-yl)acetamide
2-(5-chloro-2-(2-((3-endo)-8-(4-chlorobenzyl)-8-azabicyclo[3.2.1]octan-3-ylamino)-2-oxoethoxy)phenyl)-N,N-dimethylacetamide
N-((3-endo)-8-(4-chlorobenzyl)-8-azabicyclo[3.2.1]octan-3-yl)-2-(4-fluoro-2-ureidophenoxy)acetamide
2-(4-chloro-2-ureidophenoxy)-N-((3-endo)-8-(4-chlorobenzyl)-8-azabicyclo[3.2.1]octan-3-yl)acetamide
5-chloro-2-(2-((3-endo)-8-(4-chlorobenzyl)-8-azabicyclo[3.2.1]octan-3-ylamino)-2-oxoethoxy)benzamide
2-(2-acetamido-4-chlorophenoxy)-N-((3-endo)-8-(4-chlorobenzyl)-8-azabicyclo[3.2.1]octan-3-yl)acetamide
N-((1-(5-chloro-2-(2-((3-endo)-8-(4-chlorobenzyl)-8-azabicyclo[3.2.1]octan-3-ylamino)-2-oxoethoxy)benzoyl)piperidin-3-yl)methyl)-2,2,2-trifluoroacetamide
2-(2-(3-(aminomethyl)piperidine-1-carbonyl)-4-chlorophenoxy)-N-((3-endo)-8-(4-chlorobenzyl)-8-azabicyclo[3.2.1]octan-3-yl)acetamide
N-((3-endo)-8-(4-chlorobenzyl)-8-azabicyclo[3.2.1]octan-3-yl)-2-(4-chlorophenylamino)acetamide
N-((3-endo)-8-(4-chlorobenzyl)-8-azabicyclo[3.2.1]octan-3-yl)-2-(3-chlorophenylamino)acetamide
N-((3-endo)-8-(4-chlorobenzyl)-8-azabicyclo[3.2.1]octan-3-yl)-2-(2-(hydroxymethyl)phenoxy)acetamide
N-((3-endo)-8-(4-chlorobenzyl)-8-azabicyclo[3.2.1]octan-3-yl)-2-(4-methyl-2-(methylamino)phenoxy)acetamide
N-((3-endo)-8-(4-chlorobenzyl)-8-azabicyclo[3.2.1]octan-3-yl)-2-(4-chlorophenoxy)acetamide
N-(2-(2-((3-endo)-8-(4-chlorobenzyl)-8-azabicyclo[3.2.1]octan-3-ylamino)-2-oxoethoxy)-5-methylphenyl)acrylamide
N-((3-endo)-8-(4-chlorobenzyl)-8-azabicyclo[3.2.1]octan-3-yl)-2-(4-methyl-2-nitrophenoxy)acetamide
N-((3-endo)-8-(4-chlorobenzyl)-8-azabicyclo[3.2.1]octan-3-yl)-2-(3-cyanophenoxy)acetamide
N-((3-endo)-8-(4-chlorobenzyl)-8-azabicyclo[3.2.1]octan-3-yl)-2-(3-chlorophenoxy)acetamide
N-((3-endo)-8-(4-chlorobenzyl)-8-azabicyclo[3.2.1]octan-3-yl)-2-(4-fluoro-2-nitrophenoxy)acetamide
methyl 4-acetamido-5-chloro-2-(2-((3-endo)-8-(4-chlorobenzyl)-8-azabicyclo[3.2.1]octan-3-ylamino)-2-oxoethoxy)benzoate
N-((3-endo)-8-(4-chlorobenzyl)-8-azabicyclo[3.2.1]octan-3-yl)-2-(2-cyanophenoxy)acetamide
2-(2-((3-endo)-8-(4-chlorobenzyl)-8-azabicyclo[3.2.1]octan-3-ylamino)-2-oxoethoxy)benzamide
2-(benzo[d][1,3]dioxol-5-yloxy)-N-((3-endo)-8-(4-chlorobenzyl)-8-azabicyclo[3.2.1]octan-3-yl)acetamide
N-((3-endo)-8-(4-chlorobenzyl)-8-aza-bicyclo[3.2.1]octan-3-yl)-2-(2-nitrophenoxy)acetamide
N-((3-endo)-8-(4-chlorobenzyl)-8-azabicyclo[3.2.1]octan-3-yl)-2-(2-methoxyphenoxy)acetamide
5-chloro-2-(2-((3-endo)-8-(4-chlorobenzyl)-8-azabicyclo[3.2.1]octan-3-ylamino)-2-oxoethoxy)-N-(piperidin-4-yl)benzamide
2-(2-(4-aminopiperidine-1-carbonyl)-4-chlorophenoxy)-N-((3-endo)-8-(4-chlorobenzyl)-8-azabicyclo[3.2.1]octan-3-yl)acetamide
1-(5-chloro-2-(2-((3-endo)-8-(4-chlorobenzyl)-8-azabicyclo[3.2.1]octan-3-ylamino)-2-oxoethoxy)benzoyl)piperidine-3-carboxamide
5-chloro-2-(2-((3-endo)-8-(4-chlorobenzyl)-8-azabicyclo[3.2.1]octan-3-ylamino)-2-oxoethoxy)-N,N-dimethylbenzamide
5-chloro-2-(2-((3-endo)-8-(4-chlorobenzyl)-8-azabicyclo[3.2.1]octan-3-ylamino)-2-oxoethoxy)benzoic acid
2-(4-chloro-2-nitrophenoxy)-N-((3-endo)-8-(4-chlorobenzyl)-8-azabicyclo[3.2.1]octan-3-yl)acetamide
methyl 5-chloro-2-(2-((3-endo)-8-(4-chlorobenzyl)-8-azabicyclo[3.2.1]octan-3-ylamino)-2-oxoethoxy)benzoate
2-(2-amino-4-chlorophenoxy)-N-((3-endo)-8-(4-chlorobenzyl)-8-azabicyclo[3.2.1]octan-3-yl)acetamide
2-amino-N-(5-chloro-2-(2-((3-endo)-8-(4-chlorobenzyl)-8-azabicyclo[3.2.1]octan-3-ylamino)-2-oxoethoxy)phenyl)acetamide
3-amino-N-(5-chloro-2-(2-((3-endo)-8-(4-chlorobenzyl)-8-azabicyclo[3.2.1]octan-3-ylamino)-2-oxoethoxy)phenyl)propanamide .
N-(5-chloro-2-(2-((3-endo)-8-(4-chlorobenzyl)-8-azabicyclo[3.2.1]octan-3-ylamino)-2-oxoethoxy)phenyl)piperidine-3-carboxamide
N-(5-chloro-2-(2-((3-endo)-8-(4-chlorobenzyl)-8-azabicyclo[3.2.1]octan-3-ylamino)-2-oxoethoxy)phenyl)-2,2,2-trifluoroacetamide
2-(4-chloro-2-(methylsulfonamido)phenoxy)-N-((3-endo)-8-(4-chlorobenzyl)-8-azabicyclo[3.2.1]octan-3-yl)acetamide
2-(4-chloro-2-guanidinophenoxy)-N-((3-endo)-8-(4-chlorobenzyl)-8-azabicyclo[3.2.1]octan-3-yl)acetamide
2-(4-chloro-2-ureidophenoxy)-N-((3-exo)-8-(4-chlorobenzyl)-8-azabicyclo[3.2.1]octan-3-yl)acetamide
N-((3-endo)-8-(4-chlorobenzyl)-8-azabicyclo[3.2.1]octan-3-yl)-2-(3-chlorophenyl)acetamide
N-((3-endo)-8-(4-chlorobenzyl)-8-azabicyclo[3.2.1]octan-3-yl)-2-(2-(2,6-dichlorophenylamino)phenyl)acetamide
3-chloro-N-(2-((3-endo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)-3-(trifluoromethyl)benzamide
N-(2-((3-endo)-8-(4-chlorobenzyl)-8-azabicyclo[3.2.1]octan-3-ylamino)-2-oxoethyl)-2-(4-methylphenylsulfonamido)benzamide
N-(2-((3-endo)-8-(4-chlorobenzyl)-8-azabicyclo[3.2.1]octan-3-ylamino)-2-oxoethyl)-1-(4-chlorophenyl)cyclopentanecarboxamide
2,7-dichloro-N-(2-((3-endo)-8-(4-chlorobenzyl)-8-azabicyclo[3.2.1]octan-3-ylamino)-2-oxoethyl)-9H-xanthene-9-carboxamide
5-chloro-N-(2-((3-endo)-8-(4-chlorobenzyl)-8-azabicyclo[3.2.1]octan-3-ylamino)-2-oxoethyl)-2-ureidobenzamide
N-((3-endo)-8-(4-chlorobenzyl)-8-azabicyclo[3.2.1]octan-3-yl)-2-(3-(3-chlorophenyl)ureido)acetamide
N-(2-((3-endo)-8-(4-chlorobenzyl)-8-azabicyclo[3.2.1]octan-3-ylamino)-2-oxoethyl)-2-phenoxybenzamide
N-(2-(((3-endo)-8-((4-chtorobenzyl)-8-azabicyclo[3.2.1]oct-3-yl)amino)-2-oxoethyl)-10,11-dihydro-5H-dibenzo[a,d]cycloheptene-5-carboxamide
N-(2-((3-endo)-8-(4-chlorobenzyl)-8-azabicyclo[3.2.1]octan-3-ylamino)-2-oxoethyl)-9-methyl-9H-fluorene-9-carboxamide
N-(2-((3-endo)-8-(4-chlorobenzyl)-8-azabicyclo[3.2.1]octan-3-ylamino)-2-oxoethyl)-2,2-diphenylacetamide
2-amino-N-(2-((3-endo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)-9-methyl-9H-xanthene-9-carboxamide
N-((3-endo)-8-(4-chlorobenzyl)-8-azabicyclo[3.2.1]octan-3-yl)-2-(2-(2-(2,6-dichlorophenylamino)phenyl)acetamido)acetamide
N-((3-endo)-8-(4-chlorobenzyl)-8-azabicyclo[3.2.1]octan-3-yl)-2-(2-(3-chlorophenyl)acetamido)acetamide
N-(3-((3-endo)-8-(4-chlorobenzyl)-8-azabicyclo[3.2.1]octan-3-ylamino)-3-oxopropyl)-2-(3-(trifluoromethyl)phenylamino)benzamide
N-(2-((3-endo)-8-(4-chlorobenzyl)-8-azabicyclo[3.2.1]octan-3-ylamino)-2-oxoethyl)-N-methyl-2-(3-(trifluoromethyl)phenylamino)benzamide
3-bromo-N-(2-((3-endo)-8-(4-chlorobenzyl)-8-azabicyclo[3.2.1]octan-3-ylamino)-2-oxoethyl)benzamide
5-chloro-N-(2-((3-endo)-8-(4-chlorobenzyl)-8-azabicyclo[3.2.1]octan-3-ylamino)-2-oxoethyl)-2-methoxybenzamide
N-(2-((3-endo)-8-(4-chlorobenzyl)-8-azabicyclo[3.2.1]octan-3-ylamino)-2-oxoethyl)-3-fluorobenzamide
5-chloro-N-(2-((3-endo)-8-(4-chlorobenzyl)-8-azabicyclo[3.2.1]octan-3-ylamino)-2-oxoethyl)-2-methoxy-4-(methylamino)benzamide
3,4-dichloro-N-(2-((3-endo)-8-(4-chlorobenzyl)-8-azabicyvlo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)-3-methylbenzamide
2,5-dichloro-N-(2-((3-endo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)-3-methoxybenzamide
N-(2-((3-endo)-8-(4-chlorobenzyl)-8-azabicyclo[3.2.1]octan-3-ylamino)-2-oxoethyl)-3-(hydroxy(phenyl)methyl)benzamide
N-(2-((3-endo)-8-(4-chlorobenzyl)-8-azabicyclo[3.2.1]octan-3-ylamino)-2-oxoethyl)-3-(cyanomethyl)benzamide
5-chloro-N-(2-((3-endo)-8-(4-chlorobenzyl)-8-azabicyclo[3.2.1 ]octan-3-ylamino)-2-oxoethyl)-4-(dimethylamino)-2-methoxybenzamide
3-chloro-N-(3-((3-exo)-8-(4-chlorobenzyl)-8-azabicyclo[3.2.1]octan-3-ylamino)-3-oxopropyl)benzamide
3-chloro-N-((R)-1-((3-exo)-8-(4-chlorobenzyl)-8-azabicyclo[3.2.1]octan-3-ylamino)-1-oxopropan-2-yl)benzamide
N-(2-((3-exo)-8-(4-chlorobenzyl)-8-azabicyclo[3.2.1]octan-3-ylamino)-2-oxoethyl)-N-methyl-2-(3-(trifluoromethyl)phenylamino)benzamide
(R)-3-(3-chlorobenzamido)-4-((3-exo)-8-(4-chlorobenzyl)-8-azabicyclo[3.2.1]octan-3-ylamino)-4-oxobutanoic acid
N-((S)-1-((3-exo)-8-(4-chlorobenzyl)-8-azabicyclo[3.2.1]octan-3-ylamino)-1-oxopropan-2-yl)-2-(3-(trifluoromethyl)phenylamino)benzamide
3-chloro-N-((S)-1-((3-exo)-8-(4-chlorobenzyl)-8-azabicyclo[3.2.1]octan-3-ylamino)-1-oxopropan-2-yl)benzamide
3-chloro-N-(2-((3-exo)-8-(4-chlorobenzyl)-8-azabicyclo[3.2.1]octan-3-ylamino)-2-oxoethyl)-N-methylbenzamide
N-((S)-1-((3-exo)-8-(4-chlorobenzyl)-8-azabicyclo[3.2.1]octan-3-ylamino)-1-oxo-3-phenylpropan-2-yl)-2-(3-(trifluoromethyl)phenylamino)benzamide
N-((S)-1-((3-exo)-8-(4-chlorobenzyl)-8-azabicyclo[3.2.1]octan-3-ylamino)-4-(methylthio)-1-oxobutan-2-yl)-2-(3-(trifluoromethyl)phenylamino)benzamide
N-(3-((3-exo)-8-(4-chlorobenzyl)-8-azabicyclo[3.2.1]octan-3-ylamino)-3-oxopropyl)-2-(3-(trifluoromethyl)phenylamino)benzamide
N-((S)-1-((3-exo)-8-(4-chlorobenzyl)-8-azabicyclo[3.2.1]octan-3-ylamino)-4-methyl-1-oxopentan-2-yl)-2-(3-(trifluoromethyl)phenylamino)benzamide
3-chloro-N-(2-((3-endo)-8-(3,4-dichlorobenzyl)-8-azabicyclo[3.2.1]octan-3-ylamino)-2-oxoethyl)benzamide
3-chloro-N-(2-((3-endo)-8-(4-fluorobenzyl)-8-azabicyclo[3.2.1]octan-3-ylamino)-2-oxoethyl)benzamide
N-(2-((3-endo)-8-benzyl-8-azabicyclo[3.2.1]octan-3-ylamino)-2-oxoethyl)-3-chlorobenzamide
3-chloro-N-(2-((3-endo)-8-(2,4-dichlorobenzyl)-8-azabicyclo[3.2.1]octan-3-ylamino)-2-oxoethyl)benzamide
3-chloro-N-(2-((3-endo)-8-(1-(4-chlorophenyl)ethyl)-8-azabicyclo[3.2.1]octan-3-ylamino)-2-oxoethyl)benzamide
3-chloro-N-(2-((3-endo)-8-(1-(4-chlorophenyl)propyl)-8-azabicyclo[3.2.1]octan-3-ylamino)-2-oxoethyl)benzamide
3-chloro-N-(2-oxo-2-((3-endo)-8-(3-phenylpropyl)-8-azabicyclo[3.2.1]octan-3-ylamino)ethyl)benzamide
N-(2-((3-endo)-8-(3,4-dichlorobenzyl)-8-azabicyclo[3.2.1] octan-3-ylamino)-2-oxoethyl)-2-(3-(trifluoromethyl)phenylamino)benzamide
N-(2-((3-endo)-8-(2,4-dichlorobenzyl)-8-azabicyclo[3.2.1]octan-3-ylamino)-2-oxoethyl)-2-(3-(trifluoromethyl)phenylamino)benzamide
N-(2-((3-endo)-8-(4-methoxybenzyl)-8-azabicyclo[3.2.1]octan-3-ylamino)-2-oxoethyl)-2-(3-(trifluoromethyl)phenylamino)benzamide
N-(2-((3-endo)-8-(4-chlorobenzyl)-8-azabicyclo[3.2.1]octan-3-ylamino)-2-oxoethyl)-2-hydroxybenzamide
N-(2-((3-endo)-8-(4-chlorobenzyl)-8-azabicyclo[3.2.1]octan-3-ylamino)-2-oxoethyl)-2-(phenylamino)benzamide
N-(2-((3-endo)-8-(4-chlorobenzyl)-8-azabicyclo[3.2.1]octan-3-ylamino)-2-oxoethyl)-9H-xanthene-9-carboxamide
2-(3,5-bis(trifluoromethyl)phenylsulfonamido)-N-((3-endo)-8-(4-chlorobenzyl)-8-azabicyclo[3.2.1]octan-3-yl)acetamide
3-chloro-N-((S)-1-((3-endo)-8-(4-chlorobenzyl)-8-azabicyclo[3.2.1]octan-3-ylamino)-1-oxopropan-2-yl)benzamide
3-chloro-N-(3-((3-endo)-8-(4-chlorobenzyl)-8-azabicyclo[3.2.1]octan-3-ylamino)-3-oxopropyl)benzamide
(R)-3-(3-chlorobenzamido)-4-((3-endo)-8-(4-chlorobenzyl)-8-azabicyclo[3.2.1]octan-3-ylamino)-4-oxobutanoic acid
3-chloro-N-((R)-1-((3-endo)-8-(4-chlorobenzyl)-8-azabicyclo[3.2.1]octan-3-ylamino)-1-oxopropan-2-yl)benzamide
(R)-3-(3-chlorobenzamido)-4-((3-endo)-8-(4-chlorobenzyl)-8-azabicyclo[3.2.1]octan-3-ylamino)-4-oxobutanoic acid
N-(2-((3-endo)-8-(4-fluorobenzyl)-8-azabicyclo[3.2.1]octan-3-ylamino)-2-oxoethyl)-2-(3-(trifluoromethyl)phenylamino)benzamide
N-(2-((3-exo)-8-(4-chlorobenzyl)-8-azabicyclo[3.2.1]octan-3-ylamino)-2-oxoethyl)-2-(3-(trifluoromethyl)phenylamino)benzamide
N-(2-((3-exo)-8-(4-chlorobenzyl)-8-azabicyclo[3.2.1]octan-3-ylamino)-2-oxoethyl)-3-(trifluoromethyl)benzamide
3-chloro-N-(2-((3-exo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)-2-(3-(trifluoromethyl)phenylamino)benzamide

### Of outstanding interest are:

5-chloro-2-(3-((3-endo)-8-(4-chlorobenzyl)-8-azabicyclo[3.2.1]octan-3-ylamino)-3-oxopropoxy)-N-methylbenzamide
3-(4-chloro-2-ureidophenoxy)-N-((3-endo)-8-(4-chlorobenzyl)-8-azabicyclo[3.2.1]octan-3-yl)propanamide
5-chloro-2-(4-((3-endo)-8-(4-chlorobenzyl)-8-azabicyclo[3.2.1]octan-3-ylamino)-4-oxobutoxy)-N-methylbenzamide
N'-((3-endo)-8-(4-chlorobenzyl)-8-azabicyclo[3.2.1]octan-3-yl)-N3-(3-chlorophenyl)malonamide
2-(4-chloro-2-ureidophenoxy)-N-((3-endo)-8-(4-fluorobenzyl)-8-azabicyclo[3.2.1]octan-3-yl)acetamide
5-chloro-2-(2-((3-endo)-8-(4-chlorobenzyl)-8-azabicyclo[3.2.1]octan-3-ylamino)-2-oxoethoxy)-N-methylbenzamide
2-(4-bromo-2-ureidophenoxy)-N-((3-endo)-8-(4-chlorobenzyl)-8-azabicyclo[3.2.1]octan-3-yl)acetamide
2-(5-chloro-2-(2-((3-endo)-8-(4-chlorobenzyl)-8-azabicyclo[3.2.1]octan-3-ylamino)-2-oxoethoxy)phenyl)-N-methylacetamide
2-(2-(2-amino-2-oxoethyl)-4-chlorophenoxy)-N-((3-endo)-8-(4-chlorobenzyl)-8-azabicyclo[3.2.1]octan-3-yl)acetamide
2-(5-chloro-2-(2-((3-endo)-8-(4-chlorobenzyl)-8-azabicyclo[3.2.1]octan-3-ylamino)-2-oxoethoxy)phenyl)-N, N-dimethylacetamide
N-((3-endo)-8-(4-chlorobenzyl)-8-azabicyclo[3.2.1]octan-3-yl)-2-(4-fluoro-2-ureidophenoxy)acetamide
2-(4-chloro-2-ureidophenoxy)-N-((3-endo)-8-(4-chlorobenzyl)-8-azabicyclo[3.2.1]octan-3-yl)acetamide
5-chloro-2-(2-((3-endo)-8-(4-chlorobenzyl)-8-azabicyclo[3.2.1]octan-3-ylamino)-2-oxoethoxy)benzamide
2-(2-acetamido-4-chlorophenoxy)-N-((3-endo)-8-(4-chlorobenzyl)-8-azabicyclo[3.2.1]octan-3-yl)acetamide
N-((1-(5-chloro-2-(2-((3-endo)-8-(4-chlorobenzyl)-8-azabicyclo[3.2.1]octan-3-ylamino)-2-oxoethoxy)benzoyl)piperidin-3-yl)methyl)-2,2,2-trifluoroacetamide
2-(2-(3-(aminomethyl)piperidine-1-carbonyl)-4-chlorophenoxy)-N-((3-endo)-8-(4-chlorobenzyl)-8-azabicyclo[3.2.1]octan-3-yl)acetamide
N-((3-endo)-8-(4-chlorobenzyl)-8-azabicyclo[3.2.1]octan-3-yl)-2-(4-chlorophenylamino)acetamide
N-((3-endo)-8-(4-chlorobenzyl)-8-azabicyclo[3.2.1]octan-3-yl)-2-(3-chlorophenylamino)acetamide
N-((3-endo)-8-(4-chlorobenzyl)-8-azabicyclo[3.2.1]octan-3-yl)-2-(4-methyl-2-(methylamino)phenoxy)acetamide
5-chloro-2-(2-((3-endo)-8-(4-chlorobenzyl)-8-azabicyclo[3.2.1]octan-3-ylamino)-2-oxoethoxy)-N-(piperidin-4-yl)benzamide
2-(2-(4-aminopiperidine-1-carbonyl)-4-chlorophenoxy)-N-((3-endo)-8-(4-chlorobenzyl)-8-azabicyclo[3.2.1]octan-3-yl)acetamide
1-(5-chloro-2-(2-((3-endo)-8-(4-chlorobenzyl)-8-azabicyclo[3.2.1] octan-3-ylamino)-2-oxoethoxy)benzoyl)piperidine-3-carboxamide
5-chloro-2-(2-((3-endo)-8-(4-chlorobenzyl)-8-azabicyclo[3.2.1] octan-3-ylamino)-2-oxoethoxy)-N,N-dimethylbenzamide
5-chloro-2-(2-((3-endo)-8-(4-chlorobenzyl)-8-azabicyclo[3.2.1]octan-3-ylamino)-2-oxoethoxy)benzoic acid
2-(4-chloro-2-nitrophenoxy)-N-((3-endo)-8-(4-chlorobenzyl)-8-azabicyclo[3.2.1]octan-3-yl)acetamide
methyl 5-chloro-2-(2-((3-endo)-8-(4-chlorobenzyl)-8-azabicyclo[3.2.1]octan-3-ylamino)-2-oxoethoxy)benzoate
2-(2-amino-4-chlorophenoxy)-N-((3-endo)-8-(4-chlorobenzyl)-8-azabicyclo[3.2.1]octan-3-yl)acetamide
2-amino-N-(5-chloro-2-(2-((3-endo)-8-(4-chlorobenzyl)-8-azabicyclo[3.2.1]octan-3-ylamino)-2-oxoethoxy)phenyl)acetamide
3-amino-N-(5-chloro-2-(2-((3-endo)-8-(4-chlorobenzyl)-8-azabicyclo[3.2.1]octan-3-ylamino)-2-oxoethoxy)phenyl)propanamide
N-(5-chloro-2-(2-((3-endo)-8-(4-chlorobenzyl)-8-azabicyclo[3.2.1]octan-3-ylamino)-2-oxoethoxy)phenyl)piperidine-3-carboxamide
N-(5-chloro-2-(2-((3-endo)-8-(4-chlorobenzyl)-8-azabicyclo[3.2.1]octan-3-ylamino)-2-oxoethoxy)phenyl)-2,2,2-trifluoroacetamide
2-(4-chloro-2-(methylsulfonamido)phenoxy)-N-((3-endo)-8-(4-chlorobenzyl)-8-azabicyclo[3.2.1]octan-3-yl)acetamide
2-(4-chloro-2-guanidinophenoxy)-N-((3-endo)-8-(4-chlorobenzyl)-8-azabicyclo[3.2.1]octan-3-yl)acetamide
2-(4-chloro-2-ureidophenoxy)-N-((3-exo)-8-(4-chlorobenzyl)-8-azabicyclo[3.2.1]octan-3-yl)acetamide
3-chloro-N-(2-((3-endo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)-3-(trifluoromethyl)benzamide
N-(2-((3-endo)-8-(4-chlorobenzyl)-8-azabicyclo[3.2.1]octan-3-ylamino)-2-oxoethyl)-2-(4-methylphenylsulfonamido)benzamide
N-(2-((3-endo)-8-(4-chlorobenzyl)-8-azabicyclo[3.2.1]octan-3-ylamino)-2-oxoethyl)-1-(4-chlorophenyl)cyclopentanecarboxamide
2,7-dichloro-N-(2-((3-endo)-8-(4-chlorobenzyl)-8-azabicyclo[3.2.1]octan-3-ylamino)-2-oxoethyl)-9H-xanthene-9-carboxamide
5-chloro-N-(2-((3-endo)-8-(4-chlorobenzyl)-8-azabicyclo[3.2.1]octan-3-ylamino)-2-oxoethyl)-2-ureidobenzamide
N-((3-endo)-8-(4-chlorobenzyl)-8-azabicyclo[3.2.1]octan-3-yl)-2-(3-(3-chlorophenyl)ureido)acetamide
N-(2-((3-endo)-8-(4-chlorobenzyl)-8-azabicyclo[3.2.1]octan-3-ylamino)-2-oxoethyl)-2-phenoxybenzamide
N-(2-(((3-endo)-8-((4-chlorobenzyl)-8-azabicyclo[3.2.1]oct-3-yl)amino)-2-oxoethyl)-10,11-dihydro-5H-dibenzo[a,d]cycloheptene-5-carboxamide
N-(2-((3-endo)-8-(4-chlorobenzyl)-8-azabicyclo[3.2.1]octan-3-ylamino)-2-oxoethyl)-9-methyl-9H-fluorene-9-carboxamide
N-(2-((3-endo)-8-(4-chlorobenzyl)-8-azabicyclo[3.2.1]octan-3-ylamino)-2-oxoethyl)-2,2-diphenylacetamide
2-amino-N-(2-((3-endo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)-9-methyl-9H-xanthene-9-carboxamide
N-((3-endo)-8-(4-chlorobenzyl)-8-azabicyclo[3.2.1]octan-3-yl)-2-(2-(2-(2,6-dichlorophenylamino)phenyl)acetamido)acetamide
N-((3-endo)-8-(4-chlorobenzyl)-8-azabicyclo[3.2.1]octan-3-yl)-2-(2-(3-chlorophenyl)acetamido)acetamide
3-bromo-N-(2-((3-endo)-8-(4-chlorobenzyl)-8-azabicyclo[3.2.1]octan-3-ylamino)-2-oxoethyl)benzamide
5-chloro-N-(2-((3-endo)-8-(4-chlorobenzyl)-8-azabicyclo[3.2.1]octan-3-ylamino)-2-oxoethyl)-2-methoxybenzamide
N-(2-((3-endo)-8-(4-chlorobenzyl)-8-azabicyclo[3.2.1]octan-3-ylamino)-2-oxoethyl)-3-fluorobenzamide
5-chloro-N-(2-((3-endo)-8-(4-chlorobenzyl)-8-azabicyclo[3.2.1]octan-3-ylamino)-2-oxoethyl)-2-methoxy-4-(methylamino)benzamide
3,4-dichloro-N-(2-((3-endo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)benzamide
3-chloro-N-(3-((3-exo)-8-(4-chlorobenzyl)-8-azabicyclo[3.2.1]octan-3-ylamino)-3-oxopropyl)benzamide
3-chloro-N-((R)-1-((3-exo)-8-(4-chlorobenzyl)-8-azabicyclo[3.2.1]octan-3-ylamino)-1-oxopropan-2-yl)benzamide
3-chloro-N-(2-((3-endo)-8-(3,4-dichlorobenzyl)-8-azabicyclo[3.2.1]octan-3-ylamino)-2-oxoethyl)benzamide
3-chloro-N-(2-((3-endo)-8-(4-fluorobenzyl)-8-azabicyclo[3.2.1]octan-3-ylamino)-2-oxoethyl)benzamide
N-(2-((3-endo)-8-(3,4-dichlorobenzyl)-8-azabicyclo[3.2.1]octan-3-ylamino)-2-oxoethyl)-2-(3-(trifluoromethyl)phenylamino)benzamide
N-(2-((3-endo)-8-(4-chlorobenzyl)-8-azabicyclo[3.2.1]octan-3-ylamino)-2-oxoethyl)-2-hydroxybenzamide
N-(2-((3-endo)-8-(4-chlorobenzyl)-8-azabicyclo[3.2.1]octan-3-ylamino)-2-oxoethyl)-2-(phenylamino)benzamide
3-chloro-N-((S)-1-((3-endo)-8-(4-chlorobenzyl)-8-azabicyclo[3.2.1]octan-3-ylamino)-1-oxopropan-2-yl)benzamide
N-(2-((3-endo)-8-(4-fluorobenzyl)-8-azabicyclo[3.2.1]octan-3-ylamino)-2-oxoethyl)-2-(3-(trifluoromethyl)phenylamino)benzamide
N-(2-((3-exo)-8-(4-chlorobenzyl)-8-azabicyclo[3.2.1]octan-3-ylamino)-2-oxoethyl)-2-(3-(trifluoromethyl)phenylamino)benzamide
N-(2-((3-endo)-8-(4-chlorobenzyl)-8-azabicyclo[3.2.1]octan-3-ylamino)-2-oxoethyl)-2-(3-(trifluoromethyl)phenylamino)benzamide

The compounds of the present invention may be prepared by one of the methods described below.

### Method 1

This method finds application in the manufacture of compounds of formula (I) wherein Q represents a direct bond or a group selected from **-O-** and -NR⁹CO-.

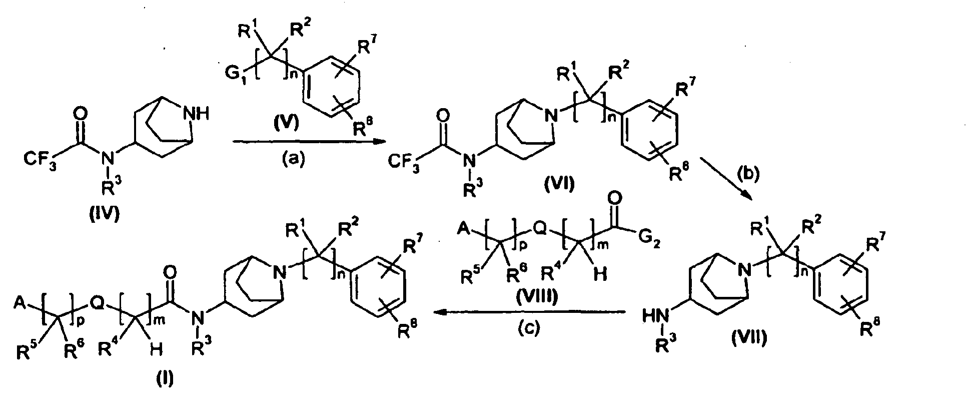

### Step a:

N-8-azabicyclo[3.2.1]oct-3-ylacetamide (IV) is reacted with compound of formula (V)
wherein G₁ represents a halogen atom, preferably a chlorine atom, in a polar aprotic solvent such as acetone for 2 to 24 hours at reflux in the presence of a base such as triethylamine to yield compound (VI).

### Step b:

Compound (VI) is hydrolized at room temperature for 2 to 12 hours with an aqueous solution of sodium hydroxide in a polar water-miscible protic solvent such as methanol to yield compound (VII).

### Step c:

Compound (VII) is reacted with compound (VIII) wherein G₂ represents a group -OH or an halogen atom using standard coupling conditions. For example when G₂ is OH, the reaction may take place using of 1-(3-dimethylaminopropyl)-3-ethylcarbodiimide hydrochloride (EDC·HCl) and 1-hydroxybenzotriazole hydrate (HOBt) in a polar aprotic solvent, such as dichloromethane at room temperature for a time period of 5 to 48 hours. In the alternative the reaction may also be carried out in toluene at reflux.

In an alternative when G₂ is a halogen atom, such as a chlorine atom the reaction may take place in a polar aprotic solvent such as dichloromethane or THF at room temperature for 0,5 to 24 hours in the presence of a base such as triethylamine.

In still another alternative when p is 0, Q is NHCO, m is 1 and R⁴ is hydrogen, the compounds of formula (VIIIc) 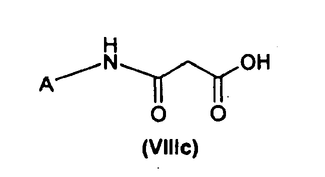 are replaced by their precursors of formula (II) 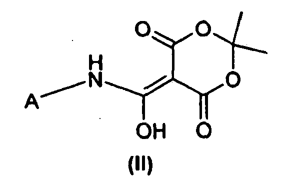 which may be obtained as described in method 13 and the reaction takes place an aprotic solvent such as toluene at reflux for 12 to 48 hours.

### Method 2

This method finds application in the manufacture of compounds of formula (I) wherein Q represents a group selected from **-O-** and -NR¹⁰-.

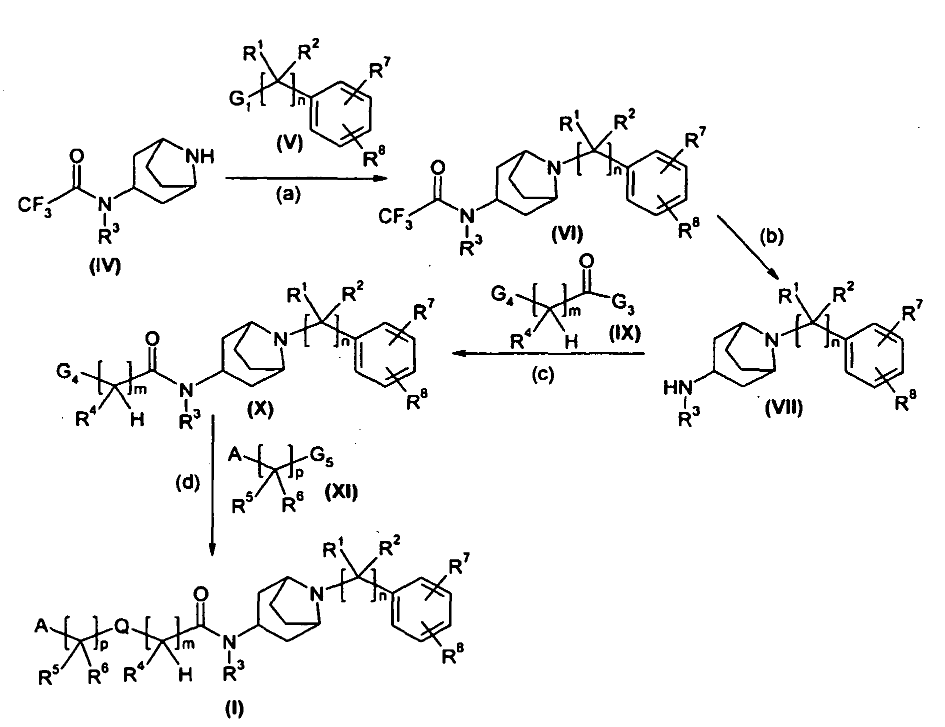

### Step a:

N-8-azabicyclo[3.2.1]oct-3-ylacetamide (IV) is reacted with compound of formula (V)
wherein G₁ represents a halogen atom, preferably a chlorine atom, in a polar aprotic solvent such as acetone for 2 to 24 hours at reflux in the presence of a base such as triethylamine to yield compound (VI)

### Step b:

Compound (VI) is hydrolized at room temperature for 2 to 12 hours with an aqueous solution of sodium hydroxide in a polar water-miscible protic solvent such as methanol to yield compound (VII).

### Step c:

Compound (X) may be obtained by reaction of compound (VII) with the compound of formula (IX) wherein both G₃ and G₄ represent chlorine atoms, in a polar aprotic solvent, such as dichloromethane at room temperature for 18 to 30 hours in the presence of a base, such as triethylamine.

### Step d:

When Q is -NR¹⁰- compounds of formula (I) may be obtained by reacting compound (X) with a compound of formula (XI) wherein G⁵ represents a -NHR⁹ group, using a base, such as potassium carbonate in an aprotic solvent, such as dimethylformamide at 50 to 70 °C for a time period of 36 to 48 hours.

When Q is **-O-** compounds of formula (I) may be obtained by reacting compound (X) with a compound of formula (XI) wherein G⁵ represents a -OH group using a base, such as potassium carbonate, in an aprotic solvent, such as dimethylformamide at 50-60°C for a time period between 3 hours and 72 hours.

### Method 3

This method finds application in the manufacture of compounds of formula (I) wherein Q represents a group-O- and R³ is a hydrogen atom.

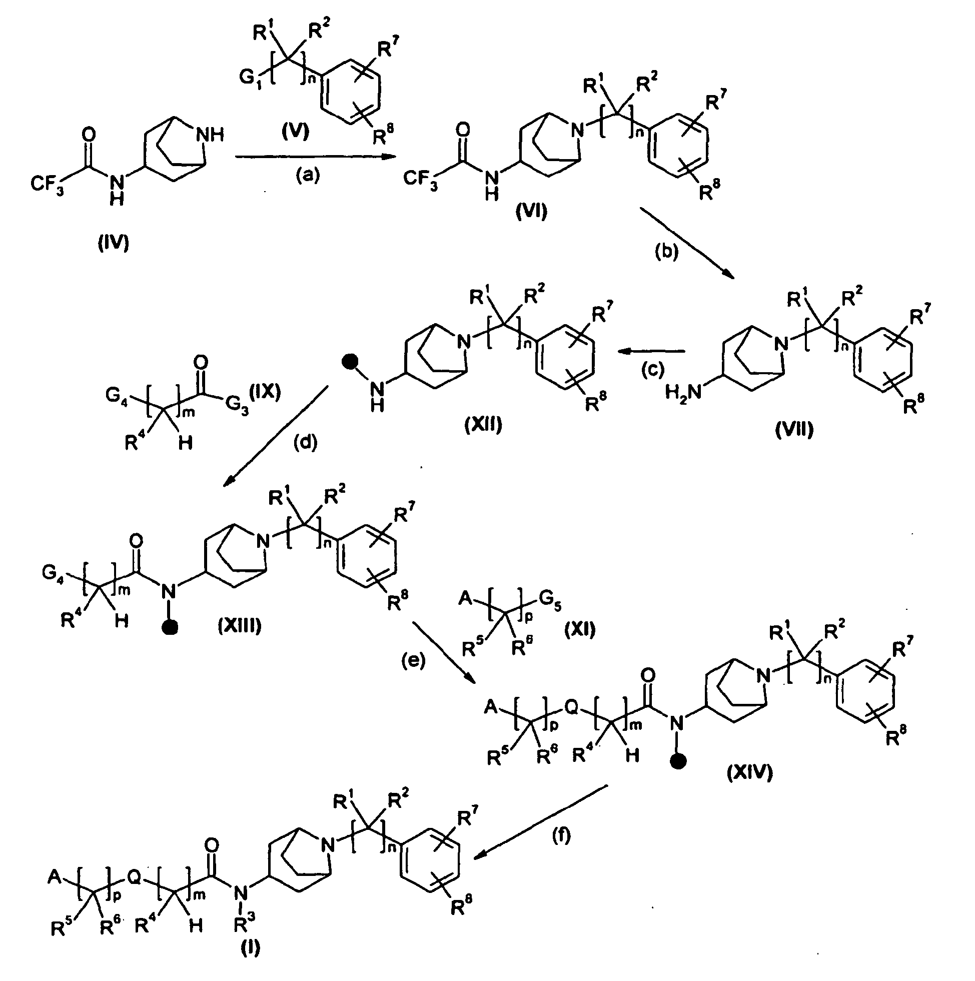

### Step a:

N-8-azabicyclo[3.2.1]oct-3-ylacetamide (IV) is reacted with a compound of formula (V)
wherein G₁ represents a halogen atom, preferably a chlorine atom, in a polar aprotic solvent such as acetone for 2 to 24 hours at reflux in the presence of a base such as triethylamine to yield compound (VI).

### Step b:

Compound (VI) is hydrolized at room temperature for 2 to 12 hours with an aqueous solution of sodium hydroxide in a polar water-miscible protic solvent such as methanol to yield compound (VII).

### Step c:

Compound (XII) may be obtained by reacting a benzaldehide functionalised resin (IRORI BAL resin) previously swollen in dimethylformamide and a suspension of compound (VII) and sodium cyanoborohydride in dimethylformamide/acetic acid (99:1). An excess of perfluorodecaline may be added. The reaction mixture is stirred at 50°-60°C for 12-24 hours and the resin (XII) is filtered, washed with dimethylformamide (3X), AcOH/dimethylformamide (1%) (2X), dimethylformamide (1X), N,N-diisopropylethylamine/dimethylformamide (5%) (2X), dimethylformamide (2X), dichloromethane (1X), methanol (1X), diethylether (2X) and dried in vacuo at 35°C.

### Step d:

Compound (XIII) may be obtained by reacting compound (XII) with compound of formula (IX) wherein G₃ represents a -OH group and G₄ represents a bromine atom, in an aprotic solvent, such as tetrahydrofuran, at room temperature for 1 to 8 hours using N,N'-diisopropylcarbodiimide.

### Step e:

Subsequently, compound (XIV) may be obtained by reacting compound (XIII) with the compound of formula (XI) wherein G₅ represents a -OH group, in dimethylsulfoxide/1-methylpyrrolidin-2-one 1:1 at room temperature for 60 to 80 hours in the presence of a base, such as 1,8-diazabicyclo[5.4.0]undec-7-ene (DBU).

### Step f

Compound (XIV) was cleaved from the resin using standard conditions such as addition to a solution of dichloromethane/trifluoroacetic acid/H2O (50:50:1) or trifluoroacetic acid/H2O (95:5) and reaction for 1 to 3 hours at room temperature to yield the final compound (I).

### Method 4

This method finds application in the manufacture of compounds of formula (I) wherein Q represents a group-O-, R³ represents a hydrogen atom and the R² substituent on the carbon atom directly attached to the benzene ring is a hydrogen atom.

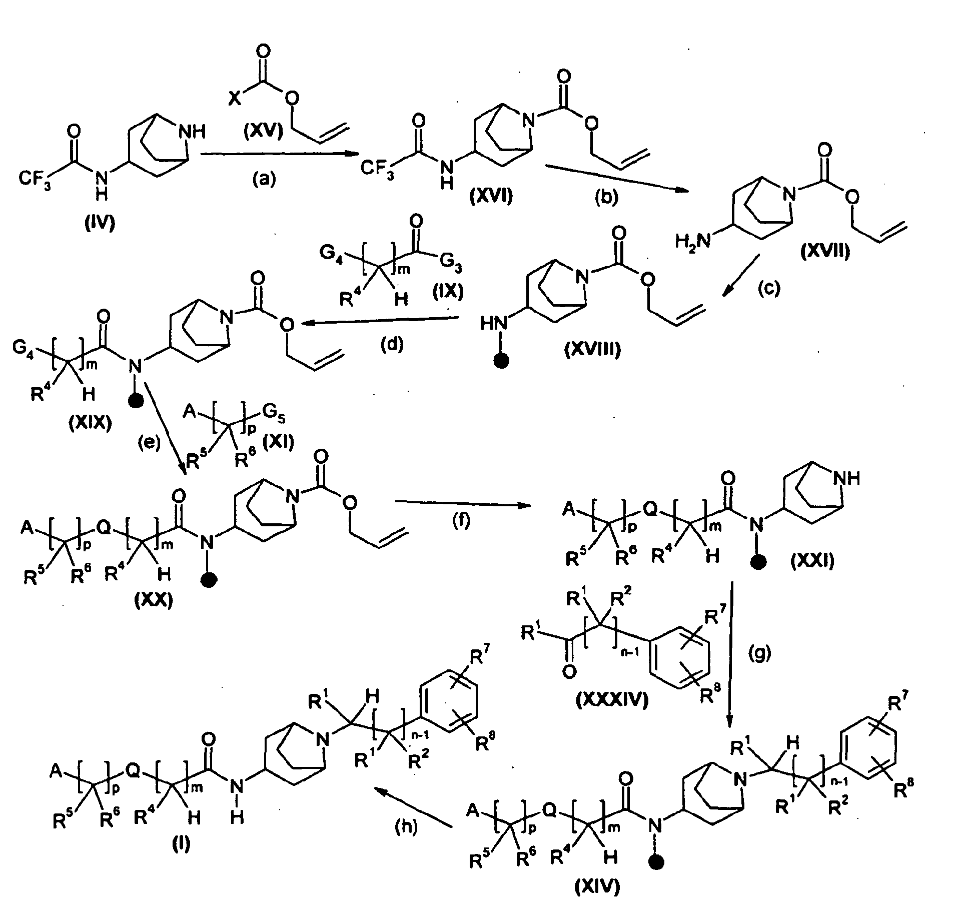

### Step a:

Reaction of compound (IV) with allyl chloroformate (XV) in the presence of a base, such as triethylamine in an aprotic solvent, such as tetrahydrofuran at room temperature for 10 to 18 hours yields compound (XVI).

### Step b:

Treatment of compound (XVI) with an aqueous solution of sodium hydroxide in a polar, water-miscible, protic solvent, such as methanol at room temperature for 2 to 12 hours yields compound (XVII).

### Step c:

Compound (XVIII) may be obtained by reacting a benzaldehide functionalised resin (IRORI BAL resin) previously swollen in dimethylformamide/methanol (8:2) and a solution of compound (XVII) and acetic acid in dimethylformamide/methanol (8:2). The mixture is stirred for 1 to 4 hours at room temperature. A solution of sodium cyanoborohydride in dimethylformamide/methanol (8:2) is added and the mixture is further stirred at room temperature for 12 to 24 hours. The resin (XVIII) was filtered, washed with methanol (3X), dimethylformamide (3X), dichloromethane (3X) and dried in vacuo at room temperature.

### Step d:

Reaction of compound (XVIII) with compound (IX) wherein G³ represent an -OH group and G⁴ represents a bromine atom with N,N'-diisopropylcarbodiimide in an aprotic solvent, such as tertahydrofuran, at room temperature for 1 to 8 hours yields compound (XIX).

### Step e:

Compound (XX) may be obtained by reacting compound (XIX) with compound (XI)
wherein G⁵ represents and -OH group in the presence of a base, such as 1,8-diazabicyclo[5.4.0]undec-7-ene (DBU), in dimethylsulfoxide/ 1-methylpyrrolidin-2-one 1:1 at room temperature for 2 to 6 days.

### Step f:

The allyloxycarbonyl (alloc) protecting group of compound (XX) may be cleaved using dimethylaminoborane and tetrakis(triphenylphosphine)palladium in an polar aprotic solvent, such as dichloromethane at room temperature for 40 to 60 min to yield compound (XXI).

### Step g:

Reaction of compound (XXI) with the corresponding substituted phenyl keto-derivative (XXXIV) using catalytic amounts of an acid, such as acetic acid and a reducing agent, such as sodium triacetoxiborohydride in an aprotic solvent, such as dimethylformamide at room temperature for 12 to 24 hours yields compound (XIV).

### Step h:

Compound (XIV) was cleaved from the resin using standard conditions such as addition to a solution of dichloromethane/trifluoroacetic acid/H2O (50:50:1) or trifluoroacetic acid/H2O (95:5) and reaction for 1 to 3 hours at room temperature to yield the final compound (I).

### Method 5

This method finds application in the manufacture of compounds of formula (I) wherein Q represents a direct bond, R³ represents a hydrogen atom and the R² substituent on the carbon atom directly attached to the benzene ring is a hydrogen atom

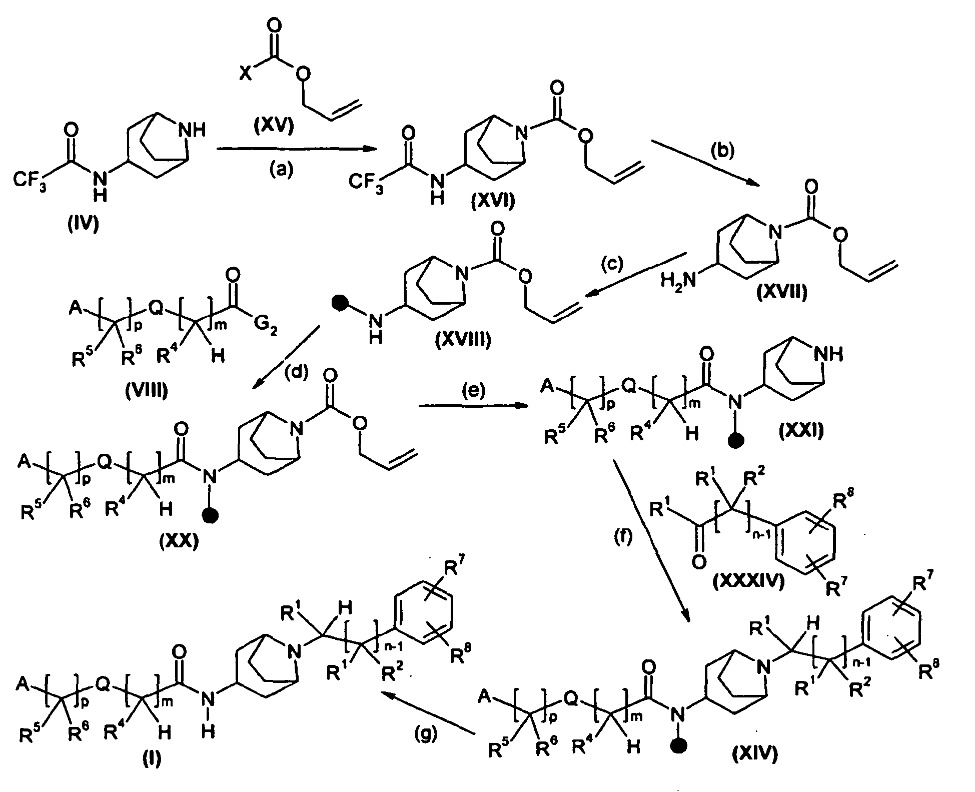

### Step a:

Reaction of compound (IV) with allyl chloroformate (XV) in the presence of a base, such as triethylamine in an aprotic solvent, such as tetrahydrofuran at room temperature for 10 to 18 hours yields compound (XVI).

### Step b:

Treatment of compound (XVI) with an aqueous solution of sodium hydroxide in a polar, water-miscible, protic solvent, such as methanol, at room temperature for 2 to 12 hours yields compound (XVII).

### Step c:

Compound (XVIII) may be obtained by reacting a benzaldehide functionalised resin (IRORI BAL resin) previously swollen in dimethylformamide/methanol (8:2) and a solution of compound (XVII) and acetic acid in dimethylformamide/methanol (8:2). The mixture is stirred for 1 to 4 hours at room temperature. A solution of sodium cyanoborohydride in dimethylformamide/methanol (8:2) is added and the mixture is further stirred at room temperature for 12 to 24 hours. The resin (XVIII) was filtered, washed with methanol (3X), dimethylformamide (3X), dichloromethane (3X) and dried in vacuo at room temperature.

### Step d:

Reaction of compound (XVIII) with compound (VIII) wherein G² represents a -OH group in the presence of N,N'-diisopropylcarbodiimide in dichloromethane/dimethylformamide 9:1 at 30-60°C for 12 to 24 hours yields compound (XX).

### Step e:

The allyloxycarbonyl (alloc) protecting group of compound (XX) may be cleaved by using dimethylamino-borane and tetrakistriphenylphosphine palladium in a polar aprotic solvent, such as dichloromethane at room temperature for 40 to 60 minutes to obtain compound (XXI).

### Step f:

Reaction of compound (XXI) with the corresponding substituted phenyl keto-derivative (XXXIV) using catalytic amounts of an acid, such as acetic acid and a reducing agent, such as sodium triacetoxiborohydride in an aprotic solvent, such as dimethylformamide at room temperature for 12 to 24 hours yields compound (XIV).

### Step g:

Compound (XIV) was cleaved from the resin using standard conditions such as addition to a solution of dichloromethane/trifluoroacetic acid/H2O (50:50:1) or trifluoroacetic acid/H2O (95:5) and reaction for 1 to 3 hours at room temperature to yield the final compound (I).

### Method 6

This method finds application in the manufacture of compounds of formula (I) wherein Q represents a group selected from -CONR⁹-, -OCONR⁹-, -NR⁹CONR¹⁰- and -SO₂NR⁹

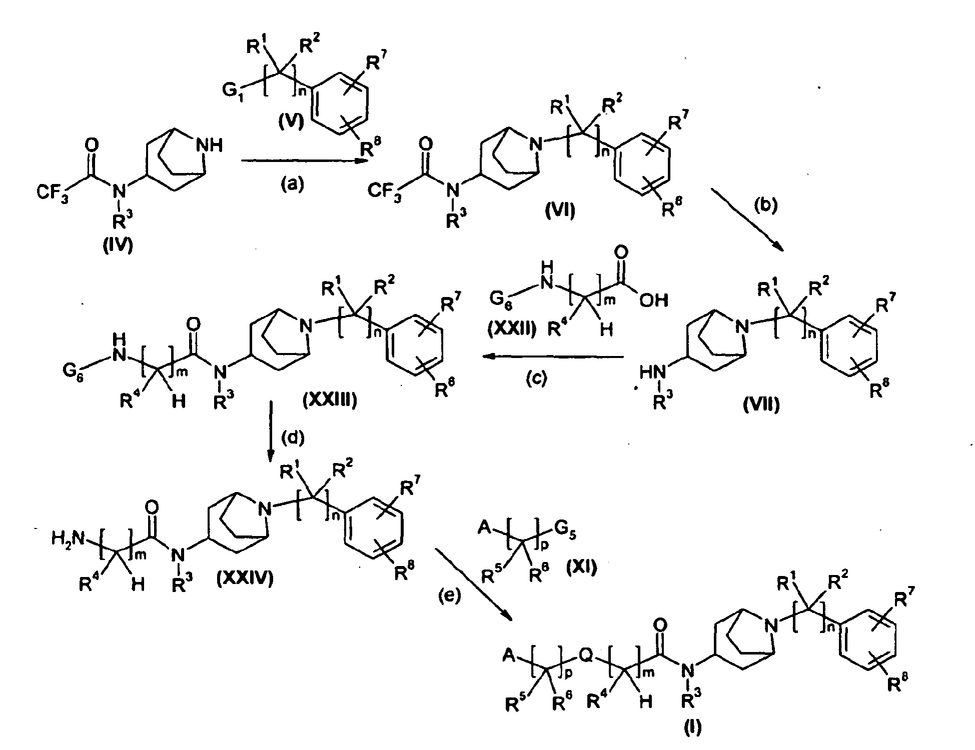

### Step a:

Reaction of compound (IV) with compound (V) wherein G¹ represents a halogen atom, preferably a chlorine atom and a base, such as triethylamine, in a polar aprotic solvent, such as acetone at reflux for 2 to 24 hours yields compound (VI).

### Step b:

Treatment of compound (VI) with an aqueous solution of sodium hydroxide in a polar, water-miscible, protic solvent, such as methanol, at room temperature for 2 to 12 hours yields compound (VII).

### Step c:

Compound (VII) may be converted into compound (XXIII) by reaction with compound (XXII) wherein G⁶ represents any standard amine-protecting group such as tert-butoxycarbonyl (BOC) using 1-(3-dimethylaminopropyl)-3-ethylcarbodiimide hydrochloride (EDC.HCl) and 1-hydroxybenzotriazole hydrate (HOBt) in a polar aprotic solvent, such as dichloromethane, at room temperature for 36 to 60 hours.

### Step d:

The N-protecting group of compound (XXIII) may be cleaved to yield compound (XXIV) by standard conditions. In case the protecting group is a tert-butoxycarbonyl (BOC), trifluoroacetic acid treatment at room temperature for 30 to 60 min is used to obtain compound (XXIV).

### Step e:

Compound (XXIV) may be reacted with compound (XI) to obtain compound (I).

When Q is a -CONR⁹- group, G⁵ represents a -COOH group and the reaction takes place in the presence of 1-(3-dimethylaminopropyl)-3-ethylcarbodiimide hydrochloride (EDC.HCI) and 1-hydroxybenzotriazole hydrate (HOBt) in a polar, aprotic solvent, such as dichloromethane or in the presence of N,N'-carbonyldiimidazole (CDI) and 4-dimethylaminopyridine (DMAP) in an aprotic solvent, such as tetrahydrofuran or dichloromethane at room temperature for a time period between 2 hours and 48 hours.

In an alternative, group G⁵ represents a -COX group wherein X is a halogen atom, such as chlorine, and the reaction takes place in a polar, aprotic solvent such as dichloromethane at room temperature for 0,5 to 24 hours in the presence of a base such as triethylamine.

When Q is a -OCONR⁹-, group G⁵ represents a -OCOX group wherein X is a halogen atom, such as chlorine, and the reaction takes place in a polar, aprotic solvent such as dichloromethane at room temperature for 2 to 24 hours in the presence of a base such as triethylamine

When Q is a -NHCONR¹⁰- group, G⁵ represents a -N=C=O group and the reaction takes place in the presence of a base such as triethylamine in an aprotic solvent such as dichloromethane, diethylether or THF at room temperature for 1 to 24 hours.

When Q is a -NR⁹CONR¹⁰- group, G⁵ represents -NR⁹COCI and the reaction may take place in the presence of a base in a polar, aprotic solvent. Examples of particular conditions are the use of N,N-diisopropylethylamine (DIEA) as a base in dichloromethane at room temperature for 2 to 24 hours and the use of triethylamine in THF at reflux for 2 to 24 hours.

When Q is a -SO₂NR⁹- group, G⁵ represents a -SO₂CI group and the reaction takes place in a polar, aprotic solvent such as dichloromethane at room temperature for 2 to 24 hours in the presence of a base such as triethylamine.

### Method 7

This method finds application in the manufacture of compounds of formula (I) wherein Q represents a group selected from -CONR⁹-, -OCONR⁹-, -NR⁹CONR¹⁰- and -SO₂NR⁹-, R³ represents a hydrogen atom and the R² substituent on the carbon atom directly attached to the benzene ring is a hydrogen atom.

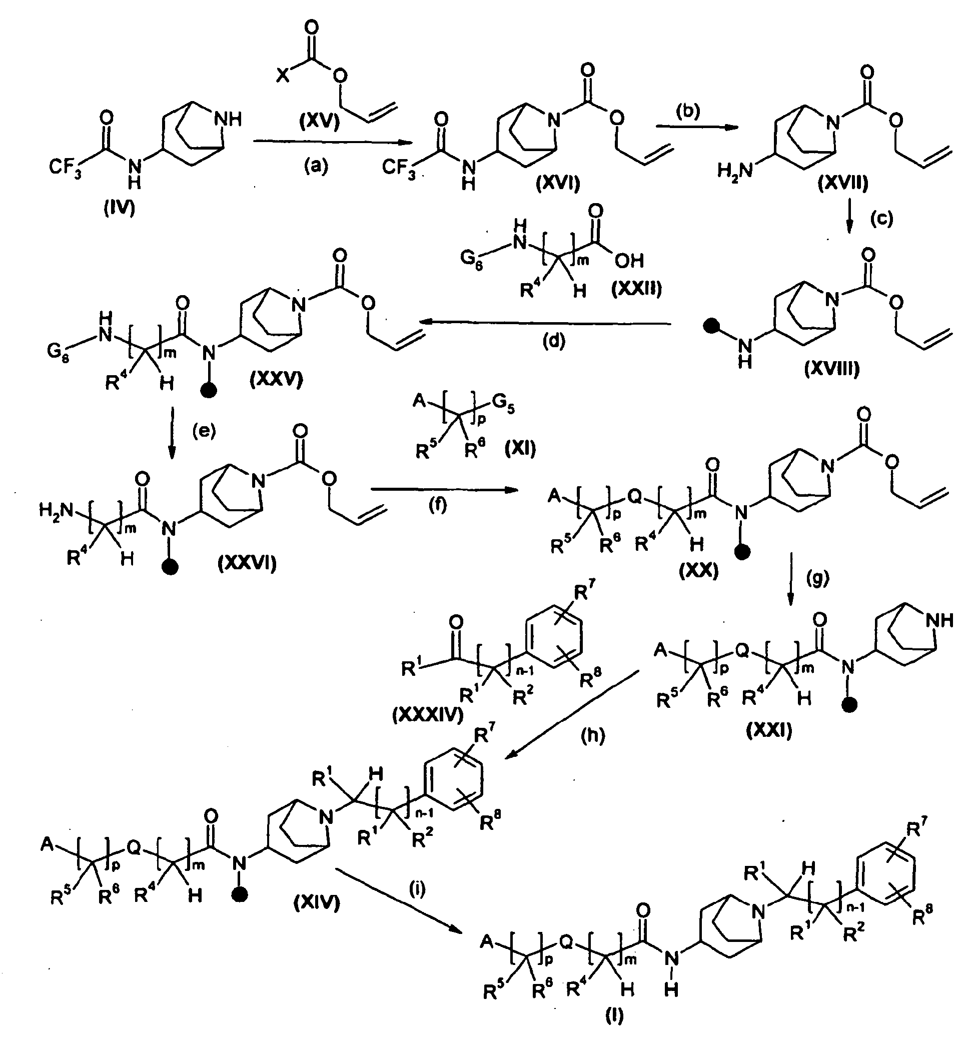

### Step a:

Reaction of compound (IV) with allyl chloroformate (XV) in the presence of a base, such as triethylamine in an aprotic solvent, such as tetrahydrofuran at room temperature for 10 to 18 hours yields compound (XVI).

### Step b:

Treatment of compound (XVI) with an aqueous solution of sodium hydroxide in a polar protic solvent, such as methanol at room temperature for 2 to 12 hours yields compound (XVII).

### Step c:

Compound (XVIII) may be obtained by reacting a benzaldehide functionalised resin (IRORI BAL resin) previously swollen in dimethylformamide/methanol (8:2) and a solution of compound (XVII) and acetic acid in dimethylformamide/methanol (8:2). The mixture is stirred for 1 to 4 hours at room temperature. A solution of sodium cyanoborohydride in dimethylformamide/methanol (8:2) is added and the mixture is further stirred at room temperature for 12 to 24 hours. The resin (XVIII) was filtered, washed with methanol (3X), dimethylformamide (3X), dichloromethane (3X) and dried in vacuo at room temperature.

### Step d:

Reaction of compound (XVIII) with compound (XXII) wherein G⁶ represents a standard amine-protecting group such as (9H-fluoren-9-ylmethoxy)carbonyl (FMOC) using N,N'-diisopropylcarbodiimide (DIC) in an aprotic solvent, such as tetrahydrofuran at 50-60°C for 20 to 30 hours and then, optionally, for up to 48 hours at room temperature yields compound (XXV).

### Step e:

The amine-protecting group of compound (XXV) may be cleaved to yield compound (XXVI) by standard conditions. In case the protecting group is a (9H-fluoren-9-ylmethoxy)carbonyl (Fmoc), a treatment with a 20% solution of piperidine in an aprotic solvent, such as dimethylformamide at room temperature for 40 to 60 minutes was used.

### Step f:

Compound (XXVI) may be reacted with compound (XI) to obtain compound (XX).

Wherein Q is a -CONR⁹- group, G⁵ represents a -COOH group and the reaction takes place in the presence of N,N'-diisopropylcarbodiimide in dichloromethane/dimethylformamide 9:1 or THF at 30-60°C for 12 to 24 hours to yield the compound (XX).

When Q is a -OCONR⁹- group, G⁵ represents a -OCOCI group and the reaction takes place in the presence of a base in a polar, aprotic solvent at room temperature for 2 to 24 hours. Examples of particular conditions are the use of N,N-diisopropylethylamine (DIEA) as a base in dichloromethane and the use of triethylamine in THF/dichloromethane (1:1).

When Q is a -NHCONR¹⁰- group, G⁵ represents a -N=C=O group and the reaction takes place in the presence of a base such as triethylamine in an aprotic solvent such as dichloromethane at room temperature for 12 to 24 hours.

When Q is a -NR⁹CONR¹⁰- group, G⁵ represents -NR⁹COCI and the reaction may take place in the presence of a base such as triethylamine in a polar, aprotic solvent such as dichloromethane at room temperature for 12 to 24 hours.

When Q is a -SO₂NR⁹- group, G⁵ represents a -SO₂CI group and the reaction takes place in pyridine/dichlomethane (1:1) at room temperature for 6 to 24 hours.

### Step g:

The allyloxycarbonyl (alloc) protecting group of compound (XX) may be cleaved using dimethylamino-borane and tetrakis(triphenylphosphine)palladium in a polar aprotic solvent, such as dichloromethane, at room temperature for 40 to 60 minutes to obtain compound (XXI).

### Step h:

Reaction of compound (XXI) with the corresponding substituted phenyl keto-derivative (XXXIV) using catalytic amounts of an acid, such as acetic acid and a reducing agent, such as sodium triacetoxiborohydride in an aprotic solvent, such as dimethylformamide at room temperature for 12 to 24 hours yields compound (XIV).

### Step i:

Compound (XIV) was cleaved from the resin using standard conditions such as addition to a solution of dichloromethane/trifluoroacetic acid/H2O (50:50:1) or trifluoroacetic acid/H2O (95:5) and reaction for 1 to 3 hours at room temperature to yield the final compound (I).

### Method 8

This method finds application in the manufacture of compounds of formula (I) wherein Q represents a group selected from -CONR⁹-, -OCONR⁹-, -NR⁹CONR¹⁰- and -SO₂NR⁹- and R³ represents a hydrogen atom.

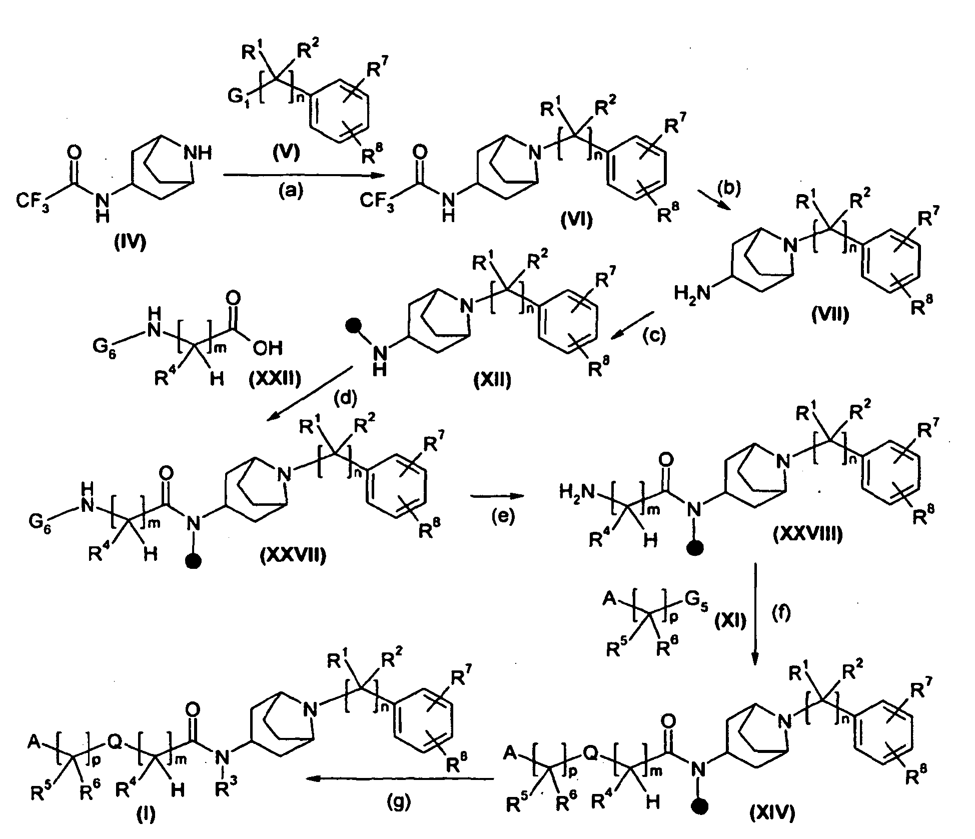

### Step a:

Reaction of compound (IV) with the corresponding halo-derivative (V) wherein G¹ represents a halogen atom, preferably a chlorine atom in the presence of a base, such as triethylamine, in a polar aprotic solvent, such as acetone at reflux for 2 to 24 hours yields compound (VI).

### Step b:

Treatment of compound (VI) with an aqueous solution of sodium hydroxide in a polar, water-miscible, protic solvent, such as methanol, at room temperature for 2 to 12 hours yields compound (VII).

### Step c:

Compound (XII) may be obtained by reacting a benzaldehide functionalised resin (IRORI BAL resin) previously swollen in dimethylformamide and a suspension of compound (VII) and sodium cyanoborohydride in dimethylformamide/acetic acid (99:1). An excess of perfluorodecaline may be added. The reaction mixture is stirred at 50°-60°C for 12-24 hours and the resin (XII) is filtered, washed with dimethylformamide (3X), AcOH/dimethylformamide (1%) (2X), dimethylformamide (1X), N,N-diisopropylethylamine/dimethylformamide (5%) (2X), dimethylformamide (2X), dichloromethane (1X), methanol (1X), diethylether (2X) and dried in vacuo at 35°C.

### Step d:

Compound (XXVII) may be obtained by reaction of compound (XII) wherein G⁶ is a standard amine-protecting group, such as a (9H-fluoren-9-ylmethoxy)carbonyl (FMOC) group with compound (XXII) using N,N'-diisopropylcarbodiimide (DIC) in an aprotic solvent, such as tetrahydrofuran at 50-60°C for about 20 to 30 hours and then, optionally, for up to 48 hours at room temperature.

### Step e:

The N-protecting group of compound (XXVII) may be cleaved to yield compound (XXVIII) by standard conditions. In case the protecting group is a (9H-fluoren-9-ylmethoxy)carbonyl (Fmoc), a treatment with a 20% solution of piperidine in an aprotic solvent, such as dimethylformamide at room temperature for 40 to 60 minutes yields compound (XXVIII).

### Step f:

Compound (XXVIII) may be reacted with compound (XI) to yield compound (XIV).

Wherein Q is a -CONR⁹- group, G⁵ represents a -COOH group and the reaction takes place in the presence of N,N'-diisopropylcarbodiimide in dichloromethane/dimethylformamide 9:1 or THF at 30-60°C for 12 to 24 hours to yield the compound (XIV).

When Q is a -OCONR⁹- group, G⁵ represents a -OCOCI group and the reaction takes place in the presence of a base in a polar, aprotic solvent at room temperature for 2 to 24 hours. Examples of particular conditions are the use of N,N-diisopropylethylamine (DIEA) as a base in dichloromethane and the use of triethylamine in THF/dichloromethane (1:1).

When Q is a -NHCONR¹⁰- group, G⁵ represents a -N=C=O group and the reaction takes place in the presence of a base such as triethylamine in an aprotic solvent such as dichloromethane at room temperature for 12 to 24 hours.

When Q is a -NR⁹CONR¹⁰- group, G⁵ represents -NR⁹COCI and the reaction may take place in the presence of a base such as triethylamine in a polar, aprotic solvent such as dichloromethane at room temperature for 12 to 24 hours.

When Q is a -SO₂NR⁹- group, G⁵ represents a -SO₂Cl group and the reaction takes place in pyridine/dichlomethane (1:1) at room temperature for 6 to 24 hours.

### Step g:

Compound (XIV) was cleaved from the resin using standard conditions such as addition to a solution of dichloromethane/trifluoroacetic acid/H2O (50:50:1) or trifluoroacetic acid/H2O (95:5) and reaction for 1 to 3 hours at room temperature to yield the final compound (I).

### Method 9

This method finds application in the manufacture of compounds of formula (I) wherein Q represents a group selected from -CONR⁹-, -OCONR⁹-, -NR⁹CONR¹⁰- and -SO₂NR⁹.

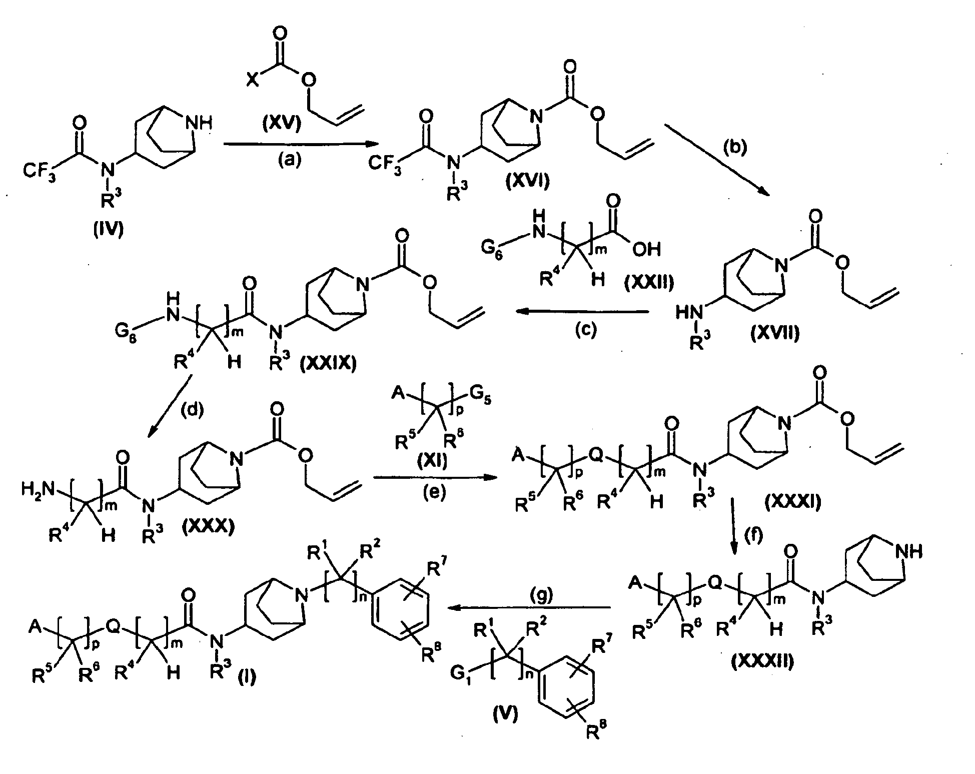

### Steep a:

Reaction of compound (IV) with allyl chloroformate (XV) in the presence of a base, such as triethylamine in an aprotic solvent, such as tetrahydrofuran at room temperature for 10 to 18 hours yields compound (XVI).

### Step b:

Treatment of compound (XVI) with an aqueous solution of sodium hydroxide in a polar, water-miscible, protic solvent, such as methanol at room temperature for 2 to 12 hours yields compound (XVII).

### Step c:

Compound (XVII) may be converted into compound (XXIX) by reaction with compound (XXII) wherein G⁶ represents any standard amine-protecting group such as tert-butoxycarbonyl (BOC) using 1-(3-dimethylaminopropyl)-3-ethylcarbodiimide hydrochloride (EDC.HCI) and 1-hydroxybenzotriazole hydrate (HOBt) in a polar aprotic solvent, such as dichloromethane, at room temperature for 36 to 60 hours.

### Step d:

The N-protecting group of compound (XXIX) may be cleaved to yield compound (XXX) by standard conditions. In case the protecting group is a tert-butoxycarbonyl (BOC), trifluoroacetic acid treatment at room temperature for 30 to 60 min is used to obtain compound (XXX).

### Step e:

Compound (XXX) may be reacted with compound (XI) to yield compound (XXXI).

Wherein Q is a -CONR⁹- group, G⁵ represents a -COOH group and the reaction takes place in the presence of 1-(3-dimethylaminopropyl)-3-ethylcarbodiimide hydrochloride (EDC.HCI) and 1-hydroxybenzotriazole hydrate (HOBt) in a polar, aprotic solvent, such as dichloromethane for a time period between 2 hours and 48 hours.

In an alternative, group G⁵ represents a -COX group wherein X is a halogen atom, such as chlorine, and the reaction takes place in a polar, aprotic solvent such as dichloromethane at room temperature for 0,5 to 24 hours in the presence of a base such as triethylamine.

When Q is a -OCONR⁹- group, G⁵ represents a -OCOCI group and the reaction takes place-OCOX group wherein X is a halogen atom, such as chlorine, and the reaction takes place in a polar, aprotic solvent such as dichloromethane at room temperature for 2 to 24 hours in the presence of a base such as triethylamine.

When Q is a -NHCONR¹⁰- group, G⁵ represents a -N=C=O group and the reaction takes place in the presence of a base such as triethylamine in an aprotic solvent such as dichloromethane, diethylether or THF at room temperature for 1 to 24 hours.

When Q is a -NR⁹CONR¹⁰- group, G⁵ represents -NR⁹COCI and the reaction may take place in the presence of a base in a polar, aprotic solvent. Examples of particular conditions are the use of N,N-diisopropylethylamine (DIEA) as a base in dichloromethane at room temperature for 2 to 24 hours and the use of triethylamine in THF at reflux for 2 to 24 hours.

When Q is a -SO₂NR⁹- group, G⁵ represents a -SO₂CI group and the reaction takes place in a polar, aprotic solvent such as dichloromethane at room temperature for 2 to 24 hours in the presence of a base such as triethylamine.

### Step f:

The allyloxycarbonyl (alloc) protecting group of compound (XXXI) may be cleaved by using an 85% aqueous solution of potassium hydroxide in 2-propanol at reflux for a time period between 10 hours and 20 hours to yield compound (XXXII).

### Step g:

Reaction of compound (XXXII) with the corresponding halo-derivative (V) wherein G¹ represents a halogen atom, preferably a chlorine atom in the presence of a base, such as trietylamine in an aprotic solvent, such as acetone at reflux for a time period between 1 hour and 20 hours yields compound (I).

### Method 10

This method finds application in the manufacture of compounds of formula (I) wherein Q represents a group-NR⁹COO-.

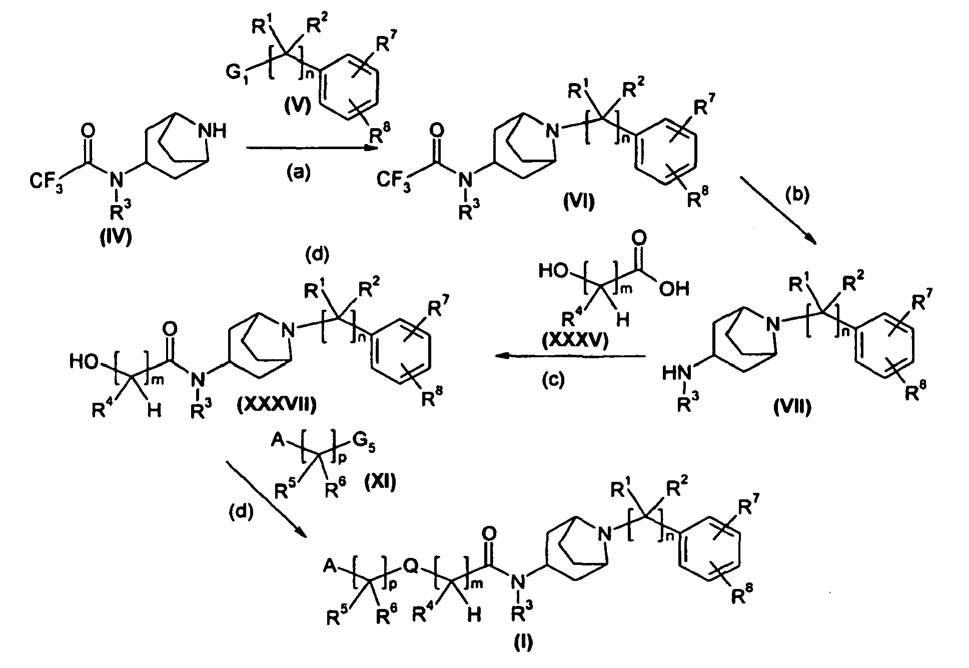

### Step a:

Reaction of compound (IV) with compound (V) wherein G¹ represents a halogen atom, preferably a chlorine atom and a base, such as triethylamine, in a polar aprotic solvent, such as acetone at reflux for 2 to 24 hours yields compound (VI).

### Step b:

Treatment of compound (VI) with an aqueous solution of sodium hydroxide in a polar, water-miscible, protic solvent, such as methanol, at room temperature for 2 to 12 hours yields compound (VII).

### Step c:

Compound (VII) may be reacted with the hydroxyacid (XXXV) in a aprotic solvent such as toluene or xylene at reflux for 12 to 36 hours to yield compound (XXXVI). In the alternative the reaction may take place in THF at reflux or DMF at room temperature using a coupling agent such as carbonyldiimidazole (CDI).

### Step d:

Compound (XXXVII) may be reacted with compound (XI) wherein G₅ represent a group selected from -N=C=O and -NR⁹-CO-Cl to obtain compound (I).

When G⁵ represents a -N=C=O group the reaction may take place in an aprotic solvent such as toluene or xylene at reflux for 2 to 24 hours.

When G⁵ represents a -NR⁹COCI group and the reaction may take place in the presence of sodium hydride in DMF at room temperature for 2 to 24 hours.

Methods 1 to 10 may be used in general to obtain the compounds of the present invention although the substituents R¹¹ of the phenyl ring may sometimes be advantageously derived from precursor substituents R¹¹. Examples of this derivatization step, which is normally effected immediately before cleavage from the resin, are as follows:
- When R¹¹ comprises a hydroxycarbonyl group (-COOH) it may be obtained by hydrolysis of the precursor -COOR group wherein R is a straight or branched C₁₋₄ alkyl group.
- When R¹¹ comprises an amino group (-NH₂) it may be obtained by reduction of the precursor -NO₂ group.
- When R¹¹ comprises an amido group (-NHCORe) it may be obtained by acylation of the precursor -NH₂ group.
- When R¹¹ comprises a sulfonamido group (-NHSO₂Rg) it may be obtained by sulfonylation of the precursor -NH₂ group.
- When R¹¹ comprises a guanidino group it may be obtained by derivatization of the precursor-NH₂ group with N,N'-di-BOC-1H-pyrazole-1-carboxamidine.
- When R¹¹ comprises an amido group (-CONRcRf) it may be obtained by reaction of the precursor -COOH or -COCI group with the amine NHRcRf.
- When R¹¹ comprises a substituted amino group (-NRcRd) it may be obtained from the precursor -NH₂ group.

The intermediates of formula (VIII) used in methods 1 and 5 are commercially available products or may be obtained by methods 11, 12 and 13 described below.

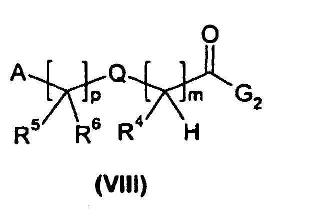

### Method 11

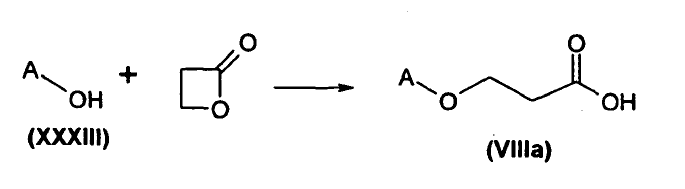

When A is a group of formula (i), p is 0, Q is oxygen, m is 2, R⁴ is a hydrogen atom and G² is a hydroxyl group, the compounds of formula (VIIIa) may be obtained by reaction of the aromatic alcohol (XXXIII) with β-propiolactone in the presence of a base, such as potassium tert-butoxide, in an aprotic solvent, such as tetrahydrofuran, at room temperature of 50-60 °C for a time period of 12-72 hours.

### Method 12

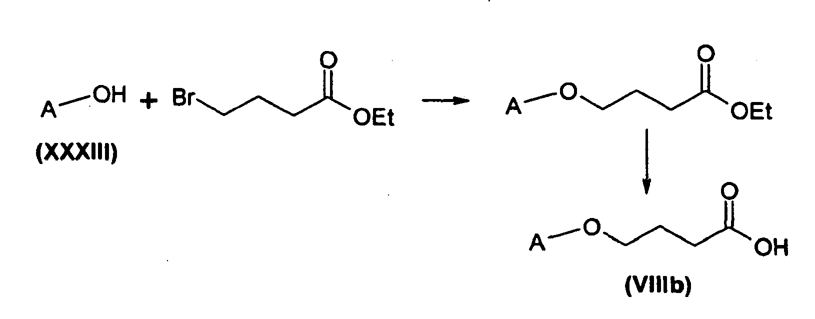

When A is a group of formula (i), p is 0, Q is oxygen, m is 3, R⁴ is a hydrogen atom and G² is a hydroxyl group, the compounds of formula (VIIIb) may be obtained by reaction of the aromatic alcohol (XXXIII) with ethyl 4-bromobutyrate in the presence of a base, such as potassium tet-butoxide, in an aprotic solvent, such as dimethylformamide at 110°C for 2-3 hours and the subsequent hydrolysis with NaOH in water/methanol at 0°C.

### Method 13

When p is 0, Q is NHCO, m is 1 and R⁴ is hydrogen, the compounds of formula (Vlllc) 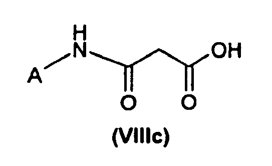 are replaced by their precursors of formula (II) which may be obtained by reaction of the aromatic isocianate (XXXIV) with Meldrum's acid in the presence of a base, such as triethylamine, in an aprotic solvent, such as dimethylformamide at room temperature for 4-5 hours.

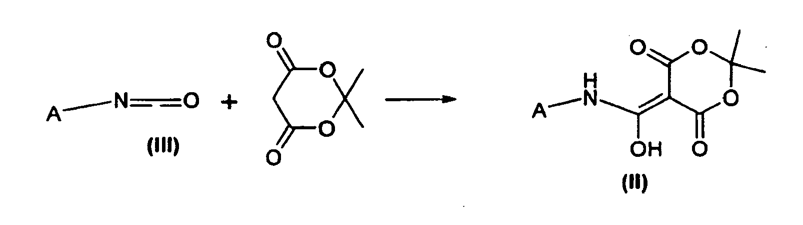

The compounds of formulae (III), (IV), (V), (IX), (XI), (XV), (XXII), (XXXIII), (XXXIV) and (XXXV) are commercially available or may be obtained by analogy with standard synthetic methods.

### Intermediates' synthesis:

### Intermediates 1 and 2 (3-endo)-8-azabicyclo[3.2.1]octan-3-amine and (3-exo)-8-azabicyclo[3.2.1]octan-3-amine

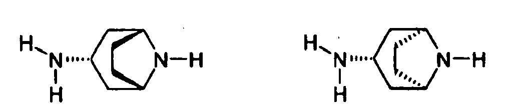

The synthesis of intermediates 1 and 2 is described in European Patent EP 0 013 138 A1.

### Intermediate 3 N-[(3-endo)-8-azabicyclo[3,2,1] oct-3-yl]-2,2,2-trifluoroacetamide

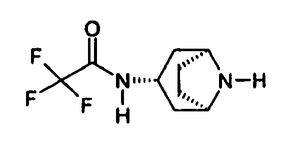

Sodium (18.4 g, 800 mmols) was added in small portions to ethanol (1460 mL) and the mixture was allowed to reach room temperature. To the solution obtained (3-endo)-8-azabicyclo[3.2.1]octan-3-amine dichlorohydrate (Intermediate 1) (73.4 g, 368 mmols) was added in small portions. The resulting mixture was stirred for 1 hour at room temperature. The solid obtained was filtered off. The filtrate was concentrated in vacuo and the oil obtained was dissolved in acetonitrile (825 mL) and water (8.25 mL). Ethyl trifluoroacetate was added (158 mL, 1325 mmols) and the resulting mixture was stirred at reflux overnight. The reaction crude obtained was concentrated in vacuo, it was dissolved in a water/ice mixture and washed with diethyl ether and dichlorometane. The aqueous layer was basified with sodium hydrogen carbonate, it was saturated with sodium chloride and extracted with ethyl acetate. The organic layer was dried over magnesium sulfate, filtered and concentrated in vacuo. The semi-solid crude obtained was dissolved in the minimum volume of ethyl acetate and the inorganic solid obtained was separated by filtration. The filtrate was concentrated in vacuo and the title compound was obtained (68.0 g).

### Intermediate 4 N-[(3-exo)-8-azabicyclo[3.2.1]oct-3-yl]-2,2,2-trifluoroacetamide

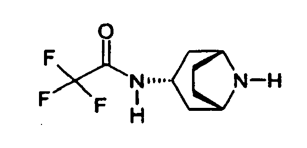

Sodium (2.3 g) was added in small portions to methanol (400 mL) and the mixture was allowed to reach room temperature. A solution of (3-exo)-8-azabicyclo[3.2.1]octan-3-amine dichlorohydrate (Intermediate 2) (20.0 g, 100 mmols) in methanol (125 mL) and water (25 mL) was added dropwise. The resulting mixture was stirred for 10 min. at room temperature and it was concentrated in vacuo. Ethanol (100 mL) was added and again it was concentrated in vacuo. The crude obtained was triturated with acetonitrile (250 mL) and it was stirred at 85°C for 10 min. The solid obtained was filtered off and to the filtrate 2.3 mL of water were added. Ethyl trifluoroacetate was added (43 mL) and the resulting mixture was stirred at reflux for overnight. The solid obtained was filtered off and the filtrate was concentrated in vacuo. The reaction crude obtained was dissolved in 20 mL of water and basified with sodium hydrogen carbonate. Ethyl acetate and more of water (20 mL) was added. The organic layer was washed with brine, dried over magnesium sulfate, filtered and concentrated in vacuo. Diethyl ether was added and the solid obtained was filtered off to give the title compound (11.9 g).

### Intermediate 5 allyl (3-endo)-3-[(trifluoroacetyl)amino]-8-azabicyclo[3.2.1]octane-8-carboxylate

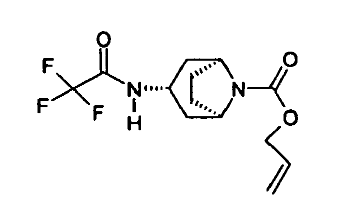

N-[(3-endo)-8-azabicyclo[3.2.1]oct-3-yl]-2,2,2-trifluoroacetamide (Intermediate 3) (53.0 g, 239 mmols) was dissolved in tetrahydrofuran (361 mL). Triethylamine was added (36.0 mL, 259 mmols) and a solution of allyl chloroformate (29 mL, 272 mmols) in tetrahydrofuran (60 mL) was added dropwise keeping the reaction mixture at room temperature and it was stirred under the same conditions overnight. The solid obtained was filtered off, washed with tetrahydrofuran and the filtrate was concentrated in vacuo. The reaction crude obtained was dissolved in ethyl acetate, washed with water, a 4% aqueous solution of sodium hydrogen carbonate and again with water. The organic layer was dried over magnesium sulfate, filtered and concentrated in vacuo to give the title compound (50.0 g). (RL-4837-004)

### Intermediate 6 allyl(3-exo)-3-[(trifluoroacetyl)amino]-8-azabicyclo[3.2.1]octane-8-carboxylate

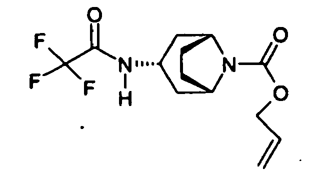

N-[(3-exo)-8-azabicyclo[3.2.1]oct-3-yl]-2,2,2-trifluoroacetamide (Intermediate 4) (3.5 g, 16 mmols) was suspended in tetrahydrofuran (35 mL). Triethylamine was added (3.1 mL, 22 mmols) and a solution of allyl chloroformate (1.2 mL, 11 mmols) in tetrahydrofuran (3 mL) was added dropwise keeping the reaction mixture at room temperature and it was stirred under the same conditions for overnight. The reaction mixture was concentrated in vacuo and it was dissolved in ethyl acetate and washed with a 4% aqueous solution of sodium hydrogen carbonate. The organic layer was dried over magnesium sulfate, filtered and concentrated in vacuo. The oil obtained was solidified with diethyl ether and the title compound was obtained (2.2 g).

### Intermediate 7 allyl (3-endo)-3-amino-8-azabicyclo[3.2.1]octane-8-carboxylate

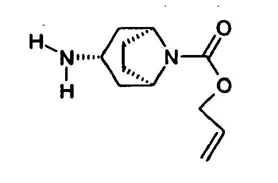

Allyl (3-endo)-3-[(trifluoroacetyl)amino]-8-azabicyclo[3.2.1]octane-8-carboxylate (Intermediate 5) (50 g, 163 mmols) was dissolved in methanol (815 mL) and a 1 N aqueous solution of sodium hydroxide (408 mL) was added dropwise keeping the mixture at room temperature and it was stirred under the same conditions overnight. The reaction mixture was concentrated in vacuo and the aqueous layer obtained was saturated with sodium chloride. It was extracted with ethyl acetate and washed with brine. The organic layer was dried over magnesium sulfate, filtered and concentrated in vacuo to give the title compound (31.0 g).

### Intermediate 8 allyl (3-endo)-3-amino-8-azabicyclo[3.2.1]octane-8-carboxylate fumarate

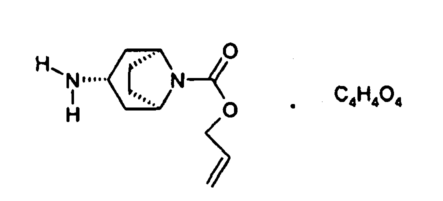

Obtained from allyl (3-endo)-3-amino-8-azabicyclo[3.2.1]octane-8-carboxylate (Intermediate 7) following standard synthetic methods.

### Intermediate 9 allyl (3-exo)-3-amino-8-azabicyclo[3.2.1]octane-8-carboxylate

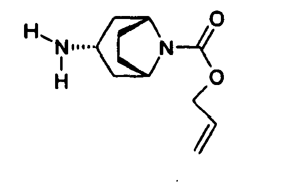

Allyl (3-exo)-3-[(trifluoroacetyl)amino]-8-azabicyclo[3.2.1]octane-8-carboxylate (Intermediate 6) (3.1 g, 10 mmols) was dissolved in methanol (62 mL) and a 1 N aqueous solution of sodium hydroxide (25 mL) was added dropwise keeping the mixture at room temperature and it was stirred under the same conditions overnight. The reaction mixture was concentrated in vacuo and the aqueous layer obtained was saturated with sodium chloride. It was extracted with ethyl acetate and washed with brine. The organic layer was dried over magnesium sulfate, filtered and concentrated in vacuo. Diethyl ether was added to the oil obtained and the solid obtained was filtered off. The filtrate was concentrated in vacuo to give the title compound (1.9 g).

### Intermediate 10 N-[(3-endo)-8-(4-chlorobenzyl)-8-azabicyclo[3.2.1]oct-3-yl]-2,2,2-trifluoroacetamide

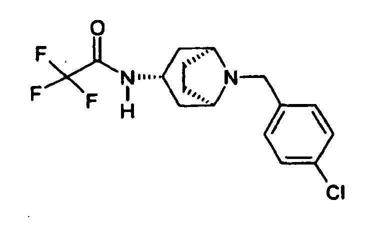

4-chlorobenzyl chloride (14.1 g, 87 mmols) was added to a solution of N-[(3-endo)-8-azabicyclo[3.2.1]oct-3-yl]-2,2,2-trifluoroacetamide (Intermediate 3) (14.1 g, 63 mmols) in acetone (250 mL). Triethylamine (25 mL, 180 mmols) was added and the mixture was stirred at reflux for 6 hours and then at room temperature for 12 hours. The solid obtained was filtered off and the filtrate was concentrated in vacuo. The reaction crude obtained was disolved in dichloromethane and the solution was washed with water and brine. The organic layer was dried over sodium sulfate, filtered and concentrated in vacuo. The oil obtained was suspended in hexane and the solid that precipitates was filtered and resuspended again in the same solvent. The solid obtained was filtered and dried to give the title compound (11.25 g).

### Intermediate 11 (3-endo)-8-(4-chlorobenzyl)-8-azabicyclo[3.2.1]octan-3-amine

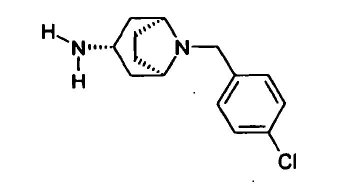

A 1N aqueous solution of sodium hydroxide (220 mL) was added to a solution of N-[(3-endo)-8-(4-chlorobenzyl)-8-azabicyclo[3.2.1 ]oct-3-yl]-2,2,2-trifluoroacetamide (Intermediate 10) (11.2 g, 32 mmols) in methanol (220 mL). The mixture was stirred for 2 hours at room temperature. It was concentrated in vacuo and the aqueous layer obtained was saturated with sodium chloride. It was extracted with dichloromethane and the organic layer obtained was washed with brine, dried over magnesium sulfate, filtered and concentrated in vacuo to give the title compound (7.61 g).

### Intermediate 12 N-[(3-endo)-8-(4-fluorobenzyl)-8-azabicyclo[3,2,1]oct-3-yl]-2,2,2-trifluoroacetamide

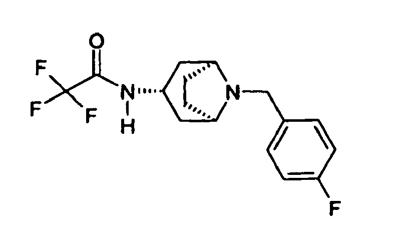

4-fluorobenzyl chloride (5.2 g, 36 mmols) was added to a solution of N-[(3-endo)-8-azabicyclo[3.2.1]oct-3-yl]-2,2,2-trifluoroacetamide (Intermediate 3) (5.0 g, 23 mmols) in acetone (90 mL). Triethylamine (9.4 mL, 68 mmols) was added and the mixture was stirred at reflux for 6 hours and then at room temperature for 12 hours. The solid obtained was filtered off and the filtrate was concentrated in vacuo. The reaction crude obtained was disolved in dichloromethane and the solution was washed with water and brine. The organic layer was dried over sodium sulfate, filtered and concentrated in vacuo. The oil obtained was washed with hexane and finally stirred at room temperature for 2-3 hours suspended on the same solvent. The semi-solid obtained was filtered and dried in vacuo to give the title compound (0.64 g).

### Intermediate 13 (3-endo)-8-(4-fluorobenzyl)-8-azabicyclo[3.2.1]octan-3-amine

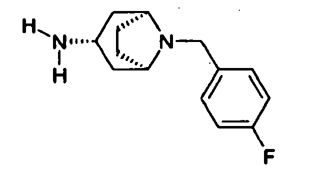

A 1 N aqueous solution of sodium hydroxide (35 mL) was added to a solution of N-[(3-endo)-8-(4-fluorobenzyl)-8-azabicyclo[3.2.1]oct-3-yl]-2,2,2-trifluoroacetamide (Intermediate 12) (0.64 g, 1.9 mmols) in methanol (35 mL). The mixture was stirred for 2 hours at room temperature. It was concentrated in vacuo and the aqueous layer obtained was saturated with sodium chloride. It was extracted with dichloromethane and the organic layer obtained was washed with brine, dried over magnesium sulfate, filtered and concentrated in vacuo to give the title compound (0.79 g).

### Intermediate 14 2-chloro-N-[(3-endo)-8-(4-fluorobenzyl)-8-azabicyclo[3.2.1]oct-3-yl]acetamide

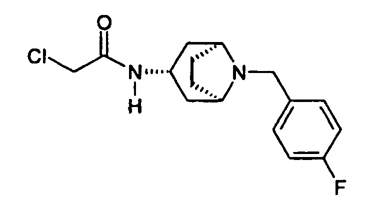

Triethylamine (0.75 mL, 5.3 mmols) was added to a solution of (3-endo)-8-(4-fluorobenzyl)-8-azabicyclo[3.2.1]octan-3-amine (Intermediate 13) (1.25 g, 5.3 mmols) in dichloromethane (20 mL) and the mixture obtained was cooled down to 0-5°C. A solution of chloroacetyl chloride (0.43 mL, 5.3 mmols) in dichloromethane (20 mL) was added dropwise to the mixture under the same temperature conditions. The mixture was allowed to reach room temperature and it was stirred for 21 hours. The reaction mixture was washed with water and a 1 N aqueous solution of hydrochloric acid. The acidic aqueous layer was washed with dichloromethane and basified with potassium carbonate. It was extracted with dichloromethane and the organic layer obtained was dried over magnesium sulfate, filtered and concentrated in vacuo to give the title compound (1.97g).

### Intermediate 15 2-chloro-N-[(3-endo)-8-(4-chlorobenzyl)-8-azabicyclo[3.2.1]oct-3-yl]acetamide

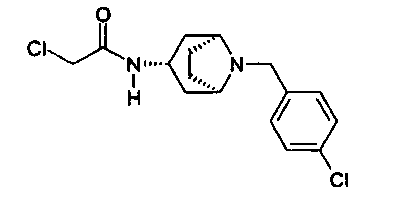

Triethylamine (1.68 mL, 12 mmols) was added to a solution of (3-endo)-8-(4-chlorobenzyl)-8-azabicyclo[3.2.1]octan-3-amine (Intermediate 11) (3.0 g, 12 mmols) in dichloromethane (45 mL) and the mixture obtained was cooled down to 0-5°C. A solution of chloroacetyl chloride (0.96 mL, 12 mmols) in dichloromethane (45 mL) was added dropwise to the mixture under the same temperature conditions. The mixture was allowed to reach room temperature and it was stirred for 21 hours. The reaction mixture was washed with water and a 1 N aqueous solution of hydrochloric acid. The acidic aqueous layer was washed with dichloromethane and basified with potassium carbonate. It was extracted with dichloromethane and the organic layer obtained was dried over magnesium sulfate, filtered and concentrated in vacuo to give the title compound (3.74 g).

### Intermediate 16 tert-butyl 2-((3-endo)-8-(4-chlorobenzyl)-8-aza-bicyclo[3.2.1]octan-3-ylamino)-2-oxoethylcarbamate

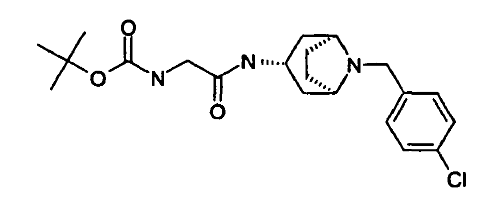

N-(tert-butoxycarbonyl)glycine (2.09 g, 12.0 mmols), 1-(3-dimethylaminopropyl)-3-ethylcarbodiimide hydrochloride (EDC.HCI) (1.62 g, 8.47 mmols) and 1-hydroxybenzotriazole hydrate (HOBt) (2.30 g, 17.0 mmols) were dissolved in dichloromethane (200 mL). It was stirred for 5 minutes at room temperature. A solution of (3-endo)-8-(4-chlorobenzyl)-8-azabicyclo[3.2.1]octan-3-amine (Intermediate 11) (2.5g, 10.0 mmols) in dichloromethane (30 mL) was added. The mixture was stirred for 48 hours at room temperature. It was washed with a saturated aqueous solution of potassium carbonate, water and brine. The organic layer was dried over sodium sulfate, filtered and concentrated in vacuo to give the title compound (4.05 g) (yield: 99.5%)

### Intermediate 17 N¹-[(3-endo)-8-(4-chlorobenzyl)-8-azabicyclo[3.2.1]oct-3-yl]glycinamide

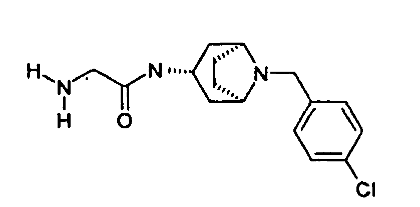

tert-butyl 2-((3-endo)-8-(4-chlorobenzyl)-8-aza-bicyclo[3.2.1]octan-3-ylamino)-2-oxoethylcarbamate (intermediate 16) (4.05 g, 10 mmols) was dissolved in trifluoroacetic acid (35 mL) and the solution was stirred for 30 minutes at room temperature. It was concentrated in vacuo and the residue was poured into water/ice. Potassium carbonate was added until a basic pH was obtained. It was extracted with chloroform, the organic layer was dried over sodium sulfate, filtered and concentrated in vacuo. The solid obtained was triturated with diethyl ether, filtered and dried in vacuo to give the title compound (1.32 g) (yield: 43%)

### Intermediate 18 allyl (3-endo)-3-{[N-(tert-butoxycarbonyl)glycyl]amino}-8-azabicyclo[3.2.1]octane-8-carboxylate

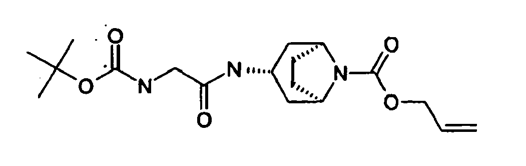

N-(tert-butoxycarbonyl)glycine (0.65 g, 3.7 mmols), 1-(3-dimethylaminopropyl)-3-ethylcarbodiimide hydrochloride (EDC.HCI) (0.71 g, 3.7 mmols) and 1-hydroxybenzotriazole hydrate (HOBt) (0.50 g, 3.7 mmols) were dissolved in dichloromethane (80 mL). It was stirred for 5 minutes at room temperature. A solution of allyl (3-endo)-3-amino-8-azabicyclo[3.2.1]octane-8-carboxylate (Intermediate 7) (0.64 g, 3.0 mmols) in dichloromethane (10 mL) was added. The mixture was stirred for 2 hours at room temperature. It was washed with a saturated aqueous solution of potassium carbonate, a 10% aqueous solution of potassium hydrogen sulfate, a saturated aqueous solution of sodium hydrogen carbonate and brine. The organic layer was dried over sodium sulfate, filtered and concentrated in vacuo to give the title compound (1.18 g).

### Intermediate 19 allyl (3-endo)-3-(glycylamino)-8-azabicyclo[3.2.1]octane-8-carboxylate

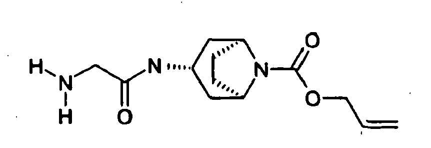

Allyl (3-endo)-3-{[N-(tert-butoxycarbonyl)glycyl]amino}-8-azabicyclo[3.2.1]octane-8-carboxylate (Intermediate 18) (10.9 g, 30 mmols) was dissolved in trifluoroacetic acid (85 mL) and the solution was stirred for 30 minutes at room temperature. It was concentrated in vacuo and the residue was poured into water/ice. Potassium carbonate was added until a basic pH was obtained. It was extracted with chloroform and the organic layer was washed with brine, dried over sodium sulfate, filtered and concentrated in vacuo to give the title compound (5.32 g) (yield: 67%).

### Intermediate 20 Allyl (3-exo)-3-{[N-(tert-butoxycarbonyl)-glycyl]amino}-8-azabicyclo[3.2.1]octane-8-carboxylate

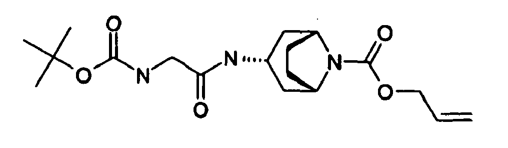

N-(tert-butoxycarbonyl)glycine (4.7 g, 26.8 mmols), 1-(3-dimethylaminopropyl)-3-ethylcarbodiimide hydrochloride (EOC.HCI) (5.1 g, 26.7 mmols) and 1-hydroxybenzotriazole hydrate (HOBt) (3.6 g, 26.7 mmols) were dissolved in dichloromethane (575 mL). It was stirred for 5 minutes at room temperature. A solution of allyl (3-exo)-3-amino-8-azabicyclo[3.2.1]octane-8-carboxylate (Intermediate 9) (4.61g, 22.0 mmols) in dichloromethane (70 mL) was added. The mixture was stirred for 3.5 hours at room temperature. It was washed with a saturated aqueous solution of potassium carbonate, a 10% aqueous solution of potassium hydrogen sulfate, a saturated aqueous solution of sodium hydrogen carbonate and brine. The organic layer was dried over sodium sulfate, filtered and concentrated in vacuo to give the title compound (9.1 g).

### Intermediate 21 allyl (3-exo)-3-(glycylamino)-8-azabicyclo[3.2.1]octane-8-carboxylate

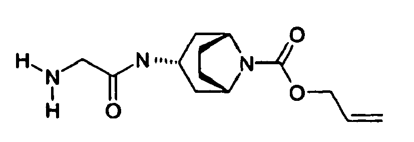

Allyl (3-exo)-3-{[N-(tert-butoxycarbonyl)glycyl]amino}-8-azabicyclo[3.2.1]octane-8-carboxylate (Intermediate 20) (9.1 g, 24.8 mmols) was dissolved in trifluoroacetic acid (75 mL) and the solution was stirred for 30 minutes at room temperature. It was concentrated in vacuo and the residue was poured into water/ice. Potassium carbonate was added until a basic pH was obtained. It was extracted with chloroform and the organic layer was washed with brine, dried over sodium sulfate, filtered and concentrated in vacuo. Chromatography on silica gel gave the title compound (2.82 g) (yield: 43%).

### Solid phase synthesis intermediates loaded to Backbone amide linker (BAL) resin:

BAL resin is commercially available (it may be purchased from Advanced ChemTech). It is abridged as PS.

### Intermediate 22 PS-(3-endo)-8-(4-chlorobenzyl)-8-azabicyclo[3.2.1]octan-3-amine

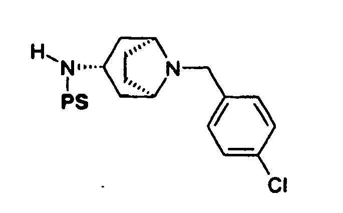

IRORI BAL resin (6.22 g, loading: 1 mmol/g, 6.22 mmols) was washed and swelled with dimethylformamide. The resin was filtered and added to a suspension of (3-endo)-8-(4-chlorobenzyl)-8-azabicyclo[3.2.1]octan-3-amine (Intermediate 11) (2.34 g, 9.33 mmols) and sodium cyanoborohydride (0.58 g, 9.33 mmols) in dimethylformamide/acetic acid 99:1 (6 mL).

50 mL of perfluorodecaline was added and the reaction mixture was stirred at 50°C overnight. It was filtered and the resin was washed with dimethylformamide (3X), AcOH/dimethylformamide (1%) (2X), dimethylformamide (1X), N,N-diisopropylethylamine/dimethylformamide (5%) (2X), dimethylformamide (2X), dichloromethane (1X), methanol (1X), diethylether (2X). The resin was dried in vacuo at 35°C.

### Intermediate 23 PS-2-bromo-N-[(3-endo)-8-(4-chlorobenzyl)-8-azabicyclo[3.2.1]oct-3-yl]acetamide

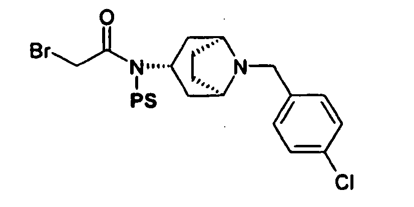

PS-(3-endo)-8-(4-chlorobenzyl)-8-azabicyclo[3.2.1]octan-3-amine (Intermediate 22) (2.2 g, loading: 1 mmol/g, 2.2 mmols) was washed and swelled with tetrahydrofuran. It was filtered and a solution of bromoacetic acid (2.37 g, 17 mmols) in tetrahydrofuran (20 mL) and a solution of N,N'-diisopropylcarbodiimide (3.5 mL) in tetrahydrofuran (30 mL) were added to the resin. The mixture was stirred for 1 hour at room temperature. The resin was filtered and washed with dimethylformamide (2X), dichloromethane (1X) and tetrahydrofuran (1X). The resin was treated for a second time under the above conditions except for the time which was 2 hours. It was filtered and washed with dimethylformamide (3X), dichloromethane (3X) and methanol (2X). The resin was dried in vacuo at 35°C.

### Intermediate 24 PS-allyl (3-exo)-3-amino-8-azabicyclo[3.2.1]octane-8-carboxylate

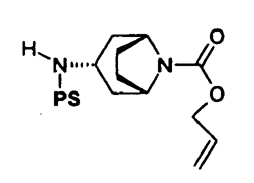

IRORI BAL resin (5.0 g, loading: 1 mmol/g, 5.0 mmols) was washed and swelled with dimethylformamide/methanol 8:2. The resin was filtered and added to a solution of allyl (3-exo)-3-amino-8-azabicyclo[3.2.1]octane-8-carboxylate (Intermediate 9) (5.54 g, 26.4 mmols) and acetic acid (0.5 mL) in dimethylformamide/methanol 8:2 (108 mL). The mixture was stirred for 1.5 hours at room temperature. A solution of sodium cyanoborohydride (1.6 g, 25.5 mmols) in dimethylformamide/methanol 8:2 (26 mL).was added and the mixture was stirred at room temperature overnight. It was filtered and washed with methanol (3X), dimethylformamide (3X), dichloromethane (3X). The resin was dried in vacuo at room temperature to give the title compound (5.17 g).

### Intermediate 25 PS-allvl (3-exo)-3-[(bromoacetyl)amino]-8-azabicyclo[3.2.1]octane-8-carboxylate

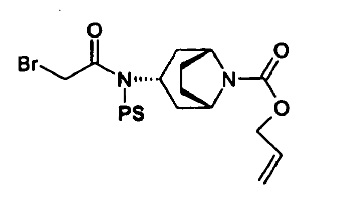

PS-allyl (3-exo)-3-amino-8-azabicyclo[3.2.1]octane-8-carboxylate (Intermediate 24) (2.0 g, loading: 1 mmol/g, 2mmols) was washed and swelled with tetrahydrofuran. It was filtered and a solution of bromoacetic acid (2.12 g, 15.3 mmols) in tetrahydrofuran (30 mL) and a solution of N,N'-diisopropylcarbodiimide (3.2 mL) in tetrahydrofuran (33 mL). The mixture was stirred for 1 hour at room temperature. It was filtered and washed with dimethylformamide (2X), dichloromethane (1X) and tetrahydrofuran (1X). The resin was treated for a second time under the above conditions except for the time which was 30 minutes. It was filtered and washed with dimethylformamide (3X), dichloromethane (3X), methanol (1X) and diethylether (2X). The resin was dried in vacuo at 35°C.

### Intermediate 26 PS-allyl (3-endo)-3-amino-8-azabicyclo[3.2.1]octane-8-carboxylate

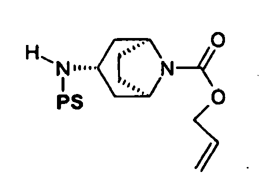

Following the same procedure described for PS-allyl (3-exo)-3-amino-8-azabicyclo[3.2.1]octane-8-carboxylate (Intermediate 24), the title compound was obtained from allyl (3-endo)-3-amino-8-azabicyclo[3.2.1]octane-8-carboxylate (Intermediate 7).

### Intermediate 27 PS-allyl (3-endo)-3-({N-[(9H-fluoren-9-ylmethoxy)carbonyl]glycyl}amino)-8-azabicyclo[3.2.1]octane-8-carboxylate

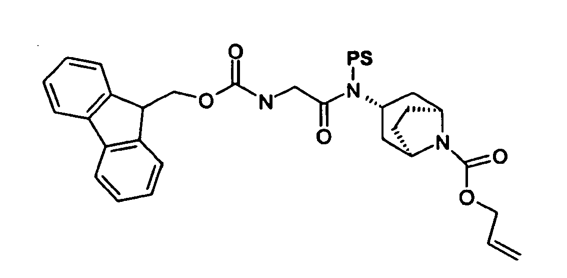

IRORI PS-allyl (3-endo)-3-amino-8-azabicyclo[3.2.1]octane-8-carboxylate (Intermediate 26) (2.8 g, loading: 1.3 mmol/g, 3.64 mmols) was washed and swelled with tetrahydrofuran. It was filtered and N-[(9H-fluoren-9-ylmethoxy)carbonyl]glycine (FMOC-glycine) (8.3 g, 28 mmols) and tetrahydrofuran (30 mL) were added. A solution of N,N'-diisopropylcarbodiimide (4.28 g, 34 mmols) in tetrahydrofuran (56 mL) was added to the mixture. It was stirred for 4 hours at 60°C. The resin was filtered and washed with dimethylformamide (2X), dichloromethane (1X) and tetrahydrofuran (1X). Again, N-[(9H-fluoren-9-ylmethoxy)carbonyl]glycine (FMOC-glycine) (4.15 g, 14 mmols), tetrahydrofuran (30 mL) and a solution of N,N'-diisopropylcarbodiimide (2.14 g, 17 mmols) in tetrahydrofuran (56 mL) were added to the mixture. It was stirred for 18 hours at 60°C and for 48 hours at room temperature. It was filtered and washed with dimethylformamide (3X) and dichloromethane (3X). The resin was dried in vacuo.

### Intermediate 28 PS-allyl (3-endo)-3-(glycylamino)-8-azabicyclo[3.2.1]octane-8-carboxylate

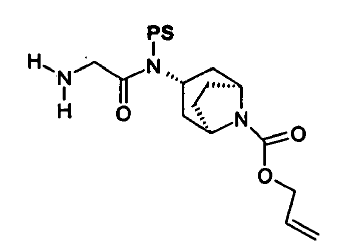

PS-allyl (3-endo)-3-({N-[(9H-fluoren-9-ylmethoxy)carbonyl]glycyl}amino)-8-azabicyclo[3.2.1]octane-8-carboxylate (Intermediate 27) (2.8 g, loading:1.3 mmols/g, 3.64 mmols) was washed and swelled with dimethylformamide. A solution of 20% piperidine in dimethylformamide (50 mL) was added and the mixture was stirred for 20 minutes at room temperature. It was filtered and washed with dimethylformamide. The resin was treated for a second time under the above conditions. It was filtered and washed with dimethylformamide (4X) and dichloromethane (4X). The resin was dried in vacuo to give the title compound (3.0 g).

### Intermediate 29 PS-N-[2-[(3-endo)-8-azabicyclo[3.2.1]oct-3-ylamino]-2-oxoethyl]-2-[[3-(trifluoromethyl)phenyl]amino]benzamide

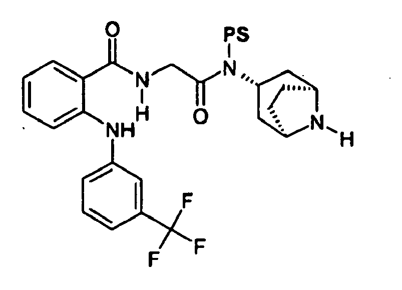

a) PS-allyl (3-endo)-3-(glycylamino)-8-azabicyclo[3.2.1]octane-8-carboxylate (Intermediate 28) (0.1 g, lading: mmol/g, 0.1 mmol) was washed and swelled with dichloromethane. A 0.5M solution of 2-{[3-(trifluoromethyl)phenyl]amino}benzoic acid in dichloromethane/dimethylformamide 9:1 (2mL) was added. A 1.2M solution of N,N'-diisopropylcarbodiimide in dichloromethane/dimethylformamide 9:1 (1 mL) was added. The mixture was stirred for 4 hours at 50°C. It was filtered and washed with dichloromethane. The resin was treated again with a 0.5M solution of 2-{[3-(trifluoromethyl)phenyl]amino}benzoic acid in dichloromethane/dimethylformamide 9:1 (2mL) and a 1.2M solution of N,N'-diisopropylcarbodiimide in dichloromethane/dimethylformamide 9:1 (1mL). The mixture was stirred at room temperature overnight. It was filtered and washed with dimethylformamide (4X) and dichloromethane (4X). The resin was dried in vacuo to give the title compound.
b) The compound of step a) (0.1 g, loading:1 mmol/g, 0.1 mmols) was washed and swelled with dichloromethane. A 2M solution of dimethylamino borane in dichloromethane (2 mL) and a 0.01 M solution of tetrakis (triphenylphosphine) palladium in dichloromethane (1 mL) were added to the resin. The mixture was stirred for 20 minutes at room temperature. It was filtered and washed with dichloromethane. The resin was treated for a second time under the above conditions. It was filtered and washed with dichloromethane (3X), a 0.2% solution of trifluoroacetic acid in dichloromethane (2X), dichloromethane (3X), a 5% solution of diisopropylethylamine in dichloromethane (2X), a solution of dioxane/water 9:1 (2X), methanol (3X), dimethylformamide (3X), dichloromethane (3X). The resin was dried in vacuo to give the title compound.

### Phenol Intermediates:

### Intermediate 30 N-(5-chloro-2-hydroxyphenyl)urea

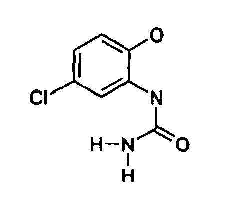

a) N-(5-chloro-2-methoxyphenyl)urea 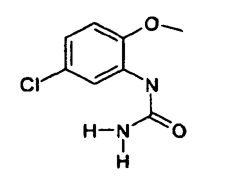 A solution of 4-chloro-2-isocyanato-1-methoxybenzene (18.0 g, 98 mmols) in tetrahydrofuran (60 mL) was added dropwise to a solution of saturated ammonia in the same solvent (240 mL) at room temperature. It was stirred under the same conditions for 3 hours. The pH of the solution obtained was acidic; therefore a stream of ammonia was passed through until a basic pH was reached. The crude was concentrated in vacuo and the solid obtained was triturated with diethylether. It was filtered, washed and dried and it was recristallized from tetrahydrofuran to give the title compound (16.7 g).
b) N-(5-chloro-2-methoxyphenyl)urea (22.1 g, 110 mmols) was suspended in dichloromethane (442 mL). The suspension obtained was cooled down to -10°C and a solution of boron tribromide (331.5 mL of a 1 M solution in dichloromethane) was added dropwise under nitrogen atmosphere. It was stirred under this conditions for 1 hour and then at room temperature overnight. The reaction crude was poured into water/ice. The organic phase was separated and the solid precipitated was dissolved in diethylether. This solution was added to the aqueous phase and it was extracted two more times with more diethyl ether. The organic phase was washed with water, a 4% aqueous solution of sodium hydrogen carbonate and brine. It was dried, filtered and concentrated in vacuo. The solid obtained was recristallized from diisopropyl ether to give N-(5-chloro-2-hydroxyphenyl)urea (15.8 g).

### Intermediate 31 5-chloro-2-hydroxy-N-methylbenzamide

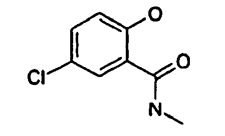

a) 5-chloro-2-methoxybenzoyl chloride 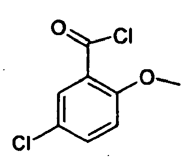 5-chloro-2-methoxybenzoic acid (26.3 g, 141 mmols) was dissolved in dry benzene (500 mL) and a solution of thionyl chloride (11.3 mL) in the same solvent (70 mL) was added dropwise at room temperature. The mixture was stirred at 100°C for 2 hours. It was concentrated in vacuo and toluene was added. It was concentrated in vacuo and the title compound was obtained (28.8 g).
b) 5-chloro-2-methoxy-N-methylbenzamide 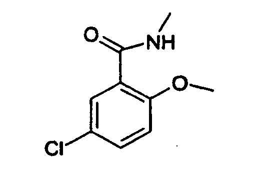 5-chloro-2-methoxybenzoyl chloride (14.1g, 69 mmols) was dissolved in dry tetrahydrofuran (80 mL). A 2M solution of methylamine in tetrahydrofuran (100 mL) was added dropwise under nitrogen atmosphere at 20°C. It was stirred at room temperature overnight. It was concentrated in vacuo and water and ice were added. It was extracted with ethyl acetate and the organic phase obtained was washed with water and brine. It was dried and concentrated in vacuo to give the title compound (13.2 g).
c) 5-chloro-2-methoxy-N-methylbenzamide (13.2 g, 66.1 mmols) was dissolved in dry dichloromethane (260 mL). The solution obtained was cooled down to -10°C and a solution of boron tribromide (200 mL of a 1 M solution in dichloromethane) was added dropwise under nitrogen atmosphere. It was stirred under this conditions for 1 hour and then at room temperature overnight. The crude was poured into water/ice and it was stirred for 10 min. It was extracted with ethyl acetate. The dichloromethane phase was concentrated in vacuo. The aqueous phase was extracted again with ethyl acetate. The residue obtained from the concentration of the dichloromethane phase was dissolved in ethyl acetate. All the ethyl acetate phases were collected together and washed with a 4% aqueous solution of sodium hydrogen carbonate and water. It was dried, filtered and concentrated in vacuo. The solid obtained was recristallized from ethanol to give 5-chloro-2-hydroxy-N-methylbenzamide (9.5 g).

### Intermediate 32 N-(5-bromo-2-hydroxyphenyl)urea

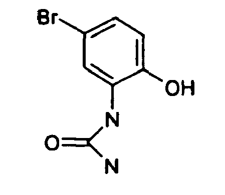

a) 4-bromo-2-isocyanato-1-methoxybenzene 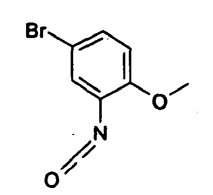 5-bromo-2-methoxyaniline (11.4 g, 56.4 mmols) was dissolved in dioxane (115 mL) and a solution of trichloromethyl chloroformate (3.4 mL) in the same solvent (30 mL) was added dropwise. It was stirred at 60°C for 18 hours. It was concentrated in vacuo and diethyl ether was added. The solid precipitated was filtered off and the mother liquors were concentrated in vacuo to give the title compound (13 g).
b) N-(5-bromo-2-methoxyphenyl)urea 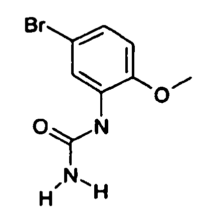 4-bromo-2-isocyanato-1-methoxybenzene (13.0 g, 57 mmols) was dissolved in tetrahydrofuran (60 mL) and it was added dropwise to a solution of saturated ammonia in the same solvent (140 mL) at room temperature. It was stirred under the same conditions for 48 hours. The solid obtained was filtered off and the filtrate was concentrated in vacuo to give the title compound (11.06 g).
c) N-(5-bromo-2-methoxyphenyl)urea (11.0 g, 45 mmols) was suspended in dry dichloromethane (275 mL). The suspension obtained was cooled down to -10°C and a solution of boron tribromide (135 mL of a 1M solution in dichloromethane) was added dropwise under nitrogen atmosphere. It was stirred under this conditions for 1 hour and then at room temperature for 16 hours. The crude was poured into water/ice and it was stirred for 15 minutes. It was concentrated in vacuo to eliminate the dichloromethane. The aqueous phase obtained was extrated with ethyl acetate. The organic phase was washed with a 4% aqueous solution of sodium hydrogen carbonate, water and brine. It was dried, filtered and concentrated in vacuo. The solid obtained was recristallized from diethyl ether to give N-(5-bromo-2-hydroxyphenyl)urea (8.33 g).

### Intermediate 33 2-(5-chloro-2-hydroxyphenyl)-N-methylacetamide

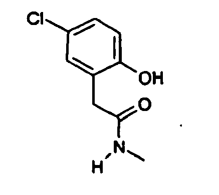

a) 2-(5-chloro-2-methoxy-phenyl)-1-morpholin-4-yl-ethanethione 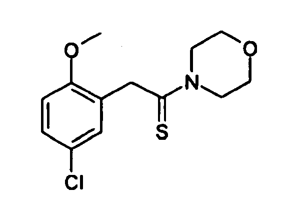 To a mixture of 5-chloro-2-methoxyacetophenone (44.0 g, 238 mmols) and sulfur (11.4 g, 355 mmols), morpholine (31.15 mL) was added using a water bath to keep the reaction mixture at room temperature. It was stirred at reflux for 18 hours. It was cooled down to 50°C and ethanol was added carefully. It was stirred for 1 hour and the solid obtained was filtered, washed with ethanol and hexane to give the title compound (62.0 g).
b) (5-chloro-2-methoxyphenyl)acetic acid 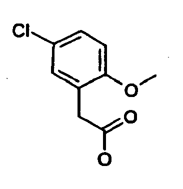 Potassium hydroxide (169.4 g, 3019 mmols) was dissolved in water (200 mL) and ethanol was added (1090 mL). 2-(5-chloro-2-methoxy-phenyl)-1-morpholin-4-yl-ethanethione (62.0 g, 217 mmols) was added to this solution and it was stirred at reflux for 18 hours. The reaction mixture was concentrated in vacuo and water/ice were added. It was washed with diethylether and the aqueous phase was acidified with hydrochloric acid. It was stirred for 15 minutes and it was extracted with diethyl ether. The organic phase was washed with water and brine, dried, filtered and concentrated in vacuo. The solid obtained was triturated with a mixture of diethyl ether (30 mL) and hexane (40 mL). It was filtered and dried to give the title compound (35.6 g).
c)(5-chloro-2-methoxyphenyl)acetyl chloride 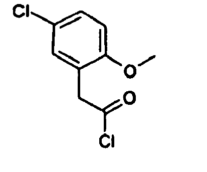 (5-chloro-2-methoxyphenyl)acetic acid (23.6 g, 118 mmols) and thionyl chloride (70.8 mL) were mixed and it was heated at 90°C for 2 hours. It was concentrated in vacuo. Toluene was added and it was concentrated in vacuo. Diethyl ether was added and the solid obtained was filtered off through celite. The filtrate was concentrated in vacuo to give the title compound (25.0 g).
d) 2-(5-chloro-2-methoxyphenyl)-N-methylacetamide 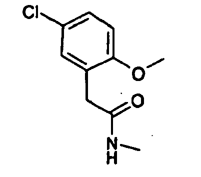 A 2M methylamine solution in tetrahydrofuran (100 mL) was cooled down to 0-5°C. A solution of (5-chloro-2-methoxyphenyl)acetyl chloride (25 g, 114 mmols) in tetrahydrofuran (100 mL) was added dropwise. It was stirred at room temperature overnight. 150 mL more of solvent were added and it was stirred for 1 hour under the same conditions. The reaction mixture was concentrated in vacuo, water/ice was added and it was extracted with ethyl acetate. The organic phase was washed with water and brine, dried and concentrated in vacuo. The solid obtained was recristallized from diethyl ether to give the title compound (16.6 g).
e) 2-(5-chloro-2-methoxyphenyl)-N-methylacetamide (16.6 g, 78 mmols) was suspended in dry dichloromethane (260 mL). The suspension obtained was cooled down to -10°C and a solution of boron tribromide (165 mL of a 1 M solution in dichloromethane) was added dropwise under nitrogen atmosphere. It was stirred under this conditions for 1 hour and then at room temperature overnight. The crude was poured into water/ice and it was stirred for 5 minutes. Ethyl acetate was added to dissolve the solid obtained. The phases were separated. The dichloromethane phase was concentrated in vacuo and the residue obtained was dissolved in ethyl acetate. The aqueous phase was extracted with ethyl acetate. All the ethyl acetate phases were collected together and washed with a 4% aqueous solution of sodium hydrogen carbonate, water and brine. It was dried, filtered and concentrated in vacuo. The solid obtained was recristallized from diisopropyl ether/methanol (70 mL/15 mL). Chromatography on silica gel gave 2-(5-chloro-2-hydroxyphenyl)-N-methylacetamide (10.4 g).

### Intermediate 34 2-(5-chloro-2-hydroxyphenyl)acetamide

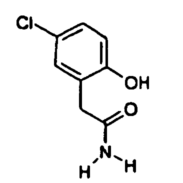

a) 2-(5-chloro-2-methoxyphenyl)acetamide 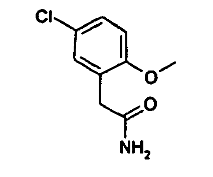 A solution of (5-chloro-2-methoxyphenyl)acetyl chloride (13.2 g, 60.3 mmols) in tetrahydrofuran (50 mL) was added dropwise at room temperature to a saturated ammonia solution in the same solvent (240 mL) and it was stirred overnight under the same conditions. It was concentrated in vacuo and water/ice were added. It was extracted with dichloromethane, the organic phase was washed with water and brine, dried, filtered and concentrated in vacuo. The solid obtained was recristallized from diethyl ether to give the title compound (6.1 g).
b) 2-(5-chloro-2-methoxyphenyl)acetamide (8.9 g, 45 mmols) was suspended in dry dichloromethane (180 mL). The suspension obtained was cooled down to -10°C and a solution of boron tribromide (135 mL of a 1M solution in dichloromethane) was added dropwise under nitrogen atmosphere. It was stirred under this conditions for 1 hour and then at room temperature overnight. The crude was poured into water/ice and it was stirred for 10 minutes. Ethyl acetate was added to dissolve the solid obtained. The phases were separated. The dichloromethane phase was concentrated in vacuo and the residue obtained was dissolved in ethyl acetate. The aqueous phase was extracted with ethyl acetate. All the ethyl acetate phases were collected together and washed with a 4% aqueous solution of sodium hydrogen carbonate, water and brine. It was dried, filtered and concentrated in vacuo. The residue obtained was recristallized from diethyl ether and the solid obtained was further purified by recristallization from methanol to give 2-(5-chloro-2-hydroxyphenyl)acetamide (4.7 g).

### Intermediate 35

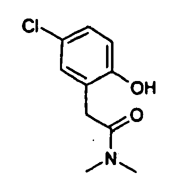

2-(5-chloro-2-hydroxyphenyl)-N,N-dimethylacetamide
a) 2-(5-chloro-2-methoxyphenyl)-N,N-dimethylacetamide 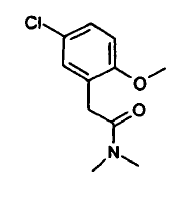 A solution of (5-chloro-2-methoxyphenyl)acetic acid (2.0 g, 10 mmols) in dichloromethane (40 mL) was cooled down to °C. N,N'-Carbonyldiimidazole (1.8 g, 11 mmols) was added and it was stirred under the same conditions for 30 minutes. A solution of dimethylamine hydrochloride (0.9 g, 11 mmols) and triethylamine (3.1 mL) in dichloromethane (20 mL) was added dropwise at 0-5°C. It was stirred under the same conditions for 30 minutes and at room temperature overnight. The crude was diluted with ethylacetate, it was washed with a 4% aqueous sodium hydrogen carbonate solution and brine. It was ddried and concentrated in vacuo to give the title compound (2.34 g).
b) 2-(5-chloro-2-methoxyphenyl)-N,N-dimethylacetamide (2.43 g, 11 mmols) was suspended in dry dichloromethane (50 mL). The suspension obtained was cooled down to -10°C and a solution of boron tribromide (32 mL of a 1M solution in dichloromethane) was added dropwise under nitrogen atmosphere. It was stirred under this conditions for 1 hour and then at room temperature overnight. Water/ice was added and it was stirred. The two phases were separated and the organic phase was washed with a 4% aqueous solution of sodium hydrogen carbonate and brine. It was dried, filtered and concentrated in vacuo. The residue obtained was recristallized from diethyl ether to give 2-(5-chloro-2-hydroxyphenyl)-N,N-dimethylacetamide (1.0 g).

### Intermediate 36 N-(5-fluoro-2-hydroxyphenyl)urea

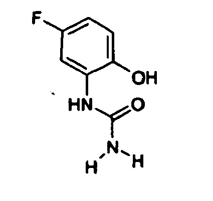

a) 4-fluoro-2-isocyanato-1-methoxybenzene 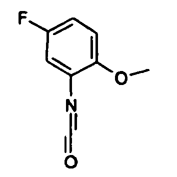 5-fluoro-2-methoxyaniline (5.0 g, 35 mmols) was dissolved in dioxane (70 mL) and a solution of trichloromethyl chloroformate (2.11 mL) in the same solvent (20 mL) was added dropwise. It was stirred at 60°C for 20 hours. It was concentrated in vacuo and diethyl ether was added. The solid precipitated was filtered off and the mother liquors were concentrated in vacuo to give the title compound (4.66 g).
b) N-(5-fluoro-2-methoxyphenyl)urea 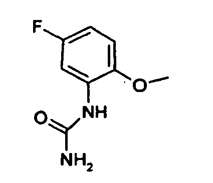 4-fluoro-2-isocyanato-1-methoxybenzene (4.6 g, 28 mmols) was dissolved in tetrahydrofuran (20 mL) and it was added dropwise to a solution of saturated ammonia in the same solvent (50 mL) at room temperature. It was stirred under the same conditions for 6 hours. The solid obtained was filtered off and the filtrate was concentrated in vacuo. The residue was recristallized from diethyl ether to give the title compound (3.5 g).
c) N-(5-fluoro-2-methoxyphenyl)urea (6.14 g, 33 mmols) was dissolved in dry dichloromethane (200 mL). The solution obtained was cooled down to -10°C and a solution of boron tribromide (100 mL of a 1M solution in dichloromethane) was added dropwise under nitrogen atmosphere. It was stirred under this conditions for 1 hour and then at room temperature overnight. Water/ice were added and it was stirred for 10 minutes. It was concentrated in vacuo to eliminate the dichloromethane. The aqueous phase obtained was extrated with ethyl acetate. The organic phase was washed with a 4% aqueous solution of sodium hydrogen carbonate, water and brine. It was dried, filtered and concentrated in vacuo. The solid obtained was recristallized from diethyl ether to give N-(5-fluoro-2-hydroxyphenyl)urea (4.87 g)

### Intermediate 37 N-(5-chloro-2-hydroxyphenyl)acetamide

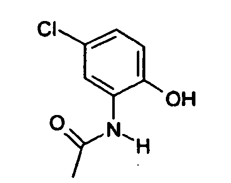

2-amino-4-chlorophenol (20 g, 139 mmols) and triethylamine (21.13 mL) were disolved in tetrahydrofuran (280 mL). It was cooled down to 0 °C and a solution of acetylchloride (10.98 mL) in tetrahydrofuran (21 mL) was added dropwise. It was stirred under the same conditions for 3 hours and then at room temperature for 2 hours. The reaction mixture was concentrated in vacuo and water/ice was added to the residue obtained. It was extracted with ethyl acetate and the organic phase was washed with a 4% aqueous solution of sodium hydrogen carbonate, water and brine. It was dried, filtered and concentrated in vacuo. Ethyl acetate was added to the solid obtained and filtered off. The filtrate was washed with a 2N aqueous solution of hydrochloric acid and then extracted with a 1 N aqueous solution of sodium hydroxide. The basic aqueous phase was acidified with a 2N aqueous solution of hydrochloric acid and then it was extracted with ethyl acetate. The organic phase was washed with water and brine, dried, filtered and concentrated in vacuo. A solid precipitated when concentrating in vacuo and it was filtered off. This solid was treated with diethyl ether to give the title compound (9.0 g).

### Intermediate 38 N-{[1-(5-chloro-2-hydroxybenzoyl)piperidin-3-yl]methyl}-2,2,2-trifluoroacetamide

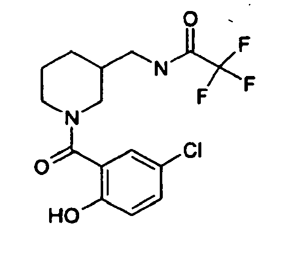

a) 2,2,2-trifluoro-N-(piperidin-3-ylmethyl)acetamide 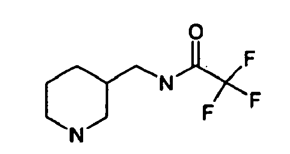 Ethyl trifluoroacetate (29 mL, 243 mmols) was added to a solution of 3-(aminomethyl)piperidine (8.11g, 71 mmols) in acetonitrile (150 mL) and water (1.5mL). The mixture was stirred at reflux for 13 hours and then at room temperature for 48 hours. It was concentrated in vacuo and the residue obtained was dissolved in ethyl acetate. It was washed with a 5% aqueous ammonia solution, water and brine. The organic layer was dried over sodium sulfate, filtered and concentrated in vacuo. Chromatography on silica gel gave the title compound (3.2 g).
b)N-{[1-(5-chloro-2-methoxybenzoyl)piperidin-3-yl]methyl}-2,2,2-trifluoroacetamide 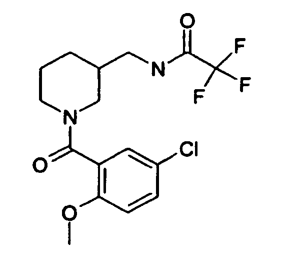 5-chloro-2-methoxybenzoyl chloride (1.0 g, 5 mmols) was dissolved in tetrahydrofuran (10 mL) and it was cooled down to 0-5°C. A solution of 2,2,2-trifluoro-N-(piperidin-3-ylmethyl)acetamide (3.07 g, 15 mmols) in tetrahydrofuran (30 mL) was added dropwise. The mixture obtained was stirred at room temperature overnight. The mixture was concentrated in vacuo and the residue was dissolved in ethyl acetate. It was washed with water and brine. The organic layer was dried over sodium sulfate, filtered and concentrated in vacuo. Chromatography on silica gel gave a product which was triturated with hexane:ethyl acetate 3:1. The solid obtained was filtered and dried in vacuo to give the title compound (1.00 g).
b.1) 5-chloro-2-methoxybenzoyl chloride 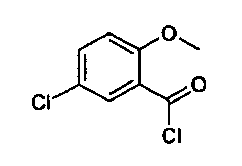 5-Chloro-2-methoxybenzoic acid (5.0 g, 27 mmols) was dissolved in toluene (95 mL) and a solution of thionyl chloride (2.15 mL, 30 mmols) in toluene (13 mL) was added dropwise at room temperature. The mixture was stirred at 100°C for 3 hours. It was concentrated in vacuo to give the title compound (5.5 g).
c)N-{[1-(5-chloro-2-methoxybenzoyl)piperidin-3-yl]methyl}-2,2,2-trifluoroacetamide (1.0 g, 2.6 mmols) was dissolved in dichloromethane (20 mL) and the solution was cooled down to -10°C. A solution of Boron tribromide in dichloromethane (8mL, 1M) was added dropwise. The reaction mixture was stirred at -10°C for 1 hour and then at room temperature overnight. The reaction crude was poured into water/ice and the two layers obtained were separated. The organic layer was washed with a 4% aqueous solution of sodium bicarbonate and brine. It was dried over sodium sulfate, filtered and concentrated in vacuo. The residue obtained was recristalized from diethyl ether to give N-{[1-(5-chloro-2-hydroxybenzoyl)piperidin-3-yl]methyl}-2,2,2-trifluoroacetamide (0.67g).

### Intermediate 39 4-methyl-2-(methylamino)phenol

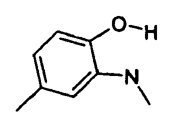

a) 3,5-dimethyl-1,3-benzoxazol-2(3H)-one 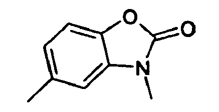 To a solution of 5-methyl-1,3-benzoxazol-2(3H)-one (14.0 g, 94 mmols) in acetone (200 mL), potassium carbonate (14.0 g, 100 mmols) was slowly added and the mixture obtained was stirred for 30 minutes at room temperature. A solution of dimethyl sulfate (9.5 mL) in acetone (15 mL) was added dropwise and the mixture was stirred at reflux for 1 hours. It was concentrated in vacuo, water was added and it was extracted with dichloromethane. The organic phase was dried, filtered and concentrated in vacuo. The solid obtained was treated with diethyl ether to give the title compound (13.8 g).
b) A 20% aqueous solution of sodium hydroxide (40 mL) was added to 3,5-dimethyl-1,3-benzoxazol-2(3H)-one (12.0 g, 74 mmols) and it was stirred at reflux for 1 hours. The mixture was acidified with concentrated hydrochloric acid. Then it was basified with solid sodium carbonate and extrated with dichloromethane. The organic phase was washed with water, dried and concentrated in vacuo. The solid obtained was treated with diethyl ether and petroleum ether to give 4-methyl-2-(methylamino)phenol (6.7 g).

### Intermediate 40 N-(2-hydroxy-5-methylphenyl)acrylamide

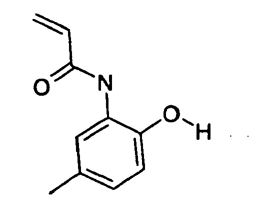

To a solution of 2-amino-4-methylphenol (20.0 g, 160 mmols) in dichloromethane (280 mL), pyridine was added (19.8 mL). The mixture was cooled down to 0°C and a solution of acryloyl chloride (16 mL, 200 mmols) in dichloromethane (120 mL) was added dropwise. It was stirred under the same conditions for 3 hours. Water was added and it was stirred at room temperature for 30 minutes. It was extrated with dichloromethane, washed with a saturated aqueous solution of sodium hydrogen carbonate and brine. It was dried, filtered and concentrated in vacuo. Chromatography on silica gel gave the title compound (10.22 g).

### Intermediate 41 methyl 4-(acetylamino)-5-chloro-2-hydroxybenzoate

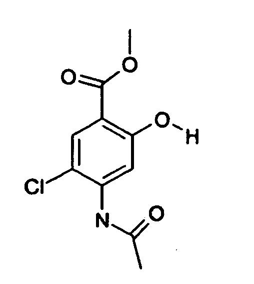

Methyl 4-acetamido-5-chloro-2-methoxybenzoate (463.8 g, 1800 mmols) in 1,2-dichloroethane (2.8 L) was heated until a solution was obtained. Anhydrous aluminium chloride (362.5 g, 2700 mmols) was carefully added in small portions under the same conditions. The mixture was stirred at reflux for 6 hours. More aluminium chloride (36.3 g) was added and it was stirred under the same conditions for 2 hours. The reaction mixture was cooled down and ice was added. A 2N aqueous hydrochloric acid solution and dichloromethane were added. It was extrated and the organic phase was washed with a saturated aqueous solution of sodium hydrogen carbonate and water. It was dried, filtered and concentrated in vacuo. The solid obtained was treated with diethyl ether to give the title compound (380 g).

### Acid Intermediates:

### Intermediate 42 2,7-dichloro-9H-xanthene-9-carboxylic acid

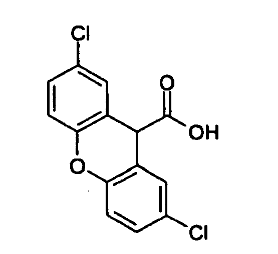

Acetic acid (80 mL) and N-chlorosuccinimide (14.7 g, 110 mmols) were added to xanthene-9-carboxylic acid (10.0 g, 44 mmols). Hydrochloric acid (2 mL) was added dropwise to the mixture at room temperature and it was stirred under these conditions for 48 hours. Water was added (150 mL) and the precipitate was filtered. The product obtained was used without further purification in the synthesis of example 54.

### Intermediate 43 10,11-dihydro-5H-dibenzo[a,d][7]annulene-5-carboxylic acid

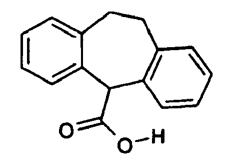

It may be obtained following the procedure described in Gualtieri, F. et al. Tetrahedron (1998), 54(10), 2251-2256 or in WO 2000061556.

### Intermediate 44 9-methyl-9H-xanthene-9-carboxylic acid

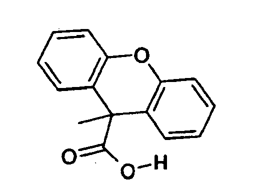

It may be obtained following the procedure described in WO 2003064417.

### Intermediate 45 5-chloro-2-methoxy-4-(methylamino)benzoic acid

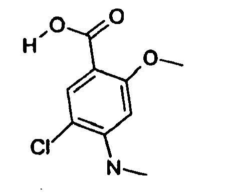

It may be obtained following the procedure described in WO 2004048326.

### Intermediate 46 3-(cyanomethyl)benzoic acid

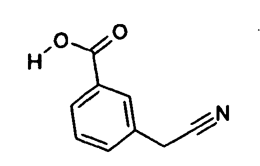

It may be obtained following the procedure described in WO 2004096781.

### Intermediate 47 5-chloro-4-(dimethylamino)-2-methoxybenzoic acid

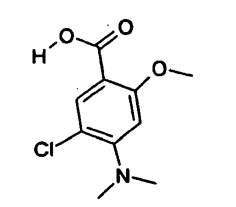

It may be obtained following the procedure described in Hirokawa, Y et al. Chemical & Pharmaceutical Bulletin (2002), 50(7), 941-959.

The intermediates 48 and 49 were made following method 11.

### Intermediate 48 3-{4-chloro-2-[(methylamino)carbonyl]phenoxy}propanoic acid

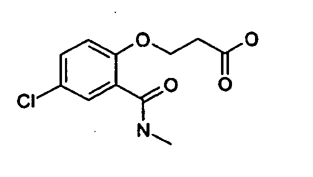

Potassium tert-butoxide (3.0 g, 27 mmols) was added to a solution of 5-chloro-2-hydroxy-N-methylbenzamide (5.0 g, 27 mmols) in tetrahydrofuran (30 mL) and the mixture was stirred at room temperature for 15 min. A solution of beta-propiolactone (1.7 mL, 27 mmols) in tetrahydrofuran (5 mL) was added dropwise to the suspension obtained. More solvent was added (100 mL) and the mixture was stirred at 50°C for 48 hours. The reaction mixture was concentrated in vacuo and water and potassium carbonate were added to the residue obtained. The aqueous solution obtained was washed with ethyl acetate. It was acidified with an aqueous solution of hydrochloric acid (2N) to reach pH=1.Ethyl acetate (600 mL) was added to dissolved the solid precipitated during the acid treatment and then the two phases were separated. The organic phase was washed with brine, dried over sodium sulfate, filtered and concentrated in vacuo. The solid obtained was triturated with diethyl ether, filtered and dried in vacuo to give the title compound (2.21 g).

### Intermediate 49 3-{2-[(aminocarbonyl)amino]-4-chlorophenoxy}propanoic acid

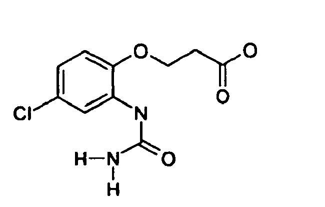

Potassium tert-butoxide (2.4 g, 21 mmols) was added to a suspension of N-(5-chloro-2-hydroxyphenyl)urea (4.0 g, 21 mmols) in tetrahydrofuran (100 mL) and the mixture was stirred at room temperature for 15 min. A solution of beta-propiolactone (1.4 mL, 22 mmols) in tetrahydrofuran (5 mL) was added dropwise to the suspension obtained. The mixture was stirred at 50°C for 36 hours. The reaction mixture was concentrated in vacuo and water and potassium carbonate were added to the residue obtained. The aqueous solution obtained was washed with ethyl acetate. It was acidified with an aqueous solution of hydrochloric acid (2N) to reach pH=1. The solid precipitated was filtered and washed with water. It was suspended in isopropyl alcohol and heated up to 60°C, cooled down to room temperature, filtered, washed and dried in vacuo to give the title compound (1.23 g). (yield.: approx.: 22%).

Intermediate 50 was made following method 12.

### Intermediate 50 4-{4-chloro-2-[(methylamino)carbonyl]phenoxy}butanoic acid

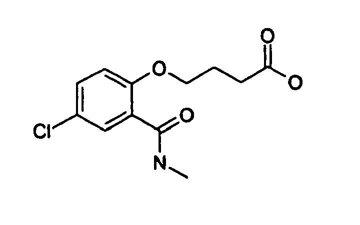

Potassium tert-butoxide (0.25 g, 2.2 mmols) was added to a suspension of 5-chloro-2-hydroxy-N-methylbenzamide (0.3 g, 1.6 mmols) in dimethylformamide (3 mL) and the mixture was stirred at room temperature until a precipitate was observed. The mixture was cooled down to 0-5°C and a solution of ethyl 4-bromobutyrate (0.32 mL, 2.2 mmols) in dimethylformamide (1 mL) was added dropwise. The reaction mixture was stirred at room temperature for 15 minutes and then it was heated to 110°C for 2.5 hours. The reaction mixture was poured into a mixture of water/ice/ethyl acetate and the two phases obtained were separated. The organic phase was washed with an aqueous solution of sodium hydroxide (2N) and brine. It was dried over sodium sulfate, filtered and concentrated in vacuo. The solid obtained was dissolved in 5 mL of methanol and an aqueous solution of sodium hydroxide (2N) (5 mL) was added. The mixture was stirred at 0-5°C for 1 hours. The reaction mixture was concentrated in vacuo and the aqueous phase obtained was diluted with more water, acidified with an aqueous solution of hydrochloric acid (2N) and extracted with dichloromethane. The organic phase was washed with brine, dried over sodium sulfate, filtered and concentrated in vacuo. The crude was triturated with diethyl ether and the solid obtained was filtered, washed and dried to give the title compound (0.2 g). (yield: 46%).

### Intermediate 51 was made following method 13.

### Intermediate 51 5-[[(3-chlorophenyl)amino](hydroxy)methylene]-2,2-dimethyl-1,3-dioxane-4,6-dione

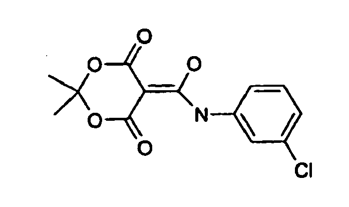

Triethylamine (0.4 mL, 2.9 mmols) was added to a solution of 2,2-dimethyl-1,3-dioxane-4,6-dione (Meldrum's acid) (0.21 g, 1.4 mmols) in dimethylformamide (1.4 mL). 1-chloro-3-isocyanatobenzene (0.22 g, 1.4 mmols) was added and the mixture obtained was stirred at room temperature for 4.5 hours. The reaction mixture was poured into a cold aqueous solution of hydrochloric acid (2N) (15 mL). The solid obtained was filtered, washed with water and dried in vacuo at 50°C overnight. The title compound was obtained (0.31 g) (yield: 75%).

### Examples

The compounds of examples 1 to 4 have been obtained using synthetic method 1.

### Example 1 5-chloro-2-(3-((3-endo)-8-(4-chlorobenzyl)-8-azabicyclo[3.2.1]octan-3-ylamino)-3-oxopropoxy)-N-methylbenzamide

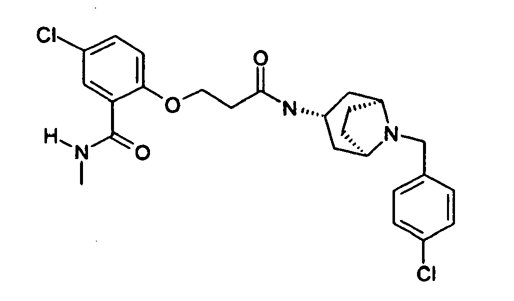

1-(3-dimethylaminopropyl)-3-ethylcarbodiimide hydrochloride (EDC.HCl) (0.71 g, 3.8 mmols) and 1-hydroxybenzotriazole hydrate (HOSt) (0.52 g, 3.8 mmols) were added to a solution of 3-{4-chloro-2-[(methylamino)carbonyl]phenoxy}propanoic acid (Intermediate 48) (1.0 g, 3.8 mmols) in dichloromethane (143 mL). The mixture was stirred at room temperature for 5 min.

(3-endo)-8-(4-chlorobenzyl)-8-azabicyclo[3.2.1]octan-3-amine (Intermediate 11) (0.8 g, 3.2 mmols) was added to the solution obtained and the mixture was stirred under the same conditions for 48 hours. The reaction mixture was washed with water, a saturated solution of potassium carbonate and brine. The organic layer was dried over sodium sulfate, filtered and concentrated in vacuo. The oil crude was recristalized from acetone and the solid obtained was filtered, washed and dried in vacuo to give the title compound (0.66 g) (yield: 42%).

### Example 2 3-(4-chloro-2-ureidophenoxy)-N-((3-endo)-8-(4-chlorobenzyl)-8-azabicyclo[3.2.1]octan-3-yl)propanamide

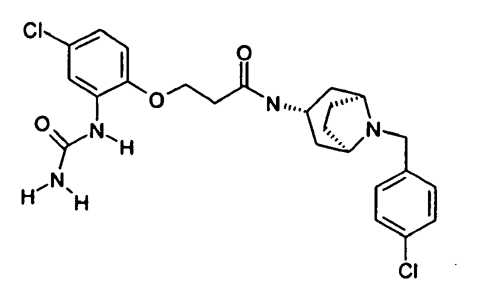

1-(3-dimethylaminopropyl)-3-ethylcarbodiimide hydrochloride (EDC.HCl) (0.91 g, 4.8 mmols) and 1-hydroxybenzotriazole hydrate (HOBt) (0.64 g, 4.8 mmols) were added to a solution of 3-{2-[(aminocarbonyl)amino]-4-chlorophenoxy}propanoic acid (Intermediate 49) (1.23 g, 4.8 mmols) in dichloromethane (175 mL).

(3-endo)-8-(4-chlorobenzyl)-8-azabicyclo[3.2.1]octan-3-amine (Intermediate 11) (1.0 g, 4.0 mmols) was added to the solution obtained and the mixture was stirred at room temperature for 4.5 hours. The reaction mixture was washed with a saturated solution of potassium carbonate, brine and water. The organic layer was dried over sodium sulfate, filtered and concentrated in vacuo. The oil crude was recristalized from isopropyl alcohol and the solid obtained was filtered, washed and dried in vacuo to give the title compound (1.13 g) (yield: 57%).

### Example 3 5-chloro-2-(4-((3-endo)-8-(4-chlorobenzyl)-8-azabicyclo[3.2.1]octan-3-ylamino)-4-oxobutoxy)-N-methylbenzamide

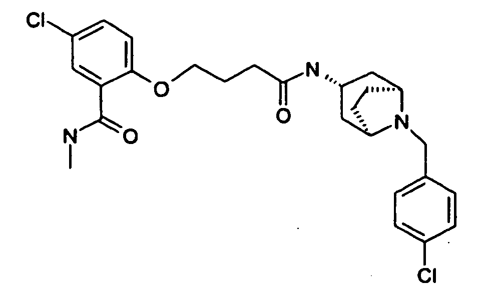

1-(3-dimethylaminopropyl)-3-ethylcarbodiimide hydrochloride (EDC.HCl) (0.15 g, 0.8 mmols) and 1-hydroxybenzotriazole hydrate (HOBt) (0.1 g, 0.8 mmols) were added to a solution of 4-{4-chloro-2-[(methylamino)carbonyl]phenoxy}butanoic acid (Intermediate 50) (0.2 g, 0.8 mmols) in dichloromethane (28 mL).

(3-endo)-8-(4-chlorobenzyl)-8-azabicyclo[3.2.1]octan-3-amine (Intermediate 11) (0.16 g, 0.7 mmols) was added to the solution obtained and the mixture was stirred at room temperature for 48 hours. The reaction mixture was washed with a saturated solution of potassium carbonate, brine and water. The organic layer was dried over sodium sulfate, filtered and concentrated in vacuo. The oil crude was recristalized from isopropyl alcohol and the solid obtained was filtered, washed and dried in vacuo to give the title compound (0.09 g) (yield: 28%).

### Example 4 N¹-((3-endo)-8-(4-chlorobenzyl)-8-azabicyclo[3.2.1]octan-3-yl)-N3-(3-chlorophenyl)malonamide

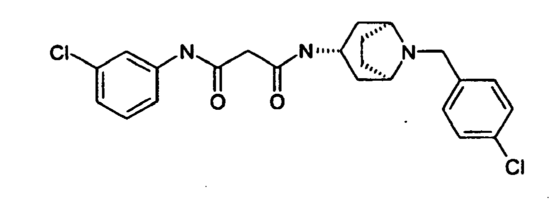

5-[[(3-chlorophenyl)amino](hydroxy)methylene]-2,2-dimethyl-1,3-dioxane-4,6-dione (Intermediate 51) (0.11 g, 0.36 mmols) and (3-endo)-8-(4-chlorobenzyl)-8-azabicyclo[3.2.1]octan-3-amine (Intermediate 11) (0.10 g, 0.4 mmols) were dissolved in toluene (3 mL). The mixture was stirred at reflux for 24 hours. It was concentrated in vacuo. Chromatography on silica gel gave the title compound (0.09 g) (yield: 57%).

The compounds of examples 5 to 32 have been obtained using synthetic method 2.

### Example 5 2-(4-chloro-2-ureidophenoxyl-N-((3-endol-8-(4-fluorobenzyl)-8-azabicyclo[3.2.1]octan-3-yl)acetamide

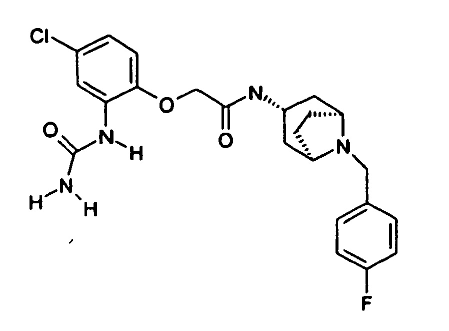

N-(5-chloro-2-hydroxyphenyl)urea (Intermediate 30) (0.15 g, 0.78 mmols) and potassium carbonate (0.09 g, 0.65 mmols) were added to a solution of 2-chloro-N-[(3-endo)-8-(4-fluorobenzyl)-8-azabicyclo[3.2.1]oct-3-yl]acetamide (Intermediate 14) (0.2 g, 0.65 mmols) in dimethylformamide (5 mL). The suspension obtained was stirred at 50°C for 5 hours and then at room temperature overnight. The reaction mixture was poured into water/ice and extracted with ethyl acetate. The organic layer was washed with a saturated aqueous solution of sodium carbonate, water and brine. It was dried over magnesium sulfate, filtered and concentrated in vacuo. Chromatography on silica gel gave the title compound (0.11 g) (yield: 38%).

### Example 6 5-chloro-2-(2-((3-endo)-8-(4-chlorobenzyl)-8-azabicyclo[3.2.1]octan-3-ylamino)-2-oxoethoxy)-N-methylbenzamide

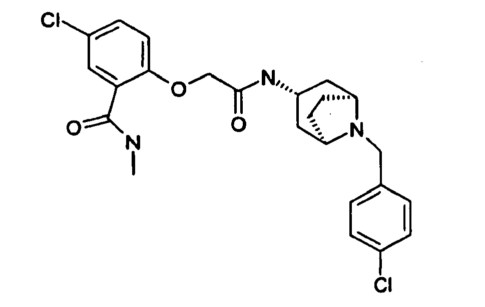

5-chloro-2-hydroxy-N-methylbenzamide (Intermediate 31) (0.135 g, 0.73 mmols) and potassium carbonate (0.08 g, 0.61 mmols) were added to a solution of 2-chloro-N-[(3-endo)-8-(4-chlorobenzyl)-8-azabicyclo[3.2.1]oct-3-yl]acetamide (Intermediate 15) (0.2 g, 0.61 mmols) in dimethylformamide (5 mL). The suspension obtained was stirred at 50°C for 6 hours and then at room temperature overnight. The reaction mixture was poured into water/ice and extracted with ethyl acetate. The organic layer was washed with a saturated aqueous solution of sodium carbonate, water and brine. It was dried over magnesium sulfate, filtered and concentrated in vacuo. The crude obtained was recristalized from isopropyl alcohol to give the title compound (0.046 g) (yield: 16%).

### Example 7 3-(4-bromo-2-ureidophenoxy)-N-((3-endo)-8-(4-chlorobenzyl)-8-azabicyclo[3.2.1]octan-3-yl)acetamide

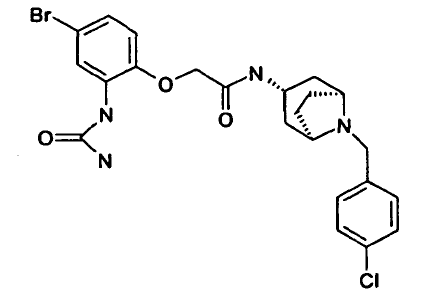

N-(5-bromo-2-hydroxyphenyl)urea (Intermediate 32) (0.085 g, 0.37 mmols) and potassium carbonate (0.043 g, 0.31 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.31 mmols) in dimethylformamide (4 mL). The suspension obtained was stirred at 50°C for 72 hours. The reaction mixture was poured into water/ice and extracted with ethyl acetate. The organic layer was washed with a saturated aqueous solution of potassium carbonate, water and brine. It was dried over magnesium sulfate, filtered and concentrated in vacuo. Chromatography on silica gel gave the title compound (0.027 g).

### Example 8 2-(5-chloro-2-(2-((3-endo)-8-(4-chlorobenzyl)-8-azabicyclo[3.2.1]octan-3-ylamino)-2-oxoethoxy)phenyl)-N-methylacetamide

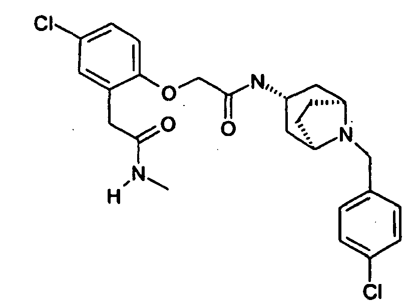

2-(5-chloro-2-hydroxyphenyl)-N-methylacetamide (Intermediate 33) (0.074 g, 0.37 mmols) and potassium carbonate (0.043 g, 0.31 mmols) were added to a solution of 2-chloro-N-[(3-endo)-8-(4-chlorobenzyl)-8-azabicyclo[3.2.1]oct-3-yl]acetamide (Intermediate, 15) (0.1 g, 0.31 mmols) in dimethylformamide (4 mL). The suspension obtained was stirred at 50°C for 72 hours. The reaction mixture was poured into water/ice and extracted with ethyl acetate. The organic layer was washed with a saturated aqueous solution of potassium carbonate, water and brine. It was dried over magnesium sulfate, filtered and concentrated in vacuo. The crude obtained was recristalized from diisopropyl ether to give the title compound (0.009 g).

### Example 9 2-(2-(2-amino-2-oxoethyl)-4-chlorophenoxyl-N-((3-endo)-8-(4-chlorobenzyl)-8-azabicyclo[3.2.1]octan-3-yl)acetamide

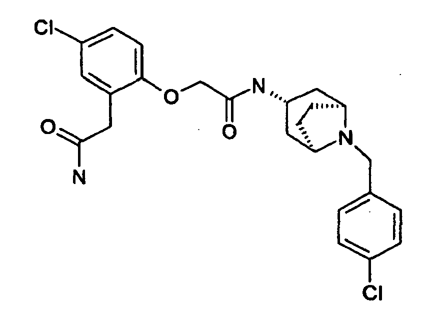

2-(5-chloro-2-hydroxyphenyl)acetamide (Intermediate 34) (0.068 g, 0.37 mmols) and potassium carbonate (0.043 g, 0.31 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.31 mmols) in dimethylformamide (4 mL). The suspension obtained was stirred at 50°C for 72 hours. The reaction mixture was poured into water/ice and extracted with ethyl acetate. The organic layer was washed with a saturated aqueous solution of potassium carbonate, water and brine. It was dried over magnesium sulfate, filtered and concentrated in vacuo. The crude obtained was recristalized from diisopropyl ether and the solid obtained was not the expected compound. The mother liquor was concentrated in vacuo and the residue obtained was recristalized from ethanol to give the title compound (0.001 g).

### Example 10 2-(5-chloro-2-(2-((3-endo)-8-(4-chlorobenzyl)-8-azabicyclo)-3.2.1]octan-3-ylamino)-2-oxoethoxy)phenyl)-N,N-dimethylacetamide

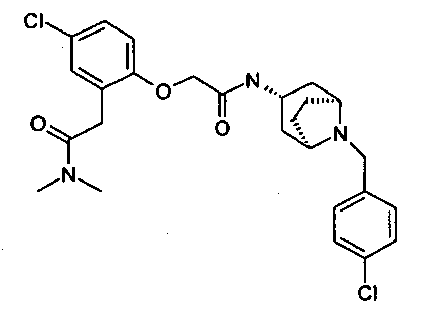

2-(5-chloro-2-hydroxyphenyl)-N,N-dimethylacetamide (Intermediate 35) (0.079 g, 0.37 mmols) and potassium carbonate (0.043 g, 0.31 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.31 mmols) in dimethylformamide (4 mL). The suspension obtained was stirred at 50°C for 72 hours. The reaction mixture was poured into water/ice and extracted with ethyl acetate. The organic layer was washed with a saturated aqueous solution of potassium carbonate, water and brine. It was dried over magnesium sulfate, filtered and concentrated in vacuo. Chromatography on silica gel gave the title compound (0.062 g).

### Example 11 N-((3-endo)-8-(4-chlorobenzyl)-8-azabicyclo[3.2.1]octan-3-yl)-2-(4-fluoro-2-ureidophenoxy)acetamide

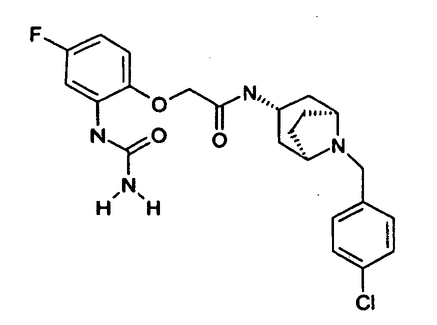

N-(5-fluoro-2-hydroxyphenyl)urea (Intermediate 36) (0.063 g, 0.37 mmols) and potassium carbonate (0.043g, 0.31 mmols) were added to a solution of 2-chloro-N-[(3-endo)-8-(4-chlorobenzyl)-8-azabicyclo[3.2.1]oct-3-yl]acetamide (Intermediate 15) (0.1g. 0.31 mmols) in dimethylformamide (4 mL). The suspension obtained was stirred at 50°C for 72 hours. The reaction mixture was poured into water/ice and extracted with ethyl acetate. The organic layer was washed with a saturated aqueous solution of potassium carbonate, water and brine. It was dried over magnesium sulfate, filtered and concentrated in vacuo. The crude obtained was recristalized from diisopropyl ether to give the title compound (0.029 g).

### Example 12 2-(4-chloro-2-ureidophenoxy)-N-((3-endo)-8-(4-chlorobenzyl)-8-azabicyclo[3.2.1]octan-3-yl)acetamide

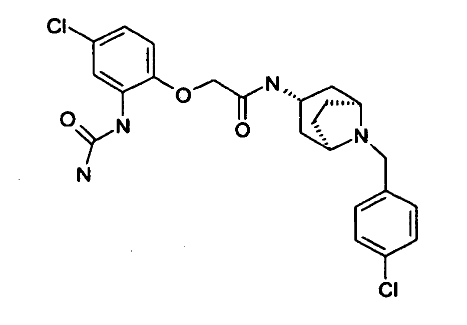

N-(5-chloro-2-hydroxyphenyl)urea (Intermediate 30) (1.05g, 5.6 mmols) and potassium carbonate (0.61 g, 4.6 mmols) were added to a solution of 2-chloro-N-[(3-endo)-8-(4-chlorobenzyl)-8-azabicyclo[3.2.1]oct-3-yl]acetamide (Intermediate 15) (1.5g, 4.6 mmols) in dimethylformamide (35 mL). The suspension obtained was stirred at 50°C for 6 hours and then overnight at room temperature. The reaction mixture was poured into water/ice and extracted with ethyl acetate. The organic layer was washed with a saturated aqueous solution of potassium carbonate, water and brine. It was dried over magnesium sulfate, filtered and concentrated in vacuo. Chromatography on silica gel gave the title compound (0.71 g) (yield: 33%).

### Example 13 5-chloro-2-(2-((3-endo)-8-(4-chlorobenzyl)-8-azabicyclo[3.2.1]octan-3-ylamino)-2-oxoethoxy)benzamide

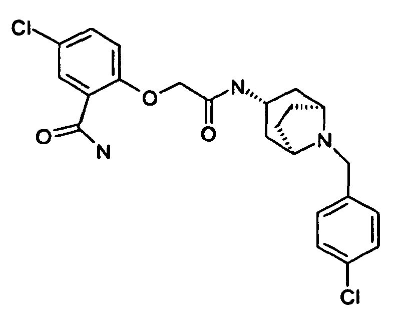

5-chloro-2-hydroxybenzamide (0.125 g, 0.73 mmols) and potassium carbonate (0.081 g, 0.61 mmols) were added to a solution of 2-chloro-N-[(3-endo)-8-(4-chlorobenzyl)-8-azabicyclo[3.2.1]oct-3-yl]acetamide (Intermediate 15) (0.2g, 0.61 mmols) in dimethylformamide (5 mL). The suspension obtained was stirred at 50°C for 5 hours and then overnight at room temperature. The reaction mixture was poured into water/ice and extracted with ethyl acetate. The organic layer was washed with a saturated aqueous solution of sodium carbonate, water and brine. It was dried over magnesium sulfate, filtered and concentrated in vacuo. The crude was recristalized from isopropyl alcohol to give the title compound (0.032 g).

### Example 14 2-(2-acetamido-4-chlorophenoxyl-N-((3-endol-8-(4-chlorobenzyl)-8-azabicyclo[3.2.1]octan-3-yl)acetamide

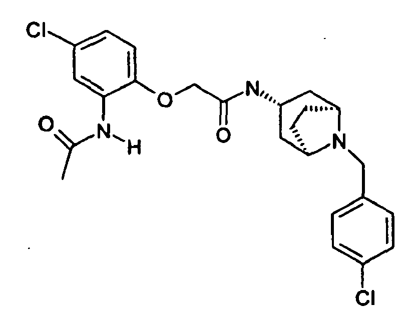

N-(5-chloro-2-hydroxyphenyl)acetamide (Intermediate 37) (0.14 g, 0.76 mmols) and potassium carbonate (0.081g, 0.61 mmols) were added to a solution of 2-chloro-N-[(3-endo)-8-(4-chlorobenzyl)-8-azabicyclo[3.2.1]oct-3-yl]acetamide (Intermediate 15) (0.2 g, 0.61 mmols) in dimethylformamide (5 mL). The suspension obtained was stirred at 50°C for 5 hours and then overnight at room temperature. The reaction mixture was poured into water/ice and extracted with ethyl acetate. The organic layer was washed with a saturated aqueous solution of sodium carbonate, water and brine. It was dried over magnesium sulfate, filtered and concentrated in vacuo. Chromatography on silica gel gave the title compound (0.050 g).

### Example 15 N-((1-(5-chloro-2-(2-((3-endo)-8-(4-chlorobenzyl)-8-azabicyclo[3.2.1]octan-3-ylamino)-2-oxoethoxy)benzoyl)piperidin-3-yl)methyl)-2.2.2-trifluoroacetamide

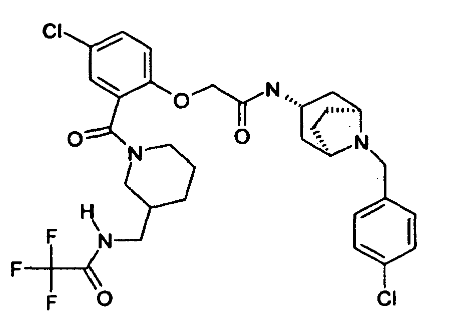

N-{[1-(5-chloro-2-hydroxybenzoyl)piperidin-3-yl]methyl}-2,2,2-trifluoroacetamide (Intermediate 38) (0.5 g, 1.4 mmols) and potassium carbonate (0.16 g, 1.1 mmols) were added to a solution of 2-chloro-N-[(3-endo)-8-(4-chlorobenzyl)-8-azabicyclo[3.2.1]oct-3-yl]acetamide (Intermediate 15) (0.37 g, 1.1 mmols) in dimethylformamide (15 mL). The suspension obtained was stirred at 50°C for 48 hours. The reaction mixture was poured into water/ice and extracted with ethyl acetate. The organic layer was washed with a saturated aqueous solution of potassium carbonate, water and brine. It was dried over magnesium sulfate, filtered and concentrated in vacuo. Chromatography on silica gel gave the title compound (0.29g).

### Example 16 2-(2-(3-(aminomethyl)piperidine-1-carbonyl)-4-chlorophenoxy)-N-((3-endo)-8-(4-chlorobenzyl)-8-azabicyclo[3.2.1]octan-3-yl)acetamide

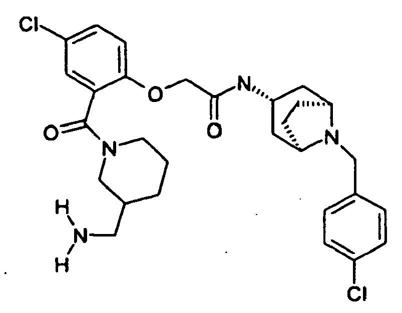

N-((1-(5-chloro-2-(2-((3-endo)-8-(4-chlorobenzyl)-8-azabicyclo[3.2.1]octan-3-ylamino)-2-oxoethoxy)benzoyl)piperidin-3-yl)methyl)-2,2,2-trifluoroacetamide (Example 15) (0.29 g, 0.44 mmols) was dissolved in methanol (8 mL) and a solution of potassium carbonate (0.13 g, 0.9 mmols) in water (0.5 mL) was added. The mixture was stirred at room temperature overnight. It was concentrated in vacuo and dichloromethane and water were added, The two layers were separated. The organic layer was washed with brine, dried over sodium sulfate, filtered and concentrated in vacuo. Chromatography on silica gel of the residue obtained gave the title compound (0.140 g).

### Example 17 N-((3-endo)-8-(4-chlorobenzyl)-8-azabicyclo[3.2.1]octan-3-yl)-2-(4-chlorophenylamino)acetamide

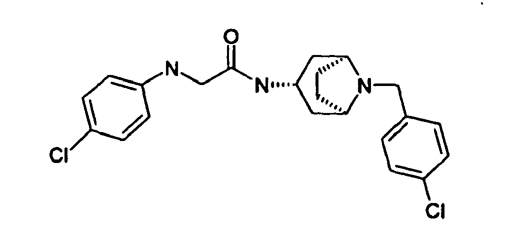

(4-chlorophenyl)amine (0.13 g, 1.0 mmols) and potassium carbonate (0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1g, 0.3 mmols) in dimethylformamide (2 mL). The suspension obtained was stirred at 60°C for 42 hours. The reaction mixture was poured into water/ice and extracted with dichloromethane. The organic layer was washed with water and brine. It was dried over magnesium sulfate, filtered and concentrated in vacuo. Reverse phase chromatography gave the title compound (0.04g).

### Example 18 N-((3-endo)-8-(4-chlorobenzyl)-8-azabicyclo[3.2.1]octan-3-yl)-2-(3-chlorophenylamino)acetamide

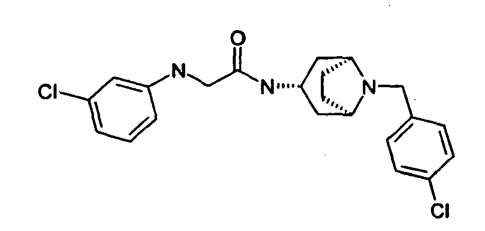

(3-chlorophenyl)amine (0.13 g, 1.0 mmols) and potassium carbonate (0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1g, 0.3 mmols) in dimethylformamide (2 mL). The suspension obtained was stirred at 60°C for 42 hours. The reaction mixture was poured into water/ice and extracted with dichloromethane. The organic layer was washed with water and brine. It was dried over magnesium sulfate, filtered and concentrated in vacuo. Reverse phase chromatography gave the title compound (0.02 g).

### Example 19 N-((3-endo)-8-(4-chlorobenzyl)-8-azabicyclo[3.2.1]octan-3-yl)-2-(2-(hydroxymethyl)phenoxy)acetamide

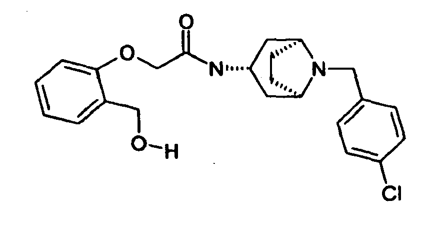

2-(hydroxymethyl)phenol (0.12 g, 1.0 mmols) and potassium carbonate (0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1g, 0.3 mmols) in dimethylformamide (2 mL). The suspension obtained was stirred at 60°C for 5.5 hours. The reaction mixture was poured into water/ice and extracted with dichloromethane. The organic layer was washed with water and brine. It was dried over magnesium sulfate, filtered and concentrated in vacuo. Chromatography on silica gel gave the title compound (0.04 g).

### Example 20 N-((3-endo)-8-(4-chlorobenzyl)-8-azabicyclo[3.2.1]octan-3-yl)-2-(4-methyl-2-(methylamino)phenoxy)acetamide

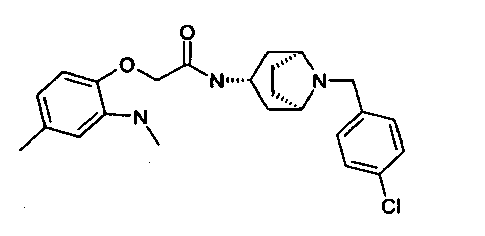

4-methyl-2-(methylamino)phenol (Intermediate 39) (0.14 g, 1.0 mmols) and potassium carbonate (0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.3 mmols) in dimethylformamide (2 mL). The mixture obtained was stirred at 60°C for 3 hours and then at room temperature overnight. The reaction mixture was poured into water and extracted with dichloromethane. The organic layer was dried and concentrated in vacuo. Reverse phase chromatography gave the title compound (0.02 g).

### Example 21 N-((3-endo)-8-(4-chlorobenzyl)-8-azabicyclo[3.2.1]octan-3-yl)-2-(4-chlorophenoxy)acetamide

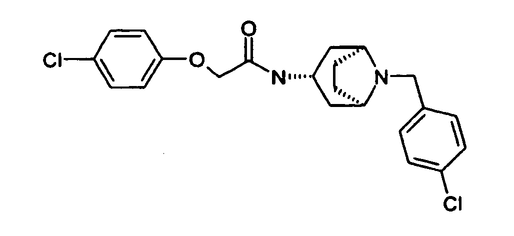

4-chlorophenol (0.13 g, 1.0 mmols) and potassium carbonate (0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.3 mmols) in dimethylformamide (2 mL). The mixture obtained was stirred at 60°C for 3 hours and then at room temperature overnight. The reaction mixture was poured into water and extracted with dichloromethane. The organic layer was dried and concentrated in vacuo and the residue was dissolved in 2 mL of methanol. Purification using a SCX ion exchange column gave the title compound (0.06 g).

### Example 22 N-(2-(2-((3-endo)-8-(4-chlorobenzyl)-8-azabicyclo[3.2.1]octan-3-ylamino)-2-oxoethoxy)-5-methylphenyl)acrylamide

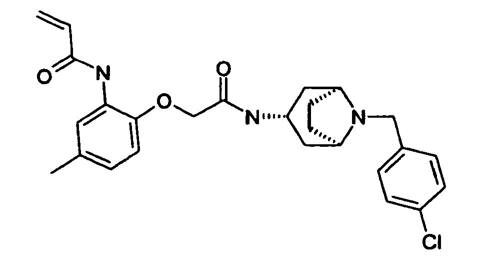

N-(2-hydroxy-5-methylphenyl)acrylamide (Intermediate 40) (0.18 g, 1.0 mmols) and potassium carbonate (0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.3 mmols) in dimethylformamide (2 mL). The mixture obtained was stirred at 60°C for 3 hours and then at room temperature overnight. The reaction mixture was poured into water and extracted with dichloromethane. The organic layer was dried and concentrated in vacuo and the residue was dissolved in 2 mL of methanol. Purification using a SCX ion exchange column gave the title compound (0.09 g).

### Example 23 N-((3-endo)-8-(4-chlorobenzyl)-8-azabicyclo[3.2.1]octan-3-yl)-2-(4-methyl-2-nitrophenoxy)acetamide

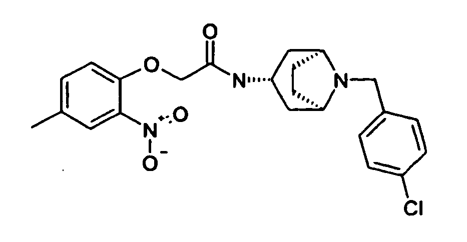

4-methyl-2-nitrophenol (0.15 g, 1.0 mmols) and potassium carbonate (0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.3 mmols) in dimethylformamide (2 mL). The mixture obtained was stirred at 60°C for 3 hours and then at room temperature overnight. The reaction mixture was poured into water and extracted with dichloromethane. The organic layer was dried and concentrated in vacuo and the residue was dissolved in 2 mL of methanol. Purification using a SCX ion exchange column gave the title compound (0.02 g).

### Example 24 N-((3-endo)-8-(4-chlorobenzyl)-8-azabicyclo[3.2.1]octan-3-yl)-2-(3-cyanophenoxy)acetamide

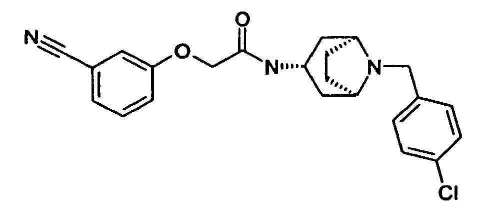

3-hydroxybenzonitrile (0.12 g, 1.0 mmols) and potassium carbonate (0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.3 mmols) in dimethylformamide (2 mL). The mixture obtained was stirred at 60°C for 3 hours and then at room temperature overnight. The reaction mixture was poured into water and extracted with dichloromethane. The organic layer was dried and concentrated in vacuo and the residue was dissolved in 2 mL of methanol. Purification using a SCX ion exchange column gave the title compound (0.07 g).

### Example 25 N-((3-endo)-8-(4-chlorobenzyl)-8-azabicyclo[3.2.1]octan-3-yl)-2-(3-chlorophenoxy)acetamide

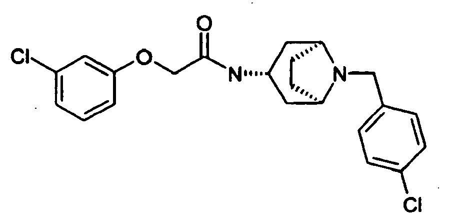

3-chlorophenol (0.13 g, 1.0 mmols) and potassium carbonate (0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.3 mmols) in dimethylformamide (2 mL). The mixture obtained was stirred at 60°C for 3 hours and then at room temperature overnight. The reaction mixture was poured into water and extracted with dichloromethane. The organic layer was dried and concentrated in vacuo and the residue was dissolved in 2 mL of methanol. Purification using a SCX ion exchange column gave the title compound (0.07 g).

### Example 26 N-((3-endo)-8-(4-chlorobenzyl)-8-azabicyclo[3.2.1]octan-3-yl)-2-(4-fluoro-2-nitrophenoxy)acetamide

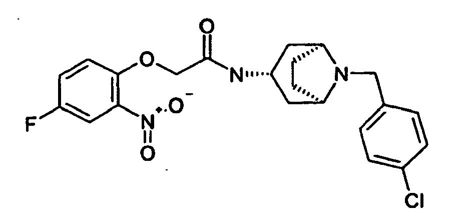

4-fluoro-2-nitrophenol (0.16 g, 1.0 mmols) and potassium carbonate ( 0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.3 mmols) in dimethylformamide (2 mL). The mixture obtained was stirred at 60°C for 3 hours and then at room temperature overnight. The reaction mixture was poured into water and extracted with dichloromethane. The organic layer was dried and concentrated in vacuo and the residue was dissolved in 2 mL of methanol. Purification using a SCX ion exchange column gave the title compound (0.007g).

### Example 27 methyl 4-acetamido-5-chloro-2-(2-((3-endo)-8-(4-chlorobenzyl)-8-azabicyclo[3.2.1]octan-3-ylamino)-2-oxoethoxy)benzoate

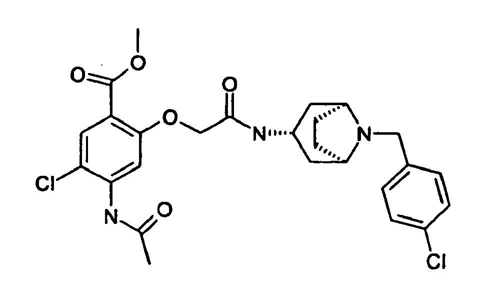

methyl 4-(acetylamino)-5-chloro-2-hydroxybenzoate (Intermediate 41) (0.24 g, 1.0 mmols) and potassium carbonate ( 0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.3 mmols) in dimethylformamide (2 mL). The mixture obtained was stirred at 60°C for 3 hours and then at room temperature overnight. The reaction mixture was poured into water and extracted with dichloromethane. The organic layer was dried and concentrated in vacuo and the residue was dissolved in 2 mL of methanol. Purification using a SCX ion exchange column gave the title compound (0.06 g).

### Example 28 N-((3-endo)-8-(4-chlorobenzyl)-8-azabicvclo[3.2.1]octan-3-yl)-2-(2-cyanophenoxy)acetamide

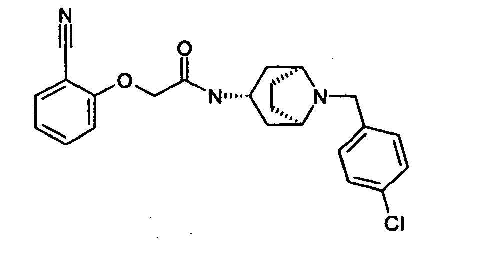

2-hydroxybenzonitrile (0.12 g, 1.0 mmols) and potassium carbonate (0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.3 mmols) in dimethylformamide (2 mL). The mixture obtained was stirred at 60°C for 3 hours and then at room temperature overnight. The reaction mixture was poured into water and extracted with dichloromethane. The organic layer was dried and concentrated in vacuo and the residue was dissolved in 2 mL of methanol. Purification using a SCX ion exchange column gave the title compound (0.008 g).

### Example 29 2-(2-((3-endo)-8-(4-chlorobenzvl)-8-azabicyclo[3.2.1]octan-3-ylamino)-2-oxoethoxy)benzamide

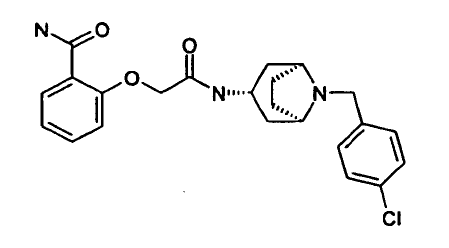

Salicylamide (0.14 g, 1.0 mmols) and potassium carbonate ( 0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.3 mmols) in dimethylformamide (2 mL). The mixture obtained was stirred at 60°C for 3 hours and then at room temperature overnight. The reaction mixture was poured into water and extracted with dichloromethane. The organic layer was dried and concentrated in vacuo and the residue was dissolved in 2 mL of methanol. Purification using a SCX ion exchange column gave the title compound (0.08 g).

### Example 30 2-(benzo[d][1,3]dioxol-5-yloxy)-N-((3-endo)-8-(4-chlorobenzyl)-8-azabicyclo[3.2.1]octan-3-yl)acetamide

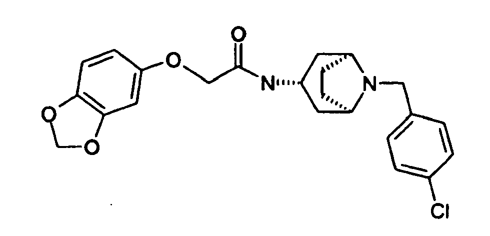

1,3-benzodioxol-5-ol (0.14 g, 1.0 mmols) and potassium carbonate (0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.3 mmols) in dimethylformamide (2 mL). The mixture obtained was stirred at 60°C for 3 hours and then at room temperature overnight. The reaction mixture was poured into water and extracted with dichloromethane. The organic layer was dried and concentrated in vacuo and the residue was dissolved in 2 mL of methanol. Purification using a SCX ion exchange column gave the title compound (0.07 g).

### Example 31 N-((3-endo)-8-(4-chlorobenzyl)-8-aza-bicyclo[3.2.1]octan-3-yl)-2-(2-nitrophenoxy)acetamide

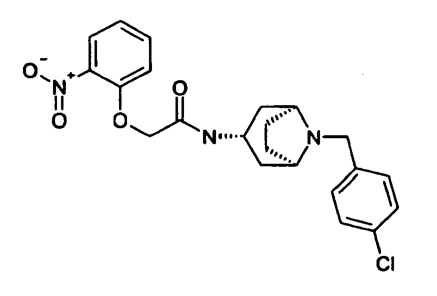

2-nitrophenol (0.14 g, 1.0 mmols) and potassium carbonate (0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.3 mmols) in dimethylformamide (2 mL). The mixture obtained was stirred at 60°C for 3 hours and then at room temperature overnight. The reaction mixture was poured into water and extracted with dichloromethane. The organic layer was dried and concentrated in vacuo and the residue was dissolved in 2 mL of methanol. Purification using a SCX ion exchange column gave the title compound (0.006 g).

### Example 32 N-((3-endol-8-(4-chlorobenzyl)-8-azabicyclo[3.2.1]octan-3-yl)-2-(2-methoxyphenoxy)acetamide

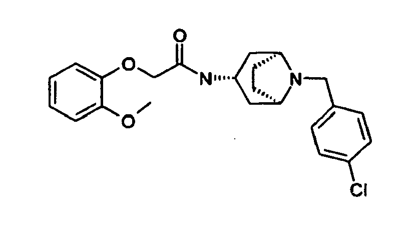

2-methoxyphenol (0.12 g, 1.0 mmols) and potassium carbonate (0.14 g, 1.0 mmols) were added to a solution of 2-chloro-N-[(3-endo)-8-(4-chlorobenzyl)-8-azabicyclo[3.2.1]oct-3-yl]acetamide (Intermediate 15) (0.1 g, 0.3 mmols) in dimethylformamide (2 mL). The mixture obtained was stirred at 60°C for 3 hours and then at room temperature overnight. The reaction mixture was poured into water and extracted with dichloromethane. The organic layer was dried and concentrated in vacuo and the residue was dissolved in 2 mL of methanol. Purification using a SCX ion exchange column gave the title compound (0.07 g).

The compounds of examples 33 to 46 have been obtained using synthetic method 3.

### Example 33 5-chloro-2-(2-((3-endo)-8-(4-chlorobenzyl)-8-azabicyclo[3.2.1]octan-3-ylamino)-2-oxoethoxy)-N-(piperidin-4-yl)benzamide

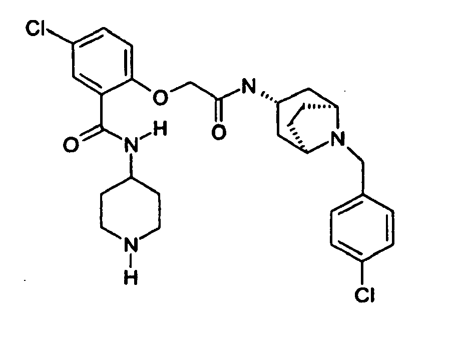

IRORI PS-5-chloro-2-(2-((3-endo)-8-(4-chlorobenzyl)-8-azabicyclo[3.2.1]octan-3-ylamino)-2-oxoethoxy)benzoic acid (Example 37) (0.1 g, loading: 1mmol/g, 0.1 mmols) was washed and swelled with dichloromethane/dimethylformamide 1:1 and it was filtered. A solution of tert-butyl 4-aminopiperidine-1-carboxylate (0.08 g, 0.4 mmols) and diisopropylethylamine (0.14 mL, 0.8 mmols) in dichloromethane/dimethylformamide 1:1 (1.5 mL) was addded to the resin. The mixture was stirred for 2 minutes at room temperature and O-(7-azabenzotriazol-1-yl)-N,N,N',N'-tetramethyluronium hexafluoro-phosphate (0.14 g, 0.38 mmols) was added. It was stirred at room temperature overnight. The resin was washed with dichloromethane/dimethylformamide 1:1, dichloromethane, diethylether, methanol, dichloromethane, diethylether, methanol, dichloromethane, dichloromethane/dimethylformamide 1:1 and, again, the resin was treated for a second time under the above conditions except for the time which was 24 hours. The resin was washed with dichloromethane/dimethylformamide 1:1 (1X), (dimethylformamide, dichloromethane, diethylether, tetrahydrofuran, water/methanol 1:1, methanol) (2X), dichloromethane (1X).

A solution of trifluoroacetic acid/water (95:5) (2mL) was added to PS-5-chloro-2-(2-((3-endo)-8-(4-chlorobenzyl)-8-azabicyclo[3.2.1]octan-3-ylamino)-2-oxoethoxy)-N-(piperidin-4-yl)benzamide (0.1g, loading:1 mmol/g, 0.1 mmol). The mixture was stirred for 2 hours at room temperature. It was filtered, washed with trifluoroacetic acid/water (95:5) and the filtrate was concentrated in vacuo. The residue obtained was dissolved in dichloromethane and the solution was washed with a 5% aqueous solution of potassium carbonate. The organic phase was dried and concentrated in vacuo to give the title compound (0.002 g).

### Example 34 2-(2-(4-aminopiperidine-1-carbonyl)-4-chlorophenoxy)-N-((3-endo)-8-(4-chlorobenzyl)-8-azabicyclo[3.2.1]octan-3-yl)acetamide

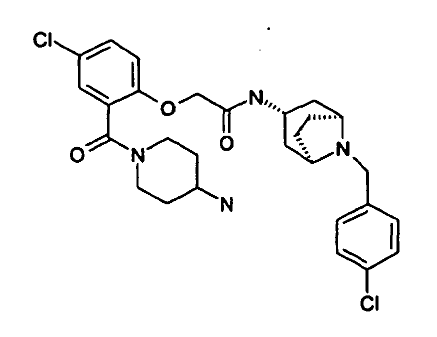

IRORI PS-5-chloro-2-(2-((3-endo)-8-(4-chlorobenzyl)-8-azabicyclo[3.2.1]octan-3-ylamino)-2-oxoethoxy)benzoic acid (Example 37) (0.1 g, loading: 1mmol/g, 0.1 mmols) was washed and swelled with dichloromethane/dimethylformamide 1:1 and it was filtered. A solution of tert-butyl piperidin-4-ylcarbamate (0.08 g, 0.4 mmols) and diisopropylethylamine (0.14 ml, 0.8 mmols) in dichloromethane/dimethylformamide 1:1 (1.5 mL) was addded to the resin. The mixture was stirred for 2 minutes at room temperature and O-(7-azabenzotriazol-1-yl)-N,N,N',N'-tetramethyluronium hexafluoro-phosphate (0.14 g, 0.38 mmols) was added. It was stirred at room temperature overnight. The resin was washed with dichloromethane/dimethylformamide 1:1, dichloromethane, diethylether, methanol, dichloromethane, diethylether, methanol, dichloromethane, dichloromethane/dimethylformamide 1:1 and, again, the resin was treated for a second time under the above conditions except for the time which was 24 hours. The resin was washed with dichloromethane/dimethylformamide 1:1 (1X), (dimethylformamide, dichloromethane, diethylether, tetrahydrofuran, water/methanol 1:1, methanol) (2X), dichloromethane (1X).

A solution of trifluoroacetic acid/water (95:5) (2mL) was added to PS-2-(2-(4-aminopiperidine-1-carbonyl)-4-chlorophenoxy)-N-((3-endo)-8-(4-chlorobenzyl)-8-azabicyclo[3.2.1]octan-3-yl)acetamide (0.1 g, loadying:1 mmol/g, 0.1 mmol). The mixture was stirred for 2 hours at room temperature. It was filtered, washed with trifluoroacetic acid/water (95:5) and the filtrate was concentrated in vacuo. The residue obtained was dissolved in dichloromethane and the solution was washed with a 5% aqueous solution of potassium carbonate. The organic phase was dried and concentrated in vacuo to give the title compound (0.003 g).

### Example 35 1-(5-chloro-2-(2-((3-endo)-8-(4-chlorobenzyl)-8-azabicyclo[3.2.1]octan-3-ylamino)-2-oxoethoxy)benzoyl)piperidine-3-carboxamide

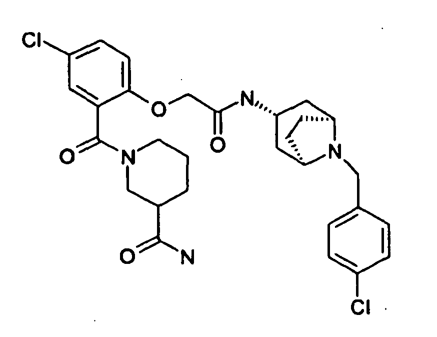

IRORI PS-5-chloro-2-(2-((3-endo)-8-(4-chlorobenzyl)-8-azabicyclo[3.2.1]octan-3-ylamino)-2-oxoethoxy)benzoic acid (Example 37) (0.1 g, loading: 1 mmol/g, 0.1 mmols) was washed and swelled with tetrahydrofuran and it was filtered. A solution of piperidine-3-carboxamide (0.13 g, 0.4 mmols) and diisopropylethylamine (0.14 mL, 0.8 mmols) in tetrahydrofuran (1 mL) was addded to the resin. The mixture was stirred for 2 minutes at room temperature and O-(7-azabenzotriazol-1-yl)-N,N,N',N'-tetramethyluronium hexafluoro-phosphate (0.14 g, 0.38 mmols) was added. It was stirred for 48 hours at room temperature. The resin was washed with tetrahydrofuran (1X), dimethylformamide (2X), dichloromethane (2X), methanol (2X), tetrahydrofuran/methanol 1:1 (1X).

A solution of trifluoroacetic acid/water (95:5) (1.5mL) was added to PS-1-(5-chloro-2-(2-((3-endo)-8-(4-chlorobenzyl)-8-azabicyclo[3.2.1]octan-3-ylamino)-2-oxoethoxy)benzoyl)piperidine-3-carboxamide (0.1 g, loading:1 mmol/g, 0.1 mmol). The mixture was stirred for 1.5 hours at room temperature. It was filtered, washed with trifluoroacetic acid/water (95:5) and the filtrate was concentrated in vacuo. The residue obtained was dissolved in dichloromethane and the solution was washed with a 5% aqueous solution of potassium carbonate. The organic phase was dried and concentrated in vacuo to give the title compound (0.004 g)

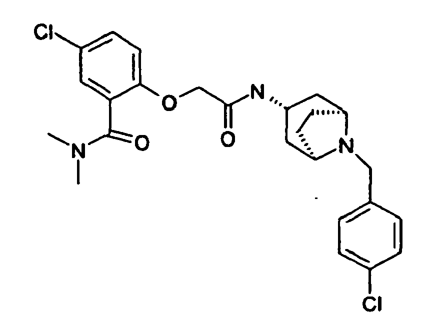

### Example 36 5-chloro-2-(2-((3-endo)-8-(4-chlorobenzyl)-8-azabicyclo[3.2.1]octan-3-ylamino)-2-oxoethoxy)-N,N-dimethylbenzamide

IRORI PS-5-chloro-2-(2-((3-endo)-8-(4-chlorobenzyl)-8-azabicyclo[3.2.1]octan-3-ylamino)-2-oxoethoxy)benzoic acid (Example 37) (0.15 g, loading: 1 mmol/g, 0.15 mmols) was washed and swelled with tetrahydrofuran and it was filtered. A solution of dimethylamine (0.3mL, 0.6 mmols) and diisopropylethylamine (0.21 mL, 1.2 mmols) in tetrahydrofuran (1.5 mL) was addded to the resin. The mixture was stirred for 2 minutes at room temperature and O-(7-azabenzotriazol-1-yl)-N,N,N',N'-tetramethyluronium hexafluoro-phosphate (0.22 g, 0.6 mmols) was added. It was stirred at room temperature overnight. The resin was washed with methanol (2X), methanol/water (1:1) (1X), a 5% aqueous solution of potassium carbonate (2X), methanol/water (1:1) (2X), methanol (2X), tetrahydrofuran (1X).

A solution of trifluoroacetic acid/water (95:5) (2 mL) was added to PS-5-chloro-2-(2-((3-endo)-8-(4-chlorobenzyl)-8-azabicyclo[3.2.1]octan-3-ylamino)-2-oxoethoxy)-N,N-dimethylbenzamide (0.15 g, loading:1 mmol/g, 0.15 mmol). The mixture was stirred for 1 hour at room temperature. It was filtered, washed with trifluoroacetic acid/water (95:5) and the filtrate was concentrated in vacuo to give the title compound (0.008 g).

### Example 37

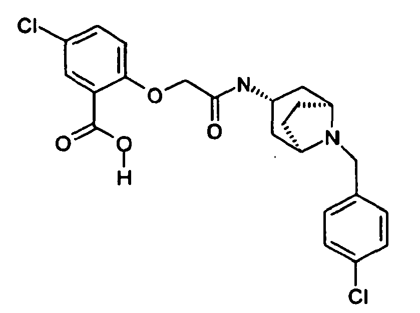

5-chloro-2-(2-((3-endo)-8-(4-chlorobenzyl)-8-azabicyclo[3.2.1]octan-3-ylamino)-2-oxoethoxy)benzoic acid

PS-methyl 5-chloro-2-(2-((3-endo)-8-(4-chlorobenzyl)-8-azabicyclo[3.2.1]octan-3-ylamino)-2-oxoethoxy)benzoate (Example 39) (1.0 g, loading: 1mmol/g, 1 mmol) was suspended in tetrahydrofuran (15 mL). Potassium trimethylsilanoate (0.33 g, 2.6 mmols) were added and the mixture was stirred at room temperature overnight. It was filtered and the resin was washed with acetic acid/methanol (1:1) (3X), methanol/water (1:1) (1X), methanol (2X), dichloromethane (3X).

A solution of dichloromethane/trifluoroacetic acid/water 50:50:1 (2 mL) was added to PS-5-chloro-2-(2-((3-endo)-8-(4-chlorobenzyl)-8-azabicyclo[3.2.1]octan-3-ylamino)-2-oxoethoxy)benzoic acid (0.050 g, loading: 1mmol/g, 0.05 mmols). The mixture was stirred for 1 hour at room temperature. It was filtered and the resin was washed with dichloromethane/trifluoroacetic acid/water 50:50:1. The filtrate was concentrated in vacuo to give the title compound (0.004 g).

### Example 38 2-(4-chloro-2-nitrophenoxy)-N-((3-endo)-8-(4-chlorobenzyl)-8-azabicyclo[3.2.1]octan-3-yl)acetamide

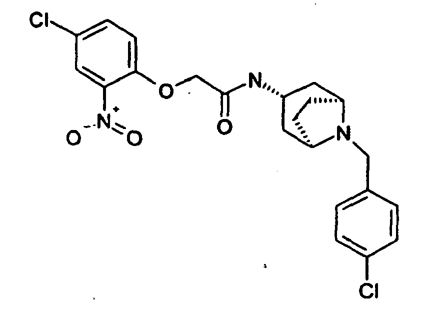

PS-2-bromo-N-[(3-endo)-8-(4-chlorobenzyl)-8-azabicyclo[3.2.1]oct-3-yl]acetamide (Intermediate 23) (1.0 g, loading: 1mmol/g, 1mmol) was washed with a solution of dimethylsulfoxide/1-methyl-2-pyrrolidinone (NMP) 1:1. A solution of 4-chloro-2-nitrophenol (1.06 g, 6.10 mmols) and 1,8-diazabicyclo[5.4.0]undec-7-ene (0.7 mL, 4.7 mmols) in dimethylsulfoxide/1-methyl-2-pyrrolidinone (NMP) 1:1 (10 mL) was added to the resin. The mixture was stirred at room temperature for 48 hours. It was filtered and the resin was washed with dimethylsulfoxide/1-methyl-2-pyrrolidinone (NMP) 1:1. The resin was treated for a second time under the above conditions except for the time which was 7 hours. It was filtered and washed with dimethylsulfoxide/1-methyl-2-pyrrolidinone (NMP) 1:1 (2X), dichloromethane (2X), methanol (2X), dichloromethane (2X), methanol (2X), diethyl ether (1X).

A solution of dichloromethane/trifluoroacetic acid/water 50:50:1 (2 mL) was added to PS-2-(4-chloro-2-nitrophenoxy)-N-((3-endo)-8-(4-chlorobenzyl)-8-azabicyclo[3.2.1]octan-3-yl)acetamide (0.065 g, loading: 1 mmol/g, 0.07 mmols). The mixture was stirred for 1 hour at room temperature. It was filtered and the resin was washed with dichloromethane/trifluoroacetic acid/water 50:50:1. The filtrate was concentrated in vacuo to give the title compound (0.006 g).

### Example 39

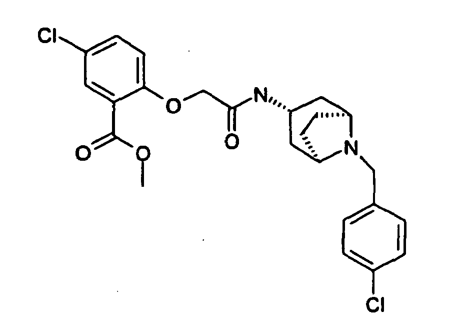

methyl 5-chloro-2-(2-((3-endo)-8-(4-chlorobenzyl)-8-azabicyclo[3.2.1]octan-3-ylamino)-2-oxoethoxy)benzoate

PS-2-bromo-N-[(3-endo)-8-(4-chlorobenzyl)-8-azabicyclo[3.2.1]oct-3-yl]acetamide (Intermediate 23) (1.0 g, loading: 1mmol/g, 1mmol) was washed with a solution of dimethylsulfoxide/1-methyl-2-pyrrolidinone (NMP) 1:1. A solution of methyl 5-chloro-2-hydroxybenzoate (1.15 g, 6.16 mmols) and 1,8-diazabicyclo[5.4.0]undec-7-ene (0.7 mL, 4.7 mmols) in dimethylsulfoxide/1-methyl-2-pyrrolidinone (NMP) 1:1 (10 mL) was added to the resin. The mixture was stirred at room temperature overnight. It was filtered and the resin was washed with dimethylsulfoxide/1-methyl-2-pyrrolidinone (NMP) 1:1. The resin was treated for a second time under the above conditions except for the time which was 48 hours. It was filtered and washed with dimethylsulfoxide/1-methyl-2-pyrrolidinone (NMP) 1:1 (2X), dichloromethane (2X), methanol (2X), dichloromethane (2X), tetrahydrofuran (1X).

A solution of dichloromethane/trifluoroacetic acid/water 50:50:1 (2 mL) was added to PS-methyl 5-chloro-2-(2-((3-endo)-8-(4-chlorobenzyl)-8-azabicyclo[3.2.1]octan-3-ylamino)-2-oxoethoxy)benzoate (0.05 g, loading:1mmol/g, 0.05 mmols). The mixture was stirred for 1 hour at room temperature. It was filtered and the resin was washed with dichloromethane/trifluoroacetic acid/water 50:50:1. The filtrate was concentrated in vacuo to give the title compound (0.003 g).

### Example 40 2-(2-amino-4-chlorophenoxy)-N-(L-endo)-8-(4-chlorobenzyl)-8-azabicyclo[3.2.1]octan-3-yl)acetamide

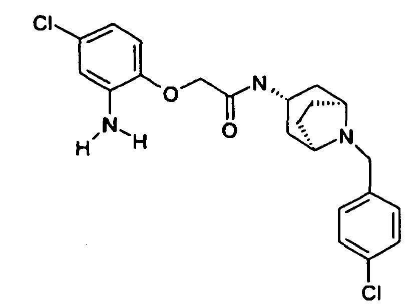

PS-2-(4-chloro-2-nitrophenoxy)-N-((3-endo)-8-(4-chlorobenzyl)-8-azabicyclo[3.2.1]octan-3-yl)acetamide (Example 38) (1.0 g, loading: 1mmol/g, 1 mmol) was suspended in a 3M solution of stannous chloride dihydrate in 1-methyl-2-pyrrolidinone (50 mL) and the mixture was stirred at 60°C overnight. It was filtered and washed with 1-methyl-2-pyrrolidinone (2X), dimethylformamide (2X), dichloromethane (2X), methanol (1X), dichloromethane (1X), methanol (1X), diethyl ether (2X). The resin was dried in vacuo at 35°C.

A solution of dichloromethane/trifluoroacetic acid/water 50:50:1 (2 mL) was added to PS-2-(2-amino-4-chlorophenoxy)-N-((3-endo)-8-(4-chlorobenzyl)-8-azabicyclo[3.2.1]octan-3-yl)acetamide (0.05 g, loading:1mmol/g, 0.05 mmols). The mixture was stirred for 1 hour at room temperature. It was filtered and the resin was washed with dichloromethane/trifluoroacetic acid/water 50:50:1. The filtrate was concentrated in vacuo to give the title compound (0.002 g).

### Example 41 2-amino-N-(5-chloro-2-(2-((3-endo)-8-(4-chlorobenzvl)-8-azabicyclo[3.2.1]octan-3-ylamino)-2-oxoethoxy)phenyl)acetamide

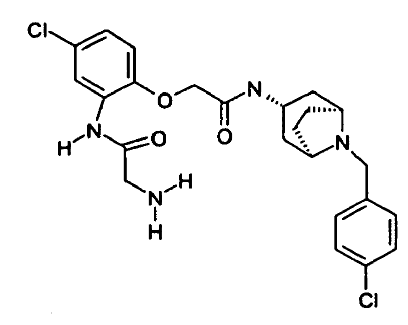

PS-2-(2-amino-4-chlorophenoxy)-N-((3-endo)-8-(4-chlorobenzyl)-8-azabicyclo[3.2.1]octan-3-yl)acetamide (Example 40) (0.06 g, loading: 1mmol/g, 0.06 mmols) was suspended in a solution of N-(tert-butoxycarbonyl)glycine (0.042 g, 0.24 mmols), diisopropylethylamine (0.062 g, 0.48 mmols) and O-(7-azabenzotriazol-1-yl)-N,N,N',N'-tetramethyluronium hexafluoro-phosphate (0.087 g, 0.23 mmols) in dichloromethane/dimethylformamide 1:1 (2 mL). The mixture was stirred at room temperature overnight. It was washed with dichloromethane/dimethylformamide 1:1, dichloromethane, diethyl ether, methanol, dichloromethane, diethyl ether and methanol. It was dried and PS-tert-butyl 2-(5-chloro-2-(2-((3-endo)-8-(4-chlorobenzyl)-8-azabicyclo[3.2.1]octan-3-ylamino)-2-oxoethoxy)phenylamino)-2-oxoethylcarbamate was obtained.

A solution of trifluoroacetic acid/water 95:5 (2 mL) was added to PS-tert-butyl 2-(5-chloro-2-(2-((3-endo)-8-(4-chlorobenzyl)-8-azabicyclo[3.2.1]octan-3-ylamino)-2-oxoethoxy)phenylamino)-2-oxoethylcarbamate (0.06 g, loading:1mmol/g, 0.06 mmols). The mixture was stirred for 2 hours at room temperature. It was filtered and the resin was washed with trifluoroacetic acid/water 95:1. The filtrate was concentrated in vacuo to give the title compound (0.004 g).

### Example 42 3-amino-N-(5-chloro-2-(2-((3-endo)-8-(4-chlorobenzyl)-8-azabicyco[3.2.1]octan-3-ylamino)-2-oxoethoxy)phenyl)propanamide

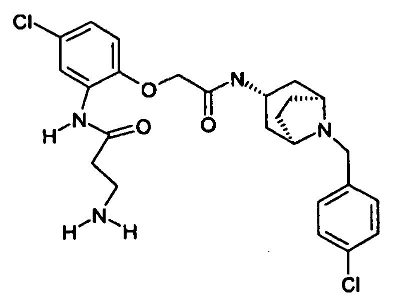

PS-2-(2-amino-4-chlorophenoxy)-N-((3-endo)-8-(4-chlorobenzyl)-8-azabicyclo[3.2.1]octan-3-yl)acetamide (Example 40) (0.06 g, loading: 1mmol/g, 0.06 mmols) was suspended in a solution of N-(tert-butoxycarbonyl)-beta-alanine (0.045 g, 0.24 mmols), diisopropylethylamine (0.062 g, 0.48 mmols) and O-(7-azabenzotriazol-1-yl)-N,N,N',N'-tetramethyluronium hexafluoro-phosphate (0.087 g, 0.23 mmols) in dichloromethane/dimethylformamide 1:1 (2 mL). The mixture was stirred at room temperature overnight. It was washed with dichloromethane/dimethylformamide 1:1, dichloromethane, diethyl ether, methanol, dichloromethane, diethyl ether and methanol. It was dried and PS-tert-butyl 3-(5-chloro-2-(2-((3-endo)-8-(4-chlorobenzyl)-8-azabicyclo[3.2.1]octan-3-ylamino)-2-oxoethoxy)phenylamino)-3-oxopropylcarbamate was obtained.

A solution of trifluoroacetic acid/water 95:5 (2 mL) was added to PS-tert-butyl 3-(5-chloro-2-(2-((3-endo)-8-(4-chlorobenzyl)-8-azabicyclo[3.2.1]octan-3-ylamino)-2-oxoethoxy)phenylamino)-3-oxopropylcarbamate (0.06 g, loading:1mmol/g, 0.06 mmols). The mixture was stirred for 2 hours at room temperature. It was filtered and the resin was washed with trifluoroacetic acid/water 95:1. The filtrate was concentrated in vacuo to give the title compound (0.004 g).

### Example 43 N-(5-chloro-2-(2-((3-endo)-8-(4-chlorobenzyl)-8-azabicyclo[3.2.1]octan-3-ylamino)-2-oxoethoxy)phenyl)piperidine-3-carboxamide

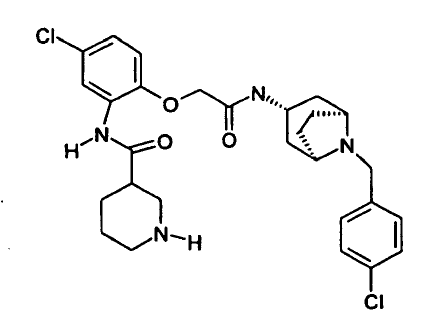

PS-2-(2-amino-4-chlorophenoxy)-N-((3-endo)-8-(4-chlorobenzyl)-8-azabicyclo[3.2.1]octan-3-yl)acetamide (Example 40) (0.06 g, loading: 1mmol/g, 0.06 mmols) was suspended in a solution of 1-(tert-butoxycarbonyl)piperidine-3-carboxylic acid (0.055 g, 0.24 mmols), diisopropylethylamine (0.062 g, 0.48 mmols) and O-(7-azabenzotriazol-1-yl)-N,N,N',N'-tetramethyluronium hexafluoro-phosphate (0.087 g, 0.23 mmols) in dichloromethane/dimethylformamide 1:1 (2 mL). The mixture was stirred at room temperature overnight. It was washed with dichloromethane/dimethylformamide 1:1, dichloromethane, diethyl ether, methanol, dichloromethane, diethyl ether and methanol. It was dried and PS-tert-butyl 3-(5-chloro-2-(2-((3-endo)-8-(4-chlorobenzyl)-8-azabicyclo[3.2.1]octan-3-ylamino)-2-oxoethoxy)phenylcarbamoyl)piperidine-1-carboxylate was obtained.

A solution of trifluoroacetic acid/water 95:5 (2 mL) was added to PS-tert-butyl 3-(5-chloro-2-(2-((3-endo)-8-(4-chlorobenzyl)-8-azabicyclo[3.2.1]octan-3-ylamino)-2-oxoethoxy)phenylcarbamoyl)piperidine-1-carboxylate (0.06 g, loading:1mmol/g, 0.06 mmols). The mixture was stirred for 2 hours at room temperature. It was filtered and the resin was washed with trifluoroacetic acid/water 95:1. The filtrate was concentrated in vacuo to give the title compound (0.004 g).

### Example 44 N-(5-chloro-2-(2-((3-endo)-8-(4-chlorobenzyl)-8-azabicyclo[3.2.1]octan-3-ylamino)-2-oxoethoxy)phenyl)-2.2.2-trifluoroacetamide

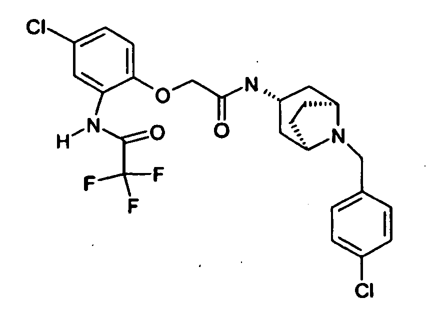

PS-2-(2-amino-4-chlorophenoxy)-N-((3-endo)-8-(4-chlorobenzyl)-8-azabicyclo[3.2.1]octan-3-yl)acetamide (Example 40) (0.06 g, loading: 1mmol/g, 0.06 mmols) was suspended in a solution of trifluoroacetic anhydride (0.050 g, 0.24 mmols) and pyridine (0.038 g, 0.48 mmols) in dichloromethane(2 mL). The mixture was stirred at room temperature overnight. The resin was washed with dichloromethane.

A solution of trifluoroacetic acid/water 95:5 (2mL) was added to PS-N-(5-chloro-2-(2-((3-endo)-8-(4-chlorobenzyl)-8-azabicyclo[3.2.1]octan-3-ylamino)-2-oxoethoxy)phenyl)-2,2,2-trifluoroacetamide (0.06 g, loading: 1mmol/g, 0.06 mmols). The mixture was stirred for 2 hours at room temperature. It was filtered and the resin was washed with trifluoroacetic acid/water 95:1. The filtrate was concentrated in vacuo to give the title compound (0.004 g).

### Example 45 2-(4-chloro-2-(methylsulfonamido)phenoxy)-N-((3-endo)-8-(4-chlorobenzyl)-8-azabicyclo[3.2.1]octan-3-yl)acetamide

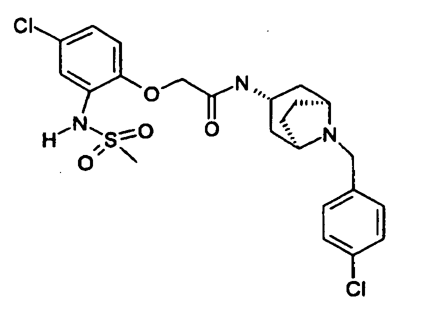

PS-2-(2-amino-4-chlorophenoxy)-N-((3-endo)-8-(4-chlorobenzyl)-8-azabicyclo[3.2.1]octan-3-yl)acetamide (Example 40) (0.06 g, loading: 1mmol/g, 0.06 mmols) was suspended in a solution of methanesulfonyl chloride(0.055 g, 0.48 mmols) and pyridine (0.038 g, 0.48 mmols) in dichloromethane(2 mL). The mixture was stirred at room temperature overnight. The resin was washed with dichloromethane.

A solution of trifluoroacetic acid/water 95:5 (2mL) was added to PS-2-(4-chloro-2-(methylsulfonamido)phenoxy)-N-((3-endo)-8-(4-chlorobenzyl)-8-azabicyclo[3.2.1]octan-3-yl)acetamide (0.06 g, loading:1mmol/g, 0.06 mmols). The mixture was stirred for 2 hours at room temperature. It was filtered and the resin was washed_with trifluoroacetic acid/water 95:1. The filtrate was concentrated in vacuo to give the title compound (0.002 g).

### Example 46 2-(4-chloro-2-guanidinophenoxy)-N-((3-endo)-8-(4-chlorobenzyl)-8-azabicyclo[3.2.1]octan3-yl)acetamide

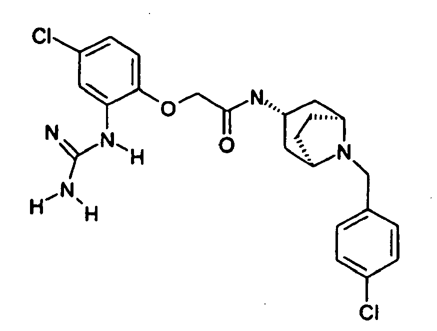

PS-2-(2-amino-4-chlorophenoxy)-N-((3-endo)-8-(4-chlorobenzyl)-8-azabicyclo[3.2.1]octan-3-yl)acetamide (Example 40) (0.1 g, loading: 1mmol/g, 0.1 mmols) was suspended in dichloroethane (2 mL). N,N'-di-BOC-1H-pyrazole-1-carboxamidine (0.06 g, 0.2 mmols) was added and the mixture was stirred for 24 hours at room temperature. It was filtered and washed with dichloroethane and the resin was trated for a second time under the above conditions except for the time which was 48 hours. It was filtered and washed with dichloromethane, methanol, dimethylformamide, methanol, dichloromethane, methanol, dichloromethane.

A solution of trifluoroacetic acid/water 95:5 (2mL) was added to PS-2-(4-chloro-2-guanidinophenoxy)-N-((3-endo)-8-(4-chlorobenzyl)-8-azabicyclo[3.2.1]octan-3-yl)acetamide (0.1 g, loading:1mmol/g, 0.1 mmols). The mixture was stirred for 2 hours at room temperature. It was filtered and the resin was washed_with trifluoroacetic acid/water 95:1. The filtrate obtained was separated: filtrate 1.

To the resin 2mL more of trifluoroacetic acid/water 95:1 were added and the mixture was stirred at room temperature for 5 hours. It was filtered and washed with trifluoroacetic acid/water 95:1 The filtrate obtained was added to filtrate 1 and it was concentrated in vacuo. Reverse phase chromatography gave the title compound (0.001 g).

The compounds of example 47 has been obtained using synthetic method 4.

### Example 47 2-(4-chloro-2-ureidophenoxy)-N-((3-exo)-8-(4-chlorobenzyl)-8-azabicyclo[3.2.1]octan-3-yl)acetamide

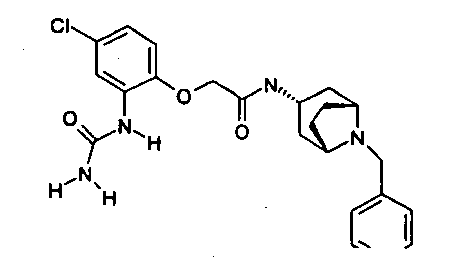

a) PS-allyl (3-exo)-3-[({2-[(aminocarbonyl)amino]-4-chlorophenoxy}acetyl)amino]-8-azabicyclo[3.2.1]octane-8-carboxylate 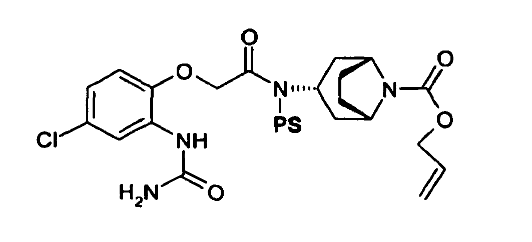
   PS-allyl-(3-exo)-3-[(bromoacetyl)amino]-8-azabicyclo[3.2.1]octane-8-carboxylate (Intermediate 25) (0.2 g, loading: 1mmol/g, 0.2 mmols) was washed and swelled with a solution of dimethylsulfoxide/1-methyl-2-pyrrolidinone (NMP) 1:1. A solution of N-(5-chloro-2-hydroxyphenyl)urea (0.229 g, 1.22 mmols) and 1,8-diazabicyclo[5.4.0]undec-7-ene (0.14 mL, 0.94 mmols) in dimethylsulfoxide/1-methyl-2-pyrrolidinone (NMP) 1:1 (2 mL) was added to the resin. The mixture was stirred for 24 hours at room temperature. It was filtered and the resin was washed with dimethylsulfoxide/1-methyl-2-pyrrolidinone (NMP) 1:1. The resin was treated for a second time under the above conditions except for the time which was 5 days. It was filtered and washed with dimethylsulfoxide/1-methyl-2-pyrrolidinone (NMP) 1:1 (1X), dichloromethane (1X), methanol (1X) and dichloromethane (2X). The resin was dried in vacuo and the title compound was obtained.
b) PS- 2-{2-[(aminocarbonyl)amino]-4-chlorophenoxyl-N-[(3-exo)-8-azabicyclo[3.2.1]oct-3-yl]acetamide 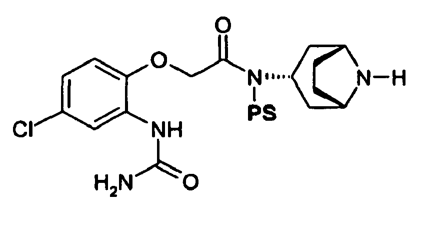
   PS-allyl (3-exo)-3-[({2-[(aminocarbonyl)amino]-4-chlorophenoxy}acetyl)amino]-8-azabicyclo[3.2.1]octane-8-carboxylate (0.2 g, loading:1 mmol/g, 0.2 mmols) was washed and swelled with dichloromethane. A solution of dimethylamino borane (0.47 g) in dichloromethane (4 mL) and a solution of tetrakis (triphenylphosphine) palladium (0.023 g) in dichloromethane (2 mL) were added to the resin. The mixture was stirred for 20 minutes at room temperature. It was filtered and washed with dichloromethane. The resin was treated for a second time under the above conditions. It was filtered and washed with dichloromethane (3X), a 0.2% solution of trifluoroacetic acid in dichloromethane (2X), dichloromethane (3X), a 5% solution of diisopropylethylamine in dichloromethane (2X), a solution of dioxane/water 9:1 (2X), methanol (3X), dimethylformamide (3X), dichloromethane (3X). The resin was dried in vacuo and the title compound was obtained.
c) PS-2-(4-chloro-2-ureidophenoxy)-N-((3-exo)-8-(4-chlorobenzyl)-8-azabicyclo[3.2.1]octan-3-yl)acetamide 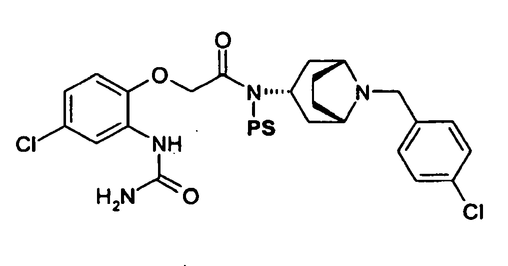
   PS- 2-{2-[(aminocarbonyl)amino]-4-chlorophenoxy}-N-[(3-exo)-8-azabicyclo[3.2.1]oct-3-yl]acetamide (0.2g, loading: 1mmol/g, 0.2 mmols) was washed and swelled with dimethylformamide. A solution of 4-chlorobenzaldehyde (0.56g, 4 mmols) in dimethylformamide (2 mL) and a 0.4% solution of acetic acid in dimethylformamide (0.87 mL) were added to the resin. The mixture was stirred at room temperature for 1 hours. A solution of sodium triacetoxiborohydride (0.74g, 3.49 mmols) in dimethylformamide (3.5 mL) was added and the mixture was stirred at room temperature overnight. It was filtered and washed with methanol (3X), dimethylformamide (3X) and dichloromethane (3X). The resin was dried in vacuo and the title compound was obtained.
d) Cleavage from the resin:
   A solution of dichloromethane/trifluoroacetic acid/water 50:50:1 (2 mL) was added to PS-2-(4-chloro-2-ureidophenoxy)-N-((3-exo)-8-(4-chlorobenzyl)-8-azabicyclo[3.2.1]octan-3-yl)acetamide (0.2 g, loading: 1mmol/g, 0.2 mmols) previously washed and swelled with dichloromethane. The mixture was stirred for 1 hour at room temperature. It was filtered and the resin was washed with dichloromethane/trifluoroacetic acid/water 50:50:1. The filtrate was concentrated in vacuo to give 2-(4-chloro-2-ureidophenoxy)-N-((3-exo)-8-(4-chlorobenzyl)-8-azabicyclo[3.2.1]octan-3-yl)acetamide (0.009 g).
   The compounds of examples 48 and 49 have been obtained using synthetic method 5.

### Example 48 N-((3-endo)-8-(4-chlorobenzyl)-8-azabicyclo[3.2.1]octan-3-yl)-2-(3 chlorophenyl)acetamide

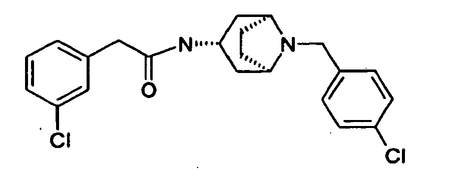

a) PS-allvl (3-endo)-3-{[(3-chlorophenyl)acetyl]amino}-8-azabicyclo[3.2.1]octane-8-carboxylate 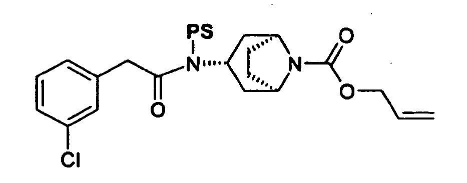
   PS-allyl (3-endo)-3-amino-8-azabicyclo[3.2.1]octane-8-carboxylate (Intermediate 7) (0.1 g, loading: 1mmol/g, 0.1 mmol) was washed with dichloromethane. A 0.5M solution of (3-chlorophenyl)acetic acid in dichloromethane/dimethylformamide 9:1 (2 mL) and a 1.2M solution of N,N'-diisopropylcarbodiimide in dichloromethane/dimethylformamide 9:1 (1 mL) were added and the mixture was stirred at 50°C for 4 hours. It was filtered and washed with dichloromethane. Again, a 0.5M solution of (3-chlorophenyl)acetic acid in dichloromethane/dimethylformamide 9:1 (2 mL) and a 1.2M solution of N,N'-diisopropylcarbodiimide in dichloromethane/dimethylformamide 9:1 (1 mL) were added and the mixture was stirred at room temperature overnight. It was filtered and washed with dimethylformamide (4X) and dichloromethane (4X). The resin was dried in vacuo to give the title compound.
b) PS-N-[(3-endo)-8-azabicyclo[3.2.1]oct-3-yl]-2-(3-chlorophenyl)acetamide 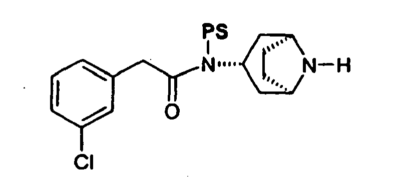
   PS- allyl (3-endo)-3-{[(3-chlorophenyl)acetyl]amino)-8-azabicyclo[3.2.1]octane-8-carboxylate (0.1 g, loading:1mmol/g, 0.1 mmols) was washed and swelled with dichloromethane. A 2M solution of dimethylamino borane in dichloromethane (2 mL) and a 0.01M solution of tetrakis (triphenylphosphine) palladium in dichloromethane (1 mL) were added to the resin. The mixture was stirred for 20 minutes at room temperature. It was filtered and washed with dichloromethane. The resin was treated for a second time under the above conditions. It was filtered and washed with dichloromethane (3X), a 0.2% solution of trifluoroacetic acid in dichloromethane (2X), dichloromethane (3X), a 5% solution of diisopropylethylamine in dichloromethane (2X), a solution of dioxane/water 9:1 (2X), methanol (3X), dimethylformamide (3X), dichloromethane (3X). The resin was dried in vacuo to give the title compound.
c)PS-N-((3-endo)-8-(4-chlorobenzyl)-8-azabicyclo[3.2.1]octan-3-yl)-2-(3-chlorophenyl)acetamide 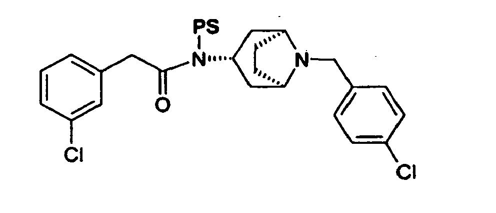 PS-N-[(3-endo)-8-azabicyclo[3.2.1]oct-3-yl]-2-(3-chlorophenyl)acetamide (0.1 g, loading: 1 mmol/g, 0.1 mmols) was washed and swelled with dimethylformamide. A 2M solution of 4-chlorobenzaldehyde in dimethylformamide (1 mL) and a 0.4% solution of acetic acid in dimethylformamide (0.5 mL) were added to the resin. The mixture was stirred at room temperature for 1 hours. A 1 M solution of sodium triacetoxiborohydride in dimethylformamide (2 mL) was added and the mixture was stirred at room temperature overnight. It was filtered and washed with methanol (3X), dimethylformamide (3X) and dichloromethane (3X). The resin was dried in vacuo to give the title compound.
d) Cleavage from the resin: A solution of dichloromethane/trifluoroacetic acid/water 50:50:1 (2 mL) was added to PS-N-((3-endo)-8-(4-chlorobenzyl)-8-azabicyclo[3.2.1]octan-3-yl)-2-(3-chlorophenyl)acetamide (0.1g, loading: 1mmol/g, 0.1 mmols) previously washed and swelled with dichloromethane. The mixture was stirred for 1 hour at room temperature. It was filtered and the resin was washed with dichloromethane/trifluoroacetic acid/water 50:50:1. The filtrate was concentrated in vacuo to give N-((3-endo)-8-(4-chlorobenzyl)-8-azabicyclo[3.2.1]octan-3-yl)-2-(3-chlorophenyl)acetamide (0.043 g)

### Example 49 N-((3-endo)-8-(4-chlorobenzyl)-8-azabicyclo[3.2.1]octan-3-yl)-2-(2-(2,6-dichlorophenylamino)phenyl)acetamide

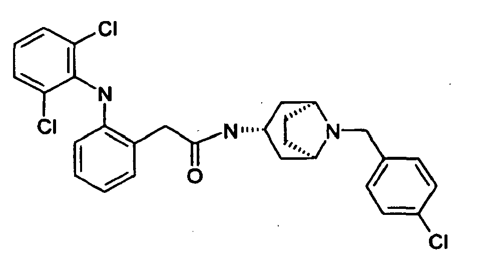

a) PS-allyl (3-endo)-3-[({2-[(2,6-dichlorophenyl)amino]phenyl}acetyl)amino]-8-azabicyclo[3.2.1]octane-8-carboxylate. 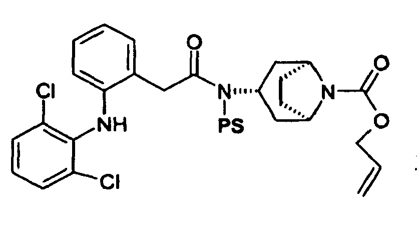 PS-allyl (3-endo)-3-amino-8-azabicyclo[3.2.1]octane-8-carboxylate (Intermediate 7) (0.1 g, loading: 1mmol/g. 0.1 mmol) was washed with dichloromethane. A 0.5M solution of {2-[(2,6-dichlorophenyl)amino]phenyl}acetic acid in dichloromethane/dimethylformamide 9:1 (2 mL) and a 1.2M solution of N,N'-diisopropylcarbodiimide in dichloromethane/dimethylformamide 9:1 (1 mL) were added and the mixture was stirred at 50°C for 4 hours. It was filtered and washed with dichloromethane. Again, a 0.5M solution of {2-[(2,6-dichlorophenyl)amino]phenyl}acetic acid in dichloromethane/dimethylformamide 9:1 (2 mL) and a 1.2M solution of N,N'-diisopropylcarbodiimide in dichloromethane/dimethylformamide 9:1 (1 mL) were added and the mixture was stirred at room temperature overnight. It was filtered and washed with dimethylformamide (4X) and dichloromethane (4X). The resin was dried in vacuo to give the title compound.
b)PS-N-[(3-endo)-8-azabicyclo[3.2.1]oct-3-yl]-2-{2-[(2,6-dichlorophenyl)amino]phenyl}acetamide 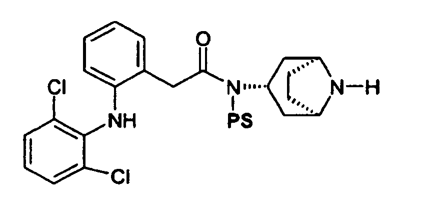 PS-allyl (3-endo)-3-[({2-[(2,6-dichlorophenyl)amino]phenyl}acetyl)amino]-8-azabicyclo[3.2.1]octane-8-carboxylate (0.1 g, loading: 1 mmol/g, 0.1 mmols) was washed and swelled with dichloromethane. A 2M solution of dimethylamino borane in dichloromethane (2 mL) and a 0.01 M solution of tetrakis (triphenylphosphine) palladium in dichloromethane (1 mL) were added to the resin. The mixture was stirred for 20 minutes at room temperature. It was filtered and washed with dichloromethane. The resin was treated for a second time under the above conditions. It was filtered and washed with dichloromethane (3X), a 0.2% solution of trifluoroacetic acid in dichloromethane (2X), dichloromethane (3X), a 5% solution of diisopropylethylamine in dichloromethane (2X), a solution of dioxane/water 9:1 (2X), methanol (3X), dimethylformamide (3X), dichloromethane (3X). The resin was dried in vacuo to give the title compound.
c)PS-N-((3-endo)-8-(4-chlorobenzyl)-8-azabicyclo[3.2.1]octan-3-yl)-2-(2-(2,6-dichlorophenylamino)phenyl)acetamide 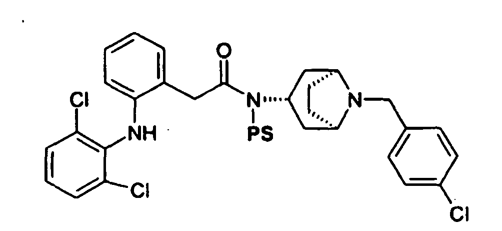
   PS-N-[(3-endo)-8-azabicyclo[3.2.1]oct-3-yl]-2-{2-[(2,6-dichlorophenyl)amino]phenyl}acetamide (0.1g, loading: 1mmol/g, 0.1 mmols) was washed and swelled with dimethylformamide. A 2M solution of 4-chlorobenzaldehyde in dimethylformamide (1 mL) and a 0.4% solution of acetic acid in dimethylformamide (0.5 mL) were added to the resin. The mixture was stirred at room temperature for 1 hours. A 1M solution of sodium triacetoxiborohydride in dimethylformamide (2 mL) was added and the mixture was stirred at room temperature overnight. It was filtered and washed with methanol (3X), dimethylformamide (3X) and dichloromethane (3X). The resin was dried in vacuo to give the title compound.
d) Cleavage from the resin:
   A solution of dichloromethane/trifluoroacetic acid/water 50:50:1 (2 mL) was added to PS-N-((3-endo)-8-(4-chlorobenzyl)-8-azabicyclo[3.2.1]octan-3-yl)-2-(2-(2,6-dichlorophenylamino)phenyl)acetamide (0.1g, loading: 1mmol/g, 0.1 mmols) previously washed and swelled with dichloromethane. The mixture was stirred for 1 hour at room temperature. It was filtered and the resin was washed with dichloromethane/trifluoroacetic acid/water 50:50:1. The filtrate was concentrated in vacuo to give N-((3-endo)-8-(4-chlorobenzyl)-8-azabicyclo[3.2.1]octan-3-yl)-2-(2-(2,6-dichlorophenylamino)phenyl)acetamide (0.012g).
   The compounds of examples 50 to 55 have been obtained using synthetic method 6.

### Example 50 3-chloro-N-(2-((3-endo)-8-(4-chlorobenzyl)-8-azabicyclo[3.2.1]octan-3-ylamino)-2-oxoethyl)benzamide

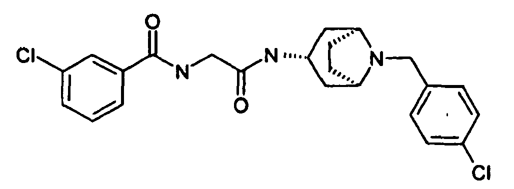

3-chlorobenzoic acid (0.61 g, 3.9 mmols), 1-(3-dimethylaminopropyl)-3-ethylcarbodiimide hydrochloride (EDC.HCl) (0.53 g, 2.7 mmols) and 1-hydroxybenzotriazole hydrate (HOBt) (0.75 g, 5.5 mmols) were dissolved in dichloromethane (140 mL). It was stirred for 10 minutes at room temperature. A solution of N¹-[(3-endo)-8-(4-chlorobenzyl)-8-azabicyclo[3.2.1]oct-3-yl]glycinamide (Intermediate 17) (1.0 g, 3.2 mmols) in dichloromethane (30 mL) was added. The mixture was stirred for 2.5 hours at room temperature. It was washed with a saturated aqueous solution of potassium carbonate and a 10% aqueous solution of potassium hydrogensulfate. The aqueous potassium hydrogensulfate phase was basified with potassium carbonate and extracted with chloroform. The organic phase obtained was washed with brine, dried over sodium sulfate, filtered and concentrated in vacuo. The residue obtained was triturated with diethyl ether and the solid obtained was filtered and dried to give the title compound (0.88 g) (yield: 62%)

### Example 51 N-(2-((3-endo)-8-(4-chlorobenzyl)-8-azabicyclo[3.2.1]octan-3-ylamino)-2-oxoethyl)-3-(trifluoromethyl)benzamide

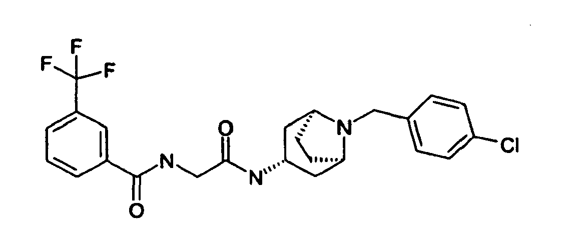

3-trifluoromethyl benzoic acid (0.19 g, 1.0 mmols), 1-(3-dimethylaminopropyl)-3-ethylcarbodiimide hydrochloride (EDC.HCl) (0.19 g, 1.0 mmols) and 1-hydroxybenzotriazole hydrate (HOBt) (0.13 g, 1.0 mmols) were dissolved in dichloromethane (35 mL). It was stirred for 15 minutes at room temperature. A solution of N¹-[(3-endo)-8-(4-ch)orobenzyl)-8-azabicyclo[3.2.1]oct-3-yl)glycinamide (Intermediate 17) (0.25 g, 0.81 mmols) in dichloromethane (8 mL) was added. The mixture was stirred for 2 hours at room temperature. It was washed with a saturated aqueous solution of potassium carbonate and water. The organic phase was dried over sodium sulfate, filtered and concentrated in vacuo. Chromatography on silica gel gave the title compound (0.23 g)

### Example 52 N-(2-((3-endo)-8-(4-chlorobenzyl)-8-azabicyclo[3.2.1]octan-3-ylamino)-2-oxoethyl)-2-(4-methylphenylsulfonamido)benzamide

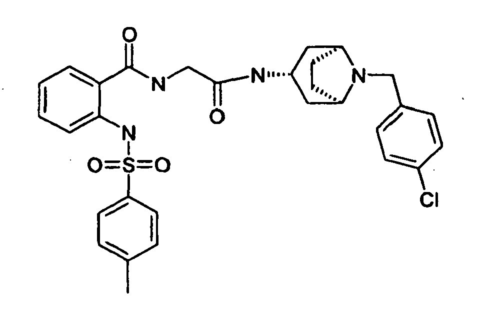

2-{[(4-methylphenyl)sulfonyl]amino}benzoic acid (0.045 g, 0.15 mmols), 1-(3-dimethylaminopropyl)-3-ethylcarbodiimide hydrochloride (EDC.HCl) (0.029 g, 0.15 mmols) and 1-hydroxybenzotriazole hydrate (HOBt) (0.021 g, 0.15 mmols) were dissolved in dichloromethane (6 mL). It was stirred for 10 minutes at room temperature. A solution of N¹-[(3-endo)-8-(4-chlorobenzyl)-8-azabicyclo[3.2.1]oct-3-yl]glycinamide (Intermediate 17) (0.04 g, 0.13 mmols) in dichloromethane (2 mL) was added. The mixture was stirred at room temperature overnight. It was washed with a saturated aqueous solution of potassium carbonate. The organic phase was dried, filtered and concentrated in vacuo. Chromatography on silica gel gave the title compound (0.037 g).

### Example 53 N-(2-((3-endo)-8-(4-chlorobenzyl)-8-azabicyclo[3.2.1]octan-3-ylamino)-2-oxoethyl)-1-(4-chlorophenyl)cyclopentanecarboxamide

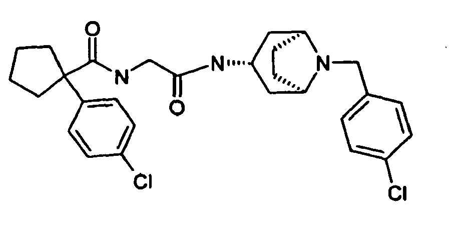

N¹-[(3-endo)-8-(4-chlorobenzyl)-8-azabicyclo[3.2.1]oct-3-yl]glycinamide (0.040 g, 0.13 mmols) and 1-(4-chlorophenyl)cyclopentanecarbonyl chloride (0.032 g, 0.13 mmols) were dissolved in dichloromethane (2 mL). Triethylamine (0.013mL) was added and the mixture was stirred at room temperature overnight. It was washed with a saturated aqueous solution of potassium carbonate. The organic phase was dried, filtered and concentrated in vacuo. Chromatography on silica gel gave the title compound (0.045 g).

### Example 54 2,7-dichloro-N-(2-((3-endo)-8-(4-chlorobenzyl)-8-azabicyclo[3.2.1]octan-3-ylamino)-2-oxoethyl)-9H-xanthene-9-carboxamide

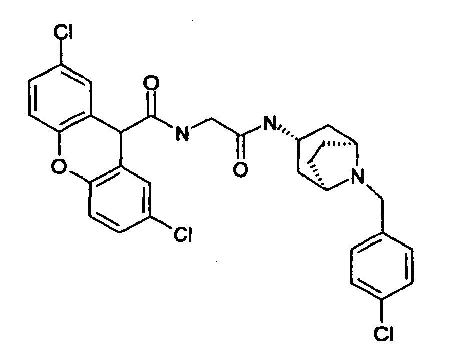

A solution of 2,7-dichloro-9H-xanthene-9-carboxylic acid (Intermediate 42) (0.094 g, 0.38 mmols) and N,N'-carbonyldiimidazole (CDI) (0.093 g, 0.57 mmols) in tetrahydrofuran (3.5 mL) was stirred for 2 hours at room temperature. A solution of N¹-[(3-endo)-8-(4-chlorobenzyl)-8-azabicyclo[3.2.1]oct-3-yl]glycinamide (Intermediate 17) (0.1g, 0.32 mmols) and 4-dimethylaminopyridine (DMAP) (0.004g, 0.03 mmols) in tetrahydrofuran (1.5 mL) was added. The mixture was stirred for 48 hours at room temperature. The reaction mixture was poured into ethyl acetate and it was washed with a saturated aqueous solution of sodium hydrogen carbonate and brine. The organic phase was dried over sodium sulfate, filtered and concentrated in vacuo. The residue was triturated with diethyl ether to give the title compound (0.038g).

### Example 55 5-chloro-N-(2-((3-endo)-8-(4-chlorobenzyl)-8-azabicyclo[3.2.1]octan-3-ylamino)-2-oxoethyl)-2-ureidobenzamide

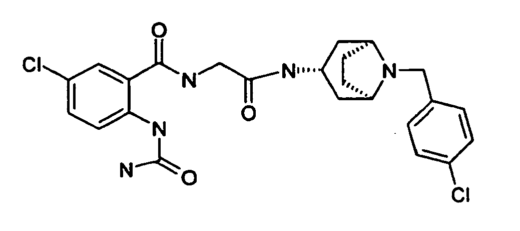

a) 2-amino-5-chloro-N-(2-((3-endo)-8-(4-chlorobenzyl)-8-azabicyclo[3.2.1]octan-3-ylamino)-2-oxoethyl) benzamide 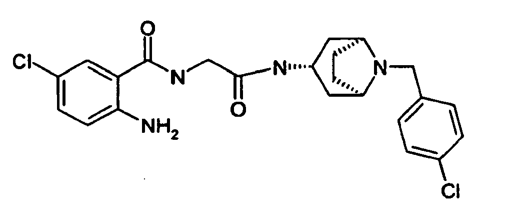
   2-amino-5-chlorobenzoic acid (0.134 g, 0.78 mmols), 1-(3-dimethylaminopropyl)-3-ethylcarbodiimide hydrochloride (EDC.HCl) (0.150 g, 0.78 mmols) and 1-hydroxybenzotriazole hydrate (HOBt) (0.11 g, 0.78 mmols) were dissolved in dichloromethane (25 mL). It was stirred for 10 minutes at room temperature. A solution of N¹-[(3-endo)-8-(4-chlorobenzyl)-8-azabicyclo[3.2.1]oct-3-yl]glycinamide (Intermediate 17) (0.2 g, 0.65 mmols) in dichloromethane (10 mL) was added. The mixture was stirred for at room temperature overnight. It was washed with a saturated aqueous solution of potassium carbonate, water and brine. The organic phase was dried over sodium sulfate, filtered and concentrated in vacuo. Chromatography on silica gel gave the title compound (0.23 g).
b)2-amino-5-chloro-N-(2-((3-endo)-8-(4-chlorobenzyl)-8-azabicyclo[3.2.1]octan-3-ylamino)-2-oxoethyl) benzamide (0.101g, 0.22 mmols) was added to a solution of acetic acid (0.5 mL) in water (1 mL). A solution of potassium cyanate (0.018g, 0.22 mmols) in water (0.5 mL) was added dropwise and the mixture was stirred for 5 hours at room temperature. A saturated aqueous solution of potassium carbonate was added to reach pH:8. Ethyl acetate was added and the two phases were separated. The organic phase was washed with water and brine, dried over magnesium sulfate and filtered. During the concentration in vacuo a precipitate was observed. It was filtered, washed and dried in vacuo to give 5-chloro-N-(2-((3-endo)-8-(4-chlorobenzyl)-8-azabicyclo[3.2.1]octan-3-ylamino)-2-oxoethyl)-2-ureidobenzamide (0.048g) (yield: 44%)

The compounds of examples 56 to 103 have been obtained using synthetic method 7.

### Example 56 N-((3-endo)-8-(4-chlorobenzyl)-8-azabicyclo[3.2.1]octan-3-yl)-2-(3-(3-chlorophenyl)ureido)acetamide

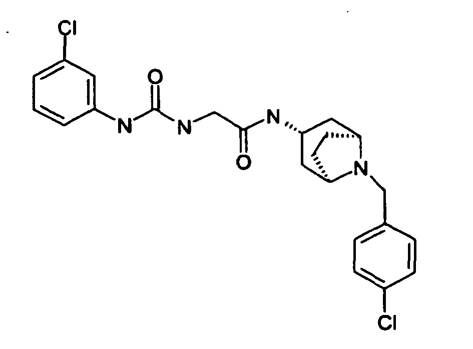

a) PS-allyl (3-endo)-3-[(N-H{[(3-chlorophenyl)amino]carbonyl}glycil)amino]-8-azabicyclo[3.2.1]octane-8-carboxylate 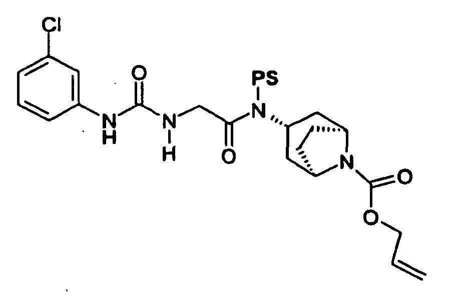
   PS-allyl (3-endo)-3-(glycylamino)-8-azabicyclo[3.2.1]octane-8-carboxylate (Intermediate 19) (0.08 g, loading: 1.3 mmols/g, 0.104 mmols) was washed and swelled with dichloromethane. A solution of 3-chlorophenyl isocyanate (0.253 mL, 2.1 mmols) and triethylamine (0.3 mL, 2.1 mmols) in dichloromethane (1 mL) was added. The mixture was stirred at room temperature overnight. It was filtered and washed with methanol (3X), dimethylformamide (3X) and dichloromethane (3X). The resin was dried in vacuo to give the title compound.
b) PS-N¹-[(3-endo)-8-azabicyclo[3.2.1]oct-3-yl]-N²-{[(3-chlorophenyl)amino]carbonyl}glycinamide 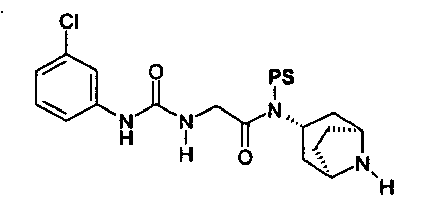
   The title compound was obtained from PS-allyl (3-endo)-3-[(N-{[(3-chlorophenyl)amino]carbonyl}glycyl)amino]-8-azabicyclo[3.2.1]octane-8-carboxylate following the same procedure described above for example 48 for the allyloxycarbonyl (alloc) deprotection.
c) PS- N-((3-endo)-8-(4-chlorobenzyl)-8-azabicyclo[3.2.1]octan-3-yl)-2-(3-(3-chlorophenyl)ureido)acetamide 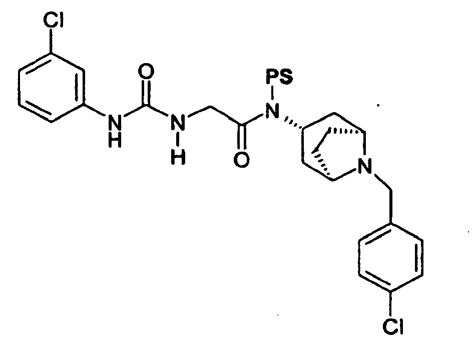 The title compound was obtained from PS-N¹-[(3-endo)-8-azabicyclo[3.2.1]oct-3-yl]-N²-{[(3-chlorophenyl)amino]carbonyl)glycinamide following the same procedure described above for example 48 for the reductive amination. The cleavage from the resin was performed following the procedure described in the same example and a reverse phase chromatography gave N-((3-endo)-8-(4-chlorobenzyl)-8-azabicyclo[3.2.1]octan-3-yl)-2-(3-(3-chlorophenyl)ureido)acetamide (0.005 g).

### Example 57 [2-((3-endo)-8-(4-chlorobenzyl)-8-azabicyclo[3.2.1]octan-3-ylamino)-2-oxoethyl)-2-phenoxybenzamide

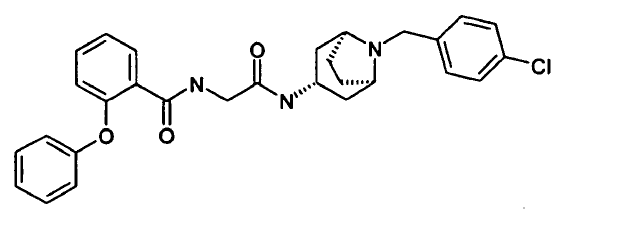

a) PS-allyl (3-endo)-3-{[N-(2-phenoxybenzol)glycyl]amino}-8-azabicyclo[3.2.1]octane-8-carboxylate 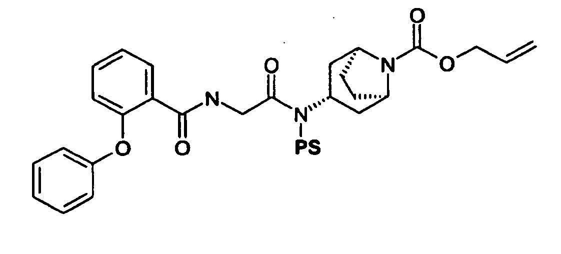
   PS-allyl (3-endo)-3-(glycylamino)-8-azabicyclo[3.2.1]octane-8-carboxylate (Intermediate 19) (0.1 g, loading:1mmol/g, 0.1 mmol) was washed and swelled with dichloromethane. A 0.5M solution of 2-phenoxybenzoic acid in dichloromethane/dimethylformamide 9:1 (2mL) was added. A 1.2M solution of N,N'-diisopropylcarbodiimide in dichloromethane/dimethylformamide 9:1 (1mL) was added. The mixture was stirred for 4 hours at 50°C. It was filtered and washed with dichloromethane. The resin was treated again with a 0.5M solution of 2-phenoxybenzoic acid in dichloromethane/dimethylformamide 9:1 (2mL) and a 1.2M solution of N,N'-diisopropylcarbodiimide in dichloromethane/dimethylformamide 9:1 (1mL). The mixture was stirred at room temperature overnight. It was filtered and washed with dimethylformamide (4X) and dichloromethane (4X). The resin was dried in vacuo to give the title compound.
b) The subsequent allyloxycarbonyl (alloc) deprotection of PS-allyl (3-endo)-3-{[N-(2-phenoxybenzoyl)glycyl]amino}-8-azabicyclo[3.2.1]octane-8-carboxylate, the reductive amination with 4-chlorobenzaldehide and the final cleavage were performed following the procedures described above for example 48 to give N-(2-((3-endo)-8-(4-chlorobenzyl)-8-azabicyclo[3.2.1]octan-3-ylamino)-2-oxoethyl)-2-phenoxybenzamide (0.039g)

### Example 58 N-(2-(((3-endo)-8-((4-chlorobenzyl)-8-azabicyclo[3.2.1]oct-3-yl)amino)-2-oxoethyl)-10,11-dihydro-5H-dibenzo[a,d]cycloheptene-5-carboxamide

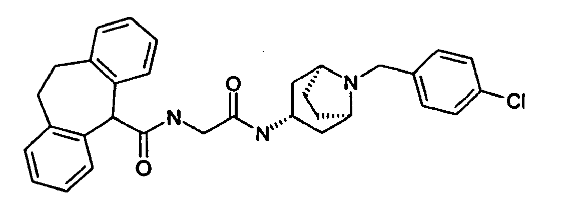

From PS-allyl (3-endo)-3-(glycylamino)-8-azabicyclo[3.2.1]octane-8-carboxylate (Intermediate 19) (0.1 g, loading: mmol/g, 0.1 mmol) the procedure described above for example 57 was followed. In this case 10,11-dihydro-5H-dibenzo[a,d][7]annulene-5-carboxylic acid was used instead to obtain the title compound (0.037 g).

### Example 59 N-(2-((3-endo)-8-(4-chlorobenzyl)-8-azabicyclo[3.2.1]octan-3-ylamino)-2-oxoethyl)-9-methyl-9H-fluorene-9-carboxamide

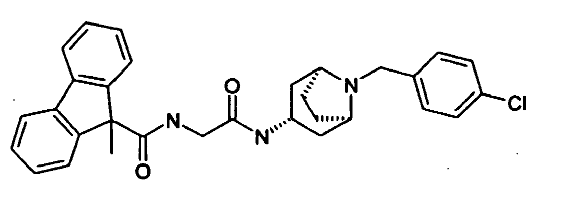

From PS-allyl (3-endo)-3-(glycylamino)-8-azabicyclo[3.2.1]octane-8-carboxylate (Intermediate 19) (0.1 g, loading:1mmol/g, 0.1 mmol) the procedure described above for example 57 was followed. In this case 9-methyl-9H-fluorene-9-carboxylic acid was used instead to obtain the title compound (0.034g).

### Example 60 N-(2-((3-endo)-8-(4-chlorobenzyl)-8-azabicyclo[3.2.1]octan-3-ylamino)-2-oxoethyl)-2,2-diphenylacetamide

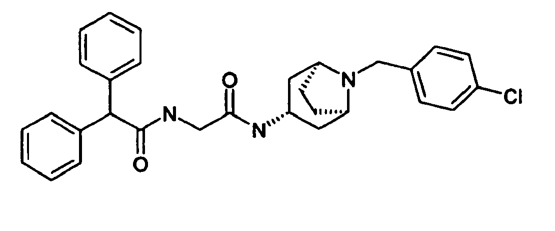

From PS-allyl (3-endo)-3-(glycylamino)-8-azabicyclo[3.2.1]octane-8-carboxylate (Intermediate 19) (0.1 g, loading:1 mmol/g, 0.1 mmol) the procedure described above for example C0074B09 was followed. In this case diphenylacetic acid was used instead to obtain the title compound (0.038 g).

### Example 61 2-amino-N-(2-((3-endo)-8-(4-chlorobenzyl)-8-azabicyclo[3.2.1]octan-3-ylamino)-2-oxoethyl)benzamide

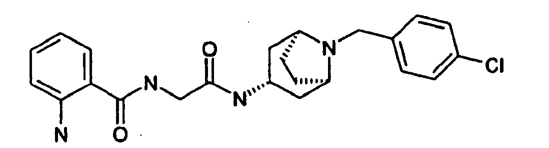

From PS-allyl (3-endo)-3-(glycylamino)-8-azabicyclo[3.2.1]octane-8-carboxylate (Intermediate 19) (0.1 g, loading:1mmol/g, 0.1 mmol) the procedure described above for example 57 was followed. In this case 2-tert-butoxycarbonylamino-benzoic acid was used instead to obtain the title compound (0.033 g).

### Example 62 N-(2-((3-endo)-8-(4-chlorobenzyl)-8-azabicyclo[3.2.1]octan-3-ylamino)-2-oxoethyl)-9-methyl-9H-xanthene-9-carboxamide

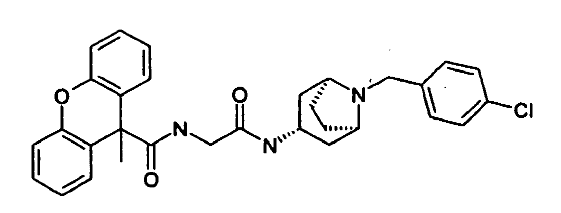

From PS-allyl (3-endo)-3-(glycylamino)-8-azabicyclo[3.2.1]octane-8-carboxylate (Intermediate 19) (0.1 g, loading:1mmol/g, 0.1 mmol) the procedure described above for example 57 was followed. In this case 9-methyl-9H-xanthene-9-carboxylic acid was used instead to obtain the title compound (0.031 g).

### Example 63 N-((3-endo)-8-(4-chlorobenzyl)-8-azabicyclo[3.2.1]octan-3-yl)-2-(2-(2-(2,6-dichlorophenylamino)phenyl)acetamido)acetamide

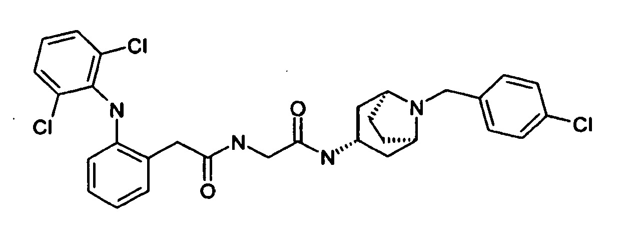

From PS-allyl (3-endo)-3-(glycylamino)-8-azabicyclo[3.2.1]octane-8-carboxylate (Intermediate 19) (0.1 g, lading: mmol/g, 0.1 mmol) the procedure described above for example 57 was followed. In this case (2-[(2,6-dichlorophenyl)amino]phenyl)acetic acid was used instead to obtain the title compound (0.007 g).

### Example 64 N-((3-endo)-8-(4-chlorobenzyl)-8-azabicyclo[3,2,1]octan-3-yl)-2-(2-(3-chlorophenyl)acetamido)acetamide

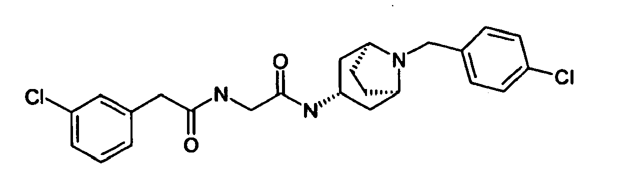

From PS-allyl (3-endo)-3-(glycylamino)-8-azabicyclo[3.2.1]octane-8-carboxylate (Intermediate 19) (0.1 g, loading: mmol/g, 0.1 mmol) the procedure described above for example 57 was followed. In this case (3-chlorophenyl)acetic acid was used instead to obtain the title compound (0.034 g).

### Example 65 N-(3-((3-endo)-8-(4-chlorobenzyl)-8-azabicyclo[3.2.1]octan-3-ylamino)-3-oxopropyl)-2-(3-(trifluoromethyl)phenylamino)benzamide

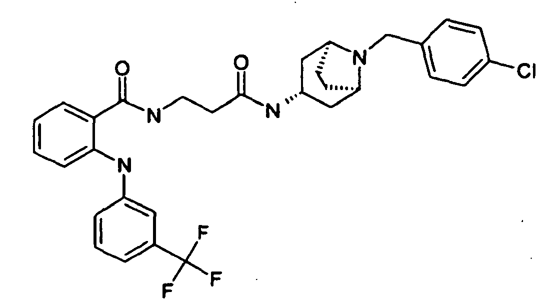

a)PS-allyl (3-endo)-3-({N-[(9H-fluoren-9-ylmethoxy)carbonyl]-beta-alanyl}amino)-8-azabicyclo[3.2.1]octane-8-carboxylate 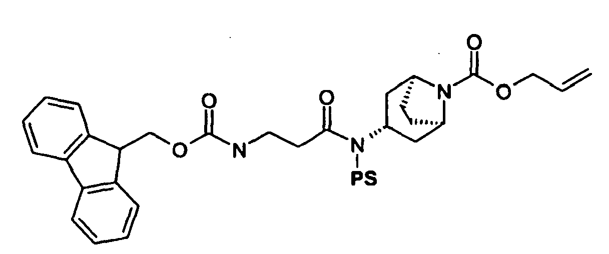
   PS-allyl (3-endo)-3-amino-8-azabicyclo[3.2.1]octane-8-carboxylate (Intermediate 19) (0.1 g, loading: 1 mmol/g, 0:1 mmols) was washed and swelled with tetrahydrofuran. It was filtered and N-[(9H-fluoren-9-ylmethoxy)carbonyl]-beta-alanine (0.31 g, 1 mmols) and tetrahydrofuran (1 mL) were added. A 0.6M solution of N,N'-diisopropylcarbodiimide in tetrahydrofuran (2 mL) was added to the mixture. It was stirred for 2.5 hours at 50°C. The resin was filtered and washed with dimethylformamide (2X), dichloromethane (1X) and tetrahydrofuran (1X). The resin was treated for a second time under the above conditions except for the time which was overnight. It was filtered and washed with dimethylformamide (4X) and dichloromethane (4X). The resin was dried in vacuo to give the title compound.
b) PS- allyl (3-endo)-3-(beta-alanylamino)-8-azabicyclo[3.2.1]octane-8-carboxylate 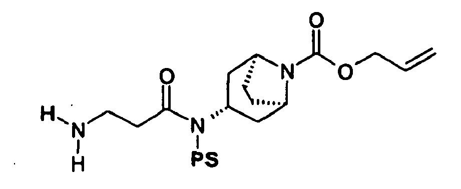 The title compound was obtained from PS-allyl (3-endo)-3-({N-[(9H-fluoren-9-ylmethoxy)carbonyl]-beta-alanyl}amino)-8-azabicyclo[3.2.1]octane-8-carboxylate following the same procedure described above for example 56 for the FMOC deprotection.
c)PS-allyl (3-endo)-3-{[N-(2-{[3-(trifluoromethyl)phenyl]amino}benzoyl)-beta-alanyl]amino}-8-azabicyclo[3.2.1]octane-8-carboxylate 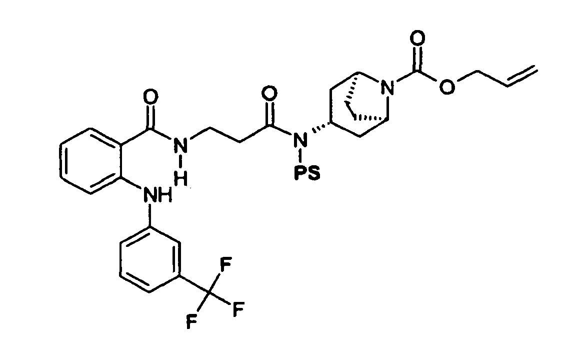
   PS- allyl (3-endo)-3-(beta-alanylamino)-8-azabicyclo[3.2.1]octane-8-carboxylate (0.1 g, loading: 1mmol/g, 0.1 mmol) was washed with dichloromethane. A 0.5M solution of 2-{[3-(trifluoromethyl)phenyl]amino}benzoic acid in dichloromethane/dimethylformamide 9:1 (2 mL) and a 1.2M solution of N,N'-diisopropylcarbodiimide in dichloromethane/dimethylformamide 9:1 (1 mL) were added and the mixture was stirred at 50°C for 4 hours. It was filtered and washed with dichloromethane. Again, a 0.5M solution of 2-([3-(trifluoromethyl)phenyl]amino)benzoic acid in dichloromethane/dimethylformamide 9:1 (2 mL) and a 1.2M solution of N,N'-diisopropylcarbodiimide in dichloromethane/dimethylformamide 9:1 (1 mL) were added and the mixture was stirred at room temperature overnight. It was filtered and washed with dimethylformamide (4X) and dichloromethane (4X). The resin was dried in vacuo to give the title compound.
d)PS-N-(3-((3-endo)-8-azabicyclo[3.2.1]octan-3-ylamino)-3-oxopropyl)-2-(3-(trifluoromethyl)phenylamino)benzamide 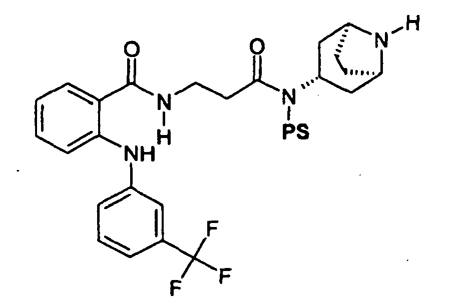
   The title compound was obtained from PS-allyl (3-endo)-3-{[N-(2-{[3-(trifluoromethyl)phenyl]amino}benzoyl)-beta-alanyl]amino}-8-azabicyclo[3.2.1]octane-8-carboxylate following the same procedure described above for example 48 for the allyloxycarbonyl (alloc) deprotection.
e) N-(3-((3-endo)-8-(4-chlorobenzyl)-8-azabicyclo[3.2.1]octan-3-ylamino)-3-oxopropyl)-2-(3-(trifluoromethyl)phenylamino)benzamide was obtained from PS-N-[3-[(3-endo)-8-azabicyclo[3.2.1]octan-3-ylamino]-3-oxopropyl]-2-[[3-(trifluoromethyl)phenyl]amino]benzamide following the same procedure described above for example 48 for the reductive amination and the subsequent cleavage from the resin (0.020g).

### Example 66 N-(2-((3-endo)-8-(4-chlorobenzyl)-8-azabicyclo[3.2.1]octan-3-ylamino)-2-oxoethyl)-N-methyl-2-(3-(trifluoromethyl)phenylamino)benzamide

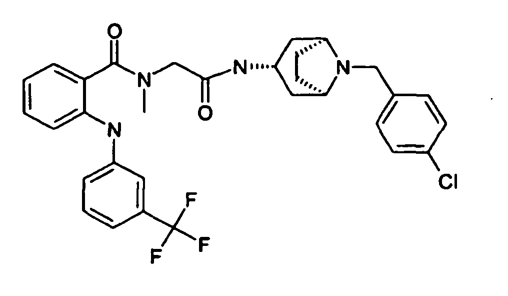

a) PS-allyl (3-endo)-3-({N-[(9H-fluoren-9-ylmethoxy)carbonyl]-N-methylglycyl}amino)-8-azabicyclo[3.2.1]octane-8-carboxylate 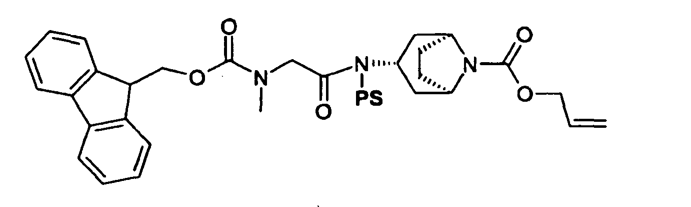
   PS-allyl (3-endo)-3-amino-8-azabicyclo[3.2.1]octane-8-carboxylate (Intermediate 7) (0.1 g, loading: 1 mmol/g, 0.1 mmols) was washed and swelled with tetrahydrofuran. It was filtered and N-[(9H-fluoren-9-ylmethoxy)carbonyl]-D-alanine (0.31 g, 1 mmols) and tetrahydrofuran (1 mL) were added. A 0.6M solution of N,N'-diisopropylcarbodiimide in tetrahydrofuran (2 mL) was added to the mixture. It was stirred for 2.5 hours at 50°C. The resin was filtered and washed with dimethylformamide (2X), dichloromethane (1X) and tetrahydrofuran (1X). The resin was treated for a second time under the above conditions except for the time which was overnight. It was filtered and washed with dimethylformamide (4X) and dichloromethane (4X). The resin was dried in vacuo to give the title compound.
b)PS-allyl (3-endo)-3-[(N-methylglycyl)amino]-8-azabicyclo[3.2.1]actane-8-carboxylate 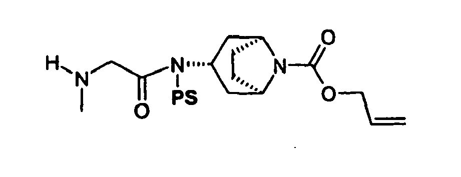
   The title compound was obtained from PS-allyl (3-endo)-3-({N-[(9H-fluoren-9-ylmethoxy)carbonyl]-N-methylglycyl}amino)-8-azabicyclo[3.2.1]octane-8-carboxylate following the same procedure described above for example 56 for the FMOC deprotection.
c)PS- allyl (3-endo)-3-{(N-methyl-N-(2-{(3-(trifluoromethyl)phenyl]amino}benzoyl)glycyl]amino}1-8-azabicyclo[3.2.1]octane-8-carboxylate 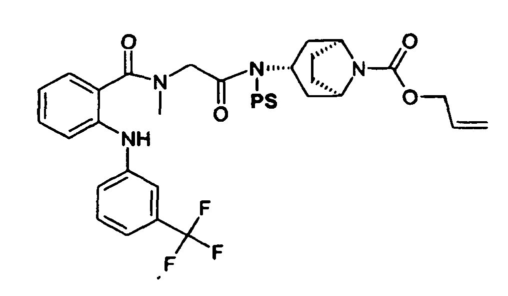 PS-allyl (3-endo)-3-[(N-methylglycyl)amino]-8-azabicyclo[3.2.1]octane-8-carboxylate (0.1 g, loading: 1mmol/g, 0.1 mmol) was washed with dichloromethane. A 0.5M solution of 2-{[3-(trifluoromethyl)phenyl]amino}benzoic acid in dichloromethane/dimethylformamide 9:1 (2 mL) and a 1.2M solution of N,N'-diisopropylcarbodiimide in dichloromethane/dimethylformamide 9:1 (1 mL) were added and the mixture was stirred at 50°C for 4 hours. It was filtered and washed with dichloromethane. Again, a 0.5M solution of 2-{[3-(trifluoromethyl)phenyl]amino}benzoic acid in dichloromethane/dimethylformamide 9:1 (2 mL) and a 1.2M solution of N,N'-diisopropylcarbodiimide in dichloromethane/dimethylformamide 9:1 (1 mL) were added and the mixture was stirred at room temperature overnight. It was filtered and washed with dimethylformamide (4X) and dichloromethane (4X). The resin was dried in vacuo.
d)PS-N-(2-((3-endo)-8-azabicyclo[3.2.1]octan-3-ylamino)-2-oxoethyl)-N-methyl-2-(3-(trifluoromethyl)phenylamino)benzamide 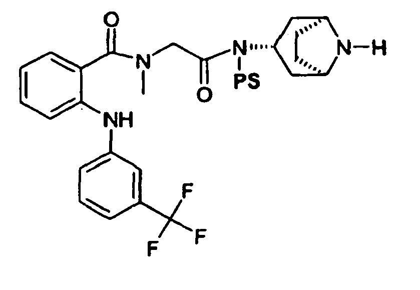
   The title compound was obtained from PS- allyl 3-{[N-methyl-N-(2-{[3-(trifluoromethyl)phenyl]amino}benzoyl)glycyl]amino}-8-azabicyclo[3.2.1]octane-8-carboxylate following the same procedure described above for example 48 for the allyloxycarbonyl (alloc) deprotection.
e) N-(2-((3-endo)-8-(4-chlorobenzyl)-8-azabicyclo[3.2.1]octan-3-ylamino)-2-oxoethyl)-N-methyl-2-(3-(trifluoromethyl)phenylamino)benzamide was obtained from PS-N-(2-((3-endo)-8-azabicyclo[3.2.1]octan-3-ylamino)-2-oxoethyl)-N-methyl-2-(3-(trifluoromethyl)phenylamino)benzamide following the same procedure described above for example 48 for the reductive amination and the subsequent cleavage from the resin (0.017 g).

### Example 67 3-bromo-N-(2-((3-endo)-8-(4-chlorobenzyl)-8-azabicyclo[3.2.1]octan-3-ylamino)-2-oxoethyl)benzamide

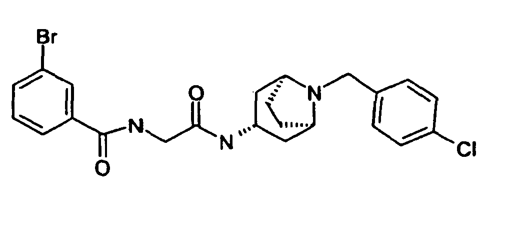

From IRORI PS-allyl (3-endo)-3-(glycylamino)-8-azabicyclo[3.2.1]octane-8-carboxylate (Intermediate 19) (0.06 g, loading:1.3mmol/g, 0.078 mmol) the procedure described above for example 57 was followed. In this case 3-bromobenzoic acid was used instead and a final reverse phase chromatography was performed to obtain the title compound (0.004 9).

### Example 68 5-chloro-N-(2-((3-endo)-8-(4-chlorobenzyl)-8-azabicyclo[3.211]octan-3-ylamino)-2-oxoethyl)-2-methoxybenzamide

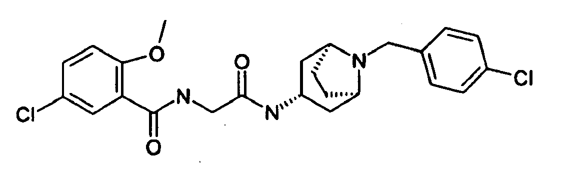

From IRORI PS-allyl (3-endo)-3-(glycylamino)-8-azabicyclo[3.2.1]octane-8-carboxylate (Intermediate 19) (0.06 g, loading:1.3mmol/g, 0.078 mmol) the procedure described above for example 57 was followed. In this case 5-chloro-2-methoxybenzoic acid was used instead to obtain the title compound (0.019 g).

### Example 69 N-(2-((3-endo)-8-(4-chlorobenzyl)-8-azabicyclo[3.2.1]octan-3-ylamino)-2-oxoethyl)-3-fluorobenzamide

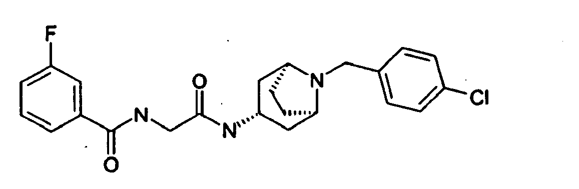

From IRORI PS-allyl (3-endo)-3-(glycylamino)-8-azabicyclo[3.2.1]octane-8-carboxylate (Intermediate 19) (0.06 g, loading:1.3mmol/g, 0.078 mmol) the procedure described above for example 57 was followed. In this case 3-fluorobenzoic acid was used instead to obtain the title compound (0.015 g).

### Example 70 5-chloro-N-(2-((3-endo)-8-(4-chlorobenzyl)-8-azabicyclo[3.2.1]octan-3-ylamino)-2-oxoethyl)-2-methoxy-4-(methylamino)benzamide

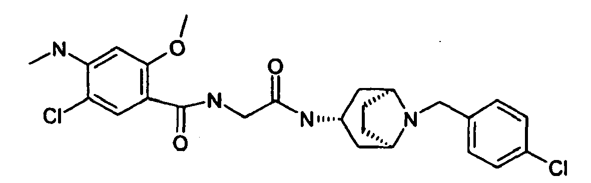

From IRORI PS-allyl (3-endo)-3-(glycylamino)-8-azabicyclo[3.2.1]octane-8-carboxylate (Intermediate 19) (0.06 g, loading:1.3mmol/g, 0.078 mmol) the procedure described above for example 57 was followed. In this case 5-chloro-2-methoxy-4-(methylamino)benzoic acid was used instead and a final reverse phase chromatography was performed to obtain the title compound (0.003 g).

### Example 71 3,4-dichloro-N-(2-((3-endo)-8-(4-chlorobenzyl)-8-azabicyclo[3.2.1]octan-3-ylamino)-2-oxoethyl)benzamide

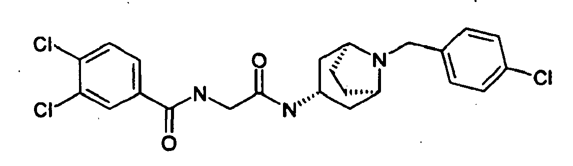

From IRORI PS-allyl (3-endo)-3-(glycylamino)-8-azabicyclo[3.2.1]octane-8-carboxylate (Intermediate 19) (0.06 g, loading:1.3mmol/g, 0.078 mmol) the procedure described above for example 57 was followed. In this case 3,4-dichlorobenzoic acid was used instead to obtain the title compound (0.008 g).

### Example 72 N-(2-((3-endo)-8-(4-chlorobenzyl)-8-azabicyclo[3.2.1]octan-3-ylamino)-2-oxoethyl)benzamide

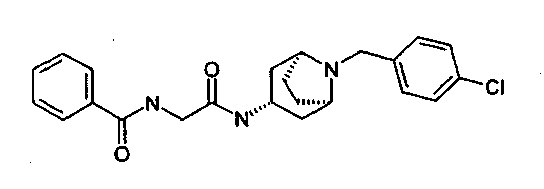

From IRORI PS-allyl (3-endo)-3-(glycylamino)-8-azabicyclo[3.2.1]octane-8-carboxylate (Intermediate 19) (0.06 g, loading:1.3mmol/g, 0.078 mmol) the procedure described above for example 57 was followed. In this case benzoic acid was used instead to obtain the title compound (0.015 g).

### Example 73 N-(2-((3-endo)-8-(4-chlorobenzyl)-8-azabicyclo[3.2.1]octan-3-ylamino)-2-oxoethyl)-3-methylbenzamide

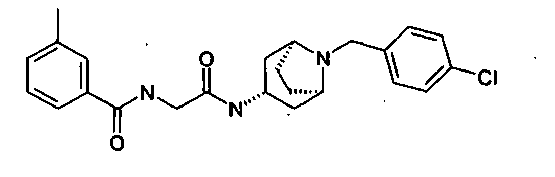

From IRORI PS-allyl (3-endo)-3-(glycylamino)-8-azabicyclo[3.2.1]octane-8-carboxylate (Intermediate 19) (0.06 g, loading:1.3mmol/g, 0.078 mmol) the procedure described above for example 57 was followed. In this case 3-methylbenzoic acid was used instead to obtain the title compound (0.017 g).

### Example 74 2,5-dichloro-N-(2-((3-endo)-8-(4-chlorobenzyl)-8-azabicyclo[3.2.1]octan-3-ylamino)-2-oxoethyl)benzamide

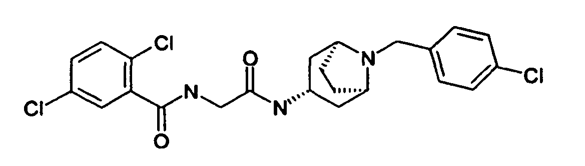

From IRORI PS-allyl (3-endo)-3-(glycylamino)-8-azabicyclo[3.2.1]octane-8-carboxylate (Intermediate 19) (0.06 g, loading: 1.3mmol/g, 0.078 mmol) the procedure described above for example 57 was followed. In this case 2,3-dichlorobenzoic acid was used instead to obtain the title compound (0.021 g).

### Example 75 N-(2-((3-endo)-8-(4-chlorobenzyl)-8-azabicyclo[3.2.1]octan-3-ylamino)-2-oxoethyl)-3-methoxybenzamide

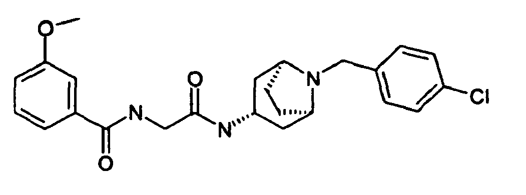

From IRORI PS-allyl (3-endo)-3-(glycylamino)-8-azabicyclo[3.2.1]octane-8-carboxylate (Intermediate 19) (0.06 g, loading:1.3mmol/g, 0.078 mmol) the procedure described above for example 57 was followed. In this case 3-methoxybenzoic acid was used instead to obtain the title compound (0.014 g).

### Example 76 N-(2-((3-endo)-8-(4-chlorobenzyl)-8-azabicyclo[3.2.1]octan-3-ylamino)-2-oxoethyl]-3-(hydroxy(phenyl)methyl)benzamide

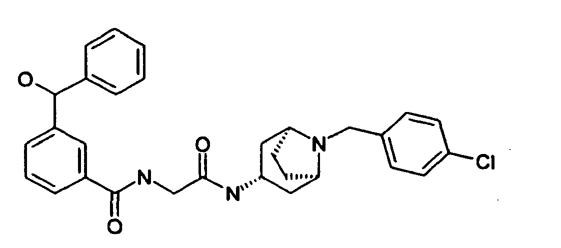

From IRORI PS-allyl (3-endo)-3-(glycylamino)-8-azabicyclo[3.2.1]octane-8-carboxylate (Intermediate 19) (0.06 g, loading:1.3mmol/g, 0.078 mmol) the procedure described above for example 57 was followed. In this case 3-benzoylbenzoic acid acid was used instead to obtain the title compound (0.020 g)

### Example 77 N-(2-((3-endo)-8-(4-chlorobenzyl)-8-azabicyclo[3.2.1]octan-3-ylamino)-2-oxoethyl)-3-(cyanomethyl)benzamide

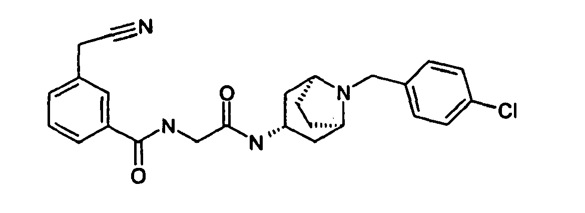

From IRORI PS-allyl (3-endo)-3-(glycylamino)-8-azabicyclo[3.2.1]octane-8-carboxylate (Intermediate 19) (0.06 g, loading:1.3mmol/g, 0.078 mmol) the procedure described above for example 57 was followed. In this case 3-(cyanomethyl)benzoic acid was used instead to obtain the title compound (0.017 g).

### Example 78 5-chloro-N-(2-((3-endo)-8-(4-chlorobenzyl)-8-azabicyclo[3.2.1]loctan-3-ylamino)-2-oxoethyl)-4-(dimethylamino)-2-methoxybenzamide

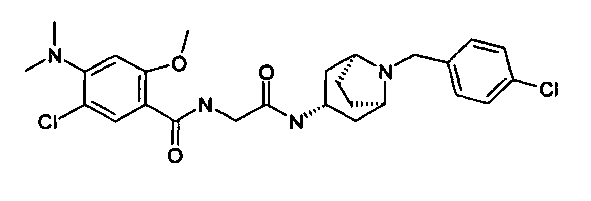

From IRORI PS-allyl (3-endo)-3-(glycylamino)-8-azabicyclo[3.2.1]octane-8-carboxylate (Intermediate 19) (0.06 g, loading:1.3mmol/g, 0.078 mmol) the procedure described above for example 57 was followed. In this case 5-chloro-4-(dimethylamino)-2-methoxybenzoic acid was used instead to obtain the title compound (0.018 g).

### Example 79 3-chloro-N-(3-((3-exol-8-(4-chlorobenzyl)-8-azabicyclo[3.2.1]octan-3-ylamino)-3-oxopropyl)benzamide

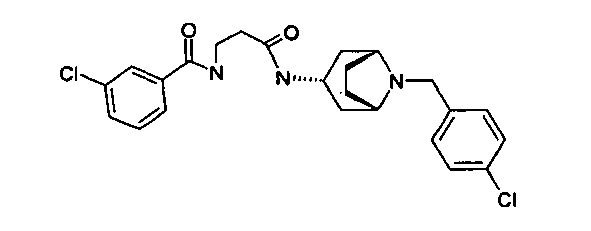

a) PS-allyl (3-exo)-3-({N-[(9H-fluoren-9-ylmethoxy)carbonyl]-beta-alanyl}amino)-8-azabicyclo[3.2.1]octane-8-carboxylate 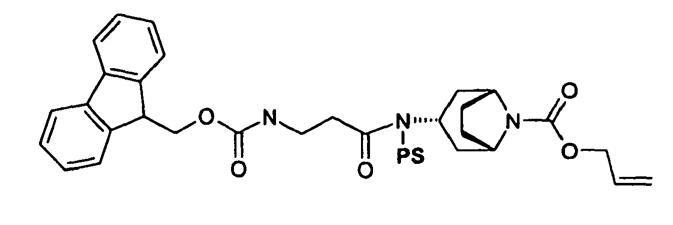
   IRORI PS-allyl (3-exo)-3-amino-8-azabicyclo[3.2.1]octane-8-carboxylate (Intermediate 9) (0.06 g, loading: 1 mmol/g, 0.06 mmols) was washed and swelled with tetrahydrofuran. It was filtered and a 1 M solution of N-[(9H-fluoren-9-ylmethoxy)carbonyl]-beta-alanine in tetrahydrofuran (0.6 mL) and a 0.6M solution of N,N'-diisopropylcarbodiimide in tetrahydrofuran (1.2 mL) were added to the mixture. It was stirred for 12 hours at 50°C. The resin was filtered and washed with dimethylformamide (2X), dichloromethane (1X) and tetrahydrofuran (1X). The resin was treated for a second time under the above conditions except for the time which was 36 hours. It was filtered and washed with tetrahydrofuran (1X), dimethylformamide (3X) and dichloromethane (3X). The resin was dried in vacuo to give the title compound.
b) PS- allyl (3-exo)-3-(beta-alanylamino)-8-azabicyclo[3.2.1]octane-8-carboxylate 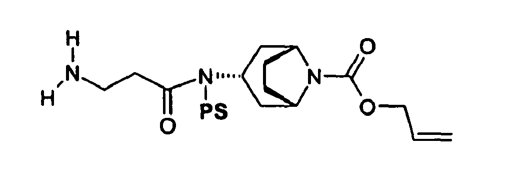 The title compound was obtained from PS- allyl (3-exo)-3-({N-[(9H-fluoren-9-ylmethoxy)carbonyl]-beta-alanyl}amino)-8-azabicyclo[3.2.1]octane-8-carboxylate following the same procedure described above for example 56 for the FMOC deprotection.
c) PS- allyl (3-exo)-3-{[N-(3-chlorobenzoyl)-beta-alanyl]amino}-8-azabicyclo[3.2.1]octane-8-carboxylate 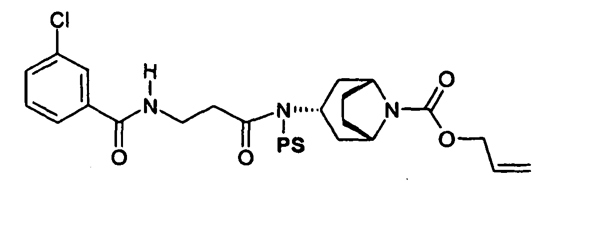 PS- allyl (3-exo)-3-(beta-alanylamino)-8-azabicyclo[3.2.1]octane-8-carboxylate (0.06 g, loading: 1mmo)/g. 0.06 mmol) was washed with dichloromethane. A 0.5M solution of 3-chlorobenzoic acid in dichloromethane/dimethylformamide 9:1 (3 mL) and N,N'-diisopropylcarbodiimide (0.22 mL) were added and the mixture was stirred at room temperature overnight. It was filtered and washed with dichloromethane (1X), dimethylformamide (3X) and dichloromethane (2X). The resin was dried in vacuo to give the title compound.
d) PS- N-(3-((3-exo)-8-azabicyclo[3.2.1]octan-3-ylamino)-3-oxopropyl)-3-chlorobenzamide 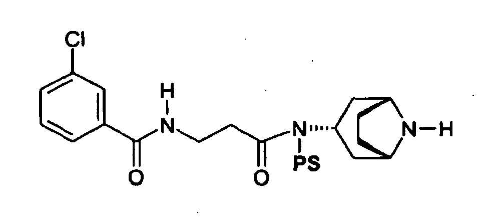 The title compound was obtained from PS-allyl (3-exo)-3-{[N-(3-chlorobenzoyl)-beta-alanyl]amino}-8-azabicyclo[3.2.1]octane-8-carboxylate following the same procedure described above for example 48 for the allyloxycarbonyl (alloc) deprotection.
e) PS-3-chloro-N-(3-((3-exo)-8-(4-chlorobenzyl)-8-azabicyclo[3.2.1]octan-3-ylamino)-3-oxopropyl)benzamide
   PS- N-(3-((3-exo)-8-azabicyclo[3.2.1]octan-3-ylamino)-3-oxopropyl)-3-chlorobenzamide (0.06 g, loading: 1 mmol/g, 0.06 mmol) was washed and swelled with dimethylformamide. A 2M solution of 4-chlorobenzaldehyde in dimethylformamide (1.5 mL) and a 0.4% solution of acetic acid in dimethylformamide (0.7 mL) were added to the resin. The mixture was stirred at room temperature for 1 hours. Sodium triacetoxiborohydride (0.32g, 1.5 mmols) was added and the mixture was stirred at room temperature overnight. It was filtered and washed with methanol (3X), dimethylformamide (3X) and dichloromethane (3X). The resin was dried in vacuo to give the title compound.
e) Cleavage from the resin:
   Following the same procedure described above for example 48 for the cleavage from the resin the title compound was obtained (0.010 g).

### Example 80 3-chloro-N-((R)-1-((3-exo)-8-(4-chlorobenzyl)-8-azabicyclo[3.2.1]octan-3-ylamino)-1-oxopropan-2-yl)benzamide

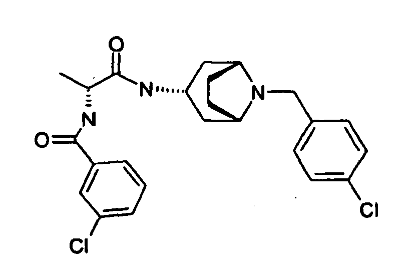

The title compound was obtained from IRORI PS-allyl (3-exo)-3-amino-8-azabicyclo[3.2.1]octane-8-carboxylate (Intermediate 9) (0.06 g, loading: 1 mmol/g, 0.06 mmols) following the same procedure described above for example 79 but using N-[(9H-fluoren-9-ylmethoxy)carbonyl]-D-alanine instead of N-[(9H-fluoren-9-ylmethoxy)carbonyl]-beta-alanine (0.010 g).

### Example 81 N-(2-((3-exo)-8-(4-chlorobenzyl)-8-azabicyclo[3.2.1]octan-3-ylamino)-2-oxoethyl)-N-methyl-2-(3-(trifluoromethyl)phenylamino)benzamide

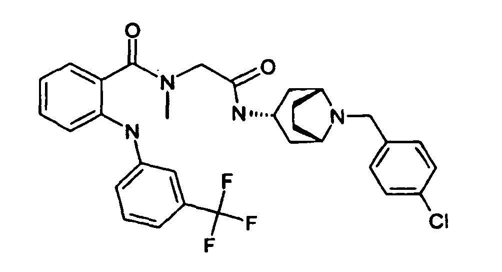

The title compound was obtained from IRORI PS-allyl (3-exo)-3-amino-8-azabicyclo[3.2.1]octane-8-carboxylate (Intermediate 9) (0.06 g, loading: 1 mmol/g, 0.06 mmols) following the same procedure described above for example 79 but using N-[(9H-fluoren-9-ylmethoxy)carbonyl]-N-methylglycine instead of N-[(9H-fluoren-9-ylmethoxy)carbonyl]-beta-alanine and 2-{(3-(trifluoromethyl)phenyl]amino}benzoic acid instead of 3-chlorobenzoic acid (0.006 g).

### Example 82 (R)-3-(3-chlorobenzamido)-4-((3-exo)-8-(4-chlorobenzyl)-8-azabicyclo[3.2.1]octan-3-ylamino)-4-oxobutanoic acid

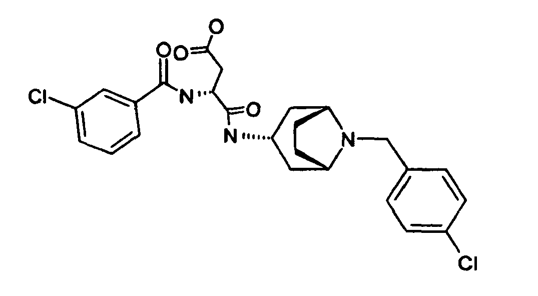

The title compound was obtained from IRORI PS-allyl (3-exo)-3-amino-8-azabicyclo[3.2.1]octane-8-carboxylate (Intermediate 9) (0.06 g, loading: 1 mmol/g, 0.06 mmols) following the same procedure described above for example 79 but using (R)-2-(((9H-fluoren-9-yl)methoxy)carbonylamino)-4-tert-butoxy-4-oxobutanoic acid instead of N-[(9H-fluoren-9-ylmethoxy)carbonyl]-beta-alanine (0.015 g).

### Example 83 N-((S)-1-((3-exo)-8-(4-chlorobenzyl)-8-azabicyclo[3.2.1]octan-3-ylamino)-1-oxopropan-2-yl)-2-(3-(trifluoromethyl)phenylamino)benzamide

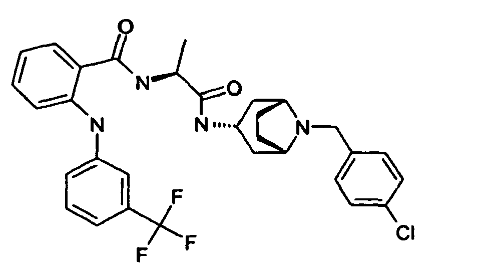

The title compound was obtained from IRORI PS-allyl (3-exo)-3-amino-8-azabicyclo[3.2.1]octane-8-carboxylate (Intermediate 9) (0.06 g, loading: 1 mmol/g, 0.06 mmols) following the same procedure described above for example 79 but using N-[(9H-fluoren-9-ylmethoxy)carbonyl]-L-alanine instead of N-[(9H-fluoren-9-ylmethoxy)carbonyl]-beta-alanine and 2-{[3-(trifluoromethyl)phenyl]amino}benzoic acid instead of 3-chlorobenzoic acid (0.005 g).

### Example 84 3-chloro-N-((S)-1-((3-exo)-8-(4-chlorobenzyl)-8-azabicyclo[3.2.1]octan-3-ylamino)-1-oxopronan-2-yl)benzamide

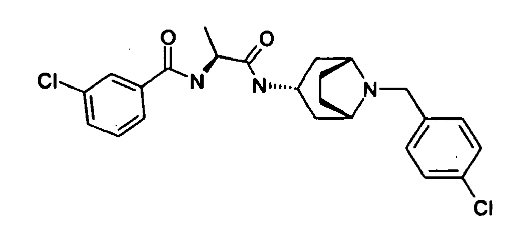

The title compound was obtained from IRORI PS-allyl (3-exo)-3-amino-8-azabicyclo[3.2.1]octane-8-carboxylate (Intermediate 9) (0.06 g, loading: 1 mmol/g, 0.06 mmols) following the same procedure described above for example 79 but using N-[(9H-fluoren-9-ylmethoxy)carbonyl]-L-alanine instead of N-[(9H-fluoren-9-ylmethoxy)carbonyl]-beta-alanine (0.011 g).

### Example 85 3-chloro-N-(2-((3-exo)-8-(4-chlorobenzyl)-8-azabicyclo[3.2.1]octan-3-ylamino)-2-oxoethyl)-N-methylbenzamide

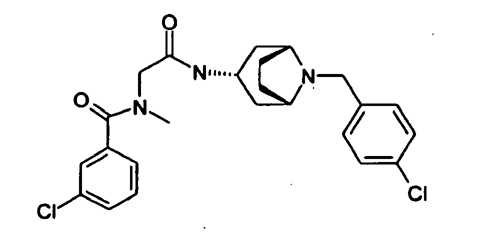

The title compound was obtained from IRORI PS-allyl (3-exo)-3-amino-8-azabicyclo[3.2.1]octane-8-carboxylate (Intermediate 9) (0.06 g, loading: 1 mmol/g, 0.06 mmols) following the same procedure described above for example 79 but using N-[(9H-fluoren-9-ylmethoxy)carbonyl]-N-methylglycine instead of N-[(9H-fluoren-9-ylmethoxy)carbonyl]-beta-alanine (0.013 g).

### Example 86 N-((S)-1-((3-exo)-8-(4-chlorobenzyl)-8-azabicyclo[3.2.1]octan-3-ylamino)-1-oxo-3-phenylpropan-2-yl)-2-(3-(trifluoromethyl)phenylamino)benzamide

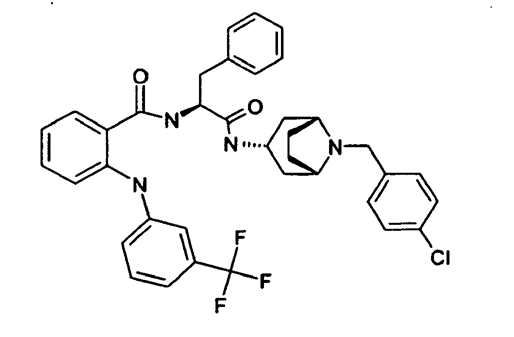

The title compound was obtained from IRORI PS-allyl (3-exo)-3-amino-8-azabicyclo[3.2.1]octane-8-carboxylate (Intermediate 9) (0.06 g, loading: 1 mmol/g, 0.06 mmols) following the same procedure described above for example 79 but using N-[(9H-fluoren-9-ylmethoxy)carbonyl]-L-phenylalanine instead of N-[(9H-fluoren-9-ylmethoxy)carbonyl]-beta-alanine and 2-{[3-(trifluoromethyl)phenyl]amino}benzoic acid instead of 3-chlorobenzoic acid (0.006 g).

### Example 87 N-((S)-1-((3-exo)-8-(4-chlorobenzyl)-8-azabicyclo[3.2.1]octan-3-ylamino)-4-(methylthio)-1-oxobutan-2-yl)-2-(3-(trifluoromethyl)phenylamino)benzamide

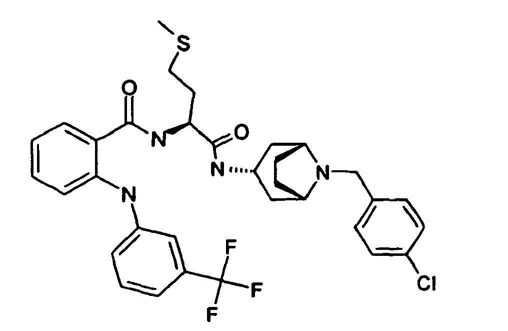

The title compound was obtained from IRORI PS-allyl (3-exo)-3-amino-8-azabicyclo[3.2.1]octane-8-carboxylate (Intermediate 9) (0.06 g, loading: 1 mmol/g, 0.06 mmols) following the same procedure described above for example 79 but using N-[(9H-fluoren-9-ylmethoxy)carbonyl]-L-methionine instead of N-[(9H-fluoren-9-ylmethoxy)carbonyl]-beta-alanine and 2-{[3-(trifluoromethyl)phenyl]amino}benzoic acid instead of 3-chlorobenzoic acid (0.007 g).

### Example 88 N-(3-((3-exo)-8-(4-chlorobenzyl)-8-azabicyclo[3.2.1]octan-3-ylamino)-3-oxopropyl)-2-(3-(trifluoromethyl)phenylamino)benzamide

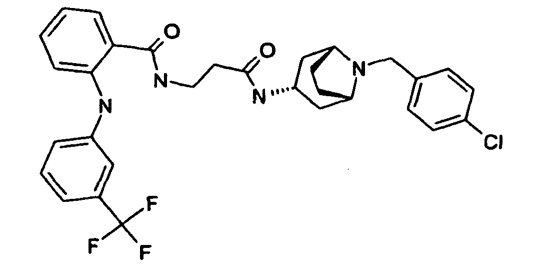

The title compound was obtained from IRORI PS-allyl (3-exo)-3-amino-8-azabicyclo[3.2.1]octane-8-carboxylate (Intermediate 9) (0.06 g, loading: 1 mmol/g, 0.06 mmols) following the same procedure described above for example 79 but using 2-{[3-(trifluoromethyl)phenyl]amino}benzoic acid instead of 3-chlorobenzoic acid (0.006 g).

### Example 89 N-((S)-1-((3-exo)-8-(4-chlorobenzyl)-8-azabicyclo[3.2.1]octan-3-ylamino)-4-methyl-1-oxopentan-2-yl)-2-(3-(trifluoromethyl)phenylamino)benzamide

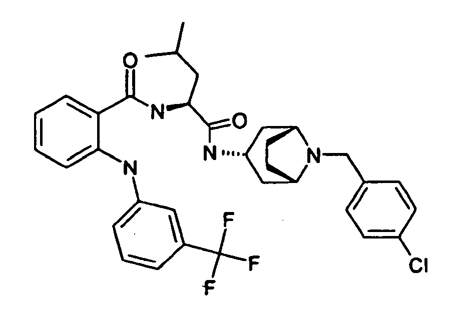

The title compound was obtained from IRORI PS-allyl (3-exo)-3-amino-8-azabicyclo[3.2.1]octane-8-carboxylate (Intermediate 9) (0.06 g, loading: 1 mmol/g, 0.06 mmols) following the same procedure described above for example 79 but using N-[(9H-fluoren-9-ylmethoxy)carbonyl]-L-leucine instead of N-[(9H-fluoren-9-ylmethoxy)carbonyl]-beta-alanine and 2-{[3-(trifluoromethyl)phenyl]amino}benzoic acid instead of 3-chlorobenzoic acid (0.006 g).

### Example 90 3-chloro-N-(2-((3-endo)-8-(3,4-dichlorobenzyl)-8-azabicyclo[3.2.1]octan-3-ylamino)-2-oxoethyl)benzamide

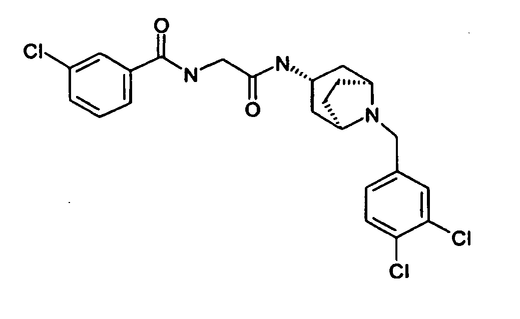

a)PS-N-(2-((3-endo)-8-azabicyclo[3.2.1]octan-3-ylamino)-2-oxoethyl)-3-chlorobenzamide 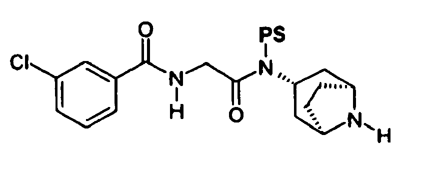
   The title compound was obtained from IRORI PS-allyl (3-endo)-3-(glycylamino)-8-azabicyclo[3.2.1]octane-8-carboxylate (Intermediate 19) (1.1 g, loading:1.3 mmol/g, 1.43 mmol) following the same procedure for the amide coupling and the allyloxycarbonyl (alloc) deprotection described above for example 57 but using 3-chlorobenzoic acid instead of 2-phenoxybenzoic acid.
b) 3-chloro-N-(2-((3-endo)-8-(3,4-dichlorobenzyl)-8-azabicyclo[3.2.1]octan-3-ylamino)-2-oxoethyl)benzamide was obtained from IRORI PS-N-(2-((3-endo)-8-azabicyclo[3.2.1]octan-3-ylamino)-2-oxoethyl)-3-chlorobenzamide (0.04 g, loading: 1.3 mmol/g, 0.052 mmol) following the same procedure for the reductive amination and for the cleavage from the resin described above for example 57 but using 3,4-dichlorobenzaldehyde instead of 4-chlorobenzaldehyde (0.019 g).

### Example 91 3-chloro-N-(2-((3-endo)-8-(4-fluorobenzyl)-8-azabicyclo[3.2.1]octan-3-ylamino)-2-oxoethyl)benzamide

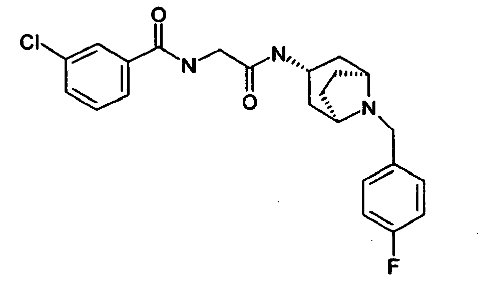

The title compound was obtained from IRORI PS-N-(2-((3-endo)-8-azabicyclo[3.2.1]octan-3-ylamino)-2-oxoethyl)-3-chlorobenzamide (Step a of Example 90) (0.04 g, loading: 1.3 mmol/g, 0.052 mmol) following the same procedure described above for example 90 but using 4-fluorobenzaldehyde instead of 3,4-dichlorobenzaldehyde (0.017g).

### Example 92 N-(2-((3-endo)-8-benzyl-8-azabicyclo[3.2.1]octan-3-ylamino)-2-oxoethyl)-3-chlorobenzamide

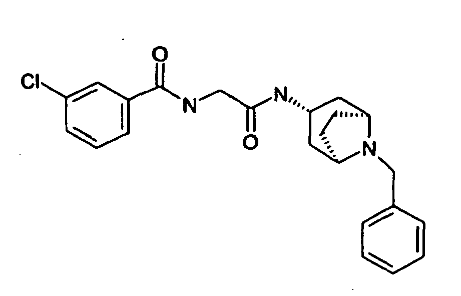

The title compound was obtained from IRORI PS-N-(2-((3-endo)-8-azabicyclo[3.2.1]octan-3-ylamino)-2-oxoethyl)-3-chlorobenzamide (Step a of example 90) (0.04 g, loading:1.3 mmol/g, 0.052 mmol) following the same procedure described above for example 90 but using benzaldehyde instead of 3,4-dichlorobenzaldehyde (0.017 g).

### Example 93 3-chloro-N-(2-((3-endo)-8-(2,4-dichlorobenzyl)-8-azabicyclo[3.2.1]octan-3-ylamino)-2-oxoethyl)benzamide

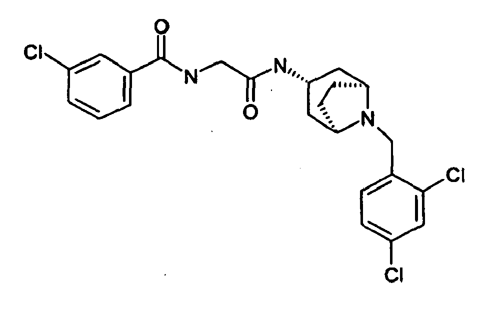

The title compound was obtained from IRORI PS-N-(2-((3-endo)-8-azabicyclo[3.2.1]octan-3-ylamino)-2-oxoethyl)-3-chlorobenzamide (Step a of example 90) (0.04 g, loading:1.3 mmol/g, 0.052 mmol) following the same procedure described above for example 90 but using 2,4-dichlorobenzaldehyde instead of 3,4-dichlorobenzaldehyde (0.018 g).

### Example 94 3-chloro-N-(2-((3-endo)-8-(1-(4-chlorophenyl)ethyl)-8-azabicyclo[3.2.1]octan-3-ylamino)-2-oxoethyl)benzamide

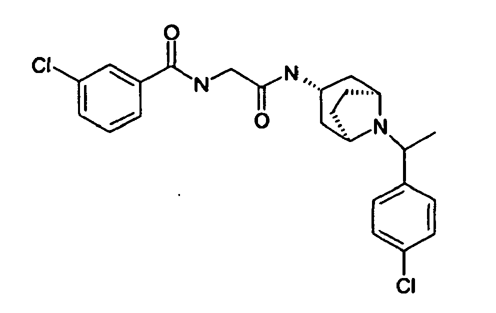

The title compound was obtained from IRORI PS-N-(2-((3-endo)-8-azabicyclo[3.2.1]octan-3-ylamino)-2-oxoethyl)-3-chlorobenzamide (Step a of example 90) (0.04 g, loading:1.3 mmol/g, 0.052 mmol) following the same procedure described above for example 90 but using 1-(4-chlorophenyl)ethanone instead of 3,4-dichlorobenzaldehyde. Reverse phase chromatography gave the pure title compound (0.003 g).

### Example 95 3-chloro-N-(2-((3-endo)-8-(1-(4-chlorophenyl)propyl)-8-azabicyclo[3.2.1]octan-3-ylamino)-2-oxoethyl)benzamide

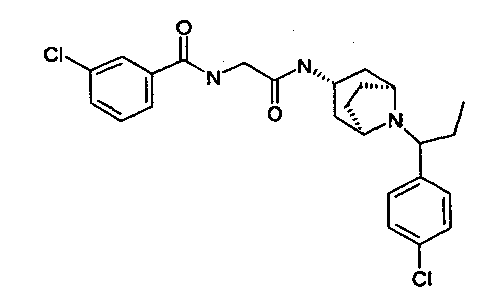

The title compound was obtained from IRORI PS-N-(2-((3-endo)-8-azabicyclo[3.2.1]octan-3-ylamino)-2-oxoethyl)-3-chlorobenzamide (Step a of example 90) (0.04 g, lading:1.3 mmol/g, 0.052 mmol) following the same procedure described above for example 90 but using 1-(4-chlorophenyl)propan-1-one instead of 3,4-dichlorobenzaldehyde. Reverse phase chromatography gave the pure title compound (0.002 g).

### Example 96 3-chloro-N-(2-oxo-2-((3-endo)-8-(3-phenylpropyl)-8-azabicyclo[3.2.1]octan-3-ylamino)ethyl)benzamide

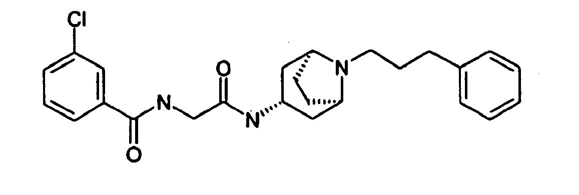

The title compound was obtained from IRORI PS-N-(2-((3-endo)-8-azabicyclo[3.2.1]octan-3-ylamino)-2-oxoethyl)-3-chlorobenzamide (Step a of example 90) (0.04 g, loading:1.3 mmol/g, 0.052 mmol) following the same procedure described above for example 90 but using 3-phenylpropanal instead of 3,4-dichlorobenzaldehyde (0.018 g).

### Example 97 N-(2-((3-endo)-8-(3,4-dichlorobenzyl)-8-azabicyclo[3.2.1]octan-3-ylamino)-2-oxoethy)-2-(3-(trifluoromethyl)phenylamino)benzamide

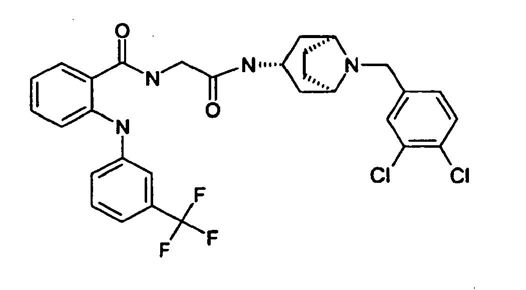

The title compound was obtained from PS-N-(2-((-endo)-8-azabicyclo[3.2.1]octan-3-ylamino)-2-oxoethyl)-2-(3-(trifluoromethyl)phenylamino)benzamide (Intermediate 29) (0.03 g, loading: 1 mmol/g, 0.03 mmol) following the same procedure described above for example 90 (0.007g).

### Example 98 N-(2-((3-endo)-8-(2,4-dichlorobenzyl)-8-azabicyclo[3.2.1]octan-3-ylamino)-2-oxoethyl)-2-(3-(trifluoromethyl)phenylamino)benzamide

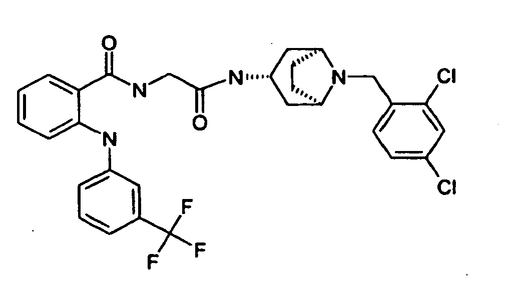

The title compound was obtained from PS-N-(2-((3-endo)-8-azabicyclo[3.2.1]octan-3-ylamino)-2-oxoethyl)-2-(3-(trifluoromethyl)phenylamino)benzamide (Intermediate 29) (0.03 g, loading:1 mmol/g, 0.03 mmol) following the same procedure described above for example 97 but using 2,4-dichlorobenzadehyde instead of 3,4-dichlorobenzaldehyde (0.008 g).

### Example 99 N-(2-((3-endo)-8-(4-methoxybenzyl)-8-azabicyclo[3.2.1]octan-3-ylamino)-2-oxoethyl)-2-(3-(trifluoromethyl)phenylamino)benzamide

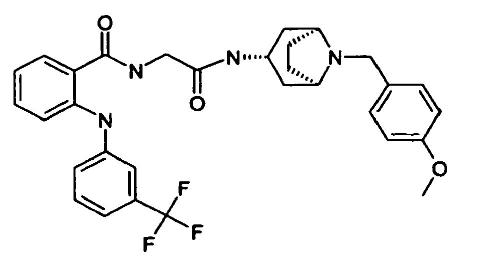

The title compound was obtained from PS-N-(2-((3-endo)-8-azabicyclo[3.2.1]octan-3-ylamino)-2-oxoethyl)-2-(3-(trifluoromethyl)phenylamino) (Intermediate 29) (0.03 g, loading:1 mmol/g, 0.03 mmol) following the same procedure described above for example 97 but using 4-methoxybenzadehyde instead of 3,4-dichlorobenzaldehyde (0.007 g).

### Example 100 N-(2-((3-endo)-8-(4-chlorobenzyl)-8-azabicyclo[3.2.1]octan-3-ylamino)-2-oxoethyl)-2-hydroxybenzamide

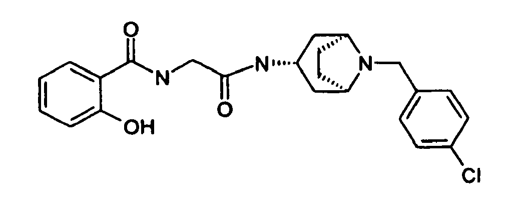

PS-allyl (3-endo)-3-(glycylamino)-8-azabicyclo[3.2.1]octane-8-carboxylate (Intermediate 19) (0.1 g, loading:1 mmol/g, 0.1 mmol) was washed and swelled with dichloromethane. A 0.5M solution of acetylsalicylic acid in dichloromethane/dimethylformamide 9:1 (2mL) was added. A 1.2M solution of N,N'-diisopropylcarbodiimide in dichloromethane/dimethylformamide 9:1 (1mL) was added. The mixture was stirred for 4 hours at 50°C. It was filtered and washed with dichloromethane. The resin was treated again with a 0.5M solution of acetylsalicylic acid in dichloromethane/dimethylformamide 9:1 (2mL) and a 1.2M solution of N,N'-diisopropylcarbodiimide in dichloromethane/dimethylformamide 9:1 (1mL). The mixture was stirred at room temperature overnight. It was filtered and washed with dimethylformamide (4X) and dichloromethane (4X). The resin was dried in vacuo.

The subsequent allyloxycarbonyl (alloc) deprotection of PS-allyl (3-endo)-3-({N-[2-(acetyloxy)benzoyl]glycyl}amino)-8-azabicyclo[3.2.1]octane-8-carboxylate, the reductive amination with 4-chlorobenzaldehide and the final cleavage were performed following the procedures described above for example 48. Reverse phase chromatography gave the title compound (0.012 g)

### Example 101 N-(2-((3-endo)-8-(4-chlorobenzyl)-8-azabicyclo[3.2.1]octan-3-ylamino)-2-oxoethyl)-2-(phenylamino)benzamide

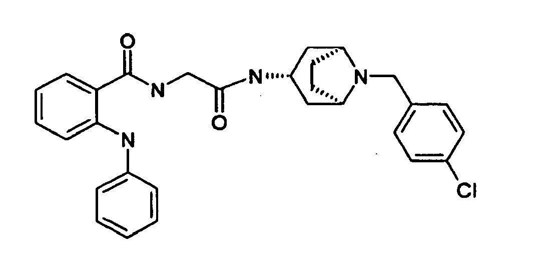

PS-allyl (3-endo)-3-(glycylamino)-8-azabicyclo[3.2.1]octane-8-carboxylate (Intermediate 19) (0.1 g, loading:1mmol/g, 0.1 mmol) was washed and swelled with dichloromethane. A 0.5M solution of N-phenylanthranilic acid in dichloromethane/dimethylformamide 9:1 (2mL) was added. A 1.2M solution of N,N'-diisopropylcarbodiimide in dichloromethane/dimethylformamide 9:1 (1mL) was added. The mixture was stirred for 4 hours at 50°C. It was filtered and washed with dichloromethane. The resin was treated again with a 0.5M solution of N-phenylanthranilic acid in dichloromethane/dimethylformamide 9:1 (2mL) and a 1.2M solution of N,N'-diisopropylcarbodiimide in dichloromethane/dimethylformamide 9:1 (1mL). The mixture was stirred at room temperature overnight. It was filtered and washed with dimethylformamide (4X) and dichloromethane (4X). The resin was dried in vacuo.

The subsequent allyloxycarbonyl (alloc) deprotection of PS-allyl (3-endo)-3-{[N-(2-anilinobenzoyl)glycyl]amino}-8-azabicyclo[3.2.1]octane-8-carboxylate the reductive amination with 4-chlorobenzaldehide and the final cleavage were performed following the procedures described above for example 48. Reverse phase chromatography gave the title compound (0.004g)

### Example 102 N-(2-((3-endo)-8-(4-chlorobenzyl)-8-azabicyclo[3.2.1]octan-3-ylamino)-2-oxoethyl)-9H-xanthene-9-carboxamide

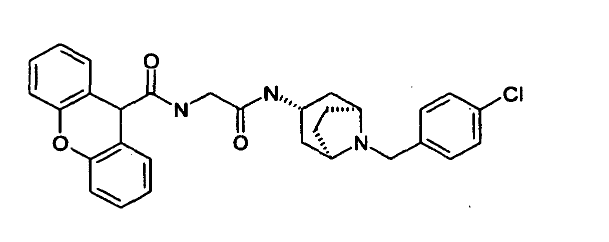

PS-allyl (3-endo)-3-(glycylamino)-8-azabicyclo[3.2.1]octane-8-carboxylate (Intermediate 19) (0.06 g, lading: 1mmol/g, 0.06 mmol) was washed and swelled with dichloromethane. A solution of 0.8 mmols of DIEA and 0.4 mmols of xanthene-9-carboxylic acid in dichloromethane/dimethylformamide 9:1 (1mL) was added and it was stirred at room temperature for 10 minutes. Then 0.4 mmols of O-(7-azabenzotriazol-1-yl)-N,N,N',N'-tetramethyluronium hexafluorophosphate (HATU) was added. The mixture was stirred for 2 hours at room temperature. It was filtered and washed with dichlorometane/dimethylformamide 9:1 (3X), dimethylformamide (3x) and dichloromethane (3X). The resin was dried in vacuo.

The subsequent allyloxycarbonyl (alloc) deprotection of PS-allyl (3-endo)-3-{[N-(9H-xanthen-9-ylcarbonyl)glycyl]amino}-8-azabicyclo[3.2.1]octane-8-carboxylate, the reductive amination with 4-chlorobenzaldehide and the final cleavage were performed following the procedures described above for example 48. Reverse phase chromatography gave the title compound (0.008 g).

### Example 103 2-(3,5-bis(trifluoromethyl)phenylsulfonamido)-N-((3-endo)-8-(4-chlorobenzyl)-8-azabicyclo[3.2.1]octan-3-yl)acetamide

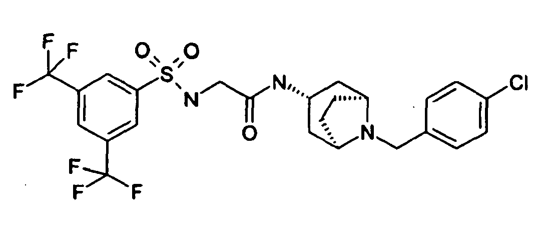

PS-allyl (3-endo)-3-(glycylamino)-8-azabicyclo[3.2.1]octane-8-carboxylate (Intermediate 19) (0.06 g, loading:1mmol/g, 0.06 mmol) was washed and swelled with dichloromethane. A solution of 0.8 mmols of DIEA in 0.5 mL of dichloromethane/dimethylformamide 9:1 and a solution of 0.4 mmols of 3,5-bis(trifluoromethyl)benzenesulfonyl chloride in dichloromethane/dimethylformamide 9:1 (1mL) were added. The mixture was stirred for 2 hours at room temperature. It was filtered and washed with dichlorometane/dimethylformamide 9:1 (3X), dimethylformamide (3x) and dichloromethane (3X). The resin was dried in vacuo.

The subsequent allyloxycarbonyl (alloc) deprotection of PS-allyl (3-endo)-3-[(N-{[3,5-bis(trifluoromethyl)phenyl]sulfonyl}glycyl)amino]-8-azabicyclo[3.2.1]octane-8-carboxylate the reductive amination with 4-chlorobenzaldehide and the final cleavage were performed following the procedures described above for example 48. Reverse phase chromatography gave the title compound (0.001 g).

The compounds of examples 104 to 108 have been obtained using synthetic method 8.

### Example 104 3-chloro-N-((S)-1-((3-endo)-8-(4-chlorobenzyl)-8-azabicyclo[3.2.1]octan-3-ylamino)-1-oxopronan-2-yl)benzamide

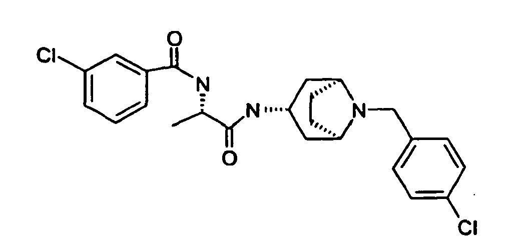

a)PS-(9H-fluoren-9-yl)methyl (S)-1-((3-endo)-8-(4-chlorobenzyl)-8-azabicyclo[3.2.1]octan-3-ylamino)-1-oxopropan-2-ylcarbamate 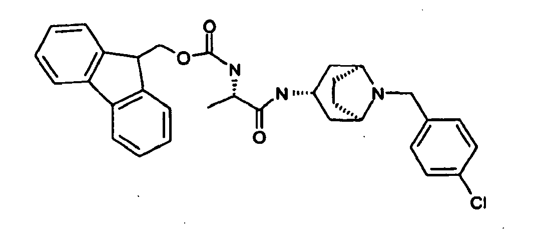
   IRORI PS-(3-endo)-8-(4-chlorobenzyl)-8-azabicyclo[3.2.1]octan-3-amine (Intermediate 11) (0.06 g, loading:1 mmol/g, 0.06 mmol) was washed and swelled with tetrahydrofuran. A 1.5M solution of N-[(9H-fluoren-9-ylmethoxy)carbonyl]-D-alanine in tetrahydrofuran (0.12mL, 0.18 mmols) and N,N'-diisopropylcarbodiimide (0.028 mL, 0.18 mmols) were added.
   To the mixture 0.5 mL more of tetrahydrofuran and 4 mL of perfluorodecaline were added.It was stirred for 12 hours at 50°C. It was filtered and washed with dimethylformamide (2X), dichloromethane (1X) and tetrahydrofuran (1X). The resin was treated again for a second time under the above conditions. It was filtered and washed with tetrahydrofuran (1X), dimethylformamide (3X) and dichloromethane (3X). The resin was dried in vacuo to give the title compound.
b)PS-N¹-[(3-endo)-8-(4-chlorobenzyl)-8-azabicyclo[3.2.1]oct-3-yl]-L-alaninamide 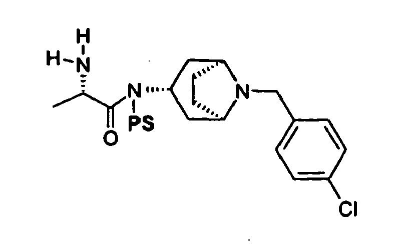
   The title compound was obtained from PS-(9H-fluoren-9-yl)methyl (S)-1-((3-endo)-8-(4-chlorobenzyl)-8-azabicyclo[3.2.1]octan-3-ylamino)-1-oxopropan-2-ylcarbamate following the same procedure described above for example 56 to perform the FMOC deprotection.
c) PS-3-chloro-N-((S)-1-((3-endo)-8-(4-chlorobenzyl)-8-azabicyclo[3.2.1]octan-3-ylamino)-1-oxopropan-2-yl)benzamide
   PS-N¹-[(3-endo)-8-(4-chlorobenzyl)-8-azabicyclo[3.2.1]oct-3-yl]-L-alaninamide (0.06 g, loading:1mmol/g, 0.06 mmol) was washed and swelled with dichloromethane. A 1.5M solution of 3-chlorobenzoic acid in dichloromethane/dimethylformamide 9:1 (0.12mL) and N,N'-diisopropylcarbodiimide (0.028 mL, 0.18 mmols) were added. To the mixture 5 mL of perfluorodecaline was added. It was stirred at room temperature overnight. It was filtered and washed with dichloromethane (1X), dimethylformamide (3X) and dichloromethane (2X). The resin was dried in vacuo to give the title compound.
d)Cleavage from the resin:
   The procedure described above for example 37 was followed to obtain the title compound (0.017 g).

### Example 105 3-chloro-N-(3-((3-endo)-8-(4-chlorobenzyl)-8-azabicyclo[3.2.1]octan-3-ylamino)-3-oxopropyl)benzamide

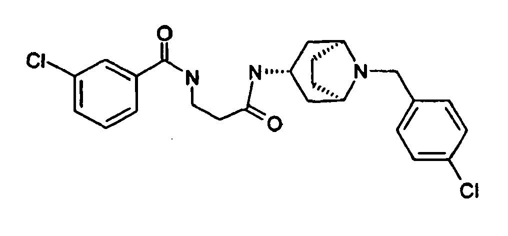

The title compound was obtained from IRORI PS-(3-endo)-8-(4-chlorobenzyl)-8-azabicyclo[3.2.1]octan-3-amine (Intermediate 11) (0.06 g, loading:1 mmol/g, 0.06 mmol) following the same procedure described above for example 104 but using N-[(9H-fluoren-9-ylmethoxy)carbonyl]-beta-alanine instead of N-[(9H-fluoren-9-ylmethoxy)carbonyl]-D-alanine (0.019 g).

### Example 106 (R)-3-(3-chlorobenzamido)-4-((3-endo)-8-(4-chlorobenzyl)-8-azabicyclo[3.2.1]octan-3-ylamino)-4-oxobutanoic acid

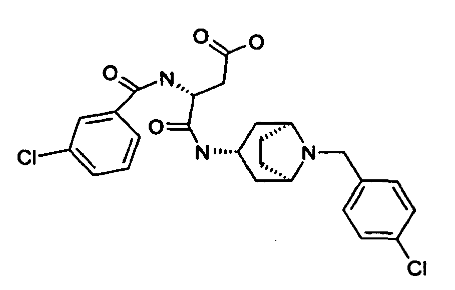

The title compound was obtained from IRORI PS-(3-endo)-8-(4-chlorobenzyl)-8-azabicyclo[3.2.1]octan-3-amine (Intermediate 11) (0.06 g, loading:1 mmol/g, 0.06 mmol) following the same procedure described above for example 104 but using (R)-2-(((9H-fluoren-9-yl)methoxy)carbonylamino)-4-tert-butoxy-4-oxobutanoic acid instead of N-[(9H-fluoren-9-ylmethoxy)carbonyl]-D-alanine (0.021g).

### Example 107 3-chloro-N-((R)-1-((3-endo)-8-(4-chlorobenzyl)-8-azabicyclo[3.2.1]octan-3-ylamino)-1-oxopropan-2-yl)benzamide

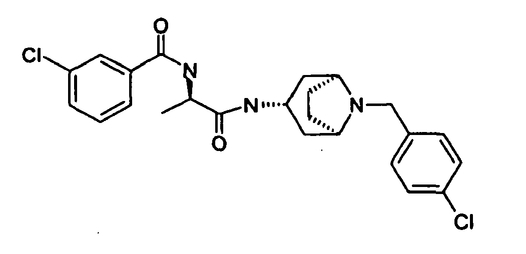

The title compound was obtained from IRORI PS-(3-endo)-8-(4-chlorobenzyl)-8-azabicyclo[3.2.1]octan-3-amine (Intermediate 11) (0,06 g, loading:1 mmol/g, 0.06 mmol) following the same procedure described above for example 104 but using N-[(9H-fluoren-9-ylmethoxy)carbonyl]-L-alanine instead of N-[(9H-fluoren-9-ylmethoxy)carbonyl]-D-alanine (0.020 g).

### Example 108 (R)-3-(3-chlorobenzamido)-4-((3-endo)-8-(4-chlorobenzyl)-8-azabicyclo[3.2.1]octan-3-ylamino)-4-oxobutanoic acid

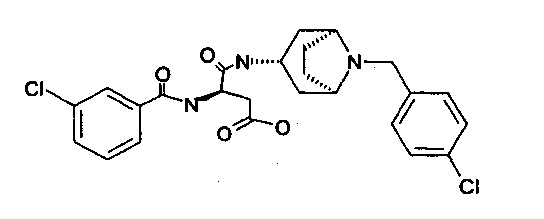

The title compound was obtained from IRORI PS-(3-endo)-8-(4-chlorobenzyl)-8-azabicyclo[3.2.1]octan-3-amine (Intermediate 11) (0.06 g, loading:1mmo)/g, 0.06 mmol) following the same procedure described above for example 104 but using (S)-2-(((9H-fluoren-9-yl)methoxy)carbonylamino)-4-tert-butoxy-4-oxobutanoic acid instead of N-[(9H-fluoren-9-ylmethoxy)carbonyl]-D-alanine (0.023 g).

The compounds of examples 109 to 113 have been obtained using synthetic method 9.

### Example 109 N-(2-((3-endo)-8-(4-fluorobenzyl)-8-azabicyclo[3.2.1]octan-3-ylamino)-2-oxoethyl)-2-(3-(trifluoromethyl)phenylamino)benzamide

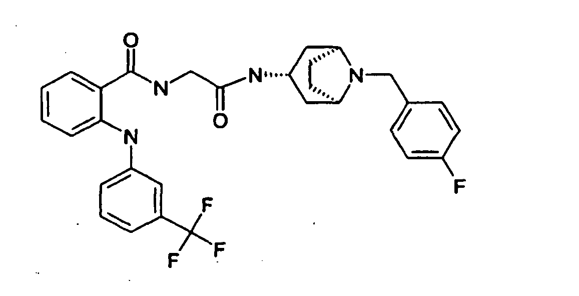

a)allyl(3-endo)-3-{[N-(2-{[3-(trifluoromethyl)phenyl]amino}benzoyl)glycyl]amino}-8-azabicyclo[3.2.1]octane-8-carboxylate 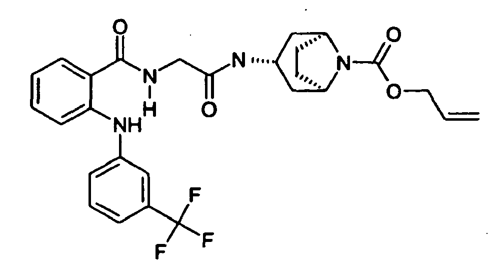
   2-{[3-(trifluoromethyl)phenyl]amino}benzoic acid (0.63 g, 2.2 mmols), 1-(3-dimethylaminopropyl)-3-ethylcarbodiimide hydrochloride (EDC.HCI) (0.43 g, 2.2 mmols) and 1-hydroxybenzotriazole hydrate (HOBt) (0.30 g, 2.2 mmols) were dissolved in dichloromethane (70 mL). It was stirred for 5 minutes at room temperature. A solution of allyl (3-endo)-3-(glycylamino)-8-azabicyclo[3.2.1]octane-8-carboxylate (Intermediate 19) (0.50 g, 1.9 mmols) in dichloromethane (10 mL) was added. The mixture was stirred for 12 hours at room temperature. It was washed with a saturated aqueous solution of potassium carbonate, a 10% aqueous solution of potassium hydrogen sulfate, a saturated aqueous solution of sodium hydrogen carbonate and brine. The organic phase was dried over sodium sulfate, filtered and concentrated in vacuo. Recristallization from diethyl eter gave the title compound (0.7 g) (yield: 70%)
b) N-(2-((3-endo)-8-azabicyclo[3.2.1]octan-3-ylamino)-2-oxoethyl)-2-(3-(trifluoromethyl)phenylamino)benzamide 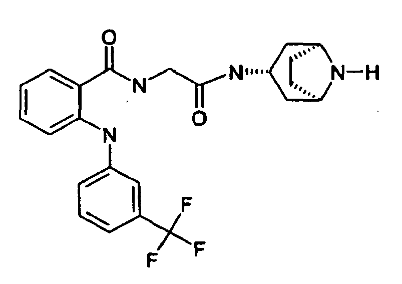
   Allyl (3-endo)-3-{[N-(2-{[3-(trifluoromethyl)phenyl]amino}benzoyl)glycyl]amino}-8-azabicyclo[3.2.1]octane-8-carboxylate (0.5 g, 0.9 mmols) was added to a 85% aqueous solution of potassium hydroxide (0.62 g, 9.4 mmols) and 4 mL of isopropyl alcohol. The mixture was stirred at reflux for 18 hours. The isopropyl alcohol was eliminated by concentration in vacuo and more water was added (20 mL). A 2N aqueous solution of HCl was added dropwise until an acidic pH was obtained. It was extreacted with dichloromethane and the aqueous phase was neutralized adding dropwise an aqueous solution of 2N sodium hydroxide. It was extracted with dichloromethane and the organic phase was dried over sodium sulfate, filtered and concentrated in vacuo to give the title compound (0.2 g) (yield: 48%)
c) 4-fluorobenzyl chloride (0.05 g, 0.3 mmols) was added to a solution of N-(2-((3-endo)-8-azabicyclo[3.2.1]octan-3-ylamino)-2-oxoethyl)-2-(3-(trifluoromethyl)phenylamino)benzamide (0.1 g, 0.22 mmols) and triethylamine (0.094mL) in dry acetone (3 mL). The mixture was stirred at reflux for 18 hours. It was concentrated in vacuo and the crude obtained was dissolved in dichloromethane. It was washed with a saturated aqueous solution of sodium hydrogen carbonate and the organic phase was dried over sodium sulfate, filtered and concentrated in vacuo. Chromatography on silica gel gave N-(2-((3-endo)-8-(4-fluorobenzyl)-8-azabicyclo[3.2.1]octan-3-ylamino)-2-oxoethyl)-2-(3-(trifluoromethyl)phenylamino)benzamide (0.05 g).

### Example 110 N-(2-((3-exo)-8-(4-chlorobenzyl)-8-azabicyclo[3.2.1]octan-3-ylamino)-2-oxoethyl)-2-(3-(trifluoromethyl)phenylamino)benzamide

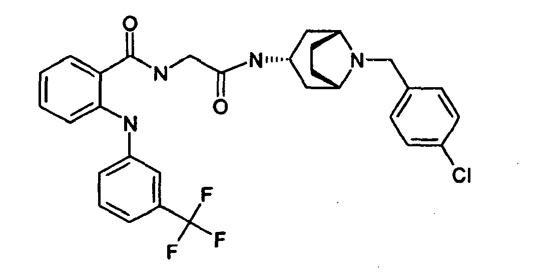

a)allyl (3-exo)-3-{[N-(2-{[3-(trifluoromethyl)phenyl]amino}benzoyl)glycyl]amino}8-azabicyclo[3.2.1]octane-8-carboxylate 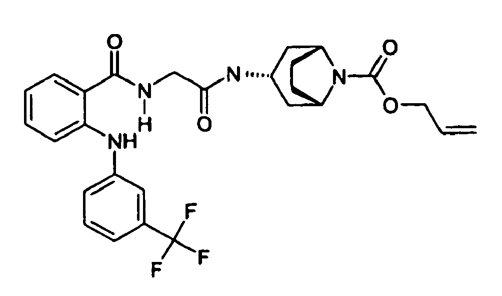
   2-{[3-(trifluoromethyl)phenyl]amino}benzoic acid (1.26 g, 4.5 mmols), 1-(3-dimethylaminopropyl)-3-ethylcarbodiimide hydrochloride (EDC.HCl) (0.60 g, 3.8 mmols) and 1-hydroxybenzotriazole hydrate (HOBt) (0.86 g, 6.4 mmols) were dissolved in dichloromethane (140 mL). It was stirred for 5 minutes at room temperature. A solution of allyl (3-exo)-3-(glycylamino)-8-azabicydo[3.2.1]octane-8-carboxylate (Intermediate 21) (1.0 g, 3.7 mmols) in dichloromethane (20 mL) was added. The mixture was stirred for 48 hours at room temperature. It was washed with a saturated aqueous solution of potassium carbonate, a 10% aqueous solution of potassium hydrogen sulfate, a saturated aqueous solution of sodium hydrogen carbonate and brine. The organic phase was dried over sodium sulfate, filtered and concentrated in vacuo to give the title compound (1.83 g) (yield: 92%)
b) N-(2-((3-exo)-8-azabicyclo[3.2.1]octan-3-ylamino)-2-oxoethyl)-2-(3-(trifluoromethyl)phenylamino)benzamide 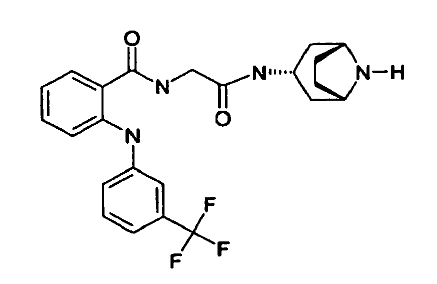
   Allyl (3-exo)-3-{[N-(2-{[3-(trifluoromethyl)phenyl]amino}benzoyl)glycyl]amino}-8-azabicyclo[3.2.1]octane-8-carboxylate (1.83 g, 3.5 mmols) was added to a 85% aqueous solution of potassium hydroxide (2.25 g, 34.1 mmols) and 15 mL of isopropyl alcohol. The mixture was stirred at reflux for 8 hours. The isopropyl alcohol was eliminated by concentration in vacuo and dichloromethane and water were added. The organic phase was washed with water, dried over sodium sulfate, filtered and concentrated in vacuo. The crude obtained was dissolved in ethyl acetate and it was washed with a 2N aqueous solution of HCl. The aqueous phase was neutralized by adding dropwise an aqueous solution of 2N sodium hydroxide. It was extracted with ethyl acetate and the organic phase was dried over magnesium sulfate, filtered and concentrated in vacuo to give the title compound (0.4 g) (yield: 26%)
c) 4-chlorobenzyl chloride (0.21g, 1.3 mmols) was added to a solution of N-(2-((3-exo)-8-azabicyclo[3.2.1]octan-3-ylamino)-2-oxoethyl)-2-(3-(trifluoromethyl)phenylamino)benzamide (0.4 g, 0.9 mmols) and triethylamine (0.37mL, 2.6 mmols) in dry acetone (15 mL). The mixture was stirred at reflux for 6 hours. It was concentrated in vacuo and the crude obtained was dissolved in dichloromethane. It was washed with a saturated aqueous solution of sodium hydrogen carbonate and the organic phase was dried over sodium sulfate, filtered and concentrated in vacuo. Chromatography on silica gel gave N-(2-((3-exo)-8-(4-chlorobenzyl)-8-azabicyclo[3.2.1]octan-3-ylamino)-2-oxoethyl)-2-(3-(trifluoromethyl)phenylamino)benzamide (0.27 g). (yield: 53%)

### Example 111 N-(2-((3-exo)-8-(4-chlorobenzyl)-8-azabicyclo[3.2.1]octan-3-ylamino)-2-oxoethyl)-3-(trifluoromethyl)benzamide

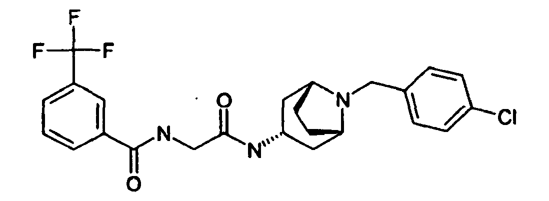

a)allyl (3-exo)-3-({N-[3-(trifluoromethyl)benzoyl]glycyl}amino)-8-azabicyclo[3.2.1]octane-8-carboxylate 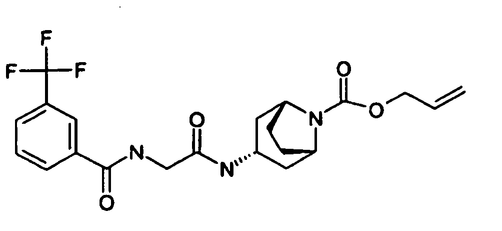
   3-(trifluoromethyl) benzoic acid (0.25 g, 1.3 mmols), 1-(3-dimethylaminopropyl)-3-ethylcarbodiimide hydrochloride (EDC.HCl) (0.17 g, 0.9 mmols) and 1-hydroxybenzotriazole hydrate (HOBt) (0.25 g, 1.9 mmols) were dissolved in dichloromethane (42 mL). It was stirred for 5 minutes at room temperature. Allyl (3-exo)-3-(glycylamino)-8-azabicyclo[3.2.1]octane-8-carboxylate (Intermediate 21) (0.3 g, 1.1 mmols) was added. The mixture was stirred for 2 hours at room temperature. It was washed with a saturated aqueous solution of potassium carbonate, a 10% aqueous solution of potassium hydrogen sulfate, a saturated aqueous solution of sodium hydrogen carbonate and brine. The organic phase was dried over sodium sulfate, filtered and concentrated in vacuo to give the title compound (0.5 g). (yield: 100%)
b) N-(2-((3-exo)-8-azabicyclo[3.2.1]octan-3-ylamino)-2-oxoethyl)-3-(trifluoromethyl)benzamide 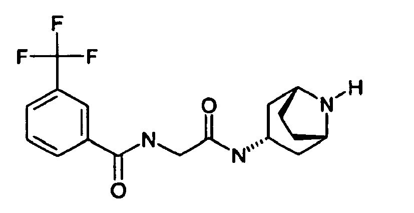
   Allyl (3-exo)-3-({N-[3-(trifluoromethyl)benzoyl]glycyl}amino)-8-azabicyclo[3.2.1]octane-8-carboxylate (0.50 g, 1.14 mmols) was added to a 85% aqueous solution of potassium hydroxide (0.05 g) and 3 mL of isopropyl alcohol. The mixture was stirred at reflux for 48 hours. 0.15 g more of 85% aqueous solution of potassium hydroxide and 15 mL of isopropyl alcohol were added and the mixture was stirred at reflux for 6 hours more.The isopropyl alcohol was eliminated by concentration in vacuo and dichloromethane and water were added. The organic phase was washed with water, dried over sodium sulfate, filtered and concentrated in vacuo to give the title compound (0.25 g) (yield: 62%).
c) 4-chlorobenzyl chloride (0.085 g, 0.53 mmols) was added to a solution of N-(2-((3-exo)-8-azabicyclo[3.2.1]octan-3-ylamino)-2-oxoethyl)-3-(trifluoromethyl)benzamide (0.125 g, 0.35 mmols) and triethylamine (0.15mL) in dry acetone (2 mL). The mixture was stirred at reflux for 12 hours. It was concentrated in vacuo and the crude obtained was dissolved in dichloromethane. It was washed with a saturated aqueous solution of sodium hydrogen carbonate and the organic phase was dried over sodium sulfate, filtered and concentrated in vacuo. Reverse phase chromatography gave N-(2-((3-exo)-8-(4-chlorobenzyl)-8-azabicyclo[3.2.1]octan-3-ylamino)-2-oxoethyl)-3-(trifluoromethyl)benzamide (0.01 g). (yield: 6%).

### Example 112 3-chloro-N-(2-((3-exo)-8-(4-chlorobenzyl)-8-azabicyclo[3.2.1]octan-3-ylamino)-2-oxoethyl)benzamide

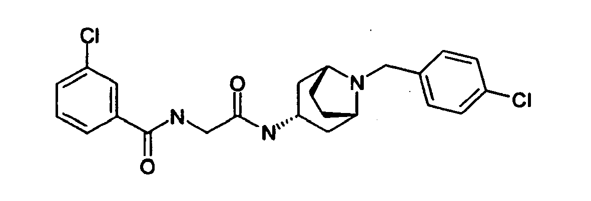

a)allyl(3-exo)-3-{[N-(3-chlorobenzoyl)glycyl]amino}-8-azabicyclo[3.2.1]octane-8-carboxylate 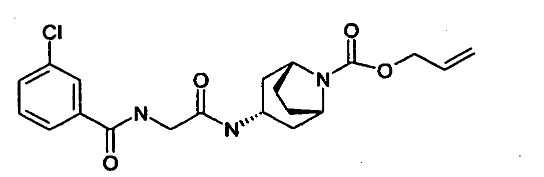
   3-chlorobenzoic acid (0.21 g, 1.3 mmols), 1-(3-dimethylaminopropyl)-3-ethylcarbodiimide hydrochloride (EDC.HCl) (0.17 g, 0.9 mmols) and 1-hydroxybenzotriazole hydrate (HOBt) (0.25 g, 1.9 mmols) were dissolved in dichloromethane (42 mL). It was stirred for 5 minutes at room temperature. Allyl (3-exo)-3-(glycylamino)-8-azabicyclo[3.2.1]octane-8-carboxylate (Intermediate 21) (0.3 g, 1.1 mmols) was added. The mixture was stirred for 2 hours at room temperature. It was washed with a saturated aqueous solution of potassium carbonate, a 10% aqueous solution of potassium hydrogen sulfate, a saturated aqueous solution of sodium hydrogen carbonate and brine. The organic phase was dried over sodium sulfate, filtered and concentrated in vacuo to give the title compound (0.33 g). (yield: 74%).
b) N-(2-((3-exo)-8-azabicyclo[3.2.1]octan-3-ylamino)-2-oxoethyl)-3-chlorobenzamide 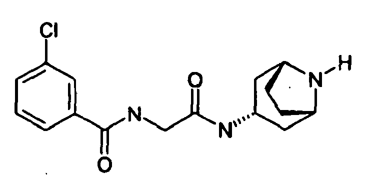 Allyl (3-exo)-3-{[N-(3-chlorobenzoyl)glycyl]amino}-8-azabicyclo[3.2.1]octane-8-carboxylate (0.33 g, 0.81 mmols) was added to a 85% aqueous solution of potassium hydroxide (0.05 g) and 3 mL of isopropyl alcohol. The mixture was stirred at reflux for 48 hours. 0.15 g more of 85% aqueous solution of potassium hydroxide and 15 mL of isopropyl alcohol were added and the mixture was stirred at reflux for 6 hours more.The isopropyl alcohol was eliminated by concentration in vacuo and dichloromethane and water were added. The organic phase was washed with water, dried over sodium sulfate, filtered and concentrated in vacuo to give the title compound (0.15 g). (yield: 58%).
c) 4-chlorobenzyl chloride (0.04 g, 0.25 mmols) was added to a solution of N-(2-((3-exo)-8-azabicyclo[3.2.1]octan-3-ylamino)-2-oxoethyl)-3-chlorobenzamide (0.150 g, 0.47 mmols) and triethylamine (0.065mL) in dry acetone (2 mL). The mixture was stirred at reflux for 2 hours. It was concentrated in vacuo and the crude obtained was dissolved in dichloromethane. It was washed with a saturated aqueous solution of sodium hydrogen carbonate and the organic phase was dried over sodium sulfate, filtered and concentrated in vacuo. Reverse phase chromatography gave 3-chloro-N-(2-((3-exo)-8-(4-chlorobenzyl)-8-azabicyclo[3.2.1]octan-3-ylamino)-2-oxoethyl)benzamide (0.007 g). (yield: 3% ).

### Example 113 N-(2-((3-endo)-8-(4-chlorobenzyl)-8-azabicyclo[3.2.1]octan-3-ylamino)-2-oxoethyl)-2-(3-(trifluoromethyl)phenylamino)benzamide

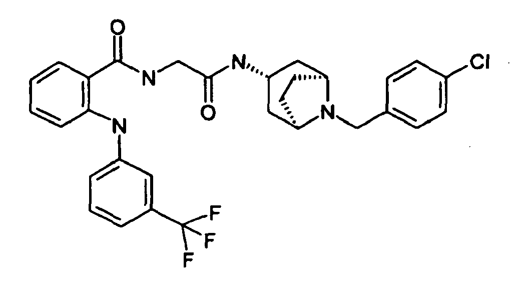

4-chlorobenzyl chloride (0.5 g, 3.1 mmols) was added to a solution of N-(2-((3-endo)-8-azabicyclo[3.2.1]octan-3-ylamino)-2-oxoethyl)-2-(3-(trifluoromethyl)phenylamino)benzamide (Intermediate 29) (0.84 g, 1.9 mmols) and triethylamine (0.87mL, 6.2 mmols) in dry acetone (20 mL). The mixture was stirred at reflux for 8 hours. It was concentrated in vacuo and the crude obtained was dissolved in dichloromethane. It was washed with a saturated aqueous solution of sodium hydrogen carbonate and the organic phase was dried over sodium sulfate, filtered and concentrated in vacuo. Chromatography on silica gel gave the title compound (0.55 g). (yield: 51%).

### PHARMACOLOGICAL ACTIVITY

### BINDING ASSAY

The binding assay was performed in 96-well microtiter plates (Costar, catalog number 3604) using a total volume of 200µl of an assay buffer composed of 50mM HEPES, pH 7.4, 5mM MgCl2, 1 mM CaCl2, 0.2% BSA, and a cocktail of protease inhibitors (Roche, Ref. 1697498).

Binding reactions were performed by incubating 2µg of membranes containing the human CCR1 receptor (Perkin Elmer, RBHCC1M) with 15pM of recombinant human [125I]-MIP1a (Amersham Biosciences, Ref. IM285, 2200 Ci/mmol) in the absence or in the presence of various concentrations of the test compound together with 1 mg/well of Wheatgerm Agglutinin SPA beads (Amersham Biosciences, RPNQ0001). Binding was allowed to proceed at room temperature for 1 hour on an orbital shaker. Bound radioactivity was determined on a Wallac Trilux Microbeta Counter. Specific binding was determined using 100µM of cold MIP1a.

All of the compounds of the invention that were tested had IC₅₀ of less than 10 µM, in CCR1 binding assay.

The N-amide derivatives of 8-azabicyclo[3.2.1]oct-3-yl of the invention are useful in the treatment or prevention of diseases known to be susceptible to improvement by treatment with an antagonist of the CCR1 receptor. Such diseases are, for example arthritis, allergic asthma, pulmonary fibrosis, respiratory virus infection, glomerulonephritis, renal transplant rejection, sepsis, atherosclerosis, multiple sclerosis, Alzheimer's disease and heart transplant rejection.

Accordingly, the N-amide derivatives of 8-azabicyclo[3.2.1]oct-3-yl of the invention and pharmaceutically acceptable salts thereof, and pharmaceutical compositions comprising such compound and/or salts thereof, may be used in a method of treatment of disorders of the human body which comprises administering to a subject requiring such treatment an effective amount of N-amide derivative of 8-azabicyclo[3.2.1]oct-3-yl of the invention or a pharmaceutically acceptable salt thereof.

The compounds of the present invention are also of benefit when administered in combination with other drugs such as oral DMARDs (Disease Modifying AntiRheumatic Drugs) such as methotrexate, leflunomide, sulfasalazine, hydroxicloroquine, azathioprine and gold salts; antagonists of the chemokine receptors CCR2 to CCR10, CXCR1 to CXCR6, XCR1, XCR2 or CX3CR1; biologicals such as anti-TNF monoclonal antibodies (Humira, Remicade), soluble antagonists of TNF receptors (Enbrel), Abatacept (CTLA-4-Ig), anti-CD20 antibodies such as Rituximab, anti-IL-6R antibodies such as Atlizumab or anti-IL-12 antibodies; small molecules that inhibit targets involved in cytokine synthesis such as PDE4 inhibitors, p38 MAPK inhibitors, MAPKAP-K2 inhibitors or P2X7 receptor antagonists; interferon beta receptor ligands; and glatiramer acetate.

The present invention also provides pharmaceutical compositions which comprise, as an active ingredient, at least an N-amide derivative of 8-azabicyclo[3.2.1]oct-3-yl of formula (I) or a pharmaceutically acceptable salt thereof in association with a pharmaceutically acceptable excipient such as a carrier or diluent. The active ingredient may comprise 0.001% to 99% by weight, preferably 0.01% to 90% by weight of the composition depending upon the nature of the formulation and whether further dilution is to be made prior to application. Preferably the compositions are made up in a form suitable for oral, topical, nasal, rectal, percutaneous or injectable administration.

The pharmaceutically acceptable excipients which are admixed with the active compound, or salts of such compound, to form the compositions of this invention are well-known *per* se and the actual excipients used depend *inter alia* on the intended method of administering the compositions.

Compositions of this invention are preferably adapted for injectable and *per* os administration. In this case, the compositions for oral administration may take the form of tablets, retard tablets, sublingual tablets, capsules, inhalation aerosols, inhalation solutions, dry powder inhalation, or liquid preparations, such as mixtures, elixirs, syrups or suspensions, all containing the compound of the invention; such preparations may be made by methods well-known in the art.

The diluents which may be used in the preparation of the compositions include those liquid and solid diluents which are compatible with the active ingredient, together with colouring or flavouring agents, if desired. Tablets or capsules may conveniently contain between 2 and 500 mg of active ingredient or the equivalent amount of a salt thereof.

The liquid composition adapted for oral use may be in the form of solutions or suspensions. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent.

Compositions for parenteral injection may be prepared from soluble salts, which may or may not be freeze-dried and which may be dissolved in pyrogen free aqueous media or other appropriate parenteral injection fluid.

Effective doses are normally in the range of 2-2000 mg of active ingredient per day. Daily dosage may be administered in one or more treatments, preferably from 1 to 4 treatments, per day.

The present invention will be further illustrated by the following examples. The examples are given by way of illustration only and are not to be construed as a limiting.

The syntheses of the compounds of the invention and of the intermediates for use therein are illustrated by the following Examples (1 to 113) including the preparation of intermediates 1 to 51 which do not limit the scope of the invention in any way.

**General. Reagents,** starting materials, and solvents were purchased from commercial suppliers and used as received. Concentration refers to evaporation under vacuum using a Büchi rotatory evaporator. Reaction products were purified, when necessary, by flash chromatography on silica gel (40-63 µm) with the solvent system indicated. Spectroscopic data were recorded on a Varian Gemini 200 spectrometer, Varian Gemini 300 spectrometer, Varian Inova 400 spectrometer and Brucker DPX-250 spectrometer. Melting points were recorded on a Büchi 535 apparatus. HPLC-MS were performed on a Gilson instrument equipped with a Gilson piston pump 321, a Gilson 864 vacuum degasser, a Gilson liquid handler 215, a Gilson 189 injection module, a Gilson Valvemate 7000, a 1/1000 splitter, a Gilson 307 make-up pump, a Gilson 170 diode array detector, and a Thermoquest Finnigan aQa detector. Semi-preparative purifications were carried out using a Symmetry C18 reverse phase column (100 Δ, 5 µm, 19 x 100 mm, purchased from WATERS), and water/ammonium formiate (0,1%, pH=3) and acetonitrile/ammonium formiate (0,1%, pH=3) as mobile phase.

The following table show the retention times of the compounds of example 1 to 113 under the conditions described in the previous paragraph.

| **Example** | **Retention time (min)** |
|---|---|
| 1 | 9,6 |
| 2 | 9,7 |
| 3 | 6,6 |
| 4 | 6,6 |
| 5 | 5,5 |
| 6 | 5,8 |
| 7 | 6,3 |
| 8 | 6,5 |
| 9 | 6,2 |
| 10 | 7 |
| 11 | 5,8 |
| 12 | 9,6 |
| 13 | 5,9 |
| 14 | 6,2 |
| 15 | 7,2 |
| 16 | 4,9 |
| 17 | 6,7 |
| 18 | 6,7 |
| 19 | 5,3 |
| 20 | 6,2 |
| 21 | 6,7 |
| 22 | 6,2 |
| 23 | 6,8 |
| 24 | 5,9 |
| 25 | 6,6 |
| 26 | 6,6 |
| 27 | 6,7 |
| 28 | 6,1 |
| 29 | 5,3 |
| 30 | 6 |
| 31 | 6,4 |
| 32 | 6,1 |
| 33 | 4,9 |
| 34 | 4,6 |
| 35 | 6,2 |
| 36 | 6,6 |
| 37 | 6,4 |
| 38 | 7,4 |
| 39 | 7,7 |
| 40 | 6,7 |
| 41 | 5,2 |
| 42 | 5,3 |
| 43 | 5,6 |
| 44 | 7,6 |
| 45 | 7 |
| 46 | 4,4 |
| 47 | 6,1 |
| 48 | 6,3 |
| 49 | 8,2 |
| 50 | 9,6 |
| 51 | 10,2 |
| 52 | 7,1 |
| 53 | 7,5 |
| 54 | 7,9 |
| 55 | 5,3 |
| 56 | 6,9 |
| 57 | 6,8 |
| 58 | 7,1 |
| 59 | 7,2 |
| 60 | 6,9 |
| 61 | 5 |
| 62 | 7,3 |
| 63 | 8 |
| 64 | 6,1 |
| 65 | 7,9 |
| 66 | 7,9 |
| 67 | 6 |
| 68 | 6,3 |
| 69 | 5,5 |
| 70 | 6,1 |
| 71 | 6,7 |
| 72 | 5,3 |
| 73 | 5,8 |
| 74 | 6 |
| 75 | 5,6 |
| 76 | 6,1 |
| 77 | 5,2 |
| 78 | 6,6 |
| 79 | 6,2 |
| 80 | 6,2 |
| 81 | 8,1 |
| 82 | 6,3 |
| 83 | 8,1 |
| 84 | 6,2 |
| 85 | 6 |
| 86 | 8,8 |
| 87 | 8,5 |
| 88 | 8,1 |
| 89 | 8,8 |
| 90 | 6,5 |
| 91 | 5,6 |
| 92 | 5,4 |
| 93 | 6,5 |
| 94 | 6,7 |
| 95 | 6,7 |
| 96 | 6,2 |
| 97 | 8,4 |
| 98 | 8,5 |
| 99 | 7,8 |
| 100 | 5,6 |
| 101 | 7,4 |
| 102 | 6,7 |
| 103 | 7,3 |
| 104 | 6,4 |
| 105 | 6,2 |
| 106 | 6,5 |
| 107 | 6,3 |
| 108 | 6,5 |
| 109 | 7,8 |
| 110 | 14,8 |
| 111 | 6,3 |
| 112 | 5,7 |
| 113 | 15 |

## Claims

1. A compound of formula (I) 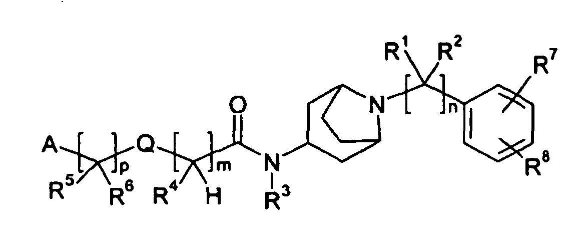
in the fom of the free base, a pharmaceutically acceptable salt, or a zwitterionic form thereof wherein
• n is an integer from 1 to 4
• m is an integer from 1 to 3
• p is an integer from 0 to 2
• R¹, R² and R³ each independently represent a hydrogen atom or a straight or branched C₁₋₄ alkyl group
• Q represents a direct bond or a group selected from -CONR⁹-, -OCONR⁹-, - NR⁹CONR¹⁰-, -NR⁹-, -NR⁹COO-, -O- , -SO₂NR⁹- and -NR⁹CO
• R⁹ and R¹⁰ each independently represent a hydrogen atom or a straight or branched C₁₋₄ alkyl group
• R⁴ represents a hydrogen atom or a group selected from straight or branched C₁₋₄ alkyl which is unsubstitued or substituted with a group selected from -COOH, phenyl or -SCH₃ with the proviso that when m is 2 or 3, only one R⁴ is as defined above and the rest of R⁴ are hydrogen atoms
• R⁵ and R⁶ each independently represent a hydrogen atom, a straight or branched C₁₋₄ alkyl or a phenyl group, or
• R⁵ and R⁶ together with the carbon atom to which they are attached form a C₃₋₈ cycloalkyl group
• R⁷ and R⁸ each independently represent a hydrogen or halogen atom or a C₁₋₄ alkoxy group
• A represents a group of formula (i) or (ii) 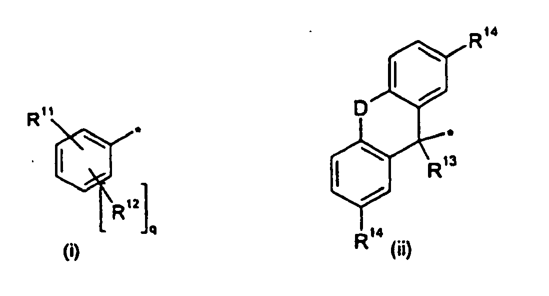
• D represents a direct bond or a group selected from -O- and -CH₂CH₂
• R¹¹ represents a hydrogen or halogen atom or a group selected from (a) straight or branched C₁₋₄ alkyl which is unsubstituted or substituted by a hydroxy, cyano, phenyl or a group -CONRaRb wherein Ra and Rb are independently selected from hydrogen atoms and C₁₋₄ alkyl groups, (b) hydroxy, phenoxy or straight or branched C₁₋₄ alkoxy, (c) nitro, (d) hydroxycarbonyl or straight or branched C₁₋₄ alkoxycarbonyl, (e) a group -NRcRd wherein Rc represents a hydrogen atom or a straight or branched C₁₋₄ alkyl and Rd represents a hydrogen atom, a straight or branched C₁₋₄ alkyl or a phenyl group which is optionally substituted by one or more groups selected from -CF₃, and halogen atoms, (f) a group - NHCORe wherein Re represents a group selected from -NH₂, straight or branched C₁₋₄ alkyl which is unsubstituted or substituted by one or more fluorine atoms or - NH₂, straight or branched C₂₋₄ alkenyl and 3-piperidinyl groups, (g) a group - CONRcRf wherein Rc is as hereinabove defined and Rf is a group selected from hydrogen atoms, straight or branched C₁₋₄ alkyl groups and 4-piperidinyl groups or Rc and Rf form together with the nitrogen atom to which they are attached a piperidinyl group which may be unsubstituted or substituted by a group selected from -NH₂, -CONH₂-, -CH₂NH₂ and -CH₂NHCOCF₃, (h) a group -NHSO₂Rg wherein Rg is selected from the group comprising straight or branched C₁₋₄ alkyl and C₁₋₄ alkylphenyl groups and (i) a guanidino group;
• R¹² each independently represent a cyano, straight or branched C₁₋₄ alkyl, straight or branched C₁₋₄ alkoxy, a halogen atom or a group -CF₃
• or R¹¹ and one of R¹² form a methanediol group
• q is an integer from 0 to 2
• R¹³ represents a hydrogen atom or a straight or branched C₁₋₄ alkyl
• R¹⁴ each independently represent a hydrogen or halogen atom, with the proviso that the compound is not 2-(4-fluorophenyl)-N-(8-benzyl-8-azabicyclo[3.2.1]-oct-3-yl)acetamide.

2. A compound according to claim 1 wherein Q represents a group selected from - CONR⁹-; -O- and NR⁹CO

3. A compound according to any preceding claim wherein n is 1.

4. A compound according to any preceding claim wherein R³ is a hydrogen atom or a methyl group.

5. A compound according to any preceding claim wherein R¹ each independently represent a hydrogen atom and R² is selected from hydrogen atoms and methyl groups.

6. A compound according to any preceding claim wherein R⁹ is a hydrogen atom.

7. A compound according to any preceding claim wherein R⁴ each independently represent a hydrogen atom or a methyl group.

8. A compound according to any preceding claim wherein R⁵ and R⁶ each independently represent a hydrogen atom or a phenyl group.

9. A compound according to any preceding claim wherein A represents a group of formula (i) and R¹¹ represents a hydrogen or halogen atom or a group selected from
(a) methyl which is substituted by a -CONRaRb group wherein Ra and Rb are independently selected from hydrogen atoms and C₁₋₄ alkyl groups, (b) hydroxyl or phenoxy, (c) nitro, (d) methoxycarbonyl, (e) a group -NHRd wherein Rd represents a hydrogen atom, a methyl group or a phenyl group which substituted by one or more groups selected from -CF₃, and halogen atoms, (f) a group -NHCORe wherein Re represents a group selected from -NH₂, -CF₃ and straight C₁₋₄ alkyl which is unsubstituted or substituted by a -NH₂ group, (g) a group -CONHRf wherein Rf is a group selected from hydrogen atoms, methyl and 4-piperidinyl groups or Rc and Rf form together with the nitrogen atom to which they are attached a piperidinyl group which may be unsubstituted or substituted by a group selected from -NH₂, -CONH₂ and -CH₂NH₂, (h) a group -NHSO₂R_{g} wherein Rg is selected from the group comprising methyl and methylphenyl groups and (i) a guanidino group.

10. A compound according to any preceding claim wherein Q represents an oxygen atom, A represents a group of formula (i), q is 1 or 2 and R¹¹ represents a group selected from (a) methyl which is substituted by a -CONRaRb group wherein Ra and Rb are independently selected from hydrogen atoms and straight or branched C₁₋₄ alkyl groups, (e) a group -NRcRd wherein Rc represents a hydrogen atom or a straight or branched C₁₋₄ alkyl and Rd represents a hydrogen atom, a straight or branched C₁₋₄ alkyl or a phenyl group which is optionally substituted by one or more groups selected from -CF₃, and halogen atoms, (f) a group - NHCORe wherein Re represents a group selected from -NH₂, straight or branched C₁₋₄ alkyl which is unsubstituted or substituted by one or more fluorine atoms or - NH₂, straight or branched C₂₋₄ alkenyl and 3-piperidinyl groups, (g) a group - CONRcRf wherein Rc is as hereinabove defined and Rf is a group selected from hydrogen atoms, straight or branched C₁₋₄ alkyl groups and 4-piperidinyl groups or Rc and Rf form together with the nitrogen atom to which they are attached a piperidinyl group which may be unsubstituted or substituted by a group selected from -NH₂, -CONH₂, -CH₂NH₂ and -CH₂NHCOCF₃, (h) a group -NHSO₂Rg wherein Rg is selected from the group comprising straight or branched C₁₋₄ alkyl and C₁₋₄ alkylphenyl groups and (i) a guanidino group.

11. A compound according to any preceding claim wherein R¹² each independently represent a halogen atom or a group -CF₃.

12. A compound according to any preceding claim wherein q is an integer from 0 to 1.

13. A compound according to any one of claims 1 to 8 wherein A represents a group of formula (ii) and R¹³ represents a hydrogen atom or a methyl group.

14. A compound according to any one of claims 1 to 8 or 13 wherein A represents a group of formula (ii) and R¹⁴ represents a hydrogen or chlorine atom.

15. A compound according to any preceding claim wherein the aminotropine has an endo configuration.

16. A compound according to any preceding claim which is one of:
5-chloro-2-(3-((3-endo)-8-(4-chlorobenzyl)-8-azabicyclo[3.2.1]octan-3-ylamino)-3-oxopropoxy)-N-methylbenzamide
3-(4-chloro-2-ureidophenoxy)-N-((3-endo)-8-(4-chlorobenzyl)-8-azabicyclo[3.2.1]octan-3-yl)propanamide
5-chloro-2-(4-((3-endo)-8-(4-chlorobenzyl)-8-azabicyclo[3.2.1]octan-3-ylamino)-4-oxobutoxy)-N-methylbenzamide
N¹-((3-endo)-8-(4-chlorobenzyl)-8-azabicyclo[3.2.1]octan-3-yl)-N3-(3-chlorophenyl)malonamide
2-(4-chloro-2-ureidophenoxy)-N-((3-endo)-8-(4-fluorobenzyl)-8-azabicyclo[3.2.1]octan-3-yl)acetamide
5-chloro-2-(2-((3-endo)-8-(4-chlorobenzyl)-8-azabicyclo[3.2.1]octan-3-ylamino)-2-oxoethoxy)-N-methylbenzamide
2-(4-bromo-2-ureidophenoxy)-N-((3-endo)-8-(4-chlorobenzyl)-8-azabicyclo[3.2.1]octan-3-yl)acetamide
2-(5-chloro-2-(2-((3-endo)-8-(4-chlorobenzyl)-8-azabicyclo[3.2.1]octan-3-ylamino)-2-oxoethoxy)phenyl)-N-methylacetamide
2-(2-(2-amino-2-oxoethyl)-4-chlorophenoxy)-N-((3-endo)-8-(4-chlorobenzyl)-8-azabicyclo[3.2.1]octan-3-yl)acetamide
2-(5-chloro-2-(2-((3-endo)-8-(4-chlorobenzyl)-8-azabicyclo[3.2.1]octan-3-ylamino)-2-oxoethoxy)phenyl)-N,N-dimethylacetamide
N-((3-endo)-8-(4-chlorobenzyl)-8-azabicyclo[3.2.1]octan-3-yl)-2-(4-fluoro-2-ureidophenoxy)acetamide
2-(4-chloro-2-ureidophenoxy)-N-((3-endo)-8-(4-chlorobenzyl)-8-azabicyclo[3.2.1]octan-3-yl)acetamide
5-chloro-2-(2-((3-endo)-8-(4-chlorobenzyl)-8-azabicyclo[3.2.1]octan-3-ylamino)-2-oxoethoxy)benzamide
2-(2-acetamido-4-chlorophenoxy)-N-((3-endo)-8-(4-chlorobenzyl)-8-azabicyclo[3.2.1]octan-3-yl)acetamide
N-((1-(5-chloro-2-(2-((3-endo)-8-(4-chlorobenzyl)-8-azabicyclo[3.2.1]octan-3-ylamino)-2-oxoethoxy)benzoyl)piperidin-3-yl)methyl)-2,2,2-trifluoroacetamide
2-(2-(3-(aminomethyl)piperidine-1-carbonyl)-4-chlorophenoxy)-N-((3-endo)-8-(4-chlorobenzyl)-8-azabicyclo[3.2.1]octan-3-yl)acetamide
N-((3-endo)-8-(4-chlorobenzyl)-8-azabicyclo[3.2.1]octan-3-yl)-2-(4-chlorophenylamino)acetamide
N-((3-endo)-8-(4-chlorobenzyl)-8-azabicyclo[3.2.1]octan-3-yl)-2-(3-chlorophenylamino)acetamide
N-((3-endo)-8-(4-chlorobenzyl)-8-azabicyclo[3.2.1]octan-3-yl)-2-(2-(hydroxymethyl)phenoxy)acetamide
N-((3-endo)-8-(4-chlorobenzyl)-8-azabicyclo[3.2.1]octan-3-yl)-2-(4-methyl-2-(methylamino)phenoxy)acetamide
N-((3-endo)-8-(4-chlorobenzyl)-8-azabicyclo[3.2.1]octan-3-yl)-2-(4-chlorophenoxy)acetamide
N-(2-(2-((3-endo)-8-(4-chlorobenzyl)-8-azabicyclo[3.2.1]octan-3-ylamino)-2-oxoethoxy)-5-methylphenyl)acrylamide
N-((3-endo)-8-(4-chlorobenzyl)-8-azabicyclo[3.2.1]octan-3-yl)-2-(4-methyl-2-nitrophenoxy)acetamide
N-((3-endo)-8-(4-chlorobenzyl)-8-azabicyclo[3.2.1]octan-3-yl)-2-(3-cyanophenoxy)acetamide
N-((3-endo)-8-(4-chlorobenzyl)-8-azabicyclo[3.2.1]octan-3-yl)-2-(3-chlorophenoxy)acetamide
N-((3-endo)-8-(4-chlorobenzyl)-8-azabicyclo[3.2.1]octan-3-yl)-2-(4-fluoro-2-nitrophenoxy)acetamide methyl 4-acetamido-5-chloro-2-(2-((3-endo)-8-(4-chlorobenzyl)-8-azabicyclo[3.2.1]octan-3-ylamino)-2-oxoethoxy)benzoate
N-((3-endo)-8-(4-chlorobenzyl)-8-azabicyclo[3.2.1]octan-3-yl)-2-(2-cyanophenoxy)acetamide
2-(2-((3-endo)-8-(4-chlorobenzyl)-8-azabicyclo[3.2.1]octan-3-ylamino)-2-oxoethoxy)benzamide
2-(benzo[d][1,3]dioxol-5-yloxy)-N-((3-endo)-8-(4-chlorobenzyl)-8-azabicyclo[3.2.1]octan-3-yl)acetamide
N-((3-endo)-8-(4-chlorobenzyl)-8-aza-bicyclo[3.2.1]octan-3-yl)-2-(2-nitrophenoxy)acetamide
N-((3-endo)-8-(4-chlorobenzyl)-8-azabicyclo[3.2.1]octan-3-yl)-2-(2-methoxyphenoxy)acetamide
5-chloro-2-(2-((3-endo)-8-(4-chlorobenzyl)-8-azabicyclo[3.2.1]octan-3-ylamino)-2-oxoethoxy)-N-(piperidin-4-yl)benzamide
2-(2-(4-aminopiperidine-1-carbonyl)-4-chlorophenoxy)-N-((3-endo)-8-(4-chlorobenzyl)-8-azabicyclo[3.2.1]octan-3-yl)acetamide
1-(5-chloro-2-(2-((3-endo)-8-(4-chlorobenzyl)-8-azabicyclo[3.2.1]octan-3-ylamino)-2-oxoethoxy)benzoyl)piperidine-3-carboxamide
5-chloro-2-(2-((3-endo)-8-(4-chlorobenzyl)-8-azabicyclo[3.2.1]octan-3-ylamino)-2-oxoethoxy)-N,N-dimethylbenzamide
5-chloro-2-(2-((3-endo)-8-(4-chlorobenzyl)-8-azabicyclo[3.2.1]octan-3-ylamino)-2-oxoethoxy)benzoic acid
2-(4-chloro-2-nitrophenoxy)-N-((3-endo)-8-(4-chlorobenzyl)-8-azabicyclo[3.2.1]octan-3-yl)acetamide
methyl 5-chloro-2-(2-((3-endo)-8-(4-chlorobenzyl)-8-azabicyclo[3.2.1]octan-3-ylamino)-2-oxoethoxy)benzoate
2-(2-amino-4-chlorophenoxy)-N-((3-endo)-8-(4-chlorobenzyl)-8-azabicyclo[3.2.1]octan-3-yl)ecetamide
2-amino-N-(5-chloro-2-(2-((3-endo)-8-(4-chlorobenzyl)-8-azabicyclo[3.2.1]octan-3-ylamino)-2-oxoethoxy)phenyl)acetamide
3-amino-N-(5-chloro-2-(2-((3-endo)-8-(4-chlorobenzyl)-8-azabicyclo[3.2.1]octan-3-ylamino)-2-oxoethoxy)phenyl)propanamide
N-(5-chloro-2-(2-((3-endo)-8-(4-chlorobenzyl)-8-azabicyclo[3.2.1]octan-3-ylamino)-2-oxoethoxy)phenyl)piperidine-3-carboxamide
N-(5-chloro-2-(2-((3-endo)-8-(4-chlorobenzyl)-8-azabicyclo[3.2.1]octan-3-ylamino)-2-oxoethoxy)phenyl)-2,2,2-trifluoroacetamide
2-(4-chloro-2-(methylsulfonamido)phenoxy)-N-((3-endo)-8-(4-chlorobenzyl)-8-azabicyclo[3.2.1]octan-3-yl)acetamide
2-(4-chloro-2-guanidinophenoxy)-N-((3-endo)-8-(4-chlorobenzyl)-8-azabicyclo[3.2.1]octan-3-yl)acetamide
2-(4-chloro-2-ureidophenoxy)-N-((3-exo)-8-(4-chlorobenzyl)-8-azabicyclo[3.2.1]octan-3-yi)acetamide
N-((3-endo)-8-(4-chlorobenzyl)-8-azabicyclo[3.2.1]octan-3-yl)-2-(3-chlorophenyl)acetamide
N-((3-endo)-8-(4-chlorobenzyl)-8-azabicyclo[3.2.1]octan-3-yl)-2-(2-(2,6-dichlorophenylamino)phenyl)acetamide
3-chloro-N-(2-((3-endo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)-3-(trifluoromethyl)benzamide
N-(2-((3-endo)-8-(4-chlorobenzyl)-8-azabicyclo[3.2.1]octan-3-ylamino)-2-oxoethyl)-2-(4-methylphenylsulfonamido)benzamide
N-(2-((3-endo)-8-(4-chlorobenzyl)-8-azabicyclo[3.2.1]octan-3-ylamino)-2-oxoethyl)-1-(4-chlorophenyl)cyclopentanecarboxamide
2,7-dichloro-N-(2-((3-endo)-8-(4-chlorobenzyl)-8-azabicyclo[3.2.1]octan-3-ylamino)-2-oxoethyl)-9H-xanthene-9-carboxamide
5-chloro-N-(2-((3-endo)-8-(4-chlorobenzyl)-8-azabicyclo[3.2.1]octan-3-ylamino)-2-oxoethyl)-2-ureidobenzamide
N-((3-endo)-8-(4-chlorobenzyl)-8-azabicyclo[3.2.1]octan-3-yl)-2-(3-(3-chlorophenyl)ureido)acetamide
N-(2-((3-endo)-8-(4-chlorobenzyl)-8-azabicyclo[3.2.1]octan-3-ylamino)-2-oxoethyl)-2-phenoxybenzamide
N-(2-(((3-endo)-8-((4-chlorobenzyl)-8-azabicyclo[3.2.1]oct-3-yl)amino)-2-oxoethyl)-10,11-dihydro-5H-dibenzo[a,d]cycloheptene-5-carboxamide
N-(2-((3-endo)-8-(4-chlorobenzyl)-8-azabicyclo[3.2.1]octan-3-ylamino)-2-oxoethyl)-9-methyl-9H-fluorene-9-carboxamide
N-(2-((3-endo)-8-(4-chlorobenzyl)-8-azabicyclo[3.2.1]octan-3-ylamino)-2-oxoethyl)-2,2-diphenylacetamide
2-amino-N-(2-((3-endo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)-9-methyl-9H-xanthene-9-carboxamide
N-((3-endo)-8-(4-chlorobenzyl)-8-azabicyclo[3.2.1]octan-3-yl)-2-(2-(2-(2,6-dichlorophenylamino)phenyl)acetamido)acetamide
N-((3-endo)-8-(4-chlorobenzyl)-8-azabicyclo[3.2.1]octan-3-yl)-2-(2-(3-chlorophenyl)acetamido)acetamide
N-(3-((3-endo)-8-(4-chlorobenzyl)-8-azabicyclo[3.2.1]octan-3-ylamino)-3-oxopropyl)-2-(3-(trifluoromethyl)phenylamino)benzamide
N-(2-((3-endo)-8-(4-chlorobenzyl)-8-azabicyclo[3.2.1]octan-3-ylamino)-2-oxoethyl)-N-methyl-2-(3-(trifluoromethyl)phenylamino)benzamide
3-bromo-N-(2-((3-endo)-8-(4-chlorobenzyl)-8-azabicyclo[3.2.1]octan-3-ylamino)-2-oxoethyl)benzamide
5-chloro-N-(2-((3-endo)-8-(4-chlorobenzyl)-8-azabicyclo[3.2.1]octan-3-ylamino)-2-oxoethyl)-2-methoxybenzamide
N-(2-((3-endo)-8-(4-chlorobenzyl)-8-azabicyclo[3.2.1]octan-3-ylamino)-2-oxoethyl)-3-fluorobenzamide
5-chloro-N-(2-((3-endo)-8-(4-chlorobenzyl)-8-azabicyclo[3.2.1]octan-3-ylamino)-2-oxoethyl)-2-methoxy-4-(methylamino)benzamide
3,4-dichloro-N-(2-((3-endo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)-3-methylbenzamide
2,5-dichloro-N-(2-((3-endo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)-3-methoxybenzamide
N-(2-((3-endo)-8-(4-chlorobenzyl)-8-azabicyclo[3.2.1]octan-3-ylamino)-2-oxoethyl)-3-(hydroxy(phenyl)methyl)benzamide
N-(2-((3-endo)-8-(4-chlorobenzyl)-8-azabicyclo[3.2.1]octan-3-ylamino)-2-oxoethyl)-3-(cyanomethyl)benzamide
5-chloro-N-(2-((3-endo)-8-(4-chlorobenzyl)-8-azabicyclo[3.2.1]octan-3-ylamino)-2-oxoethyl)-4-(dimethylamino)-2-methoxybenzamide
3-chloro-N-(3-((3-exo)-8-(4-chlorobenzyl)-8-azabicyclo[3.2.1]octan-3-ylamino)-3-oxopropyl)benzamide
3-chloro-N-((R)-1-((3-exo)-8-(4-chlorobenzyl)-8-azabicyclo[3.2.1]octan-3-ylamino)-1-oxopropan-2-yl)benzamide
N-(2-((3-exo)-8-(4-chlorobenzyl)-8-azabicyclo[3.2.1]octan-3-ylamino)-2-oxoethyl)-N-methyl-2-(3-(trifluoromethyl)phenylamino)benzamide
(R)-3-(3-chlorobenzamido)-4-((3-exo)-8-(4-chlorobenzyl)-8-azabicyclo[3.2.1]octan-3-ylamino)-4-oxobutanoic acid
N-((S)-1-((3-exo)-8-(4-chlorobenzyl)-8-azabicyclo[3.2.1]octan-3-ylamino)-1-oxopropan-2-yl)-2-(3-(trifluoromethyl)phenylamino)benzamide
3-chloro-N-((S)-1-((3-exo)-8-(4-chlorobenzyl)-8-azabicyclo[3.2.1]octan-3-ylamino)-1-oxopropan-2-yl)benzamide
3-chloro-N-(2-((3-exo)₋8-(4-chlorobenzyl)-8-azabicyclo[3.2.1]octan-3-ylamino)-2-oxoethyl)-N-methylbenzamide
N-((S)-1-((3-exo)-8-(4-chlorobenzyl)-8-azabicyclo[3.2.1]octan-3-ylamino)-1-oxo-3-phenylpropan-2-yl)-2-(3-(trifluoromethyl)phenylamino)benzamide
N-((S)-1-((3-exo)-8-(4-chlorobenryl)-8-azabicyclo[3.2.1]octan-3-ylamino)-4-(methylthio)-1-oxobutan-2-yl)-2-(3-(trifluoromethyl)phenylamino)benzamide
N-(3-((3-exo)-8-(4-chlorobenzyl)-8-azabicyclo[3.2.1]octan-3-ylamino)-3-oxopropyl)-2-(3-(trifluoromethyl)phenylamino)benzamide
N-((S)-1-((3-exo)-8-(4-chlorobenzyl)-8-azabicyclo[3.2.1]octan-3-ylamino)-4-methyl-1-oxopentan-2-yl)-2-(3-(trifluoromethyl)phenylamino)benzamide
3-chloro-N-(2-((3-endo)-8-(3,4-dichlorobenzyl)-8-azabicyclo[3.2.1]octan-3-ylamino)-2-oxoethyl)benzamide
3-chloro-N-(2-((3-endo)-8-(4-fluorobenzyl)-8-azabicyclo[3.2.1]octan-3-ylamino)-2-oxoethyl)benzamide
N-(2-((3-endo)-8-benzyl-8-azabicyclo[3.2.1]octan-3-ylamino)-2-oxoethyl)-3-chlorobenzamide
3-chloro-N-(2-((3-endo)-8-(2,4-dichlorobenzyl)-8-azabicyclo[3.2.1]octan-3-ylamino)-2-oxoethyl)benzamide
3-chloro-N-(2-((3-endo)-8-(1-(4-chlorophenyl)ethyl)-8-azabicyclo[3.2.1]octan-3-ylamino)-2-oxoethyl)benzamide
3-chloro-N-(2-((3-endo)-8-(1-(4-chlorophenyl)propyl)-8-azabicyclo[3.2.1]octan-3-ylamino)-2-oxoethyl)benzamide
3-chloro-N-(2-oxo-2-((3-endo)-8-(3-phenylpropyl)-8-azabicyclo[3.2.1]octan-3-ylamino)ethyl)benzamide
N-(2-((3-endo)-8-(3,4-dichlorobenzyl)-8-azabicyclo[3.2.1]octan-3-ylamino)-2-oxoethyl)-2-(3-(trifluoromethyl)phenylamino)benzamide
N-(2-((3-endo)-8-(2,4-dichlorobenzyl)-8-azabicyclo[3.2.1]octan-3-ylamino)-2-oxoethyl)-2-(3-(trifluoromethyl)phenylamino)benzamide
N-(2-((3-endo)-8-(4-methoxybenzyl)-8-azabicyclo[3.2.1]octan-3-ylamino)-2-oxoethyl)-2-(3-(trifluoromethyl)phenylamino)benzamide
N-(2-((3-endo)-8-(4-chlorobenzyl)-8-azabicyclo[3.2.1]octan-3-ylamino)-2-oxoethyl)-2-hydroxybenzamide
N-(2-((3-endo)-8-(4-chlorobenzyl)-8-azabicyclo[3.2.1]octan-3-ylamino)-2-oxoethyl)-2-(phenylamino)benzamide
N-(2-((3-endo)-8-(4-chlorobenzyl)-8-azabicyclo[3.2.1]octan-3-ylamino)-2-oxoethyl)-9H-xanthene-9-carboxamide
2-(3,5-bis(trifluoromethyl)phenylsulfonamido)-N-((3-endo)-8-(4-chlorobenzyl)-8-azabicyclo[3.2.1]octan-3-yl)acetamide
3-chloro-N-((S)-1-((3-endo)-8-(4-chlorobenzyl)-8-azabicyclo[3.2.1]octan-3-ylamino)-1-oxopropan-2-yl)benzamide
3-chloro-N-(3-((3-endo)-8-(4-chlorobenzyl)-8-azabicyclo[3.2.1]octan-3-ylamino)-3-oxopropyl)benzamide
(R)-3-(3-chlorobenzamido)-4-((3-endo)-8-(4-chlorobenzyl)-8-azabicyclo[3.2.1]octan-3-ylamino)-4-oxobutanoic acid
3-chloro-N-((R)-1-((3-endo)-8-(4-chlorobenzyl)-8-azabicyclo[3.2.1]octan-3-ylamino)-1-oxopropan-2-yl)benzamide
(R)-3-(3-chlorobenzamido)-4-((3-endo)-8-(4-chlorobenzyl)-8-azabicyclo[3.2.1]octan-3-ylamino)-4-oxobutanoic acid
N-(2-((3-endo)-8-(4-fluorobenzyl)-8-azabicyclo[3.2.1]octan-3-ylamino)-2-oxoethyl)-2-(3-(trifluoromethyl)phenylamino)benzamide
N-(2-((3-exo)-8-(4-chlorobenzyl)-8-azabicyclo[3.2.1]octan-3-ylamino)-2-oxoethyl)-2-(3-(trifluoromethyl)phenylamino)benzamide
N-(2-((3-exo)-8-(4-chlorobenzyl)-8-azabicyclo[3.2.1]octan-3-ylamino)-2-oxoethyl)-3-(trifluoromethyl)benzamide
3-chloro-N-(2-((3-exo)-8-(4-chlorobenzyl)-8-azabicyclo[3.2.1]octan-3-ylamino)-2-oxoethyl)benzamide
N-(2-((3-endo)-8-(4-chlorobenzyl)-8-azabicyclo[3.2.1]octan-3-ylamino)-2-oxoethyl)-2-(3-(trifluoromethyl)phenylamino)benzamide

17. A compound according to any one of claims 1 to 16 for use in the treatment of a pathological condition or disease susceptible to amelioration by antagonism of the CCR1 receptor.

18. A compound according to any one of claims 1 to 16 for use in the treatment of a pathological condition or disease wherein the pathological condition or disease is rheumatoid arthritis, allergic asthma, pulmonary fibrosis, respiratory virus infection, glomerulonephritis, renal transplant rejection, sepsis, atherosclerosis, systemic lupus erythematosus (SLE), multiple sclerosis, Alzheimer's disease or heart transplant rejection.

19. A pharmaceutical composition comprising a compound as defined in any one of claims 1 to 16 in admixture with a pharmaceutically acceptable diluent or carrier.

20. Use of a compound as defined in any one of claims 1 to 16 in the manufacture of a medicament for the treatment of a pathological condition for disease susceptible of being improved by antagonism of the CCR1 receptor.

21. Use of a compound according to claim 20 wherein the pathological condition or disease is rheumatoid arthritis, allergic asthma, pulmonary fibrosis, respiratory virus infection, glomerulonephritis, renal transplant rejection, sepsis, atherosclerosis, systemic lupus erythematosus (SLE), multiple sclerosis, Alzheimer's disease or heart transplant rejection.

22. A combination product comprising a compound according to any one of claims 1 to 16; and another compound selected from (a) methotrexate, laflunomide, (c) sulfasalazine, (d) hydroxychloroquine, (e) azathioprine, (f) gold salts, (h) antagonists of the chemokine receptors CCR2 to CCR10, (i) antagonists of the chemokine receptors CXCR1 to CXCR6, (j) antagonists of the chemokine receptors XCR1, XCR2 or CX3CR1, (k) anti-TNF monoclonal antibodies, (I) soluble antagonists of TNF receptors, (m) Abatacept, (n) anti-CD20 antibodies, (o) anti-IL-6R antibodies, (p) PDE4 inhibitors, (q) p38 MAPK inhibitors, (r) MAPKAP-K2 inhibitors, (a) P2X7 receptor antagonists, (t) interferon beta receptor ligands, (u) glatiramer acetate and (v) anti-IL-12 antibodies for simultaneous, separate or sequential use.

## Patentansprüche

1. Verbindung von Formel (I) 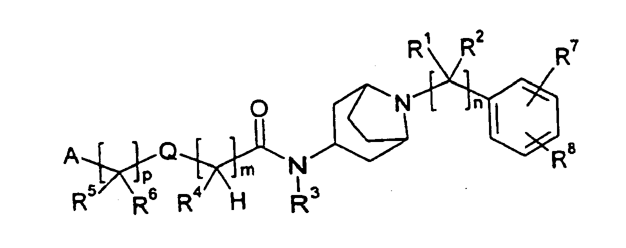 in der Form der freien Base, eines pharmazeutisch verträglichen Salzes oder einer zwitterionischen Form davon, worin
• n eine ganze Zahl von 1 bis 4 ist
• m eine ganze Zahl von 1 bis 3 ist
• p eine ganze Zahl von 0 bis 2 ist
• R¹, R² und R³ jeweils unabhängig für ein Wasserstoffatom oder eine gerade oder verzweigte C₁₋₄-Alkylgruppe stehen
• Q für eine direkte Bindung oder eine Gruppe steht, die ausgewählt ist aus -CONR⁹-, -OCONR⁹-, - NR⁹CONR¹⁰-, -NR⁹-, -NR⁹COO-, -O- , -SO₂NR⁹- und -NR⁹CO
• R⁹ und R¹⁰ jeweils unabhängig für ein Wasserstoffatom oder eine gerade oder verzweigte C₁₋₄-Alkylgruppe stehen
• R⁴ für ein Wasserstoffatom oder eine Gruppe steht, die ausgewählt ist aus geradem oder verzweigtem C₁₋₄-Alkyl, das unsubstituiert oder mit einer Gruppe substituiert ist, die ausgewählt ist aus -COOH, Phenyl oder -SCH₃, mit der Maßgabe, dass, wenn m 2 oder 3 ist, nur ein R⁴ ist, wie oben definiert, und der Rest von R⁴ Wasserstoffatome sind
• R⁵ und R⁶ jeweils unabhängig für ein Wasserstoffatom, ein gerades oder verzweigtes C₁₋₄-Alkyl oder eine Phenylgruppe stehen oder
• R⁵ und R⁶ zusammen mit dem Kohlenstoffatom, an das sie gebunden sind, eine C₃₋₈-Cycloalkylgruppe bilden
• R⁷ und R⁸ jeweils unabhängig für ein Wasserstoff- oder Halogenatom oder eine C₁₋₄-Alkoxygruppe stehen
• A für eine Gruppe von Formel (i) oder (ii) steht 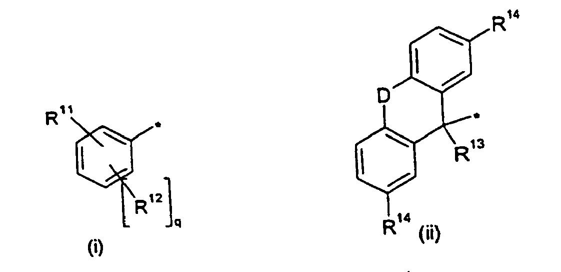
• D für eine direkte Bindung oder eine Gruppe steht, die ausgewählt ist aus -O-und -CH₂CH₂
• R¹¹ für ein Wasserstoff- oder Halogenatom oder eine Gruppe steht, die ausgewählt ist aus **(a)** geradem oder verzweigtem C₁₋₄-Alkyl, das unsubstituiert oder mit einem Hydroxy, Cyano, Phenyl oder einer Gruppe -CONRaRb substituiert ist, wobei Ra und Rb unabhängig ausgewählt sind aus Wasserstoffatomen und C₁₋₄-Alkylgruppen, **(b)** Hydroxy, Phenoxy oder geradem oder verzweigtem C₁₋₄-Alkoxy, **(c)** Nitro, **(d)** Hydroxycarbonyl oder geradem oder verzweigtem C₁₋₄-Alkoxycarbonyl, **(e)** einer Gruppe -NRcRd, wobei Rc für ein Wasserstoffatom oder ein gerades oder verzweigtes C₁₋₄-Alkyl steht und Rd für ein Wasserstoffatom, ein gerades oder verzweigtes C₁₋₄-Alkyl oder eine Phenylgruppe steht, die gegebenenfalls mit einer oder mehreren Gruppen substituiert ist, die ausgewählt sind aus -CF₃ und Halogenatomen, **(f)** einer Gruppe -NHCORe, wobei Re für eine Gruppe steht, die ausgewählt ist aus -NH₂, geradem oder verzweigtem C₁₋₄-Alkyl, das unsubstituiert oder mit einem oder mehreren Fluoratomen oder -NH₂ substituiert ist, geradem oder verzweigtem C₂₋₄-Alkenyl und 3-Piperidinylgruppen, **(g)** einer Gruppe -CONRcRf, wobei Rc ist, wie hierin zuvor definiert, und Rf eine Gruppe ist, die ausgewählt ist aus Wasserstoffatomen, geraden oder verzweigten C₁₋₄-Alkylgruppen und 4-Piperidinylgruppen, oder Rc und Rf zusammen mit dem Stickstoffatom, an das sie gebunden sind, eine Piperidinylgruppe bilden, die unsubstituiert oder mit einer Gruppe substituiert sein kann, die ausgewählt ist aus -NH₂, -CONH₂-, -CH₂NH₂ und -CH₂NHCOCF₃, **(h)** einer Gruppe -NHSO₂R_{g}, wobei Rg ausgewählt ist aus der Gruppe, die gerade oder verzweigte C₁₋₄-Alkyl- und C₁₋₄-Alkylphenylgruppen umfasst, und **(i)** einer Guanidinogruppe;
• R¹² jeweils unabhängig für ein Cyano, gerades oder verzweigtes C₁₋₄-Alkyl, gerades oder verzweigtes C₁₋₄-Alkoxy, ein Halogenatom oder eine Gruppe-CF₃ steht,
• oder R¹¹ und eines von R¹² eine Methandiolgruppe bilden
• q eine ganze Zahl von 0 bis 2 ist
• R¹³ für ein Wasserstoffatom oder ein gerades oder verzweigtes C₁₋₄-Alkyl steht
• R¹⁴ jeweils unabhängig für ein Wasserstoff- oder Halogenatom stehen, mit der Maßgabe, dass die Verbindung nicht 2-(4-Fluorphenyl)-N-(8-benzyl-8-azabicyclo[3.2.1]-oct-3-yl)acetamid ist.

2. Verbindung nach Anspruch 1, wobei Q für eine Gruppe steht, die ausgewählt ist aus -CONR⁹-, -O- und -NR⁹CO-.

3. Verbindung nach einem vorangehenden Anspruch, wobei n 1 ist.

4. Verbindung nach einem vorangehenden Anspruch, wobei R³ ein Wasserstoffatom oder eine Methylgruppe ist.

5. Verbindung nach einem vorangehenden Anspruch, wobei R¹ jeweils unabhängig für ein Wasserstoffatom stehen und R² ausgewählt ist aus Wasserstoffatomen und Methylgruppen.

6. Verbindung nach einem vorangehenden Anspruch, wobei R⁹ ein Wasserstoffatom ist.

7. Verbindung nach einem vorangehenden Anspruch, wobei R⁴ jeweils unabhängig für ein Wasserstoffatom oder eine Methylgruppe stehen.

8. Verbindung nach einem vorangehenden Anspruch, wobei R⁵ und R⁶ jeweils unabhängig für ein Wasserstoffatom oder eine Phenylgruppe stehen.

9. Verbindung nach einem vorangehenden Anspruch, wobei A für eine Gruppe von Formel (i) steht und R¹¹ für ein Wasserstoff- oder Halogenatom oder eine Gruppe steht, die ausgewählt ist aus **(a)** Methyl, das mit einer -CONRaRb-Gruppe substituiert ist, wobei Ra und Rb jeweils unabhängig ausgewählt sind aus Wasserstoffatomen und C₁₋₄-Alkylgruppen, **(b)** Hydroxyl oder Phenoxy, **(c)** Nitro, **(d)** Methoxycarbonyl, **(e)** einer Gruppe -NHRd, wobei Rd für ein Wasserstoffatom, eine Methylgruppe oder eine Phenylgruppe steht, die mit einer oder mehreren Gruppen substituiert ist, die ausgewählt sind aus -CF₃ und Halogenatomen, **(f)** einer Gruppe -NHCORe, wobei Re für eine Gruppe steht, die ausgewählt ist aus -NH₂, -CF₃ und geradem C₁₋₄-Alkyl, das unsubstituiert oder mit einer -NH₂-Gruppe substituiert ist, **(g)** einer Gruppe -CONHRf, wobei Rf eine Gruppe ist, die ausgewählt ist aus Wasserstoffatomen, Methyl und 4-Piperidinylgruppen, oder Rc und Rf zusammen mit dem Stickstoffatom, an das sie gebunden sind, eine Piperidinylgruppe bilden, die unsubstituiert oder mit einer Gruppe substituiert sein kann, die ausgewählt ist aus -NH₂, -CONH₂ und -CH₂NH₂, **(h)** einer Gruppe -NHSO₂Rg, wobei Rg ausgewählt ist aus der Gruppe, die Methyl- und Methylphenylgruppen umfasst, und **(i)** einer Guanidinogruppe.

10. Verbindung nach einem vorangehenden Anspruch, wobei Q für ein Sauerstoffatom steht, A für eine Gruppe von Formel (i) steht, q 1 oder 2 ist und R¹¹ für eine Gruppe steht, die ausgewählt ist aus **(a)** Methyl, das mit einer -CONRaRb-Gruppe substituiert ist, wobei Ra und Rb unabhängig ausgewählt sind aus Wasserstoffatomen und geraden oder verzweigten C₁₋₄-Alkylgruppen, **(e)** einer Gruppe -NRcRd, wobei Rc für ein Wasserstoffatom oder ein gerades oder verzweigtes C₁₋₄-Alkyl steht und Rd für ein Wasserstoffatom, ein gerades oder verzweigtes C₁₋₄-Alkyl oder eine Phenylgruppe steht, die gegebenenfalls mit einer oder mehreren Gruppen substituiert ist, die ausgewählt sind aus -CF₃ und Halogenatomen, **(f)** einer Gruppe -NHCORe, wobei Re für eine Gruppe steht, die ausgewählt ist aus -NH₂, geradem oder verzweigtem C₁₋₄-Alkyl, das unsubstituiert oder mit einem oder mehreren Fluoratomen oder -NH₂ substituiert ist, geradem oder verzweigtem C₂₋₄-Alkenyl und 3-Piperidinylgruppen, **(g)** einer Gruppe -CONRcRf, wobei Rc ist, wie hierin zuvor definiert, und Rf eine Gruppe ist, die ausgewählt ist aus Wasserstoffatomen, geraden oder verzweigten C₁₋₄-Alkylgruppen und 4-Piperidinylgruppen, oder Rc und Rf zusammen mit dem Stickstoffatom, an das sie gebunden sind, eine Piperidinylgruppe bilden, die unsubstituiert oder mit einer Gruppe substituiert sein kann, die ausgewählt ist aus -NH₂, -CONH₂, -CH₂NH₂ und -CH₂NHCOCF₃, **(h)** einer Gruppe -NHSO₂Rg, wobei Rg ausgewählt ist aus der Gruppe, die gerade oder verzweigte C₁₋₄-Alkyl- und C₁₋₄-Alkylphenylgruppen umfasst, und **(i)** einer Guanidinogruppe.

11. Verbindung nach einem vorangehenden Anspruch, wobei R¹² jeweils unabhängig für ein Halogenatom oder eine Gruppe -CF₃ steht.

12. Verbindung nach einem vorangehenden Anspruch, wobei q eine ganze Zahl von 0 bis 1 ist.

13. Verbindung nach einem der Ansprüche 1 bis 8, wobei A für eine Gruppe von Formel (ii) steht und R¹³ für ein Wasserstoffatom oder eine Methylgruppe steht.

14. Verbindung nach einem der Ansprüche 1 bis 8 oder 13, wobei A für eine Gruppe von Formel (ii) steht und R¹⁴ für ein Wasserstoff- oder Chloratom steht.

15. Verbindung nach einem vorangehenden Anspruch, wobei das Aminotropin eine endo-Konfiguration aufweist.

16. Verbindung nach einem vorangehenden Anspruch, die eine der folgenden ist:
5-Chlor-2-(3-((3-endo)-8-(4-chlorbenzyl)-8-azabicyclo[3.2.1]octan-3-ylamino)-3-oxopropoxy)-N-methylbenzamid
3-(4-Chlor-2-ureidophenoxy)-N-((3-endo)-8-(4-chlorbenzyl)-8-azabicyclo [3.2.1]-octan-3-yl)propanamid
5-Chlor-2-(4-((3-endo)-8-(4-chlorbenzyl)-8-azabicyclo[3.2.1]octan-3-ylamino)-4-oxobutoxy)-N-methylbenzamid
N¹-((3-endo)-8-(4-Chlorbenzyl)-8-azabicyclo[3.2.1]octan-3-yl)-N3-(3-chlorphenyl)malonamid
2-(4-Chlor-2-ureidophenoxy)-N-((3-endo)-8-(4-fluorbenzyl)-8-azabicyclo [3.2.1]octan-3-yl)acetamid
5-Chlor-2-(2-((3-endo)-8-(4-chlorbenzyl)-8-azabicyclo[3.2.1]octan-3-ylamino)-2-oxoethoxy)-N-methylbenzamid
2-(4-Brom-2-ureidophenoxy)-N-((3-endo)-8-(4-chlorbenzyl)-8-azabicyclo[3.2.1]-octan-3-yl)acetamid
2-(5-Chlor-2-(2-((3-endo)-8-(4-chlorbenzyl)-8-azabicyclo [3.2.1]octan-3-ylamino)-2-oxoethoxy)phenyl)-N-methylacetamid
2-(2-(2-Amino-2-oxoethyl)-4-chlorphenoxy)-N-((3-endo)-8-(4-chlorbenzyl)-8-azabicyclo[3.2.1]octan-3-yl)acetamid
2-(5-Chlor-2-(2-((3-endo)-8-(4-chlorbenzyl)-8-azabicyclo[3.2.1]octan-3-ylamino)-2-oxoethoxy)phenyl)-N,N-dimethylacetamid
N-((3-endo)-8-(4-Chlorbenzyl)-8-azabicyclo[3.2.1]octan-3-yl)-2-(4-fluor-2-ureidophenoxy)acetamid
2-(4-Chlor-2-ureidophenoxy)-N-((3-endo)-8-(4-chlorbenzyl)-8-azabicyclo[3.2.1]octan-3-yl)acetamid
5-Chlor-2-(2-((3-endo)-8-(4-chlorbenzyl)-8-azabicyclo[3.2.1]octan-3-ylamino)-2-oxoethoxy)benzamid
2-(2-Acetamido-4-chlorphenoxy)-N-((3-endo)-8-(4-chlorbenzyl)-8-azabicyclo [3.2.1]octan-3-yl)acetamid
N-((1-(5-Chlor-2-(2-((3-endo)-8-(4-chlorbenzyl)-8-azabicyclo[3.2.1]octan-3-ylamino)-2-oxoethoxy)benzoyl)piperidin-3-yl)methyl)-2,2,2-trifluoracetamid
2-(2-(3-(Aminomethyl)piperidin-1-carbonyl)-4-chlorphenoxy)-N-((3-endo)-8-(4-chlorbenzyl)-8-azabicyclo[3.2.1]octan-3-yl)acetamid
N-((3-endo)-8-(4-Chlorbenzyl)-8-azabicyclo[3.2.1]octan-3-yl)-2-(4-chlorphenylamino)acetamid
N-((3-endo)-8-(4-Chlorbenzyl)-8-azabicyclo[3.2.1]octan-3-yl)-2-(3-chlorphenylamino)acetamid
N-((3-endo)-8-(4-Chlorbenzyl)-8-azabicyclo[3.2.1]octan-3-yl)-2-(2-(hydroxymethyl)phenoxy)acetamid
N-((3-endo)-8-(4-Chlorbenzyl)-8-azabicyclo[3.2.1]octan-3-yl)-2-(4-methyl-2-(methylamino)phenoxy)acetamid
N-((3-endo)-8-(4-Chlorbenzyl)-8-azabicyclo[3.2.1]octan-3-yl)-2-(4-chlorphenoxy)acetamid
N-(2-(2-((3-endo)-8-(4-Chlorbenzyl)-8-azabicyclo[3.2.1]octan-3-ylamino)-2-oxoethoxy)-5-methylphenyl)acrylamid
N-((3-endo)-8-(4-Chlorbenzyl)-8-azabicyclo[3.2.1]octan-3-yl)-2-(4-methyl-2-nitrophenoxy)acetamid
N-((3-endo)-8-(4-Chlorbenzyl)-8-azabicyclo[3.2.1]octan-3-yl)-2-(3-cyanophenoxy)acetamid
N-((3-endo)-8-(4-Chlorbenzyl)-8-azabicyclo[3.2.1]octan-3-yl)-2-(3-chlorphenoxy)acetamid
N-((3-endo)-8-(4-Chlorbenzyl)-8-azabicyclo[3.2.1]octan-3-yl)-2-(4-fluor-2-nitrophenoxy)acetamid
Methyl-4-acetamido-5-chlor-2-(2-((3-endo)-8-(4-chlorbenzyl)-8-azabicyclo[3.2.1]-octan-3-ylamino)-2-oxoethoxy)benzoat
N-((3-endo)-8-(4-Chlorbenzyl)-8-azabicyclo[3.2.1]octan-3-yl)-2-(2-cyanophenoxy)acetamid
2-(2-((3-endo)-8-(4-Chlorbenzyl)-8-azabicyclo[3.2.1]octan-3-ylamino)-2-oxoethoxy)benzamid
2-(Benzo[d][1,3]dioxol-5-yloxy)-N-((3-endo)-8-(4-chlorbenzyl)-8-azabicyclo[3.2.1]octan-3-yl)acetamid
N-((3-endo)-8-(4-Chlorbenzyl)-8-azabicyclo[3.2.1]octan-3-yl)-2-(2-nitrophenoxy)acetamid
N-((3-endo)-8-(4-Chlorbenzyl)-8-azabicyclo[3.2.1]octan-3-yl)-2-(2-methoxyphenoxy)acetamid
5-Chlor-2-(2-((3-endo)-8-(4-chlorbenzyl)-8-azabicyclo[3.2.1]octan-3-ylamino)-2-oxoethoxy)-N-(piperidin-4-yl)benzamid
2-(2-(4-Aminopiperidin-1-carbonyl)-4-chlorphenoxy)-N-((3-endo)-8-(4-chlorbenzyl)-8-azabicyclo[3.2.1]octan-3-yl)acetamid
1-(5-Chlor-2-(2-((3-endo)-8-(4-chlorbenzyl)-8-azabicyclo[3.2.1]octan-3-ylamino)-2-oxoethoxy)benzoyl)piperidin-3-carboxamid
5-Chlor-2-(2-((3-endo)-8-(4-chlorbenzyl)-8-azabicyclo[3.2.1]octan-3-ylamino)-2-oxoethoxy)-N,N-dimethylbenzamid
5-Chlor-2-(2-((3-endo)-8-(4-chlorbenzyl)-8-azabicyclo[3.2.1]octan-3-ylamino)-2-oxoethoxy)benzoesäure
2-(4-Chlor-2-nitrophenoxy)-N-((3-endo)-8-(4-chlorbenzyl)-8-azabicyclo[3.2.1]octan-3-yl)acetamid
Methyl-5-chlor-2-(2-((3-endo)-8-(4-chlorbenzyl)-8-azabicyclo[3.2.1]octan-3-ylamino)-2-oxoethoxy)benzoat
2-(2-Amino-4-chlorphenoxy)-N-((3-endo)-8-(4-chlorbenzyl)-8-azabicyclo [3.2.1]-octan-3-yl)acetamid
2-Amino-N-(5-chlor-2-(2-((3-endo)-8-(4-chlorbenzyl)-8-azabicyclo[3.2.1]octan-3-ylamino)-2-oxoethoxy)phenyl)acetamid
3-Amino-N-(5-chlor-2-(2-((3-endo)-8-(4-chlorbenzyl)-8-azabicyclo[3.2.1]octan-3-ylamino)-2-oxoethoxy)phenyl)propanamid
N-(5-Chlor-2-(2-((3-endo)-8-(4-chlorbenzyl)-8-azabicyclo[3.2.1]octan-3-ylamino)-2-oxoethoxy)phenyl)piperidin-3-carboxamid
N-(5-Chlor-2-(2-((3-endo)-8-(4-chlorbenzyl)-8-azabicyclo[3.2.1]octan-3-ylamino)-2-oxoethoxy)phenyl)-2,2,2-trifluoracetamid
2-(4-Chlor-2-(methylsulfonamido)phenoxy)-N-((3-endo)-8-(4-chlorbenzyl)-8-azabicyclo[3.2.1]octan-3-yl)acetamid
2-(4-Chlor-2-guanidinophenoxy)-N-((3-endo)-8-(4-chlorbenzyl)-8-azabicyclo[3.2.1]octan-3-yl)acetamid
2-(4-Chlor-2-ureidophenoxy)-N-((3-exo)-8-(4-chlorbenzyl)-8-azabicyclo[3.2.1]octan-3-yl)acetamid
N-((3-endo)-8-(4-Chlorbenzyl)-8-azabicyclo[3.2.1]octan-3-yl)-2-(3-chlorphenyl)acetamid
N-((3-endo)-8-(4-Chlorbenzyl)-8-azabicyclo[3.2.1]octan-3-yl)-2-(2-(2,6-dichlorphenylamino)phenyl)acetamid
3-Chlor-N-(2-((3-endo)-8-(4-chlorbenzyl)-8-azabicyclo[3.2.1]octan-3-ylamino)-2-oxoethyl)benzamid
N-(2-((3-endo)-8-(4-Chlorbenzyl)-8-azabicyclo[3.2.1]octan-3-ylamino)-2-oxoethyl)-3-(trifluormethyl)benzamid
N-(2-((3-endo)-8-(4-Chlorbenzyl)-8-azabicyclo[3.2.1]octan-3-ylamino)-2-oxoethyl)-2-(4-methylphenylsulfonamido)benzamid
N-(2-((3-endo)-8-(4-Chlorbenzyl)-8-azabicyclo[3.2.1]octan-3-ylamino)-2-oxoethyl)-1-(4-chlorphenyl)cyclopentancarboxamid
2,7-Dichlor-N-(2-((3-endo)-8-(4-chlorbenzyl)-8-azabicyclo[3.2.1]octan-3-ylamino)-2-oxoethyl)-9H-xanthen-9-carboxamid
5-Chlor-N-(2-((3-endo)-8-(4-chlorbenzyl)-8-azabicyclo[3.2.1]octan-3-ylamino)-2-oxoethyl)-2-ureidobenzamid
N-((3-endo)-8-(4-Chlorbenzyl)-8-azabicyclo[3.2.1]octan-3-yl)-2-(3-(3-chlorphenyl)ureido)acetamid
N-(2-((3-endo)-8-(4-Chlorbenzyl)-8-azabicyclo[3.2.1]octan-3-ylamino)-2-oxoethyl)-2-phenoxybenzamid
N-(2-(((3-endo)-8-((4-Chlorbenzyl)-8-azabicyclo[3.2.1]oct-3-yl)amino)-2-oxoethyl)-10,11-dihydro-5H-dibenzo[a,d]cyclohepten-S-carboxamid
N-(2-((3-endo)-8-(4-Chlorbenzyl)-8-azabicyclo[3.2.1]octan-3-ylamino)-2-oxoethyl)-9-methyl-9H-fluoren-9-carboxamid
N-(2-((3-endo)-8-(4-Chlorbenzyl)-8-azabicyclo[3.2.1]octan-3-ylamino)-2-oxoethyl)-2,2-diphenylacetamid
2-Amino-N-(2-((3-endo)-8-(4-chlorbenzyl)-8-azabicyclo[3.2.1]octan-3-ylamino)-2-oxoethyl)benzamid
N-(2-((3-endo)-8-(4-Chlorbenzyl)-8-azabicyclo[3.2.1]octan-3-ylamino)-2-oxoethyl)-9-methyl-9H-xanthen-9-carboxamid
N-((3-endo)-8-(4-Chlorbenzyl)-8-azabicyclo[3.2.1]octan-3-yl)-2-(2-(2-(2,6-dichlorphenylamino)phenyl)acetamido)acetamid
N-((3-endo)-8-(4-Chlorbenzyl)-8-azabicyclo[3.2.1]octan-3-yl)-2-(2-(3-chlorphenyl)acetamido)acetamid
N-(3-((3-endo)-8-(4-Chlorbenzyl)-8-azabicyclo[3.2.1]octan-3-ylamino)-3-oxopropyl)-2-(3-(trifluormethyl)phenylamino)benzamid
N-(2-((3-endo)-8-(4-Chlorbenzyl)-8-azabicyclo[3.2.1]octan-3-ylamino)-2-oxoethyl)-N-methyl-2-(3-(trifluormethyl)phenylamino)benzamid
3-Brom-N-(2-((3-endo)-8-(4-chlorbenzyl)-8-azabicyclo[3.2.1]octan-3-ylamino)-2-oxoethyl)benzamid
5-Chlor-N-(2-((3-endo)-8-(4-chlorbenzyl)-8-azabicyclo[3.2.1]octan-3-ylamino)-2-oxoethyl)-2-methoxybenzamid
N-(2-((3-endo)-8-(4-Chlorbenzyl)-8-azabicyclo[3.2.1]octan-3-ylamino)-2-oxoethyl)-3-fluorbenzamid
5-Chlor-N-(2-((3-endo)-8-(4-chlorbenzyl)-8-azabicyclo[3.2.1]octan-3-ylamino)-2-oxoethyl)-2-methoxy-4-(methylamino)benzamid
3,4-Dichlor-N-(2-((3-endo)-8-(4-chlorbenzyl)-8-azabicyclo[3.2.1]octan-3-ylamino)-2-oxoethyl)benzamid
N-(2-((3-endo)-8-(4-Chlorbenzyl)-8-azabicyclo[3.2.1]octan-3-ylamino)-2-oxoethyl)benzamid
N-(2-((3-endo)-8-(4-Chlorbenzyl)-8-azabicyclo[3.2.1]octan-3-ylamino)-2-oxoethyl)-3-methylbenzamid
2,5-Dichlor-N-(2-((3-endo)-8-(4-chlorbenzyl)-8-azabicyclo[3.2.1]octan-3-ylamino)-2-oxoethyl)benzamid
N-(2-((3-endo)-8-(4-Chlorbenzyl)-8-azabicyclo[3.2.1]octan-3-ylamino)-2-oxoethyl)-3-methoxybenzamid
N-(2-((3-endo)-8-(4-Chlorbenzyl)-8-azabicyclo[3.2.1]octan-3-ylamino)-2-oxoethyl)-3-(hydroxy(phenyl)methyl)benzamid
N-(2-((3-endo)-8-(4-Chlorbenzyl)-8-azabicyclo[3.2.1]octan-3-ylamino)-2-oxoethyl)-3-(cyanomethyl)benzamid
5-Chlor-N-(2-((3-endo)-8-(4-chlorbenzyl)-8-azabicyclo[3.2.1]octan-3-ylamino)-2-oxoethyl)-4-(dimethylamino)-2-methoxybenzamid
3-Chlor-N-(3-((3-exo)-8-(4-chlorbenzyl)-8-azabicyclo[3.2.1]octan-3-ylamino)-3 - oxopropyl)benzamid
3-Chlor-N-((R)-1-((3-exo)-8-(4-chlorbenzyl)-8-azabicyclo[3.2.1]octan-3-ylamino)-1-oxopropan-2-yl)benzamid
N-(2-((3-exo)-8-(4-Chlorbenzyl)-8-azabicyclo[3.2.1]octan-3-ylamino)-2-oxoethyl)-N-methyl-2-(3-(trifluorethyl)phenylamino)benzamid
(R)-3-(3-Chlorbenzamido)-4-((3-exo)-8-(4-chlorbenzyl)-8-azabicyclo[3.2.1]octan-3-ylamino)-4-oxobuttersäure
N-((S)-1-((3-exo)-8-(4-Chlorbenzyl)-8-azabicyclo[3.2.1]octan-3-ylamino)-1-oxopropan-2-yl)-2-(3-(trifluormethyl)phenylamino)benzamid
3-Chlor-N-((S)-1-((3-exo)-8-(4-chlorbenzyl)-8-azabicyclo[3.2.1]octan-3-ylamino)-1-oxopropan-2-yl)benzamid
3-Chlor-N-(2-((3-exo)-8-(4-chlorbenzyl)-8-azabicyclo[3.2.1]octan-3-ylamino)-2-oxoethyl)-N-methylbenzamid
N-((S)-1-((3-exo)-8-(4-Chlorbenzyl)-8-azabicyclo [3.2.1]octan-3-ylamino)-1-oxo-3-phenylpropan-2-yl)-2-(3-(trifluormethyl)phenylamino)benzamid
N-((S)-1-((3-exo)-8-(4-Chlorbenzyl)-8-azabicyclo[3.2.1]octan-3-ylamino)-4-(methylthio)-1-oxobutan-2-yl)-2-(3-(trifluormethyl)phenylamino)benzamid
N-(3-((3-exo)-8-(4-Chlorbenzyl)-8-azabicyclo[3.2.1]octan-3-ylamino)-3-oxopropyl)-2-(3-(trifluormethyl)phenylamino)benzamid
N-((S)-1-((3-exo)-8-(4-Chlorbenzyl)-8-azabicyclo[3.2.1]octan-3-ylamino)-4-methyl-1-oxopentan-2-yl)-2-(3-(trifluormethyl)phenylamino)benzamid
3-Chlor-N-(2-((3-endo)-8-(3,4-dichlorbenzyl)-8-azabicyclo[3.2.1]octan-3-ylamino)-2-oxoethyl)benzamid
3-Chlor-N-(2-((3-endo)-8-(4-fluorbenzyl)-8-azabicyclo[3.2.1]octan-3-ylamino)-2-oxoethyl)benzamid
N-(2-((3-endo)-8-Benzyl-8-azabicyclo [3.2.1]octan-3-ylamino)-2-oxoethyl)-3-chlorbenzamid
3-Chlor-N-(2-((3-endo)-8-(2,4-dichlorbenzyl)-8-azabicyclo[3.2.1]octan-3-ylamino)-2-oxoethyl)benzamid
3-Chlor-N-(2-((3-endo)-8-(1-(4-chlorphenyl)ethyl)-8-azabicyclo [3.2.1]octan-3-ylamino)-2-oxoethyl)benzamid
3-Chlor-N-(2-((3-endo)-8-(1-(4-chlorphenyl)propyl)-8-azabicyclo [3.2.1]octan-3-ylamino)-2-oxoethyl)benzamid
3-Chlor-N-(2-oxo-2-((3-endo)-8-(3-phenylpropyl)-8-azabicyclo[3.2.1]octan-3-ylamino)ethyl)benzamid
N-(2-((3-endo)-8-(3,4-Dichlorbenzyl)-8-azabicyclo[3.2.1]octan-3-ylamino)-2-oxoethyl)-2-(3-(trifluormethyl)phenylamino)benzamid
N-(2-((3-endo)-8-(2,4-Dichlorbenzyl)-8-azabicyclo[3.2.1]octan-3-ylamino)-2-oxoethyl)-2-(3-(trifluormethyl)phenylamino)benzamid
N-(2-((3-endo)-8-(4-Methoxybenzyl)-8-azabicyclo[3.2.1]octan-3-ylamino)-2-oxoethyl)-2-(3-(trifluormethyl)phenylamino)benzamid
N-(2-((3-endo)-8-(4-Chlorbenzyl)-8-azabicyclo[3.2.1]octan-3-ylamino)-2-oxoethyl)-2-hydroxybenzamid
N-(2-((3-endo)-8-(4-Chlorbenzyl)-8-azabicyclo[3.2.1]octan-3-ylamino)-2-oxoethyl)-2-(phenylamino)benzamid
N-(2-((3-endo)-8-(4-Chlorbenzyl)-8-azabicyclo[3.2.1]octan-3-ylamino)-2-oxoethyl)-9H-xanthen-9-carboxamid
2-(3,5-Bis(trifluormethyl)phenylsulfonamido)-N-((3-endo)-8-(4-chlorbenzyl)-8-azabicyclo[3.2.1]octan-3-yl)acetamid
3-Chlor-N-((S)-1-((3-endo)-8-(4-chlorbenzyl)-8-azabicyclo [3.2.1]octan-3-ylamino)-1-oxopropan-2-yl)benzamid
3-Chlor-N-(3-((3-endo)-8-(4-chlorbenzyl)-8-azabicyclo[3.2.1]octan-3-ylamino)-3-oxopropyl)benzamid
(R)-3-(3-Chlorbenzamido)-4-((3-endo)-8-(4-chlorbenzyl)-8-azabicyclo[3.2.1]octan-3-ylamino)-4-oxobuttersäure
3-Chlor-N-((R)-1-((3-endo)-8-(4-chlorbenzyl)-8-azabicyclo[3.2.1]octan-3-ylamino)-1-oxopropan-2-yl)benzamid
(R)-3-(3-Chlorbenzamido)-4-((3-endo)-8-(4-chlorbenzyl)-8-azabicyclo[3.2.1]octan-3-ylamino)-4-oxobuttersäure
N-(2-((3-endo)-8-(4-Fluorbenzyl)-8-azabicyclo[3.2.1]octan-3-ylamino)-2-oxoethyl)-2-(3-(trifluormethyl)phenylamino)benzamid
N-(2-((3-exo)-8-(4-Chlorbenzyl)-8-azabicyclo[3.2.1] octan-3 -ylamino)-2-oxoethyl)-2-(3-(trifluormethyl)phenylamino)benzamid
N-(2-((3-exo)-8-(4-Chlorbenzyl)-8-azabicyclo[3.2.1]octan-3-ylamino)-2-oxoethyl)-3-(trifluormethyl)benzamid
3-Chlor-N-(2-((3-exo)-8-(4-chlorbenzyl)-8-azabicyclo[3.2.1]octan-3-ylamino)-2-oxoethyl)benzamid
N-(2-((3-endo)-8-(4-Chlorbenzyl)-8-azabicyclo[3.2.1]octan-3-ylamino)-2-oxoethyl)-2-(3-(trifluormethyl)phenylamino)benzamid

17. Verbindung nach einem der Ansprüche 1 bis 16 zur Verwendung bei der Behandlung eines pathologischen Zustandes oder einer Erkrankung, die empfänglich ist für Linderung durch Antagonismus des CCR1-Rezeptors.

18. Verbindung nach einem der Ansprüche 1 bis 16 zur Verwendung bei der Behandlung eines pathologischen Zustandes oder einer Erkrankung, wobei der pathologische Zustand oder die Erkrankung rheumatoide Arthritis, allergisches Asthma, Lungenfibrose, Atemwegsvirusinfektion, Glomerulonephritis, Nierentransplantatabstoßung, Sepsis, Atherosklerose, systemischer Lupus erythematosus (SLE), multiple Sklerose, Alzheimer-Krankheit und Herztransplantatabstoßung ist.

19. Pharmazeutische Zusammensetzung, die eine Verbindung, wie definiert in einem der Ansprüche 1 bis 16, in Vermischung mit einem pharmazeutisch verträglichen Verdünnungsmittel oder Trägerstoff umfasst.

20. Verwendung einer Verbindung, wie definiert in einem der Ansprüche 1 bis 16, zur Herstellung eines Arzneimittels zur Behandlung eines pathologischen Zustandes oder einer Erkrankung, die dafür empfänglich ist, durch Antagonismus des CCR1-Rezeptors verbessert zu werden.

21. Verwendung einer Verbindung nach Anspruch 20, wobei der pathologische Zustand oder die Erkrankung rheumatoide Arthritis, allergisches Asthma, Lungenfibrose, Atemwegsvirusinfektion, Glomerulonephritis, Nierentransplantatabstoßung, Sepsis, Atherosklerose, systemischer Lupus erythematosus (SLE), multiple Sklerose, Alzheimer-Krankheit und Herztransplantatabstoßung ist.

22. Kombinationsprodukt, das eine Verbindung nach einem der Ansprüche 1 bis 16; und eine weitere Verbindung umfasst, die ausgewählt ist aus (a) Methotrexat, (b) Leflunomid, (c) Sulfasalazin, (d) Hydroxychloroquin, (e) Azathioprin, (f) Goldsalzen, (h) Antagonisten der Chemokin-Rezeptoren CCR2 bis CCR10, (i) Antagonisten der Chemokin-Rezeptoren CXCR1 bis CXCR6, (j) Antagonisten der Chemokin-Rezeptoren XCR1, XCR2 oder CX3CR1, (k) monoklonalen anti-TNF-Antikörpern, (1) löslichen Antagonisten von TNF-Rezeptoren, (m) Abatacept, (n) anti-CD20-Antikörpern, (o) anti-IL-6R-Antikörpern, (p) PDE4-Inhibitoren, (q) p38-MAPK-Inhibitoren, (r) MAPKAP-K2-Inhibitoren, (s) P2X7-Rezeptorantagonisten, (t) Interferon-beta-Rezeptorliganden, (u) Glatirameracetat und (v) anti-IL-12-Antikörpern, für gleichzeitige, getrennte oder aufeinanderfolgende Verwendung.

## Revendications

1. Composé de formule (I) 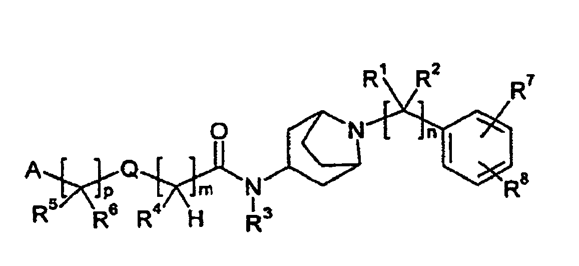 sous la forme d'une base libre, d'un sel pharmaceutiquement acceptable, ou d'une forme zwittérionique de celui-ci, dans lequel :
n est un nombre entier de 1 à 4
m est un nombre entier de 1 à 3
p est un nombre entier de 0 à 2
R¹, R² et R³ représentent chacun indépendamment un atome d'hydrogène ou un groupe alkyle en C₁₋₄ droit ou ramifié
Q représente une liaison directe ou un groupe choisi parmi -CONR⁹-, -OCONR⁹-, - NR⁹CONR¹⁰- -NR⁹-, -NR⁹COO-, -O-, -SO₂NR⁹- et NR⁹CO
R⁹ et R¹⁰ représentent chacun un atome d'hydrogène ou un groupe alkyle en C₁₋₄ droit
ou ramifié
R⁴ représente un atome d'hydrogène ou un groupe choisi parmi un alkyle en C₁₋₄ droit ou ramifié qui est non substitué ou substitué avec un groupe choisi parmi -COOH, un phényle ou -SCH₃ à condition que lorsque m est égal à 2 ou 3, seul un R⁴ soit comme défini ci-dessus et le reste de R⁴ est constitué d'atomes d'hydrogène
R⁵ et R⁶ représentent chacun indépendamment un atome d'hydrogène, un alkyle en C₁₋₄ droit ou ramifié ou un groupe phényle, ou
R⁵ et R⁶, avec l'atome de carbone auquel ils sont fixés forment un groupe cycloalkyle en C₃₋₈
R⁷ et R⁸ représentent chacun indépendamment un atome d'hydrogène ou d'halogène ou un groupe alcoxy en C₁₋₄
A représente un groupe de formule (i) ou (ii) 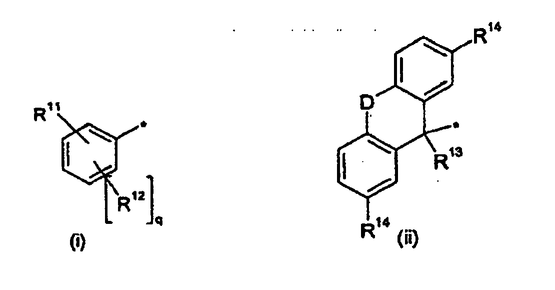 D représente une liaison directe ou un groupe choisi parmi -O- et -CH₂CH₂
R¹¹ représente un atome d'hydrogène ou d'halogène ou un groupe choisi parmi (a) un alkyle en C₁₋₄ droit ou ramifié qui est non substitué ou substitué par un hydroxy, un cyano, un phényle ou un groupe -CONRaRb, dans lequel Ra et Rb sont indépendamment choisis parmi des atomes d'hydrogène et des groupes alkyle en C₁₋₄, (b) un hydroxy, un phénoxy, ou un alcoxy en C₁₋₄ ramifié, (c) un nitro, (d) un hydroxycarbonyle ou un alcoxycarbonyle en C₁₋₄ droit ou ramifié, (e) un groupe NRcRd dans lequel Rc représente un atome d'hydrogène ou un alkyle en C₁₋₄ droit ou ramifié, et Rd représente un atome d'hydrogène, un alkyle en C₁₋₄ droit ou ramifié ou un groupe phényle qui est éventuellement substitué par un ou plusieurs groupes choisis parmi -CF₃, et des atomes d'halogène, (f) un groupe - NHCORe dans lequel Re représente un groupe choisi parmi -NH₂, un alkyle en C₁₋₄ droit ou ramifié qui est non substitué ou substitué par un ou plusieurs atomes de fluor ou -NH₂, un alcényle en C₂₋₄ droit
ou ramifié et des groupes 3-pipéridinyle, (g) un groupe -CONRcRf dans lequel Rc est comme défini ci-dessus et Rf est un groupe choisi parmi les atomes d'hydrogène, les groupes alkyle en C₁₋₄ droits ou ramifiés et des groupes 4-pipéridinyle ou Rc et Rf forment ensemble avec l'atome d'azote auquel ils sont fixés un groupe pipéridinyle qui peut être non substitué ou substitué par un groupe choisi parmi -NH₂, -CONH₂-, -CH₂NH₂ et -CH₂NHCOCF₃, (h) un groupe -NHSO₂Rg dans lequel Rg est choisi dans le groupe constitué de groupes alkyle en C₁₋₄ droit ou ramifié et alkylphényle en C₁₋₄ et (i) un groupe guanidino ;
R¹² représentent chacun indépendamment un cyano, un alkyle en C₁₋₄ ramifié, un alcoxy en C₁₋₄ droit ou ramifié, un atome d'halogène ou un groupe -CF₃,
ou R¹¹ et un des R¹² forment un groupe méthanediol
q est un nombre entier de 0 à 2
R¹³ représente un atome d'hydrogène ou un alkyle en C₁₋₄ droit ou ramifié
R¹⁴ représentent indépendamment chacun un atome d'hydrogène ou d'halogène, à condition que le composé ne soit pas un 2-(4-fluorophényl)-N-(8-benzyl-8-azabicyclo [3.2.1]-oct-3-yl)acétamide.

2. Composé selon la revendication 1, dans lequel Q représente un groupe choisi parmi un -CONR⁹-, -O- et NR⁹CO

3. Composé selon l'une quelconque des revendications précédentes dans lequel n est égal à 1.

4. Composé selon l'une quelconque des revendications précédentes dans lequel R³ est un atome d'hydrogène ou un groupe méthyle.

5. Composé selon l'une quelconque des revendications précédentes dans lequel les R¹ représentent chacun indépendamment un atome d'hydrogène et R² est choisi parmi un atome d'hydrogène et des groupes méthyles.

6. Composé selon l'une quelconque des revendications précédentes dans lequel R⁹ est un atome d'hydrogène.

7. Composé selon l'une quelconque des revendications précédentes dans lequel les R⁴ représentent chacun indépendamment un atome d'hydrogène ou un groupe méthyle.

8. Composé selon l'une quelconque des revendications précédentes dans lequel R⁵ et R⁶ représentent chacun indépendamment un atome d'hydrogène ou un groupe phényle.

9. Composé selon l'une quelconque des revendications précédentes, dans lequel A représente un groupe de formule (i) et R¹¹ représente un atome d'hydrogène ou d'halogène ou un groupe choisi parmi (a) un méthyle qui est substitué par un groupe -CONRaRb dans lequel Ra et Rb sont indépendamment choisis parmi des atomes d'hydrogène et des groupes alkyle en C₁₋₄, (b) un hydroxyle ou phénoxy, (c) un nitro, (d) un méthoxycarbonyle, (e) un groupe - NHRd dans lequel Rd représente un atome d'hydrogène, un groupe méthyle, ou un groupe phényle qui est substitué par un ou plusieurs groupes choisis parmi -CF₃, et des atomes d'halogène, (f) un groupe NHCORe dans lequel Re représente un groupe choisi parmi le - NH₂, -CF₃ et un alkyle en C₁₋₄ droit, qui est non substitué ou substitué par un groupe -NH₂, (g) un groupe -CONHRf dans lequel Rf est un groupe choisi parmi des atomes d'hydrogène, des groupes méthyle et 4-pipéridinyle ou Rc et Rf forment ensemble, avec l'atome d'azote auquel ils sont fixés, un groupe pipéridinyle qui peut être non substitué ou substitué par un groupe choisi parmi -NH₂, -CONH₂ et -CH₂NH₂, (h) un groupe -NHSO₂Rg dans lequel Rg est choisi dans le groupe comprenant des groupes méthyle et méthylphényle et (i) un groupe guanidino.

10. Composé selon l'une quelconque des revendications précédentes, dans lequel Q représente un atome d'oxygène, A représente un groupe de formule (i), q est égal à 1 ou 2 et R¹¹ représente un groupe choisi parmi (a) un méthyle qui est substitué par un groupe - CONRaRb dans lequel Ra et Rb sont indépendamment choisis parmi des atomes d'hydrogène et des groupes alkyle en C₁₋₄ droits ou ramifiés, (e) un groupe NHRcRd, dans lequel Rc représente un atome d'hydrogène ou un alkyle en C₁₋₄ droit ou ramifié et Rd représente un atome d'hydrogène ou un alkyle en C₁₋₄ droit ou ramifié, ou un groupe phényle qui est éventuellement substitué par un ou plusieurs groupes choisis parmi -CF₃, et des atomes d'halogène, (f) un groupe -NHCORe dans lequel Re représente un groupe choisi parmi le - NH₂, un alkyle en C₁₋₄ droit ou ramifié, qui est non substitué ou substitué par un ou plusieurs atomes de fluor ou des groupes -NH₂, alcényle en C₂₋₄ droit ou ramifié et 3-pipéridinyle, (g) un groupe -CONRcRf dans lequel Rc est comme défini ci-dessus et Rf est un groupe choisi parmi des atomes d'hydrogène, des groupes alkyle en C₁₋₄ droits ou ramifiés et des groupes 4-pipéridinyle ou Rc et Rf forment ensemble, avec l'atome d'azote auquel ils sont fixés, un groupe pipéridinyle qui peut être non substitué ou substitué par un groupe choisi parmi -NH₂, -CONH₂, -CH₂NH₂ et -CH₂NHCOCF₃, (h) un groupe NHSO₂Rg dans lequel Rg est choisi dans le groupe comprenant des groupes alkyle en C₁₋₄ et alkylphényle en C₁₋₄ droits ou ramifiés et (i) un groupe guanidino.

11. Composé selon l'une quelconque des revendications précédentes dans lequel les R¹² représentent chacun indépendamment un atome d'halogène ou un groupe -CF₃.

12. Composé selon l'une quelconque des revendications précédentes dans lequel q est un nombre entier de 0 à 1.

13. Composé selon l'une quelconque des revendications 1 à 8 dans lequel A représente un groupe de formule (ii) et R¹³ représente un atome d'hydrogène ou un groupe méthyle.

14. Composé selon l'une quelconque des revendications 1 à 8 ou 13 dans lequel A représente un groupe de formule (ii) et R¹⁴ représente un atome d'hydrogène ou de chlore.

15. Composé selon l'une quelconque des revendications précédentes, dans lequel l'aminotropine a une configuration endo.

16. Composé selon l'une quelconque des revendications précédentes, qui est un composé parmi :
5-chloro-2-(3-3-endo)-8-(4-chlorobenzyl)-8-azabicydo[3.2.1]octan-3-ylamino)-3-oxopropoxy)-N-méthylbenzamide
3-(4-chloro-2-uréidophénoxy)-N-((3-endo)-8-(4-chlorobenzyl)-8-azabicyclo [3.2.1]octan-3-yl)propanamide
5-chloro-2-(4-((3-endo-8-(4-chlorobenzyl)-8-azabicyclo[3.2.1]octan-3-ylamino)-4-oxobutoxy)-N-méthylbenzamide
N¹-((3-endo)-8-(4-chlorobenzyl)-8-azabicyclo[3.2.1]octan-3-yl)-N3-(3-chlorophényl)-malonamide
2-(4-chloro-2-uréidophénoxy)-N-((3-endo)-8-(4-fluorobenzyl)-8-azabicyclo[3.2.1]octan-3-yl)acétamide
5-chloro-2-(2-((3-endo)-8-(4-chlorobenzyl)-8-azabicyclo[3.2.1 ]octan-3-ylamino)-2-oxoéthoxy)-N-méthylbenzamide
2-(4-bromo-2-uréidophénoxy)-N-((3-endo)-8-(4-chlorobenzyl)-8-azabicyclo[3.2.1]octan-3-yl)acétamide
2-(5-chloro-2-(2-((3-endo)-8-(4-chlorobenzyl)-8-azabicyclo[3.2.1]octan-3-ylamino)-2-oxoéthoxy)phényl)-N-méthylacétamide
2-(2-(2-amino-2-oxoéthyl)-4-chlorophénoxy)-N-((3-endo)-8(4-chlorobenzyl)-8-azabicyclo[3.2.1]octan-3-yl)acétamide
2-(5-chloro-2-(2-((3-endo)-8-(4-chlorobenzyl)-8-azabicyclo[3.2.1]octan-3-ylamino)-2-oxoéthoxy)phényl)-N,N-diméthylacétamide
N-((3-endo)-8-(4-chlorobenzyl)-8-azabicyclo[3.2.1]octan-3-yl)-2-(4-fluoro-2-uréidophénoxy)acétamide
2-(4-chloro-2-uréidophénoxy)-N-((3-endo)-8-(4-chlorobenzyl)-8-azabicyclo[3.2.1]octan-3-yl)acétamide
5-chloro-2-(2-((3-endo)-8-(4-chlorobenzyl)-8-azabicyclo[3.2.1]octan-3-ylamino)-2-oxoéthoxy)benzamide
2-(2-acétamido-4-chlorophénoxy)-N-((3-endo)-8-(4-chlorobenzyl)-8-azabicyclo[3.2.1]octan-3-yl)acétamide
N-((1-(5-chloro-2-(2-((3-endo)-8-(4-chlorobenzyl)-8-azabicyclo[3.2.1]octan-3-ylamino)-2-oxoéthoxy)benzoyl)pipéridin-3-yl)méthyl)-2,2,2-trifluoroacétamide
2-(2-(3-aminométhyl)pipéridine-1-carbonyl)-4-chlorophénoxy)-N-((3-endo)-8-(4-chlorobenzyl)-8-azabicyclo[3.2.1]octan-3-yl)acétamide
N-((3-endo)-8-(4-chlorobenzyl)-8-azabicyclo[3.2.1]octan-3-yl)-2-(4-chlorophénylamino)acétamide
N-((3-endo)-8-(4-chlorobenzyl)-8-azabicyclo[3.2.1]octan-3-yl)-2-(3-chlorophénylamino)-acétamide
N-((3-endo)-8-(4-chlorobenzyl)-8-azabicyclo[3.2.1]octan-3-yl)-2-(2-(hydroxyméthyl)phénoxy)acétamide
N-((3-endo)-8-(4-chlorobenzyl)-8-azabicyclo[3.2.1]octan-3-yl)-2-(4-méthyl-2-(méthylamino)phénoxy)acétamide
N-((3-endo)-8-(4-chlorobenzyl)-8-azabicyclo[3.2.1]octan-3-yl)-2-(4-chlorophénoxy)acétamide
N-(2-(2-((3-endo-8-(4-chlorobenzyl)-8-azabicyclo[3.2.1]octan-3-ylamino)-2-oxoéthoxy)-5-méthylphényl)acrylamide
N-((3-endo)-8-(4-chlorobenzyl)-8-azabicyclo[3.2.1]octan-3-yl)-2-(4-méthyl-2-nitrophénoxy)acétamide
N-((3-endo)-8-(4-chlorobenzyl)-8-azabicyclo[3.2.1]octan-3-yl)-2-(3-cyanophénoxy)acétamide
N-((3-endo)-8-(4-chlorobenzyl)-8-azabicyclo[3.2.1]octan-3-yl)-2-(3-chlorophénoxy)acétamide
N-((3-endo)-8-(4-chlorobenzyl)-8-azabicyclo[3.2.1]octan-3-yl)-2-(4-fluoro-2-nitrophénoxy)acétamide
4-acétamido-5-chloro-2-(2-((3-endo)-8-(4-chlorobenzyl)-8-azabicyclo[3.2.1]octan-3-ylamino)-2-oxoéthoxy)benzoate de méthyle
N-((3-endo)-8-(4-chlorobenzyl)-8-azabicyclo[3.2.1]octan-3-yl)-2-(2-cyanophénoxy)acétamide
2-(2-((3-endo)-8-(4-chlorobenzyl)-8-azabicyclo[3.2.1]octan-3-ylamino)-2-oxoéthoxy)acétamide
2-(benzo[d][1,3]dioxol-5-yloxy)-N-((3-endo)-8-(4-chlorobenzyl)-8-azabicyclo[3.2.1]octan-3-yl)acétamide
N-((3-endo)-8-(4-chlorobenzyl)-8-azabicyclo[3.2.1]octan-3-yl)-2-(2-nitrophénoxy)-acétamide
N-((3-endo)-8-(4-chlorobenzyl)-8-azabicyclo[3.2.1]octan-3-yl)-2-(2-méthoxyphénoxy)-acétamide
5-chloro-2-(2-((3-endo)-8-(4-chlorobenzyl)-8-azabicyclo[3.2.1]octan-3-ylamino)-2-oxoéthoxy)-N-(pipéridin-4-yl)benzamide
2-(2-(4-aminopipéridine-1-carbonyl)-4-chlorophénoxy)-N-((3-endo)-8-(4-chlorobenzyl)-8-azabicyclo[3.2.1]octan-3-yl)acétamide
1-(5-chloro-2-(2-((3-endo)-8-(4-chlorobenzyl)-8-azabicyclo[3.2.1]octan-3-ylamino)-2-oxoéthoxy)benzoyl)pipéridine-3-carboxamide
5-chloro-2-(2-((3-endo)-8-(4-chlorobenzyl)-8-azabicyclo[3.2.1]octan-3-ylamino)-2-oxoéthoxy)-N,N-diméthylbenzamide
Acide 5-chloro-2-(2-((3-endo)-8-(4-chlorobenzyl)-8-azabicyclo[3.2.1]octan-3-ylamino)-2-oxoéthoxy)-benzoïque
2-(4-chloro-2-nitrophénoxy)-N-((3-endo)-8-(4-chlorobenzyl)-8-azabicyclo[3.2.1]octan-3-yl)acétamide
5-chloro-2-(2-((3-endo)-8-(4-chlorobenzyl)-8-azabicyclo[3.2.1]octan-3-ylamino)-2-oxoéthoxy)benzoate de méthyle
2-(2-amino-4-chlorophénoxy)-N-((3-endo)-8-(4-chlorobenzyl)-8-azabicyclo[3.2.1]octan-3-yl)acétamide
2-amino-N-(5-chloro-2-(2-((3-endo)-8-(4-chlorobenzyl)-8-azabicyclo[3.2.1]octan-3-ylamino)-2-oxoéthoxy)phényl)acétamide
3-amino-N-(5-chloro-2-(2-((3-endo)-8-(4-chlorobenzyl)-8-azabicyclo[3.2.1]octan-3-ylamino)-2-oxoéthoxy)phényl)propanamide
N-(5-chloro-2-(2-((3-endo)-8-(4-chlorobenzyl)-8-azabicyclo[3.2.1]octan-3-ylamino)-2-oxoéthoxy)phényl)pipéridine-3-carboxamide
N-(5-chloro-2-(2-((3-endo)-8-(4-chlorobenzyl)-8-azabicyclo[3.2.1]octan-3-ylamino)-2-oxoéthoxy)phényl)-2,2,2-trifluoroacétamide
2-(4-chloro-2-(méthylsulfonamido)phénoxy)-N-((3-endo)-8-(4-chlorobenzyl)-8-azabicyclo[3.2.1]octan-3-yl)acétamide
2-(4-chloro-2-guanidinophénoxy)-N-((3-endo)-8-(4-chlorobenzyl)-8-azabicyclo[3.2.1]octan-3-yl)acétamide
2-(4-chloro-2-uréidophénoxy)-N-((3-exo)-8-(4-chlorobenzyl)-8-azabicyclo[3.2.1]octan-3-yl)acétamide
N-((3-endo)-8-(4-chlorobenzyl)-8-azabicyclo[3.2.1]octan-3-yl)-2-(3-chlorophényl) acétamide
N-((3-endo)-8-(4-chlorobenzyl)-8-azabicyclo[3.2.1]octan-3-yl)-2-(2-(2,6-dichlorophénylamino)phényl)acétamide
3-chloro-N-(2-((3-endo)-8-(4-chlorobenzyl)-8-azabicyclo[3.2.1]octan-3-ylamino)-2-oxoéthyl)benzamide
N-(2-((3-endo)-8-(4-chlorobenzyl)-8-azabicyclo[3.2.1]octan-3-ylamino)-2-oxoéthyl)-3-(trifluorométhyl)benzamide
N-(2-((3-endo)-8-(4-chlorobenzyl)-8-azabicyclo[3.2.1]octan-3-ylamino)-2-oxoéthyl)-2-(4-méthylphénylsulfonamido)benzamide
N-(2-((3-endo)-8-(4-chlorobenzyl)-8-azabicyclo[3.2.1]octan-3-ylamino)-2-oxoéthyl)-1-(4-chlorophényl)cyclopentanecarboxamide
2,7-dichloro-N-(2-((3-endo)-8-(4-chlorobenzyl)-8-azabicyclo[3.2.1]octan-3-ylamino)-2-oxoéthyl)-9H-xanthène-8-carboxamide
5-chloro-N-(2-((3-endo)-8-(4-chlorobenzyl)-8-azabicyclo[3.2.1]octan-3-yl)-2-(3-(3-chlorophényl)uréido)acétamide
N-((3-endo)-8-(4-chlorobenzyl)-8-azabicyclo[3.2.1]octan-3-yl)-2-(3-(3-chlorophényl)uréido)acétamide
N-(2-((3-endo)-8-(4-chlorobenzyl)-8-azabicyclo[3.2.1]octan-3-ylamino)-2-oxoéthyl)-2-phénoxybenzamide
N-(2-(((3-endo)-8-((4-chlorobenzyl)-8-azabicyclo[3.2.1]oct-3-yl)amino)-2-oxoéthyl)-10,11-dihydro-5H-dibenzo[a,d]cycloheptène-5-carboxamide
N-(2-((3-endo)-8-(4-chlorobenzyl)-8-azabicyclo[3.2.1]octan-3-ylamino)-2-oxoéthyl)-9-méthyl-9H-fluorène-9-carboxamide
N-(2-((3-endo)-8-(4-chlorobenzyl)-8-azabicyclo[3.2.1]octan-3-ylamino)-2-oxoéthyl)-2,2-diphénylacétamide
2-amino-N-(2-((3-endo)-8-(4-chlorobenzyl)-8-azabicyclo[3.2.1]octan-3-ylamino)-2-oxoéthyl)benzamide
N-(2-((3-endo)-8-(4-chlorobenzyl)-8-azabicyclo[3.2.1]octan-3-ylamino)-2-oxoéthyl)-9-méthyl-9H-xanthène-9-carboxamide
N-((3-endo)-8-(4-chlorobenzyl)-8-azabicyclo[3.2.1]octan-3-yl)-2-(2-(2-(2,6-dichlorphénylamino)phényl)acétamido)acétamide
N-((3-endo)-8-(4-chlorobenzyl)-8-azabicyclo[3.2.1]octan-3-yl)-2-(2-(3-chlorophényl)acétamido)acétamide
N-(3-((3-endo)-8-(4-chlorobenzyl)-8-azabicyclo[3.2.1]octan-3-ylamino)-3-oxopropyl)-2-(3-(trifluorométhyl)phénylamino)benzamide
N-(2-((3-endo)-8-(4-chlorobenzyl)-8-azabicyclo[3.2.1]octan-3-ylamino)-2-oxoéthyl)-N-méthyl-2-(3-(trifluorométhyl)phénylamino)benzamide
3-bromo-N-(2-((3-endo)-8-(4-chlorobenzyl)-8-azabicyclo[3.2.1]octan-3-ylamino)-2-oxoéthyl)benzamide
5-chloro-N-(2-((3-endo)-8-(4-chlorobenzyl)-8-azabicyclo[3.2.1]octan-3-ylamino)-2-oxoéthyl)-2-méthoxybenzamide
N-(2-((3-endo)-8-(4-chlorobenzyl)-8-azabicyclo[3.2.1]octan-3-ylamino)-2-oxoéthyl)-3-fluorobenzamide
5-chloro-N-(2-((3-endo)-8-(4-chlorobenzyl)-8-azabicyclo[3.2.1]octan-3-ylamino)-2-oxoéthyl)-2-méthoxy-4-(méthylamino)benzamide
3,4-dichloro-N-(2-((3-endo)-8-(4-chlorobenzyl)-8-azabicyclo[3.2.1]octan-3-ylamino)-2-oxoéthyl)benzamide
N-(2-((3-endo)-8-(4-chlorobenzyl)-8-azabicyclo[3.2.1]octan-3-ylamino)-2-oxoéthyl)benzamide
N-(2-((3-endo)-8-(4-chlorobenzyl)-8-azabicyclo[3.2.1]octan-3-ylamino)-2-oxoéthyl)-3-méthylbenzamide
2,5-dichloro-N-(2-((3-endo)-8-(4-chlorobenzyl)-8-azabicyclo[3.2.1]octan-3-ylamino)-2-oxoéthyl)benzamide
N-(2-((3-endo)-8-(4-chlorobenzyl)-8-azabicyclo[3.2.1]octan-3-ylamino)-2-oxoéthyl)-3-méthoxybenzamide
N-(2-((3-endo)-8-(4-chlorobenzyl)-8-azabicyclo[3.2.1]octan-3-ylamino)-2-oxoéthyl)-3-hydroxy(phényl)méthyl)benzamide
N-(2-((3-endo)-8-(4-chlorobenzyl)-8-azabicyclo[3.2.1]octan-3-ylamino)-2-oxoéthyl)-3-(cyanométhyl)benzamide
5-chloro-N-(2-((3-endo)-8-(4-chlorobenzyl)-8-azabicyclo[3.2.1]octan-3-ylamino)-2-oxoéthyl)-4-(diméthylamino)-2-méthoxybenzamide
3-chloro-N-(3-((3-exo)-8-(4-chlorobenzyl)-8-azabicyclo[3.2.1]octan-3-ylamino)-3-oxopropyl)benzamide
3-chloro-N-((R)-1-((3-exo)-8-(4-chlorobenzyl)-8-azabicyclo[3.2.1]octan-3-ylamino)-1-oxopropane-2-yl)benzamide
N-(2-((3-exo-8-(4-chlorobenzyl)-8-azabicyclo[3.2.1]octan-3-ylamino)-2-oxoéthyl)-N-méthyl-2-(3-(trifluorométhyl)phénylamino)benzamide
Acide (R)-3-(3-chlorobenzamido)-4-((3-exo)-8-(4-chlorobenzyl)-8-azabicyclo[3.2.1]octan-3-ylamino)-4-oxobutanoïque
N-((S)-1-((3-exo)-8-(4-chlorobenzyl)-8-azabicyclo[3.2.1]octan-3-ylamino)-1-oxopropan-2-yl)-2-(3-(trifluorométhyl)phénylamino)benzamide
3-chloro-N-((S)-1-(3-exo)-8-(4-chlorobenzyl)-8-azabicyclo[3.2.1]octan-3-ylamino)-1-oxopropan-2-yl)benzamide
3-chloro-N-(2-((3-exo)-8-(4-chlorobenzyl)-8-azabicyclo[3.2.1]octan-3-ylamino)-2-oxoéthyl)-N-méthylbenzamide
N-((S)-1-((3-exo)-8-(4-chlorobenzyl)-8-azabicyclo[3.2.1]octan-3-ylamino)-1-oxo-3-phénylpropan-2-yl)-2-(3-(trifluorométhyl)phénylamino)benzamide
N-((S)-1-((3-exo)-8-(4-chlorobenzyl)-8-azabicyclo[3.2.1]octan-3-ylamino)-4-(méthylthio)-1-oxobutan-2-yl)-2-(3-(trifluorométhyl)phénylamino)-benzamide
N-(3-((3-exo)-8-(4-chlorobenzyl)-8-azabicyclo[3.2.1]octan-3-ylamino)-3-oxopropyl)-2-(3-(trifluorométhyl)phénylamino)benzamide
N-((S)-1-((3-exo)-8-(4-chlorobenzyl)-8-azabicyclo[3.2.1]octan-3-ylamino)-4-méthyl-1-oxopentan-2-yl)-2-(3-(trifluorométhyl)phénylamino)benzamide
3-chloro-N-(2-((3-endo)-8-(3,4-dichlorobenzyl)-8-azabicyclo[3.2.1]octan-3-ylamino)-2-oxoéthyl)benzamide
3-chloro-N-(2-((3-endo)-8-(3,4-fluorobenzyl)-8-azabicyclo[3.2.1]octan-3-ylamino)-2-oxoéthyl)benzamide
N-(2-((3-endo)-8-benzyl-8-azabicyclo[3.2.1]octan-3-ylamino)-2-oxoéthyl)-3-chlorobenzamide
3-chloro-N-(2-((3-endo)-8-(2,4-dichlorobenzyl)-8-azabicyclo[3.2.1]octan-3-ylamino)-2-oxoéthyl)benzamide
3-chloro-N-(2-((3-endo)-8-(1-(4-chlorophényl)éthyl)-8-azabicyclo[3.2.1]octan-3-ylamino)-2-oxoéthyl)benzamide
3-chloro-N-(2-((3-endo)-8-(1-(4-chlorophényl)propyl)-8-azabicyclo[3.2.1]octan-3-ylamino)-2-oxoéthyl)benzamide
3-chloro-N-(2-oxo-2-((3-endo)-8-(3-phénylpropyl)-8-azabicyclo[3.2.1]octan-3-ylamino)éthyl)benzamide
N-(2-((3-endo)-8-(3,4-dichlorobenzyl)-8-azabicyclo[3.2.1]octan-3-ylamino)-2-oxoéthyl)-2-(3-(trifluorométhyl)phénylamino)benzamide
N-(2-((3-endo)-8-(2,4-dichlorobenzyl)-8-azabicyclo[3.2.1]octan-3-ylamino)-2-oxoéthyl)-2-(3-(trifluorométhyl)phénylamino)benzamide
N-(2-((3-endo)-8-(4-méthoxybenzyl)-8-azabicyclo[3.2.1]octan-3-ylamino)-2-oxoéthyl)-2-(3-(trifluorométhyl)phénylamino)benzamide
N-(2-((3-endo)-8-(4-chlorobenzyl)-8-azabicyclo[3.2.1]octan-3-ylamino)-2-oxoéthyl)-2-hydroxybenzamide
N-(2-((3-endo)-8-(4-chlorobenzyl)-8-azabicyclo[3.2.1]octan-3-ylamino)-2-oxoéthyl)-2-(phénylamino)benzamide
N-(2-((3-endo)-8-(4-chlorobenzyl)-8-azabicyclo[3.2.1]octan-3-ylamino)-2-oxoéthyl)-9H-xanthène-9-carboxamide
2-(3,5-bis(trifluorométhyl)phénylsulfonamido)-N-((3-endo)-8-(4-chlorobenzyl)-8-azabicyclo[3.2.1]octan-3-yl)acétamide
3-chloro-N-((S)-1-((3-endo)-8-(4-chlorobenzyl)-8-azabicyclo[3.2.1]octan-3-ylamino)-1-oxopropan-2-yl)benzamide
3-chloro-N-(3-((3-endo)-8-(4-chlorobenzyl)-8-azabicyclo[3.2.1]octan-3-ylamino)-3-oxopropyl)benzamide
Acide (R)-3-(3-chlorobenzamido)-4-((3-endo)-8-(4-chlorobenzyl)-8-azabicyclo[3.2.1]octan-3-ylamino)-4-oxobutanoïque
3-chloro-N((R)-1-((3-endo)-8-(4-chlorobenzyl)-8-azabicyclo[3.2.1]octan-3-ylamino)-1-oxopropan-2-yl)benzamide
Acide (R)-3-(3-chlorobenzamido)-4-((3-endo)-8-(4-chlorobenzyl)-8-azabicyclo[3.2.1]octan-3-ylamino)-4-oxobutanoïque
N-(2-((3-endo)-8-(4-fluorobenzyl)-8-azabicyclo[3.2.1]octan-3-ylamino)-2-oxoéthyl)-2-(3-trifluorométhyl)phénylamino)benzamide
N-(2-((3-exo)-8-(4-chlorobenzyl)-8-azabicyclo[3.2.1]octan-3-ylamino)-2-oxoéthyl)-3-(trifluorométhyl)phénylamido)benzamide
N-(2-((3-exo)-8-(4-chlorobenzyl)-8-azabicyclo[3.2.1]octan-3-ylamino)-2-oxoéthyl)-3-(trifluorométhyl)benzamide
3-chloro-N-(2-((3-exo)-8-(4-chlorobenzyl)-8-azabicyclo[3.2.1]octan-3-ylamino)-2-oxoéthyl)benzamide
N-(2-((3-endo)-8-(4-chlorobenzyl)-8-azabicyclo[3.2.1]octan-3-ylamino)-2-oxoéthyl)-2-(3-(trifluorométhyl)phénylamino)benzamide

17. Composé selon l'une quelconque des revendications 1 à 16, à utiliser dans le traitement d'une condition pathologique ou d'une maladie susceptible d'amélioration par l'antagonisme du récepteur CCR1.

18. Composé selon l'une quelconque des revendications 1 à 16, à utiliser dans le traitement d'une condition pathologique ou d'une maladie, dans laquelle la condition pathologique ou maladie est la polyarthrite rhumatoïde, l'asthme allergique, la fibrose pulmonaire, les infections virales respiratoires, la glomérulonéphrite, le rejet de transplantation rénale, la septicémie, l'athérosclérose, le lupus érythémateux systémique (SLE), la sclérose en plaque, la maladie d'Alzheimer ou le rejet d'une transplantation cardiaque.

19. Composition pharmaceutique comprenant un composé tel que défini dans l'une quelconque des revendications 1 à 16, mélangé à un diluant ou à un excipient pharmaceutiquement acceptable.

20. Utilisation d'un composé comme défini dans l'une quelconque des revendications 1 à 16, dans la fabrication d'un médicament pour le traitement d'une condition pathologique
ou maladie susceptible d'être améliorée par l'antagonisme du récepteur de CCR1.

21. Utilisation d'un composé selon la revendication 20, dans lequel la condition pathologique ou maladie est la polyarthrite rhumatoïde, l'asthme allergique, la fibrose pulmonaire, l'infection virale respiratoire, la glomérulonéphrite, le rejet de transplantation rénale, la septicémie, l'athérosclérose, le lupus érythémateux systémique (SLE), la sclérose en plaques, la maladie d'Alzheimer ou le rejet d'une transplantation cardiaque.

22. Produit de combinaison comprenant un composé selon l'une quelconque des revendications 1 à 16, et un d'autre composé choisi parmi (a) le méthotrexate, (b) le léfluno-mide, (c) la sulfasalazine, (d) l'hydroxychloroquine, (e) l'azathioprine, (f) les sels d'or, (h) les antagonistes de récepteurs de chimiokine CCR2 à CCR10, (i) les antagonistes des récepteurs de chimiokine CXCR1 à CXCR6, (j) les antagonistes des récepteurs de chimiokine XCR1, XCR2 ou CX3CR1, (k) des anticorps monoclonaux anti-TNF, (1) des antagonistes solubles de récepteurs de TNF, (m) l'Abatacept, (n) des anticorps anti-CD20, (o) des anticorps anti-IL-6R, (p) des inhibiteurs de PDE4, (q) des inhibiteurs de MAPK p38, (r) des inhibiteurs de MAPKAP-K2, (s) des antagonistes de récepteur de P2X7, (t) des ligands de récepteur d'interféron bêta, (u) de l'acétate de glatiramère et (v) des anticorps anti-IL 12 pour utilisation simultanée, séparée ou séquentielle.
